(19) Europäisches Patentamt
European Patent Office
Office européen des brevets

(11) EP 2 855 490 B1

(12) FASCICULE DE BREVET EUROPEEN

(45) Date de publication et mention de la délivrance du brevet:
**07.03.2018 Bulletin 2018/10**

(21) Numéro de dépôt: **13731374.8**

(22) Date de dépôt: **30.05.2013**

(51) Int Cl.:
***C07F 3/06*** (2006.01) ***C07F 5/06*** (2006.01)
***C07F 7/00*** (2006.01) ***A61K 9/14*** (2006.01)
***C07F 15/02*** (2006.01) ***A61K 47/08*** (2006.01)
***C07F 9/38*** (2006.01) ***G01N 33/58*** (2006.01)
***A61P 35/00*** (2006.01)

(86) Numéro de dépôt international:
**PCT/FR2013/051219**

(87) Numéro de publication internationale:
**WO 2013/178954 (05.12.2013 Gazette 2013/49)**

(54) **SOLIDE HYBRIDE ORGANIQUE INORGANIQUE AMELIOEE À SURFACE EXTERNE MODIFIÉE**

VERBESSERTER ORGANISCH-ANORGANISCHER HYBRIDFESTSTOFF MIT MODIFIZIERTER AUSSENOBERFLÄCHE

IMPROVED ORGANIC-INORGANIC HYBRID SOLID HAVING A MODIFIED OUTER SURFACE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.05.2012 FR 1255065**

(43) Date de publication de la demande:
**08.04.2015 Bulletin 2015/15**

(73) Titulaires:
- **Centre National de la Recherche Scientifique (C.N.R.S.)**
  **75794 Paris Cedex 16 (FR)**
- **Université de Versailles Saint-Quentin-en-Yvelines**
  **78000 Versailles (FR)**
- **Université Paris-Sud**
  **91405 Orsay Cedex (FR)**

(72) Inventeurs:
- **GREF, Ruxandra**
  **F-91370 Verrieres-le Buisson (FR)**
- **AGOSTONI, Valentina**
  **8049 Zürich (CH)**
- **DAOUD-MAHAMMED, Samia**
  **F-92330 Sceaux (FR)**
- **RODRIGUEZ-RUIZ, Violeta**
  **F-75017 Paris (FR)**
- **MALANGA, Milo**
  **I-40138 Bologne (IT)**
- **JICSINSZKY, Laszlo**
  **H-1192 Budapest (HU)**
- **HORCAJADA-CORTES, Patricia**
  **F-92370 Chaville (FR)**
- **SERRE, Christian**
  **F-78730 Plaisir (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
- **LIU, BEI ET AL: "Molecular simulation studies of separation of CH4/H2 mixture in metal-organic frameworks with interpenetration and mixed-ligand", CHEMICAL ENGINEERING SCIENCE , 66(13), 3012-3019 CODEN: CESCAC; ISSN: 0009-2509, 2011, XP002693314,**
- **SAVONNET, MARIE ET AL: "Combinatorial synthesis of metal-organic frameworks libraries by click-chemistry", NEW JOURNAL OF CHEMISTRY , 35(9), 1892-1897 CODEN: NJCHE5; ISSN: 1144-0546, 2011, XP002693315,**
- **AN, JIHYUN ET AL: "Cation-Triggered Drug Release from a Porous Zinc-Adeninate Metal-Organic Framework", JOURNAL OF THE AMERICAN CHEMICAL SOCIETY , 131(24), 8376-8377 CODEN: JACSAT; ISSN: 0002-7863, 2009, XP002693316,**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- SAPCHENKO, SERGEY A. ET AL: "Microporous sensor: gas sorption, guest exchange and guest-dependant luminescence of metal-organic framework", DALTON TRANSACTIONS , 40(10), 2196-2203 CODEN: DTARAF; ISSN: 1477-9226, 2011, XP002693317,
- ZHAO, XIAOLIANG ET AL: "A porous metal-organic framework ( MOF ) with unusual 2D .fwdarw. 3D polycatenation based on honeycomb layers", DALTON TRANSACTIONS , 41(7), 1928-1930 CODEN: DTARAF; ISSN: 1477-9226, 2012, XP002693318,
- BAUER, CHRISTINA A. ET AL: "Influence of Connectivity and Porosity on Ligand-Based Luminescence in Zinc Metal-Organic Frameworks", JOURNAL OF THE AMERICAN CHEMICAL SOCIETY , 129(22), 7136-7144 CODEN: JACSAT; ISSN: 0002-7863, 2007, XP002693319,
- PARK, HYE JEONG ET AL: "Post-synthetic reversible incorporation of organic linkers into single-crystal-to-single-crystal transformations and modification of gas sorption properties", CHEMISTRY--A EUROPEAN JOURNAL , 16(38), 11662-11669, S11662/1-S11662/46 CODEN: CEUJED; ISSN: 0947-6539, 2010, XP002693320,
- NAN, JIANGPU ET AL: "Formation mechanism of metal-organic framework membranes derived from reactive seeding approach", MICROPOROUS AND MESOPOROUS MATERIALS , 155, 90-98 CODEN: MIMMFJ; ISSN: 1387-1811, 2012, XP002693321,
- DATABASE CA [Online] CHEMICAL ABSTRACTS SERVICE, COLUMBUS, OHIO, US; KIM, JONG SIK ET AL: "Method for supercritical treatment of porous metal organic framework", XP002693322, extrait de STN Database accession no. 2010:1518319
- RAVON, UGO ET AL: "Engineering of coordination polymers for shape selective alkylation of large aromatics and the role of defects", MICROPOROUS AND MESOPOROUS MATERIALS , VOLUME DATE 2010, 129(3), 319-329 CODEN: MIMMFJ; ISSN: 1387-1811, 2009, XP002693313,

## Description

### Domaine technique

La présente invention se rapporte à un solide de réseau métal-organique ou « Metal-Organic Framework » (MOF) amélioré, cristallin poreux, à surface externe modifiée, ainsi, notamment, qu'à son procédé de préparation.

Le solide MOF de la présente invention est utilisable par exemple comme agent de contraste et/ou pour le transport de composés pharmaceutiques. Le solide de la présente invention peut également être utilisé pour des applications dans le domaine du stockage, de la séparation, catalyse, cosmétique ou agro-alimentaire. Il peut également être utilisé pour la vectorisation et/ou le suivi de composés pharmaceutiques dans un organisme. Il peut être également utilisé pour la détoxification. Il peut se présenter, par exemple, sous la forme de cristaux, de poudre, de particules ou de nanoparticules.

Les références entre crochets **[X]** renvoient à la liste des références à la fin des exemples.

### Etat de la technique

L'utilisation de transporteurs et vecteurs de molécules d'intérêt, notamment des molécules à effet thérapeutique ou des marqueurs, est devenu un enjeu important pour le développement de nouvelles méthodes de diagnostique ou de nouveaux médicaments. En effet, les molécules d'intérêt présentent des caractéristiques qui ont une influence sur la pharmacocinétique et la biodistribution de ces molécules et qui ne sont pas toujours favorables ou adaptables vis-à-vis du milieu dans lequel elles sont introduites. Ce sont par exemple des caractéristiques physico-chimiques telles que l'instabilité, la forte tendance à la cristallisation, la faible hydro/liposolubilité et/ou des caractéristiques biologiques telles que la toxicité, la biodégradabilité, etc.

C'est dans ce contexte que des nanovecteurs originaux ont été élaborés à partir de matériaux très prometteurs, jamais utilisés jusqu'alors dans le domaine biomédical : les solides hybrides organiques-inorganiques poreux cristallisés.(cf. WO 2009/077670 **[1]** et WO 2009/077671 **[2]**)

Les réseaux métal-organiques ou « Metal-Organic Framework » (MOF) sont des polymères de coordination de charpente hybride inorganique-organiques comprenant des ions métalliques et des ligands organiques coordinés aux ions métalliques. Ces matériaux sont organisés en réseau mono-, bi- ou tri-dimensionnels où les entités inorganiques sont reliées entre elles par des ligands espaceurs de façon périodique. Ces matériaux ont une structure cristalline, sont le plus souvent poreux et pourraient être utilisés dans de nombreuses applications industrielles telles que le stockage de gaz, l'adsorption de liquides, la séparation de liquides ou de gaz, la catalyse, etc.

Les nanoparticules hybrides organiques-inorganiques (nanoMOFs) à base de carboxylate de fer poreux ont été récemment élaborées afin de relever certains défis da la galénique moderne. Comme évoqué précédemment, la recherche dans ce domaine est partie du constat qu'il subsistait un nombre de principes actifs à demi-vie plasmatique très courte, franchissant mal les barrières naturelles de l'organisme, ou conduisant à des phénomènes de résistance ou toxicité, pour lesquels la nanoencapsulation constituerait une alternative intéressante. Certaines de ces molécules d'intérêt (notamment à activité anticancéreuse ou antivirale) n'avaient pas pu être encapsulées avec succès dans les nanovecteurs connus (liposomes, nanoparticules à base de polymère ou inorganiques...). La raison principale résultait de l'incompatibilité de ces molécules actives, en terme d'interaction suffisante afin de les encapsuler convenablement, avec les matériaux actuellement utilisés pour élaborer les nanovecteurs (polymères, lipides, huiles, ...).

Les nanoMOFS sont par exemple formés à partir des unités de fer(III) qui engendrent par pontage avec des acides polycarboxyliques endo ou exogènes, comme l'acide fumarique ou l'acide trimésique, de grandes cages amphiphiles de taille définie (3 à 60 Å) (1 Å = $10^{-10}$ m). Il est possible de moduler la taille des pores, la structure ainsi que le microenvironnement interne (balance hydrophile/hydrophobe) en jouant sur la nature et la fonctionnalisation des acides carboxyliques utilisés lors de la synthèse des nanoMOFs.

Grâce à leur volume poreux et surface spécifique importantes, ces nanoparticules ou nanoMOFs de carboxylate de fer se sont révélées capables d'adsorber par simple imprégnation dans des solutions de principe actif, des quantités très importantes de ces molécules thérapeutiques, pouvant dépasser les 40% en poids dans le cas de plusieurs molécules hydrophiles, hydrophobes ou amphiphiles qui n'avaient jamais auparavant pu être encapsulées de manière efficace (quantités encapsulées <1 ou au mieux 5% en poids).

La dégradabilité de ces nanoMOFs dans l'organisme, ainsi que leur biocompatibilité a été démontrée. Par exemple, l'injection de doses répétées allant jusqu'à 220 mg/kg n'a mis en évidence aucun signe de toxicité chez les rats traités (comportement des animaux, poids, histologie, modification de marqueurs biologiques). La capacité de ces nanoMOFS à produire in vivo un signal en imagerie résonance magnétique (IMN) a également été mise en évidence (marquage du foie et de la rate). Le contraste a été attribué à la fois aux atomes de fer paramagnétiques et aux canaux interconnectés remplis d'eau, coordonnée aux sites métalliques et/ou libres. Cette observation a ouvert des perspectives séduisantes en théranostique, permettant le suivi du devenir *in vivo* de nanoparticules chargées en principes actifs.

On pourra se référer par exemple à la demande internationale WO 2009/077670 **[1]** pour une description de ces nanoparticules et leurs propriétés.

Des méthodes de fonctionnalisation de la surface des nanoMOFs ont été explorées, afin de pouvoir contrôler leur interaction avec le milieu vivant et de permettre leur adressage sélectif *in vivo.* Ceci est important dans la mesure où les nanoparticules non modifiées sont rapidement reconnues comme corps étrangers et sont éliminées au bout de quelques minutes par le système réticulo-endothélial (accumulation dans le foie et la rate). D'autres types de vecteurs (liposomes, nanoparticules, ...) à surface modifiée, développés depuis quelques années déjà, sont capables d'atteindre des cibles biologiques.

La demande internationale WO 2009/077671 **[2]** décrit des méthodes de modification de surface de nanoMOFs. Par exemple, il y est proposé de coupler des chaînes de polyéthylèneglyol linéaires (PEG) à la surface des nanoMOFs in situ pendant leur synthèse ou post-synthèse afin de rendre les nanoMOFs « furtifs », c'est-à-dire capables d'éviter l'accumulation dans le foie et la rate, et de changer leur biodistribution.

Cependant, cette stratégie de modification de surface a des inconvénients, en raison notamment du caractère poreux des matériaux MOFs. Ceux-ci se manifestent notamment par une capacité d'encapsulation diminuée, et une plus grande difficulté à contrôler la libération des principes actifs encapsulés (cf. Exemple 11).

La demande internationale WO 2009/077671 **[2]** décrit également l'utilisation de polymères portant des groupes hydrophobes capables d'interagir avec la surface externe des MOFs (tel que le dextrane greffé avec des groupements fluorescéine et biotine) pour recouvrir (fonctionnaliser) la surface des MOFs. Cependant, ces méthodes de recouvrement présentes des problèmes de stabilité, notamment en milieu physiologique, ce qui représente un obstacle à l'utilisation de ces matériaux MOF à surface externe modifiée pour des applications biomédicales *in vivo* (cf Exemple 12).

Il reste donc de nombreuses améliorations à apporter en termes de fonctionnalisation de la surface externe de particules de MOFs. En particulier, il existe un réel besoin de disposer de composés améliorés capables d'échapper au système immunitaire et/ou à leur capture rapide par certains organes, par exemple le foie, évitant ainsi leur accumulation dans ces organes, et capables de vectoriser des principes actifs vers des cibles spécifiques.

**Exposé de l'invention**

La présente invention a précisément pour but de répondre à ces besoins et inconvénients de l'art antérieur en fournissant un solide MOF cristallin poreux à surface externe modifiée comprenant une succession tridimensionnelle de motifs identiques ou différents répondant à la formule (I) suivante :

$M_mO_kX_1L_p$ Formule (I)

dans laquelle :

chaque occurrence de **M** représente indépendamment un ion métallique choisi dans le groupe comprenant $Fe^{2+}$, $Fe^{3+}$, $Zn^{2+}$, $Zr^{4+}$, $Ti^{4+}$, $Ca^{2+}$, $Mg^{2+}$ et $Al^{3+}$;

- **m, k, l et p** sont des nombres ≥ 0 choisis de façon à respecter la neutralité des charges du motif ; de préférence, m, k, 1 et p sont indépendamment 0 à 4, par exemple m et p sont indépendamment 1, 2 ou 3 et/ou k et l sont indépendamment 0 ou 1 ;

- **X** est un ligand choisi dans le groupe comprenant $OH^-$, $Cl^-$, $F^-$, $I^-$, $Br^-$, $SO_4^{2-}$, $NO_3^-$, $ClO_4^-$, $R^1-(COO)_n^-$, $R^1-(S0_3)_n^-$, $R^1-(PO_3)_n^-$, où $R^1$ est un hydrogène, un alkyle en $C_1$ à $C_8$, linéaire ou ramifié, n = 1 à 6 ; et

- **L** est un ligand espaceur (polyfonctionnalisé) comprenant un radical $R^0$ et q occurrences d'un groupe complexant A, où

  - q est un nombre entier compris entre 2 et 6 ;

  - chaque occurrence de A est indépendamment :

    (i) un carboxylate

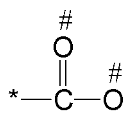 ;

(ii) un phosphonate

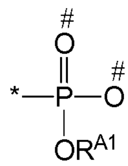

;

ou

(iii) un groupe imidazolate

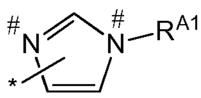

;

dans lequel $R^{A1}$ représente un atome d'hydrogène ou un radical $C_{1-6}$alkyle ;

où * désigne le point d'attachement du groupement A avec le radical $R^0$ ;

# désigne les points d'attachement possibles du groupement A à l'ion métallique M ;

- $R^0$ représente
  - un radical $C_{1-12}$alkylène, $C_{2-12}$alcènylène ou $C_{2-12}$alcynylène ;
  - un radical aryle mono- ou poly-cyclique, fusionné ou non, comprenant de 6 à 50 atomes de carbone,
  - un radical hétéroaryle mono- ou poly-cyclique, fusionné ou non, comprenant de 4 à 50 atomes de carbone,

le radical $R^0$ étant éventuellement substitué par un ou plusieurs groupes indépendamment choisis dans le groupe halogène, OH, $NH_2$, $NO_2$ ou un alkyle en $C_1$ à $C_6$ ;
dans lequel la surface externe du MOF est modifiée en ce qu'elle comprend au moins un agent de surface organique en interaction avec (par exemple complexé à) un centre métallique M ou à un ligand L situé sur la surface externe du solide MOF cristallin.

Dans l'ensemble de la présente description, la variable q étant au moins 2, le ligand L est de façon inhérente, polyfonctionnalisé.
De préférence le ligand L représente un ligand di-, tri-, tétra- ou hexa-carboxylate.
Avantageusement, l'agent de surface comprend: i) au moins un groupement phosphate, phosphonate, bisphosphonate, sulfate, carboxylate, hydroxy, cathécolate, thiolate (-SR ou $-S^-$), hétérocyclique azoté, amido ($-C(=O)N(R)_2$), amino ($-N(R)_2$), ou une combinaison de ces groupes, où chaque occurrence de R représente indépendamment H, $C_{1-6}$alkyle ou phényle) ;

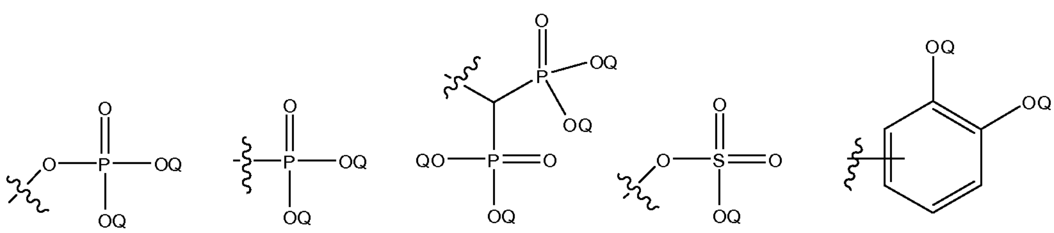

Phosphate Phosphonate Bisphosphonate Sulfate Cathécolate

où chaque occurrence de Q représente indépendamment H ou un cation de métal alcalin ;
et/ou ii) une section rigide supérieure à la taille des fenêtres d'accès aux pores de plus grande dimension du matériau

MOF (par exemple, des cyclodextrines).

La taille des fenêtres d'accès aux pores des matériaux MOF décrits dans la présente est définie par les paramètres M, X, L, m, k, l et p. Les matériaux MOFs sont bien connus dans la littérature scientifique, et le lecteur saura sans difficulté à partir de la sélection des paramètres M, X, L, m, k, l et p opérée (et donc du choix du matériau MOF en question) déterminer la structure du MOF, y compris les tailles des fenêtres des pores, et en particulier la taille des fenêtres d'accès aux pores de plus grande dimension du matériau MOF.

Avantageusement, lorsque la section rigide de l'agent de surface est inférieure à la taille des fenêtres d'accès aux pores de plus grande dimension du matériau MOF (et donc lorsque l'agent de surface est susceptible de par sa taille et conformation de pénétrer dans les pores du solide MOF), l'agent de surface possède de préférence une pluralité de groupements phosphate, phosphonate, bisphosphonate, sulfate, carboxylate, hydroxy, cathécolate, thiolate (-SR ou $-S^-$), hétérocyclique azoté, amido ($-C(=O)N(R)_2$), amino ($-N(R)_2$), ou une combinaison de ces groupes, où chaque occurrence de R représente indépendamment H, $C_{1-6}$alkyle ou phényle), répartis sur toute la longueur de sa chaine principale (par exemple, dextrane greffé avec des alendronates repartis statistiquement), évitant ainsi une adsorption importante de l'agent de surface dans les pores.

Avantageusement, l'agent de surface organique peut être choisi parmi un monomère, oligomère ou polymère de cyclodextrine; un polyéthylèneglycol ramifié (e.g., « étoilé » ou dendrimère); une protéine ; un polysaccharide portant ou non une pluralité de chaînes latérales polyéthylèneglycol (PEG) elles-mêmes éventuellement couplées ou non en bout de chaîne avec des ligands spécifiques; ou un polysaccharide comme le chitosane, insoluble dans l'eau à 6<pH<8 et soluble dans l'eau à pH<5.

Avantageusement, ledit agent de surface organique étant en interaction avec (par exemple complexé à) un centre métallique M ou à un ligand L situé en surface du solide MOF cristallin via ledit un ou plusieurs groupe(s) phosphate, phosphonate, sulfate, carboxylate, hydroxy, cathécolate, thiolate, azote hétérocyclique, amide ou amino, ou une combinaison de ces groupes.

Avantageusement, les chaines de PEG pourront porter à leur extrémité libre un ligand spécifique (anticorps, peptide, folate etc) permettant le changement de leur biodistribution, notamment un ciblage des nanoparticules.

Dans le cas d'un polysaccharide portant une pluralité de chaînes latérales polyéthylèneglycol, plus l'on greffe des chaines de PEG, plus l'hydrophilie du copolymère augmente. Ainsi, avantageusement, il conviendra dans ce cas de greffer également des fonctions plus complexantes, telles que les alendronates, afin d'assurer une bonne stabilité du recouvrement.

Avantageusement, l'agent de surface organique interagit avec un centre métallique M ou avec un ligand L situé en surface du solide MOF cristallin *via* au moins un groupement parmi ceux cités précédemment. L'interaction a lieu de préférence par des interactions covalentes, iono-covalentes, ioniques ou des interactions faibles (i.e. liaisons d'hydrogène) (par exemple, coordination fer-phosphate). En revanche, les interactions de type hydrophobes (exemple chainon alkyle-MOF) sont de préférence exclues, car trop faible pour assurer une bonne stabilité du recouvrement.

Par « bonne stabilité du recouvrement » on entend une stabilité suffisante pour permettre, dans un milieu biologique, de réaliser une fonction d'intérêt biomédical : par exemple, atteindre une cible (ex tumeur, tissu infecté etc) ; interagir avec la muqueuse intestinale et/ou de circuler suffisamment longtemps dans le flux sanguin.

Avantageusement, dans des conditions *in vitro,* une « bonne stabilité du recouvrement » peut représenter un décrochage de moins de 20% du recouvrement (c'est-à-dire de l'agent de surface présent sur la surface externe dumatériau MOF) pendant une heure d'incubation sous agitation, à 37°C dans une solution tampon phosphate 0.15 M, pH 7.4.

Avantageusement, lorsque la section rigide de l'agent de surface est inférieure à la taille des fenêtres d'accès aux pores de plus grande dimension du matériau MOF, la densité des points d'ancrage pourra de préférence être augmentée si un ou plusieurs des critères suivants sont remplis :

- Groupes possédant une moindre capacité d'interaction avec le matériau (par exemple, les groupes carboxylate, amino ou sulfate interagissent moins fortement que les phosphates) ;
- Grande solubilité de l'agent de surface dans le milieu dispersant;
- Grande flexibilité de l'agent de surface et/ou section rigide faible.

Par exemple, l'agent de surface peut être dextrane greffé à la fois avec des chaines de PEG et des groupes bisphosphonate Alendronate, tel qu'exemplifié dans la présente demande. La méthode de synthèse selon la présente invention permet de faire varier à la fois la densité des chaines de PEG et des points d'ancrage (alendronate).

Avantageusement, les agents de surface spécifiquement décrits dans la demande WO 2009/0077671 sont exclus.

Avantageusement, l'agent de surface organique peut être choisi parmi un monomère, oligomère ou polymère de cyclodextrine; un groupement polyéthylèneglycol ramifié ; un polysaccharide portant une pluralité de chaînes latérales polyéthylèneglycol; ou un polysaccharide insoluble dans l'eau à 6<pH<8 et soluble dans l'eau à pH<5.

Dans le cadre de la présente invention, les diverses occurrences de **M** dans les motifs de formule (I) peuvent être identiques ou différentes. De préférence, chaque occurrence de **M** représente indépendamment un ion métallique $Fe^{2+}$, $Fe^{3+}$, $Zn^{2+}$ ou $Ca^{2+}$.

On entend par « solide » au sens de la présente invention tout type de matériau cristallin. Ledit solide peut par exemple se présenter sous forme de cristaux, de poudre, de particules de formes variées, par exemple de forme sphérique, cubique, parallèpipédique, rhomboédrique, lamellaire, etc. Les particules peuvent être sous la forme de nanoparticules.

Par « nanoparticule », on entend une particule de taille inférieure à 1 $\mu$m. En particulier, les nanoparticules de solide MOF selon l'invention peuvent avoir un diamètre inférieur à 1000 nanomètres, de préférence inférieur à 500 nm, de manière plus préférée inférieur à 250 nm, tout particulièrement inférieur à 100 nm.

Par « section rigide » d'une molécule ou d'un agent de surface, on entend la dimension du petit axe du volume rigide occupé par la molécule ou l'agent de surface dans un milieu donné, avantageusement un milieu liquide. Avantageusement, il peut s'agir du milieu liquide dans lequel la molécule ou l'agent de surface est mis(e) en contact avec le matériau MOF, en particulier lors de la modification de la surface externe du matériau MOF avec la molécule ou l'agent de surface en question. Avantageusement, il peut également ou alternativement s'agir du milieu liquide dans lequel le matériau MOF, modifié avec la molécule ou l'agent de surface selon la présente invention, se trouve. Avantageusement, le volume rigide représente le volume occupé par la molécule ou de l'agent de surface dans sa conformation la plus stable dans le milieu en question, que ce soit lors de la modification de la surface externe du matériau MOF selon la présente invention, ou une fois que la modification de surface est réalisée. Dans tous les cas, avantageusement, le milieu peut être de l'eau, un liquide physiologique, un solvant organique ou un mélange eau/solvant organique. La section rigide peut être calculée à l'aide de la simulation numérique (logiciel Materials Studio, Accelrys; connu par l'homme du métier) une fois que la conformation la plus stable de la molécule ou de l'agent de surface dans le milieu donné est déterminée par minimisation d'énergie. Par exemple, le logiciel Accelrys, Materials Studio version 5.0 (2010) peut être utilisé.

Par « monomère de cyclodextrine », on entend une unité cyclodextrine, laquelle peut être par exemple une $\alpha$-, $\beta$- ou $\gamma$-cyclodextrine.

Par « oligomère de cyclodextrine », on entend un enchaînement de 2 à 9 unités cyclodextrine identiques ou différentes. Les unités cyclodextrine peuvent être par exemple des $\alpha$-, $\beta$- ou $\gamma$-cyclodextrines.

Par « polymère de cyclodextrine » ou « polycyclodextrine », on entend un polymère comprenant au moins 10 unités cyclodextrine identiques ou différentes. Avantageusement, le polymère de cyclodextrine contient au moins 10 unités cyclodextrine, de préférence au moins 15 unités cyclodextrines, et avantageusement au moins 20 unités cyclodextrines. De façon particulièrement avantageuse, on préfère que les polymères de cyclodextrine portant des cyclodextrines comprennent en moyenne au moins 100 unités cyclodextrines, de préférence au moins 200 unités cyclodextrines, et avantageusement au moins 300 unités cyclodextrines. Typiquement, les polymères de cyclodextrine comprennent en moyenne au moins 400 unités cyclodextrines. Avantageusement, le polycyclodextrine contient en moyenne entre 10 et 1500 unités cyclodextrine au sein de sa structure, de préférence en moyenne entre 10 et 1000 unités cyclodextrine, de préférence en moyenne entre 15 et 800 unités cyclodextrine, de préférence en moyenne entre 50 et 600 unités cyclodextrine, et avantageusement en moyenne entre 100 et 400 unités cyclodextrine.

Les unités cyclodextrines présentes au sein du polycyclodextrine peuvent, de façon générale, être des $\alpha$-cyclodextrines, des $\beta$-cyclodextrines, des $\gamma$-cyclodextrines, ou bien des mélanges d'au moins deux de ces types de cyclodextrines.

Le nombre moyen d'unités cyclodextrines présentes dans les polymères de l'invention peut par exemple être établi par chromatographie d'exclusion stérique et par résonance magnétique nucléaire.

Au sein des polycyclodextrines, les unités cyclodextrine peuvent être liées entre elles par des chaînes hydrocarbonées de 3 à 50 atomes de carbone, linéaires ou ramifiées, éventuellement interrompues par un ou plusieurs atomes d'oxygène, et ces chaînes étant de préférence des chaînes alkyles, alcényles, ou alcynyles de 3 à 50 atomes de carbone, ou bien encore des chaînes polyéthers de 3 à 50 atomes de carbone, ces chaînes pouvant être substituées par des groupements hydrophiles (groupements hydroxy ou amino par exemple). Les chaînes liant entre elles les unités cyclodextrines peuvent comporter au moins 3 atomes de carbone et de préférence de 4 à 50 atomes de carbone, le chemin le plus court entre deux unités cyclodextrines étant de préférence constitué par une chaîne comportant entre 3 et 8 atomes de carbone.

Avantageusement, les chaînes hydrocarbonées liant entre elles deux unités cyclodextrines au sein d'un polycyclodextrine répondent à la formule générale un groupement de formule $-O-(CH_2-CHOR^1-CH_2)_n-O-$ où n est un entier compris entre 1 et 50 (généralement compris entre 2 et 10) et où, dans chacune des n unités $(CH_2-CHOR^1-CH_2)$, $R^1$ désigne soit un atome d'hydrogène, soit une chaîne $-CH_2-CHOH-CH_2-O-$ reliée à une unité cyclodextrine du polymère.

Ainsi, les polycyclodextrines peuvent typiquement être obtenus par une réticulation de molécules de cyclodextrines avec des composés bifonctionnels capables de former des liaisons covalentes avec les groupements hydroxyles des cyclodextrines. Par exemple, il peut s'agir d'acides dicarboxyliques tels que l'acide citrique, l'acide sébacique, l'acide

fumarique, l'acide glutamique, l'acide maléique, l'acide malique, l'acide malonique, l'acide aspartique, l'acide oxalique, l'acide succinique, l'acide glutarique, l'acide trans, trans muconique, l'acide téréphtalique, l'acide isophtalique, l'acide oxaloacétique, l'acide phtalique, l'acide adipique ou l'acide butanedioïque.

Par exemple, les polycyclodextrines peuvent être obtenus par polycondensation de molécules de cyclodextrines et d'épichlorhydrine, généralement en milieu basique (généralement dans un milieu aqueux additionné de soude, à une concentration massique de 10 à 40%), le ratio molaire cyclodextrines/épichlorhydrine étant de préférence compris entre 1 : 15 et 1 : 1, et avantageusement entre 1 : 15 et 1 : 8. Pour plus de détails concernant cette synthèse et contrôle du nombre moyen d'unités cyclodextrines intégrées au sein des polymères à base d'unités cyclodextrine obtenus selon ce procédé, on pourra notamment se référer aux articles suivants :

- E. Renard et al., European Polymer Journal, vol. 33, No 1, pp 49-57 (1997) **[6]**
- Gref et al., International Journal of Pharmaceutics, Vol. 332, Issues 1-2, Pages 185-191 (2007) **[7]**
- Gref et al., J. Control Release, 111(3):316-24 (2006) **[8]**
- Gref et al., Journal of colloid and interface science, 307 (1) :83-93 (2007) **[9]**
- Blanchemain et al., Acta Biomaterialia, Volume 4, Issue 6, November 2008, Pages 1725-1733 **[10]**

Les polycyclodextrines peuvent également être obtenus par polycondensation de molécules de cyclodextrines et d'hexaméthylène diisocyanate, comme décrit par exemple dans Elif Yilmaz Ozmen et al. Bioresource Technology, Volume 99, Issue 3, Pages 526-531 (2008) **[11].**

Les polycyclodextrines peuvent également être obtenus par polycondensation de molécules de cyclodextrines et un polyéthylèneglycol fonctionnalisé, comme décrit par exemple dans :

- Cesteros et al., European Polymer Journal, Volume 45, Issue 3, Pages 674-679 (2009) (PEG acylé) **[12]**
- Salmaso et al., International Journal of Pharmaceutics, Volume 345, Issues 1-2, Pages 42-50 (2007) (PEG diaminé) **[13]**

Les polycyclodextrines peuvent également être obtenus par polycondensation de molécules de cyclodextrines et plusieurs branches oligoethylimine, pour former un polymère étoilé, comme décrit par exemple dans Yang et al., Biomaterials, Volume 28, Issue 21, Pages 3245-3254 (2007). **[14]**

Quelle que soit la nature exacte des chaînes hydrocarbonées liant entre elles les unités cyclodextrines, en général, la masse totale des motifs cyclodextrines présents au sein des polycyclodextrines représente au moins 30%, avantageusement au moins 40 %, et encore plus préférentiellement au moins 50%, de la masse totale des desdits polymères, cette masse totale des motifs cyclodextrine représentant généralement entre 30 et 80%, et de préférence entre 40 et 75% de la masse totale des polymères à base d'unités cyclodextrine.

Ce pourcentage massique de cyclodextrines dans les polymères utilisables dans le contexte de la présente invention peut par exemple être déterminé par résonance magnétique nucléaire (RMN).

Dans certains modes de réalisation, le polycyclodextrine peut être un poly-β-cyclodextrine répondant à la Formule I suivante :

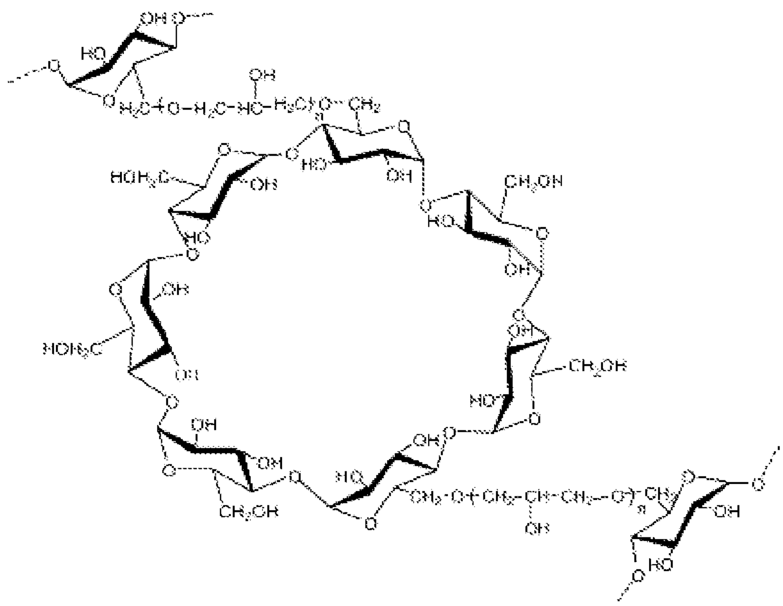

Formule I

dans laquelle n représente un entier compris entre 1 et 50, de préférence entre 2 et 10 ; et le nombre d'unités beta-cyclodextrine est en moyenne compris entre 10 et 1500, de préférence en moyenne entre 10 et 1000, de préférence en moyenne entre 15 et 800, de préférence en moyenne entre 50 et 600, et avantageusement en moyenne entre 100 et 400 unités.

Dans la structure ci-dessus, les monosaccharides aux extrémités de la structure (i.e., avec des liaisons représentées en pointillés) schématisent la continuité du polymère (i.e., enchaînement d'unités β-cyclodextrines formant le reste du polymère).

Le polycyclodextrine peut être un poly-β-cyclodextrine de masse molaire plus élevée, et peut répondre à la formule I ci-dessus, dans laquelle n représente un entier compris entre 1 et 50, de préférence entre 2 et 10 ; et le nombre d'unités beta-cyclodextrine est en moyenne compris entre 10 et 2000, de préférence en moyenne entre 100 et 1800 unités, de préférence en moyenne entre 500 et 1600 unités, et avantageusement en moyenne entre 800 et 1500 unités.

Le polycyclodextrine peut être un poly-α-cyclodextrine répondant à la Formule I ci-dessus dans laquelle les unités α-cyclodextrines sont remplacées par des α-cyclodextrines.

Le polycyclodextrine peut être un poly-γ-cyclodextrine répondant à la Formule I ci-dessus dans laquelle les unités γ-cyclodextrines sont remplacées par des γ-cyclodextrines.

Selon l'invention, le groupement polyéthylèneglycol ramifié peut être un dendrimère de polyéthylèneglycol.

Dans la présente, le terme « dendrimère » se rapporte à une molécule dont l'architecture reprend celle des branches d'un arbre. Il s'agit d'une macromolécule de structure tridimensionnelle où les monomères branchés sont associés selon un processus arborescent autour d'un coeur central multivalent. Les dendrimères prennent généralement une forme globulaire très régulière ou sphérique, hautement ramifiée et plurifonctionnalisée. Ils sont constitués de trois régions spécifiques :

- un coeur central multivalent,
- un nombre défini (constituant la multivalence) de branches dendritiques intermédiaires connectées au coeur central multivalent où chaque branche dendritique est constituée d'un certain nombre de génération de ramification,
- et la périphérie constituée d'une multitude de groupes terminaux fonctionnels.

Ces dendrimères présentent à la fois des cavités internes et un grand nombre de groupements terminaux à la périphérie, facilement accessibles, qui peuvent être responsables de propriétés et de réactivités très variées.

Les dendrimères sont construits étape par étape à l'aide d'une succession de séquences, chacune aboutissant à une nouvelle génération. Le contrôle structural est déterminant pour les propriétés spécifiques de ces macromolécules. Des méthodes de synthèse sont connues dans l'art. Par exemple, on peut citer :

Navath R S, Menjoge A R, Dai H, Romero R , Kannan S, Kannan R M., Injectable PAMAM dendrimer-PEG hydrogels for the treatment of génital infections: formulation and in vitro and in vivo evaluation, Mol Pharm. 2011 Aug

1;8(4):1209-23 [34]. R C. Hedden et B. J. Bauer,Structure and Dimensions of PAMAM/PEG Dendrimer-Star Polymers, Macromolecules, 2003, 36 (6), pp 1829-1835 **[35].**

Certains de ces dendrimères PEG sont commerciaux, il peut s'agir d'un PEG ramifié répondant à l'une des structures suivante :

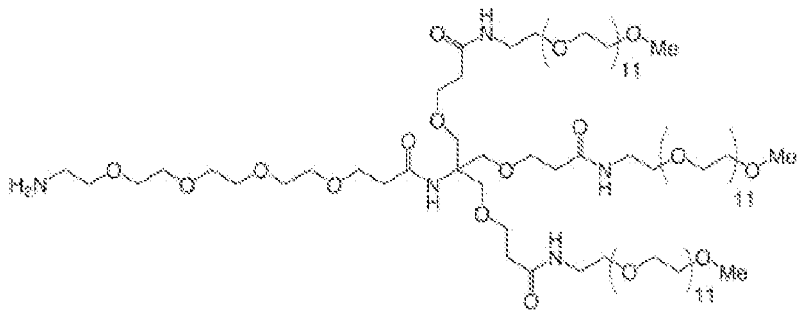

(H2N-dPEG(4)-[dPEG(12)-OMe]3 ou C99H197N5047 Iris Biotech, ref PEG1325.0100), ou

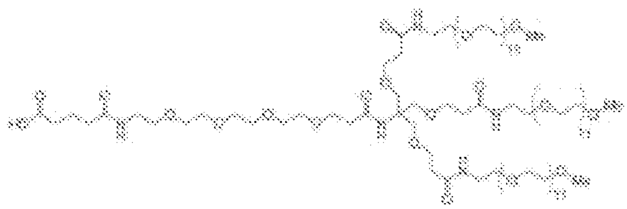

(HOOC-dPEG(4)-[dPEG(12)-OMe]3 ou C104H203N5050 Iris Biotech, ref PEG1490.0100).

Il peut s'agir également du kit PAMAM-PEG Dendrimère, générations 3-6 commercialisé par la société Sigma-Aldrich sous la référence 683493.

L'agent de surface peut être une protéine. Dans ce cas, plusieurs types d'interactions peuvent avoir lieu avec la surface du MOF par le biais à la fois de fonctions $NH_2$ et COOH présentes sur la protéine, par exemple. La protéine peut être enzymatique, de structure, de transport, de signalisation, régulatrice ou motrice, tels que de l'albumine ou les immunoglobulines.

Dans la présente, on entend par « polysaccharide portant une pluralité de chaînes latérales polyéthylèneglycol » un polysaccharide sur lequel sont greffés des groupes polyéthylèneglycol. Le polysaccharide peut être un polysaccharide naturel ou synthétique. Par exemple, il peut s'agir de l'acide hyaluronique, l'acide alginique, le chitosane, la chitine, le scleroglucane, le dextrane, l'amilose, l'amilopectine, un dérivé de la cellulose, l'amidon, le pullulane, la pectine, un alginate, l'héparine, l'ulvane, un caragheenane, le fucane, le curdlan, le xylane, l'acide polyguluronique, le xanthane, l'arabinane, des polysaccharides contenant l'acide sialique ou l'acide polymannuronique. Avantageusement, le polysaccharide peut être le chitosane.

Des méthodes pour greffer des groupes polyéthylèneglycol à des polysaccharides naturels ou synthétiques sont connues. Par exemple, l'homme du métier pourra s'inspirer des méthodes décrites dans :

- N Bhattarai, H R. Ramay, J Gunn, F A. Matsen, M Zhan, PEG-grafted chitosane as an injectable thermosensitive hydrogel for sustained protein release, J Controlled Release, 103 (3), 609-624, 2005. **[36]** où plus de 40% en poids de PEG ont pu être gréffés au chitosane

- J.A Wieland, T.L. Houchin-Ray, L.D. Shea, Non-viral vector delivery from PEG-hyaluronic acid hydrogels, J. Controlled Release, 120 (3), 233-241, 2007. **[37],** dans lequel, avantageusement, le PEG étoilé (4 bras) portant des fonctionnalités de type acryle a été gréffé à l'acide hyaluronique par photopolymérisation.

Selon l'invention, le polysaccharide insoluble dans l'eau à 6<pH<8 et soluble dans l'eau à pH<5 peut être le chitosane. En faisant varier le pH, le diamètre hydrodynamique du polysaccharide peut être accru, ce qui l'empêche de pénétrer dans les pores des particules de MOF, et ainsi de permettre un meilleur contrôle du profil de libération des composés éventuellement encapsulés dans les MOFs. Le chitosane est hydrosoluble à pH faible, mais forme des agrégats à pH neutre. Le chitosane peut être donc utilisé pour recouvrir par une technique apparentée à la coacervation les nanoMOFs de la présente invention en jouant sur le pH.

Des cyclodextrines phosphatées sont connues dans l'art antérieur. Par exemple, on pourra citer la beta-cyclodextrine-phosphate commercialisée par la société Cyclolab.

Des cyclodextrines sulfatées sont connues dans l'art antérieur. Par exemple, on pourra citer la beta-cyclodextrine-sulfate commercialisée par la société Cyclolab Des cyclodextrines bisphosphonatées sont connues dans l'art antérieur. Par exemple, on pourra se référer à la publication X.M. Liu, H. Lee, R. Reinhardt, L. Marky, W Dong, J. Controlled Release vol 122, 2007, 54-62, Novel biomineral-binding cyclodextrins for controlled drug delivery in the oral cavity (beta-cyclodextrine-alendronate). **[38]**

Les méthodes de synthèse pour fonctionnaliser les alpha et/ou gamma-cyclodextrines avec les groupements phosphate, sulfate et bisphosphonate peuvent être adaptées des méthodes de synthèse utilisées pour la préparation de leur homologues beta-cyclodextrine citées ci-dessus.

Le groupement biphosphonate peut se présenter sous la forme d'un groupement alendronate ou zoledronate.

Avantageusement, on privilégiera les groupements complexant les plus forts des sites métalliques, par exemple les groupes phosphate, cathécolate, carboxylate, sulfate phosphonates et/ou biphosphonate.

Les groupements moins complexants, comme les hydroxy, thiolate, groupes hétérocycliques azotés, amido ou amino, peuvent également être utilisés. Dans ce cas, on utilisera de préférence un agent de surface organique, tel que défini précédemment, fonctionnalisé avec plusieurs groupements amino, afin de compenser le faible pouvoir complexant de ces derniers.

On notera que les groupements COOH ou $NH_2$ présents sur les protéines par exemple, peuvent également participer à l'ancrage de la protéine (comme agent de surface) sur la surface des MOFs.

De manière générale, le terme « substitué », précédé ou non du terme « éventuellement », et les substituants décrits dans les formules de la présente demande, désignent le remplacement d'un radical hydrogène dans une structure donnée avec le radical d'un substituant spécifié. Le terme « substitué » désigne par exemple le remplacement d'un radical hydrogène dans une structure donnée par un radical $R^2$ tel que défini précédemment. Lorsque plus d'une position peut être substituée, les substituants peuvent être les mêmes ou différents à chaque position.

On entend par « ligand espaceur » au sens de la présente invention, un ligand (incluant par exemple les espèces neutres et les ions) coordiné à au moins deux sites métalliques M, participant à l'éloignement entre ces sites métalliques et à la formation d'espaces vides ou pores. Le ligand espaceur peut comprendre plusieurs fonctions complexantes comprenant carboxylates, phosphonates, imidazolates, de préférence de 2 à 6 groupements fonctionnels qui peuvent être mono, bi tri ou tétradentates, c'est à dire comprendre 1, 2, 3 ou 4 points d'attachement au site métallique.

On entend par « surface externe » au sens de la présente invention, la surface extérieure des matériaux MOFs, c'est-à-dire excluant la surface des pores (micropores et/ou mesopores) des MOFs.

On entend par « alkyle » au sens de la présente invention, un radical carboné linéaire, ramifié ou cyclique, saturé ou insaturé, éventuellement substitué, comprenant 1 à 25 atomes de carbone, par exemple 1 à 10 atomes de carbone, par exemple 1 à 8 atomes de carbone, par exemple 1 à 6 atomes de carbone.

On entend par « alkylène » au sens de la présente invention, un radical carboné divalent linéaire, ramifié ou cyclique, saturé, éventuellement substitué, comprenant 1 à 25 atomes de carbone, par exemple 1 à 10 atomes de carbone, par exemple 1 à 8 atomes de carbone, par exemple 1 à 6 atomes de carbone.

On entend par « alcènylène » au sens de la présente invention, un radical alkylène, tel que défini précédemment, présentant au moins une double liaison carbone-carbone.

On entend par « alcynylène » au sens de la présente invention, un radical alkylène, tel que défini précédemment, présentant au moins une triple liaison carbone-carbone.

On entend par « aryle » au sens de la présente invention, un système aromatique comprenant au moins un cycle satisfaisant la règle d'aromaticité de Hückel. Ledit aryle est éventuellement substitué et peut comprendre de 6 à 50 atomes de carbone, par exemple 6 à 20 atomes de carbone, par exemple 6 à 10 atomes de carbone.

On entend par « hétéroaryle » au sens de la présente invention, un système comprenant au moins un cycle aromatique de 5 à 50 chaînons parmi lesquels au moins un chaînon du cycle aromatique est un hétéroatome, notamment choisi dans le groupe comprenant le soufre, l'oxygène, l'azote, le bore. Ledit hétéroaryle est éventuellement substitué et peut comprendre de 1 à 50 atomes de carbone, de préférence 1 à 20 atomes de carbone, de préférence 3 à 10 atomes de carbone.

On entend par « amino » au sens de la présente invention, un système de formule $-N(R)_2$ où chaque occurrence de R représente indépendamment H, $C_{1-6}$alkyle, ou $C_{6-10}$aryle, de préférence H, $C_{1-6}$alkyle, ou phényle.

On entend par « amido » au sens de la présente invention, un système de formule $-C(=O)N(R)_2$ où chaque occurrence de R représente indépendamment H, $C_{1-6}$alkyle, ou $C_{6-10}$aryle, de préférence H, $C_{1-6}$alkyle, ou phényle.

On entend par « hétérocyclique azoté » au sens de la présente invention, un système cyclique mono- ou polycyclique saturé ou insaturé et non-aromatique comprenant de 5 à 20 chaînons, et comprenant éventuellement un ou plusieurs cycles à 5 ou 6 chaînons ayant au moins un atome d'azote, et éventuellement entre 1 à 2 autres hétéroatomes sélectionnés indépendamment les uns des autres parmi S, O, et N, dans lesquels (i) chaque cycle à 5 chaînons a de 0 à 2 double liaisons, et chaque cycle à 6 chaînons a de 0 à 3 double liaisons, (ii) les atomes de soufre et/ou d'azote sont

éventuellement oxydés, et (iii) les atomes d'azote sont éventuellement sous forme de sel quaternaires. Par exemple, un radical hétérocyclique peut être un groupe pyrrolidinyle, pyrazolinyle, pyrazolidinyle, imidazolinyle, imidazolidinyle, piperidinyle, piperazinyle, oxazolidinyle, isoxazolidinyle, morpholinyle, thiazolidinyle, isothiazolidinyle, ou tétrahydrofuryle.

Par « structure tridimensionnelle », on entend une succession ou répétition tridimensionnelle de motifs de formule (I) tel que l'on entend de façon conventionnelle dans le domaine des matériaux MOFs, que l'on caractérise également comme « polymères de coordination métallo-organiques ».

Sauf indication contraire, les divers modes de réalisation qui suivent concernant les matériaux MOF s'appliquent autant à leurs utilisations qu'à leur procédé de préparation selon la présente invention.

On entend par « agent de surface » selon l'invention une molécule recouvrant en partie ou en totalité la surface du solide permettant de moduler les propriétés de surface du matériau, par exemple :

- de modifier sa biodistribution, par exemple pour éviter sa reconnaissance par le système réticulo endothélial (« furtivité »), et/ou
- de lui conférer des propriétés de bioadhésion intéressantes lors de l'administration par voies orale, oculaire, nasale, et/ou rectale
- de lui permettre un ciblage spécifique de certaines organes/tissus malades, etc.

Selon l'invention, plusieurs agents de surface peuvent être utilisés pour combiner les propriétés précitées.

Selon l'invention un agent de surface combinant au moins deux des propriétés précitées peut être utilisé.

Selon l'invention, l'agent de surface organique peut être choisi par exemple dans le groupe comprenant :

- les $\alpha$-, $\beta$- ou $\gamma$-cyclodextrines ;
- les oligomères de $\alpha$-, $\beta$- ou $\gamma$-cyclodextrines ;
- les poly-$\alpha$, poly-$\beta$ ou poly-$\gamma$-cyclodextrines,
- les copolymères de $\alpha$, $\beta$ et/ou $\gamma$-cyclodextrines,
- les PEG dendrimères,
- le chitosane,
- le chitosane portant une pluralité de chaînes latérales PEG,
- l'albumine, immunoglobulines,..

lequel agent de surface comprend un ou plusieurs groupe(s) phosphate, phosphonate, bisphosphonate, sulfate, carboxylate, hydroxy, cathécolate, thiolate, hétérocyclique azoté (par exemple, azolate, imidazolate), amido ou amino.

Selon l'invention, les unités cyclodextrine du poly-$\alpha$, poly-$\beta$ ou poly-$\gamma$-cyclodextrine ou le copolymère de $\alpha$, $\beta$ et/ou $\gamma$-cyclodextrine peuvent être liées entre elles par des chaînes hydrocarbonées répondant à la formule $-O-(CH_2-CHOR^1-CH_2)_n-O-$où n est un entier compris entre 1 et 50 et, dans chacune des unités ($CH_2-CHOR^1-CH_2$), $R^1$ désigne soit un atome d'hydrogène, soit une chaine $-CH_2-CHOH-CH_2-O-$ reliée à une unité cyclodextrine dudit polymère ou copolymère.

Selon l'invention, le poly-$\alpha$, poly-$\beta$ ou poly-$\gamma$-cyclodextrine ou le copolymère de $\alpha$, $\beta$ et/ou $\gamma$-cyclodextrine peut être obtenu par polycondensation de molécules de cyclodextrine et d'épichlorhydrine.

Le solide MOF selon l'invention peut comprendre des atomes de métaux di-, tri- ou tétravalents. Les atomes métalliques peuvent avoir une géométrie octaédrique, pentaédrique, tétraédrique, voire être en coordinence supérieure dans la structure du matériau.

Par « coordinence » ou « nombre de coordination », on entend le nombre de liaisons pour laquelle les deux électrons partagés dans la liaison proviennent du même atome. L'atome donneur d'électrons acquiert une charge positive alors que l'atome accepteur d'électrons acquiert une charge négative.

En outre, les atomes métalliques peuvent être isolés ou regroupés en entités inorganiques. Le solide MOF selon l'invention peut par exemple être construit à partir de chaînes de polyèdres, de dimères, trimères,tétramères, pentamères ou hexamères de polyèdres ou d'une combinaison de ces entités. Par exemple, le solide MOF selon

l'invention peut être construit à partir de chaînes d'octaèdres, de dimères, trimères ou tétramères d'octaèdres. Par exemple, les matériaux MOF carboxylate de fer selon l'invention peuvent être construits à partir de chaines d'octaèdres liés par les sommets ou les arêtes ou de trimères d'octaèdres connectés par un atome d'oxygène central.

On entend par « entité inorganique » au sens de la présente invention un ensemble d'atomes contenant au moins deux métaux liés par des liaisons ionocovalentes, soit directement par des anions, par exemple O, OH, Cl, F, etc., soit par le ligand organique.

De plus, le solide MOF selon l'invention peut se présenter sous différentes formes ou « phases » compte tenu des divers possibilités d'organisation et de connections des ligands au métal ou au groupement métallique.

On entend par « phase » au sens de la présente invention une composition hybride comprenant au moins un métal et au moins un ligand organique possédant une structure cristalline définie.

L'organisation spatiale cristalline du solide de la présente invention est à la base des caractéristiques et propriétés particulières de ce matériau, et régit notamment la taille des pores, qui a une influence sur la surface spécifique du matériau et sur les caractéristiques d'adsorption, mais également la densité du matériau, celle-ci étant relativement faible, la proportion de métal dans ce matériau, la stabilité du matériau, la rigidité et la flexibilité de sa structure, etc.

En particulier, le solide MOF selon l'invention peut être isoréticulaire, c'est à dire comprendre des réseaux de même topologie.

En outre, le solide de la présente invention peut comprendre des motifs qui contiennent soit un seul type d'ion métallique, soit plusieurs types d'ions métalliques.

Par exemple, le solide de la présente invention peut comprendre une succession tridimensionnelle de trois motifs différents. Par exemple également, le solide de la présente invention peut comprendre une succession tridimensionnelle de deux motifs différents.

En outre, la taille des pores peut être ajustée par le choix de ligands espaceurs appropriés.

Avantageusement, le ligand L du motif de formule (I) des solides MOF de la présente invention peut être un ligand portant plusieurs fonctions complexantes comprenant les carboxylates, phosphonates, imidazolates, de préférence le groupe carboxylate est un di-, tri-, tétra- ou hexa-carboxylate choisi dans le groupe comprenant :

| | |
|---|---|
| 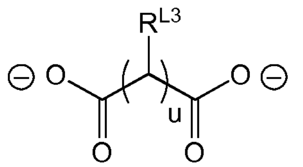 | 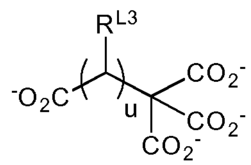 |
| 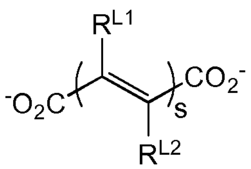 | 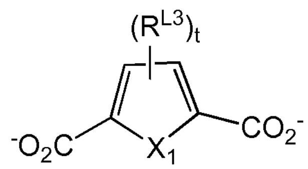 |
| 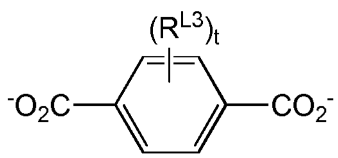 | 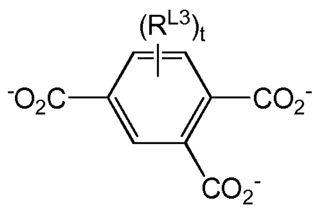 |
| 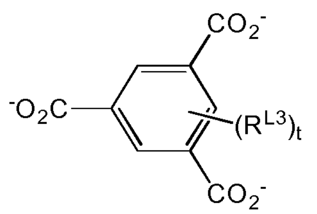 | 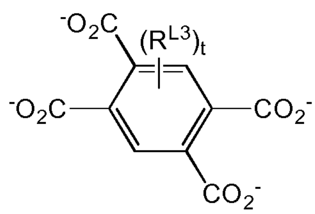 |

(suite)

| | |
|---|---|
| 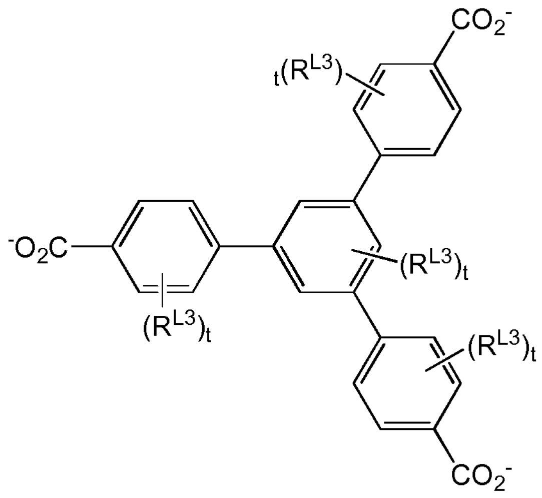 | 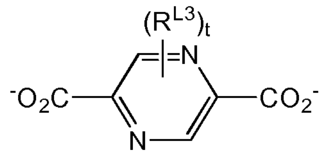 |
| 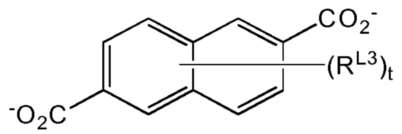 | 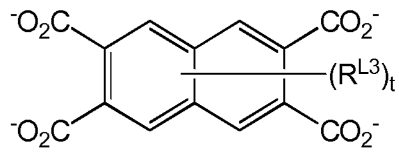 |
| 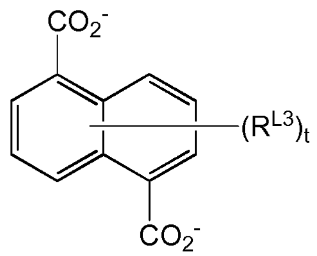 | 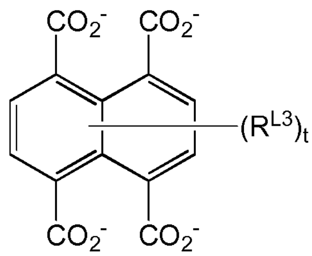 |
| 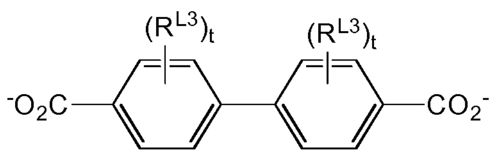 | |
| 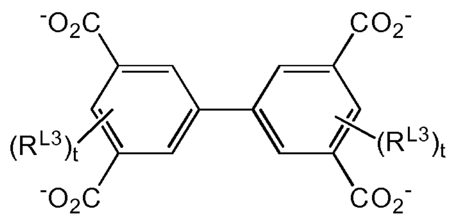 | |
| 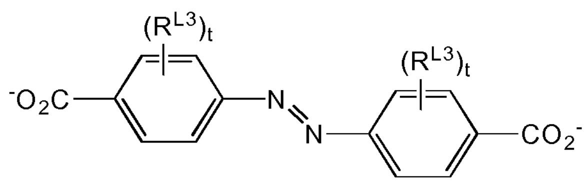 | |

(suite)

| |
|---|
| 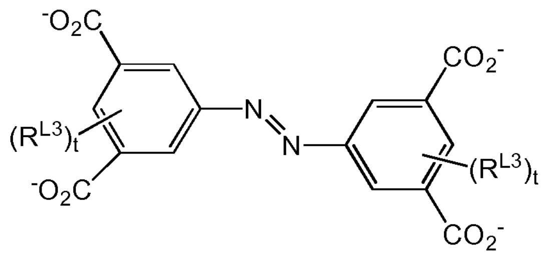 |
| 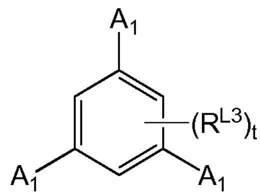 où $A_1$, $A_2$ et $A_3$ représentent indépendamment 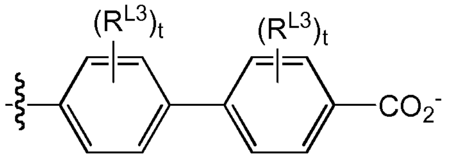 |

dans lesquels :

$X_1$ représente O ou S,

s représente un entier de 1 à 4,

chaque occurrence de t représente indépendamment un entier de 1 à 4,

u représente un entier de 1 à 7,

$R^{L1}$ et $R^{L2}$ représentent indépendamment H, un halogène ou un alkyle en $C_1$ à $C_6$ (de préférence méthyle ou éthyle), et

chaque occurrence de $R^{L3}$ représente indépendamment H, un halogène (de préférence F, Cl ou Br), OH, $NH_2$, $NO_2$ ou un alkyle en $C_1$ à $C_6$ (de préférence méthyle ou éthyle).

En particulier, le ligand L du motif de formule (I) de la présente invention peut être un ligand di-, tri- ou tétracarboxylate choisi dans le groupe comprenant : $C_2H_2(CO_2^-)_2$ (fumarate), $C_2H_4(CO_2^-)_2$ (succinate), $C_3H_6(CO_2^-)_2$ (glutarate), $C_4H_4(CO_2^-)_2$ (muconate), $C_4H_8(CO_2^-)_2$ (adipate), $C_7H_{14}$ $(CO_2^-)_2$ (azelate), $C_5H_3S(CO_2^-)_2$ (2,5-thiophènedicarboxylate), $C_6H_4(CO_2^-)_2$ (téréphtalate), $C_6H_2N_2(CO_2^-)_2$ (2,5-pyrazine dicarboxylate), $C_{10}H_6(CO_2^-)_2$ (naphtalène-2,6-dicarboxylate), $C_{12}H_8$ $(CO_2^-)_2$ (biphényle-4,4'-dicarboxylate), $C_{12}H_8N_2(CO_2^-)_2$ (azobenzènedicarboxylate), $C_6H_3(CO_2^-)_3$ (benzène-1,2,4-tricarboxylate), $C_6H_3(CO_2^-)_3$ (benzène-1,3,5-tricarboxylate), $C_{24}H_{15}(CO_2^-)_3$ (benzène-1, 3, 5-tribenzoate), $C_6H_2(CO_2^-)_4$ (benzène-1,2,4,5-tétracarboxylate, $C_{10}H_4$ $(CO_2^-)_4$ (naphtalène-2,3,6,7-tétracarboxylate), $C_{10}H_4$ $(CO_2^-)_4$ (naphtalène-1,4,5,8-tétracarboxylate), $C_{12}H_6(CO_2^-)_4$ (biphényl-3,5,3',5'-tétracarboxylate), et les analogues modifiés choisis dans le groupe comprenant le 2-aminotéréphtalate, le 2-nitrotéréphtalate, le 2-méthyltéréphtalate, le 2-chlorotéréphtalate, le 2-bromotéréphtalate, le 2,5-dihydroxotéréphtalate, le tétrafluorotéréphtalate, le tétraméthyltéréphtalate, le diméthyl-4,4'-biphénydicarboxylate, le tétraméthyl-4,4'-biphénydicarboxylate, le dicarboxy-4,4'-biphénydicarboxylate, le 2,5-pyrazyne dicarboxylate. Le ligand L du motif de formule (I) de la présente invention peut également représenter le 2,5 diperfluorotéréphthalate, azobenzène 4,4'-dicarboxylate, 3,3'-dichloro azobenzène 4,4'-dicarboxylate, 3,3'-dihydroxo azobenzène 4,4'-dicarboxylate, 3,3'-diperfluoro azobenzène 4,4'-dicarboxylate, 3,5,3',5'-azobenzène tétracarboxylate, 2,5-diméthyl téréphthalate, perfluorosuccinate, perfluoromuconate, perfluoro glutarate, 3,5,3',5' perfluoro-4,4'-azobenzène dicarboxylate, 3,3'-diperfluoro azobenzène 4,4'-dicarboxylate.

Avantageusement, le ligand L du motif de formule (I) de la présente invention peut être aussi un ligand imidazolate, tétrazolate, phosphate ou phosphonate tels que l'imidazole, 2-méthylimidazolate, 2-éthylimidazole, 4,(-imidazol-dicarboxyic acid, 1,4-(butanediyl)bis(imidazole), purine, pyrimidine, benzimidazolate, pipérazinediphosphonate, tétrazolylbenzoate.

La plupart des ligands listés ci-dessus sont commerciaux. Le lecteur pourra se référer à la partie Exemples et/ou aux demandes internationales WO 2009/077670 et WO 2009/077671 pour la préparation des ligands non commerciaux.

Le ligand L peut présenter une activité biologique. Les solides hybrides nanoporeux selon l'invention possèdent une partie minérale, le métal (fer), et une partie organique, un ligand avec deux ou plusieurs fonctions complexantes (carboxylate, phosphate, amide, etc). L'incorporation de ligands organiques qui possèdent une activité biologique a l'avantage de permettre une libération contrôlée de molécules actives en fonction de la vitesse de dégradation du matériau (il s'agit des ligands biologiquement actifs précités qui sont libérés lors de la dégradation du matériau MOF). Ainsi, le matériau MOF lui-même peut être « bioactif », c'est-à-dire qu'il est susceptible de libérer des composants ayant une activité biologique.

En outre, la libération de ces molécules actives qui font partie du réseau MOF peut être combinée avec la libération d'autres principes actifs encapsulés dans les solides MOF selon l'invention. Cet aspect d'encapsulation de principes actifs est décrit *infra* dans le présent document.

Ainsi, la présente invention concerne également des solides MOF comprenant des ligands biologiquement actifs et/ou encapsulant un ou plusieurs principes actifs, avec une activité peut-être complémentaire ou différente, et leur utilisation pour thérapies combinées. La thérapie combinée est mise en oeuvre par relargage (i) du principe actif encapsulé dans les pores du matériau MOF et (ii) des ligands biologiquement actifs incorporés dans le réseau (la charpente) du matériau MOF cristallin.

Il existe de nombreuses molécules organiques biologiquement actives comprenant des fonctions complexantes, susceptibles de former solides hybrides poreux selon la présente invention.

Par exemple, il peut s'agir de l'acide azelaique ($HO_2C(CH_2)_7CO_2H$, agent dermatologique avec une activité antinéoplasique), du meprobamate (anticonvulsivante, sédatif, relaxant musculaire, anti-anxiété), de l'acide aminosalicylique (antituberculose), du chlodronate, pamidronate, zoledronate, alendronate et etidronate (traitement curatif de l'ostéoporose post-ménopausique), des azobenzènes (activité antimicrobienne, inhibiteurs de la COX), des porphyrines ou des aminoacides (Lys, Arg, Asp, Cys, Glu, Gln, etc.), de l'acide 4-aminosalycique, pyrazinamide (antituberculeux), de l'acide dibenzofuran-4,6-dicarboxylique (inhibiteur de transtryretin), de l'acide dipicolinique (inhibiteur de la dihydrodipicolinate reductase), de l'acide glutamique, de l'acide fumarique, de l'acide succinique, de l'acide subérique, de l'acide adipique, de l'acide nicotinique, du nicotinamide, de purines, pyrimidines....

Citons par exemple, l'activité antimicrobienne ou anti-inflammatoire (NSAIDs, inhibiteurs COX) des azobenzènes. À ce titre, le lecteur pourra se référer aux références suivantes : G. Oros, T. Cserhati, E. Forgacs, Chemosphere 52, 2003, 185 **[15],** A.M. Badawi, E.M.S. Azzam, S.M.I. Morsy, Bioorg. Med. Chem., 14, 2006, 8661 **[16]** et W-J. Tsai, Y-J Shiao, S-J Lin, W-F Chiou, L-C Lin, T-H Yang, C-M teng, T-S Wu, L-M Yang, Bioorg. Med. Chem. Letters 16, 2006, 4440 **[17].**

Ainsi, le ligand L peut être un ligand biologiquement actif choisi dans le groupe comprenant $C_7H_{14}(CO_2^-)_2$ (azelate) ; aminosalicylate (groupes carboxylique, amino et hydroxo) ; chlodronate, pamidrontate, alendronate et etidronate (comportant des groupes phosphonate) ; meprobamate (comportant des groupes carbamate) ; des porphyrines comprenant des groupes carboxylates, phosphonates et/ou amino ; des aminoacides (Lys, Arg, Asp, Cys, Glu, Gln, etc.) qui comportent des groupes amino, carboxylate, amide et/ou imine ; des azobenzènes comprenant des groupes carboxylates, phosphonates, et/ou amino ; le dibenzofuran-4,6-dicarboxylate, le dipicolinate (ligand mixte de type pyridine avec des groupes carboxyliques) ; le glutamate, le fumarate, le succinate, le suberate, l'adipate, le nicotinate, nicotinamide, purines, pyrimidines...

L'anion X du motif de formule (I) de la présente invention peut être choisi dans le groupe comprenant $OH^-$, $Cl^-$, $Br^-$, $F^-$, $R-(COO)_n^-$, $PF_6^-$, $NO_3^-$, $SO_4^{2-}$, $ClO_4^-$, avec R et n tels que définis précédemment.

En particulier, l'anion X du motif de formule (I) de la présente invention peut être choisi dans le groupe comprenant $OH^-$, $Cl^-$, $F^-$, $CH_3-COO^-$, $PF_6^-$, $ClO_4^-$, ou alors un ligand choisi dans la liste ci-dessus.

Avantageusement, l'anion X peut être choisi dans le groupe comprenant $OH^-$, $Cl^-$, $F^-$ et $R-(COO)_n^-$ où R représente $-CH_3$, $-C_6H_3$, $-C_6H_4$, $-C_{10}H_4$ ou $-C_6(CH_3)_4$.

Dans un mode de réalisation, l'anion X peut être sous une forme isotopique adaptée aux techniques d'imagerie telles que la tomograhie par émission de positrons (TEP).

La **tomographie par émission de positrons** (TEP) est une méthode d'imagerie médicale nucléaire qui permet de mesurer en trois dimensions l'activité métabolique d'un organe grâce aux émissions produites par les positrons issus de la désintégration d'un produit radioactif injecté au préalable. La TEP repose sur le principe général de la scintigraphie qui consiste à injecter un traceur dont on connaît le comportement et les propriétés biologiques pour obtenir une image du fonctionnement d'un organe. Ce traceur est marqué par un atome radioactif (carbone, fluor, azote, oxygène...) qui émet des positrons dont l'annihilation produit elle-même deux photons. La détection de la trajectoire de ces photons

par le collimateur de la caméra TEP permet de localiser le lieu de leur émission et donc la concentration du traceur en chaque point de l'organe. C'est cette information quantitative que l'on représente sous la forme d'une image faisant apparaitre en couleurs les zones de forte concentration du traceur.

Ainsi la TEP permet de visualiser les activités du métabolisme des cellules : on parle d'imagerie fonctionnelle par opposition aux techniques d'imagerie dite structurelle comme celles basées sur les rayons X (radiologie ou CT-scan) qui se limitent aux images de l'anatomie. Par conséquent, la tomographie par émission de positrons est un outil diagnostic qui permet de déceler certaines pathologies qui se traduisent par une altération de la physiologie normale comme les cancers. La TEP est aussi utilisée en recherche biomédicale, par exemple en imagerie cérébrale où elle permet de révéler les régions actives du cerveau lors de telle ou telle activité cognitive de manière analogue à ce qui se fait avec l'imagerie par résonance magnétique fonctionnelle.

Par exemple, X peut représenter $^{18}F^-$, qui est un émetteur de positrons et permet donc l'utilisation des solides MOF de l'invention pour des applications impliquant l'imagerie TEP.

Ainsi, dans un mode de réalisation, dans le motif de formule (I), au moins une occurrence du ligand X est $^{18}F^-$.

Le solide MOF selon l'invention, peut comprendre un pourcentage de métal en phase sèche de 5 à 40%, de préférence de 18 à 31%.

Le pourcentage massique (%m) est une unité de mesure utilisée en chimie et en métallurgie pour désigner la composition d'un mélange ou d'un alliage, c'est-à-dire les proportions de chaque composant dans le mélange.

1 %m d'un composant = 1g du composant pour 100 g de mélange ou encore 1 kg dudit composant pour 100 kg de mélange.

Les solides MOF de la présente invention présentent notamment l'avantage d'avoir une stabilité thermique jusqu'à une température de 400 °C.

En particulier, le solide MOF de la présente invention présente notamment l'avantage d'avoir une stabilité thermique de 120°C à 400°C.

En particulier, le solide MOF selon l'invention, peut être sous forme particulaire avec un diamètre des particules inférieur à 4 $\mu$m, de préférence inférieur à 1000 nanomètres.

En particulier, le solide MOF selon l'invention peut avoir une taille de pores de 0,4 à 6 nm, de préférence de 0,5 à 5,2 nm, et de manière plus préférée de 0,5 à 3,4 nm.

En particulier, le solide MOF selon l'invention peut avoir une surface spécifique (BET) de 5 à 6000 $m^2$/g, de préférence de 5 à 4500 $m^2$/g.

En particulier, le solide MOF selon l'invention peut avoir un volume poreux de 0,05 à 4 $cm^2$/g, de préférence de 0,05 à 2 $cm^2$/g.

Dans le cadre de l'invention, le volume poreux signifie le volume accessible pour les molécules de gaz et/ou de liquide.

Les inventeurs ont mis en évidence que les matériaux MOF comprenant une structure tridimensionnelle de motifs de formule (I) peuvent se présenter sous la forme d'une structure rigide ou flexible.

Le solide MOF de la présente invention peut se présenter sous la forme d'une structure robuste, qui a une charpente rigide et ne se contracte que très peu lorsque les pores se vident, ou sous la forme d'une structure flexible, qui peut se gonfler et se dégonfler faisant varier l'ouverture des pores en fonction de la nature des molécules adsorbées.

Ces molécules adsorbées qui peuvent être, par exemple, des solvants et/ou des gaz.

On entend par « structure rigide », au sens de la présente invention des structures qui se gonflent ou se contractent que très faiblement, c'est à dire avec une amplitude jusqu'à 10%.

En particulier, le solide MOF selon l'invention, peut avoir une structure rigide qui se gonfle ou se contracte avec une amplitude de 0 à 10%.

On entend par « structure flexible », au sens de la présente invention des structures qui se gonflent ou se contractent avec une grande amplitude, notamment avec une amplitude supérieure à 10%, par exemple supérieure à 50%.

En particulier, un matériau MOF de structure flexible peut se gonfler ou se contracter avec une amplitude de 10% à 300%, de préférence de 50 à 300%.

En particulier, le solide MOF selon l'invention, peut avoir une structure flexible qui se gonfle ou se contracte avec une amplitude supérieure à 10%, par exemple de 50 à 300%.

La présente invention peut être mise en oeuvre avec des matériaux MOF de structure rigide ou flexible.

Les propriétés de ces solides MOFs sont décrites par exemple dans les demandes internationales WO 2009/77670 et WO 2009/77671.

Différents matériaux MOF ont été élaborés par les inventeurs à l'Institut Lavoisier de Versailles avec des phases variées, nommées « MIL » (pour « Matériau Institut Lavoisier »). L'appellation « MIL » de ces structures est suivie d'un nombre arbitraire n donnée par les inventeurs pour identifier les différentes phases.

Dans le présent document, le sigle « ZIF » est l'abréviation du terme anglais « Zeolite Imidazolate Framework » (réseau zéolite à base d'imidazolates).

Dans le présent document, le sigle « UiO » est l'abréviation du terme anglais « University of Oslo » (Université d'Oslo).

Dans le présent document, le sigle « AEPF » est l'abréviation du terme anglais « alkaline-earth polymer framework» (solide de réseau polymère d'alcalino-terreux, en français).

Les inventeurs ont également mis en évidence que certains solides selon l'invention peuvent présenter un nombre plus élevé de phases possibles par rapport aux matériaux MOF classiquement rencontrés dans la littérature. Par exemple, différentes phases ont été obtenues pour les solides selon l'invention carboxylates de fer (III), par exemple MIL-53, MIL-69, MIL-88A, MIL-88B, MIL-88Bt, MIL-88C, MIL-88D, MIL-89, MIL-100, MIL-101, MIL-102. Ces différentes phases sont présentées *inter alia* dans les demandes internationales WO 2009/77670 et WO 2009/77671.

Les caractéristiques cristallographiques de ces structures sont connues, et ont fait l'objet de nombreux rapports. Il en est de même pour la description et le calcul des fenêtres d'accès aux pores de plus grande dimension des matériaux MOF décrits dans le présent document (le lecteur pourra trouver ces informations dans les publications citées dans le présent document pour chaque type de MOF spécifique cité). Par ailleurs, les dénominations « MIL » précitées sont des bien connues de l'homme du métier. On citera par exemple :

**MIL-53:** Whitfield, T. R.; Wang, X.; Liu, L.; Jacobson, A. J. Solid State Sci. 2005, 7, 1096.**[18]**;

**MIL-69:** T. Loiseau et al, C. R. Chimie, 8 765 (2005). **[19]**

**MIL-88A** : (a) Serre et al., « Role of solvent-host interactions that lead to very large swelling of hybrid frameworks », Science, 2007, Vol. 315, 1828-1831 **[20];** (b) Surblé et al., « A new isoreticular class of metal-organic frameworks with the MIL-88 topology », Chem. Comm., 2006, 284-286 **[21];** (c) Mellot-Draznieks et al., « Very large swelling in hybrid frameworks : a combined computational and power diffraction study », J. Am. Chem. Soc., 2005, Vol. 127, 16273-16278 **[22];** Chalati et al., « Optimisation of the synthesis of MOF nanoparticules made of flexible porous iron fumarate MIL-88A », J .Mater. Chem., 2011, 21, 2220 **[39].**

**MIL-88B, MIL-88C et MIL-88D**. Pour ces types structuraux, le lecteur pourra se référer aux publications concernant le type MIL-88A ci-dessus, à savoir, (a) Serre et al., « Role of solvent-host interactions that lead to very large swelling of hybrid frameworks », Science, 2007, Vol. 315, 1828-1831 **[20];** (b) Surblé et al., « A new isoreticular class of metal-organic frameworks with the MIL-88 topology », Chem. Comm., 2006, 284-286 **[21].**

**MIL-89** : C. Serre, F. Millange, S. Surblé, G. Férey Angew. Chem. Int. Ed. 2004, 43, 6286: A new route to the synthesis of trivalent transition metals porous carboxylates with trimeric SBU. **[23]**

**MIL-100** : Horcajada et al., « Synthesis and catalytic properties of MIL-100(Fe), an iron(III) carboxylate with large pores », Chem. Comm., 2007, 2820-2822. **[24]**

**MIL-101 :** Férey et al., « A chromium terephthalate-based solid with unusally large pore volumes and surface area », Science, 2005, Vol. 309, 2040-2042**.[25]**

**MIL-102 :** S. Surblé, F. Millange, C. Serre, T. Düren, M. Latroche, S. Bourrelly, P.L. Llewellyn and G. Férey « MIL-102: A Chromium Carboxylate Metal Organic Framework with Gas Sorption Analysis » J. Am. Chem. Soc. 128 (2006), 46, 14890. **[26]**

**UiO-66:** Pour ce type structural, le lecteur pourra se référer aux publications : (a) Cavka, J.; Jakobsen, S.; Olsbye, U.; Guillou, N.; Lamberti, C.; Bordiga, S.; Lillerud, K., J. Am. Chem. Soc. 2008, 130, 13850 **[40].** (b) Kandiah, M.; Nilsen, M.H.; Usseglio, S.; Jakobsen, S.; Olsbye, U.; Tilset, M.; Larabi, C.; Quadreli, E.A.; Bonino, F.; Lillerud K.P., Chem. Mater., 2010, 22(24), 6632 **[41]**. (c) Garibay S.J.; Cohen S.M., Chem. Commun., 2010, 46, 7700 **[42]**

**ZIF-8** : Pour ce type structural, le lecteur pourra se référer à Park et al., « Exceptional chemical and thermal stability of zeolitic imidazolate frameworks », Proc. Natl. Acad. Sci. U.S.A., 2006, 103, 10186 **[43]**

**MIL-125(Ti)** et **MIL-125(Ti)_$NH_2$.** Pour ce type structural, le lecteur pourra se référer aux publications suivantes : (a) M. Dan-Hardi, C. Serre, T. Frot, L. Rozes, G. Maurin, C. Sanchez and G. Férey : J. Am. Chem. Soc. Comm., 131, 2009, 10857-10859 A New Photoactive Crystalline Highly Porous Titanium (IV) Dicarboxylate **[44];** (b) C. Zlotea, D. Phanon, M. Mazaj, D. Heurtaux, V. Guillerm, C. Serre, P. Horcajada, T. Devic, E. Magnier, F. Cuevas, G. Férey, P. L. Llewellyn and M. Latroche : "Effet of NH2 and CF3 functionalization on the hydrogen sorption properties of MOFs" Dalton Trans., 2011, 40, 4879-4881 **[45]**

**AEPF-1(Ca)** et d'autre MOFs à base de calcium : **AEPF=alkaline-earth polymer framework** Pour ce type structural, le lecteur pourra se référer aux publications A.E. Platero-Prats, V.A. de la Pena-O'Shea, N. Snejko, A. Monge,E. Gutierrez-Puebla, « Dynamic calcium metal-organic framework acts as a selective organic solvent sponge », Chemistry, 16(38), 11632 **[46];** C. Volkringer, J. Marrot, G. Ferey, T. Loiseau, »Hydrothermal crystallization of three calcium-based hybrid solids with 2,6-naphthalene or 4,4'-biphenyl-dicarboxylates » Crystal Growth Design, 2008, 8, 685 **[47]**

**MIL-88B_9CH$_3$, MIL-88B_CH$_3$, MIL-88B_2CF$_3$, MIL-88B_2OH, MIL-88B_NO$_2$, MIL-88B_NH$_2$, MIL-88B_Cl, MIL-88B_Br, MIL-88B_4F:** Pour ce type structural, le lecteur pourra se référer aux publications concernant le type MIL-88 ci-dessus, à savoir, (a) Serre et al., « Role of solvent-host interactions that lead to very large swelling of hybrid frameworks », Science, 2007, Vol. 315, 1828-1831 **[20];** (b) Surblé et al., « A new isoreticular class of metal-organic frameworks with the MIL-88 topology », Chem. Comm., 2006, 284-286 **[21];** (c) Mellot-Draznieks et al., « Very large swelling in hybrid frameworks : a combined computational and power diffraction study », J. Am. Chem. Soc., 2005, Vol. 127, 16273-16278 **[22],** c) Horcajada et al., « How linker's modification controls swelling properties of highly flexible iron(III) dicarboxylates MIL-88 », J. Am. Chem. Soc., 2011, 133, 17839 **[48]**

En particulier, le solide MOF selon l'invention, peut avoir un motif de formule choisie dans le groupe comprenant :

$Fe(OH)[C_6H_4(CO_2)_2]$ de structure flexible, par exemple MIL-53 et ses formes fonctionnalisées MIL-53(Fe)_X(X=Cl, Br, CH3, 2CF$_3$...) (voir référence Devic et al., "Functionalization in flexible porous solids: effects on the pore opening and the host-guest interactions", J. Am. Chem. Soc., 2010, 132, 1127 **[49])**

$Fe_3OX[C_2H_2(CO_2)_2]_3$ de structure flexible, par exemple MIL-88A $Fe_3OX[C_4H_4(CO_2)_2]_3$ de structure flexible, par exemple MIL-89 (voir référence : C. Serre, S. Surblé, C. Mellot-Draznieks, Y. Filinchuk, G. Férey Dalton Trans., 2008, 5462-5464 : Evidence of flexibility in the nanoporous iron(III) carboxylate MIL-89 **[50])**

$Fe_3OX[C_6H_4(CO_2)_2]_3$ de structure flexible, par exemple MIL-88B

$Fe_3OX[O_2C\text{-}C_6(CH_3)_4\text{-}CO_2]_3.nH_2O$ de structure flexible, par exemple MIL-88Bt

$Fe_3OX[C_6H_4(CO_2)_2]_3$ de structure rigide, par exemple MIL-101

$Fe_3OX[C_6H_3(CO_2)_3]_3$ de structure rigide, par exemple MIL-100

$Al_3OX[C_6H_3(CO_2)_3]_3$ de structure rigide, par exemple MIL-100

$Fe_3OX[C_{10}H_6(CO_2)_2]_3$ de structure flexible, par exemple MIL-88C

$Fe_3OX[C_{12}H_8(CO_2)_2]_3$ de structure flexible, par exemple MIL-88D

$Zn_6N_{24}C_{48}H_{60}$ de structure rigide, par exemple ZIF-8

$Zr_6O_4(OH)_{4[}(CO_2)2C_6H_{4]6}$ de structure rigide, par exemple UiO-66

Ti8O8(OH)4[(CO2)2C6H4]6 de structure rigide, par exemple MIL-125

dans laquelle X est tel que défini précédemment.

Tout particulièrement, le solide MOF selon l'invention, peut avoir un motif de formule choisie dans le groupe comprenant :

MIL-101 (Fe) ou $Fe_3O[C_6H_4\text{-}(CO_{2)2}]_3.X.nH_2O$ (X=F, Cl, OH) de structure rigide

MIL-101 -Cl (Fe) ou $Fe_3O[Cl\text{-}C_6H_3\text{-}(CO_{2)2}]_3.X.nH_2O$ (X=F, Cl, OH) de structure rigide

MIL-101-NH$_2$ (Fe) ou $Fe_3O[NH_2\text{-}C_6H_3\text{-}(CO_{2)2}]_3.X.nH_2O$ (X=F, Cl, OH) de structure rigide

MIL-101-2CF$_3$ (Fe) ou $Fe_3O[(CF_3)_2\text{-}C_6H_2\text{-}(CO_{2)2}]_3.X.nH_2O$ (X=F, Cl, OH) de structure rigide

MIL-88B-$NO_2$ (Fe) ou $Fe_3O[C_6H_3NO_2$-$(CO_{2)2}]_3.X.nH_2O$ (X=F, Cl, OH) de structure flexible

MIL-88B-20H (Fe) ou $Fe_3O[C_6H_2(OH)_2$-$(CO_{2)2}]_3.X.nH_2O$ (X=F, Cl, OH) de structure flexible

MIL-88B-$NH_2$ (Fe) ou $Fe_3O[C_6H_3NH_2$-$(CO_{2)2}]_3.X.nH_2O$ (X=F, Cl, OH) de structure flexible

MIL-88B-$CH_3$ (Fe) ou $Fe_3O[C_6H_3CH_3$-$(CO_{2)2}]_3.X.nH_2O$ (X=F, Cl, OH) de structure flexible

MIL-88B-2$CH_3$ (Fe) ou $Fe_3O[C_6H_2(CH_3)_2$-$(CO_{2)2}]_3.X.nH_2O$ (X=F, Cl, OH) de structure flexible

MIL-88B-Cl (Fe) ou $Fe_3O[C_6H_3Cl$-$(CO_{2)2}]_3.X.nH_2O$ (X=F, Cl, OH) de structure flexible

MIL-88B-4$CH_3$ (Fe) ou $Fe_3O[C_6(CH_3)_4$-$(CO_{2)2}]_3.X.nH_2O$ (X=F, Cl, OH) de structure flexible

MIL-88B-4F (Fe) ou $Fe_3O[C_6F_4$-$(CO_{2)2}]_3.X.nH_2O$ (X=F, Cl, OH) de structure flexible

MIL-88B-Br (Fe) ou $Fe_3O[C_6H_3Br$-$(CO_{2)2}]_3.X.nH_2O$ (X=F, Cl, OH) de structure flexible

MIL-88B-2$CF_3$ (Fe) ou $Fe_3O[(CF_3)_2$-$C_6H_2$-$(CO_2)_2]_3.X.nH_2O$ (X=F, Cl, OH) de structure flexible

MIL-88D 4$CH_3$ (Fe) ou $Fe_3O[C_{12}H_4(CH_3)_4$-$(CO_{2)2}]_3.X.nH_2O$ (X=F, Cl, OH) de structure flexible

MIL-88D 2$CH_3$ (Fe) ou $Fe_3O[C_{12}H_6(CH_3)_2$-$(CO_{2)2}]_3.X.nH_2O$ (X=F, Cl, OH) de structure flexible

MIL-88E (Pyr) (Fe) ou $Fe_3O[C_4H_3N_2$-$(CO_{2)2}]_3.X.nH_2O$ (X=F, Cl, OH) de structure flexible

MIL-88F (Thio) (Fe) ou $Fe_3O[C_4H_2S$-$(CO_{2)2}]_3.X.nH_2O$ (X=F, Cl, OH) de structure flexible

MIL-53-20H (Fe) ou $FeO(OH)[C_6H_2(OH)_2$-$(CO_2)_2].X.nH_2O$ (X=F, Cl, OH) de structure flexible

MIL-53-$NH_2$ (Fe) ou $FeO(OH)[C_6H_2$-$NH_2(CO_{2)2}].X.nH_2O$ (X=F, Cl, OH) de structure flexible

MIL-53-Cl (Fe) ou $FeO(OH)[C_6H_2Cl$-$(CO_2)_2].X.nH_2O$ (X=F, Cl, OH) de structure flexible

MIL-53-Br (Fe) ou $FeO(OH)[C_6H_2Br$-$(CO_{2)2}].X.nH_2O$ (X=F, Cl, OH) de structure flexible

MIL-53-2$CF_3$ (Fe) ou $FeO(OH)[C_6H_2(CF_3)_2$-$(CO_{2)2}].X.nH_2O$ (X=F, Cl, OH) de structure flexible

MIL-53-$CH_3$ (Fe) ou $FeO(OH)[C_6H_3CH_3$-$(CO_{2)2}].X.nH_2O$ (X=F, Cl, OH) de structure flexible

MIL-53-2COOH (Fe) ou $FeO(OH)[C_6H_3$-$(CO_2)_4].X.nH_2O$ (X=F, Cl, OH) de structure flexible

MIL-88G (AzBz) (Fe) ou $Fe_3O[C_{12}H_8N_2$-$(CO_{2)2}]_3.X.nH_2O$ (X=F, Cl, OH) de structure flexible

MIL-88G 2Cl (AzBz-2Cl) (Fe) ou $Fe_3O[C_{12}H_6N_2Cl_2$-$(CO_{2)2}]_3.X.nH_2O$ (X=F, Cl, OH) de structure flexible.

En outre, à partir d'un même ligand acide carboxylique L et des mêmes bases de fer (chaînes ou trimères), les inventeurs ont pu obtenir des matériaux MOF de même formule générale (I) mais de structures différentes. C'est par exemple le cas des solides MIL-88B et MIL-101. En effet, la différence des solides MIL-88B et MIL-101 réside dans le mode de connexions des ligands aux trimères d'octaèdre : dans le solide MIL-101, les ligands L s'assemblent sous forme de tétraèdres rigides, tandis que dans le solide MIL-88B, ils forment des bipyramides trigonales, rendant possible l'écartement entre les trimères.

Ces différents matériaux sont présentés dans les demandes internationales WO 2009/77670 et WO 2009/77671. Le mode d'assemblage de ces ligands peut être contrôlé lors de la synthèse par exemple par l'ajustement du pH. Par exemple, le solide MIL-88 est obtenu en milieu moins acide que le solide MIL-101 comme décrit dans la demande internationale WO 2009/77671.

En particulier, le solide MOF de la présente invention peut avoir une phase choisie dans le groupe comprenant : MIL-53, MIL-88, MIL-100, MIL-101, MIL-102 comme décrit dans la demande internationale WO 2009/77671, ZIF-8 ,

MIL-125 et UiO-66.

Le solide MOF selon l'invention, peut comprendre au moins un métal possédant des propriétés paramagnétiques ou diamagnétiques. De préférence, le solide MOF selon l'invention peut comprendre un ou plusieurs métaux paramagnétiques, identiques ou différents, qui peuvent être le fer. En particulier, le solide MOF selon l'invention peut comprendre un ou plusieurs ions métalliques paramagnétiques, identiques ou différents, qui peuvent être choisi parmi $Fe^{2+}$ et $Fe^{3+}$.

Par ailleurs, le solide MOF selon l'invention peut être utilisé en imagerie. En outre, l'invention concerne également l'utilisation du solide MOF selon l'invention comme agent de contraste.

En effet, les agents de contraste sont caractérisés par leur relaxivité. Plus elle est importante, plus l'effet des agents de contraste est important. La relaxivité correspond à la capacité des agents de contraste à modifier les temps de relaxation des protons de l'eau du milieu suite à l'application d'un champ magnétique. Elle dépend des propriétés paramagnétiques des métaux utilisés mais également de la quantité et de la mobilité des molécules d'eau qui se coordinent au métal dans la première sphère interne, amenant la contribution la plus importante, ainsi que dans la sphère externe. Ces « sphères de coordination » représentent les atomes immédiatement attachés au centre métallique dans le cas de la 1ère sphère ; pour la sphère externe, cela représente les atomes immédiatement situés au-delà de la 1ère sphère.

Dans le cas du solide de l'invention, outre la susceptibilité magnétique du métal, dans cet exemple le fer(III), les caractéristiques structurales du solide de la présente invention permettent à l'eau de se coordiner autour de la 1ère sphère de coordination et de circuler dans les pores ce qui induit un effet sur les temps de relaxation longitudinal T1 et transversal T2 des protons de l'eau. En particulier, la relaxivité r2 du solide est suffisante pour une utilisation *in vivo* lors des expériences d'écho de gradient.

Par ailleurs, les travaux de recherche menés par les inventeurs leur ont permis d'élaborer une méthode de synthèse souple et modulable permettant d'obtenir les solides MOF selon l'invention ayant une organisation structurale isoréticulaire particulière avec de bons rendements. En outre, le procédé permet d'obtenir des nanoparticules de dimensions souhaitées et des tailles de particules et de pores homogènes.

Ainsi, l'invention concerne également un procédé de préparation d'un solide tel que défini dans la présente invention, comprenant au moins une étape réactionnelle (i) consistant à mélanger dans un solvant polaire :

au moins une solution comprenant au moins un précurseur inorganique métallique se présentant sous la forme de métal M, d'un sel de métal M ou d'un complexe de coordination comprenant l'ion métallique M dans laquelle M est tel que défini précédemment.

au moins un ligand L' de formule $-R^0(COR^3)_q$,

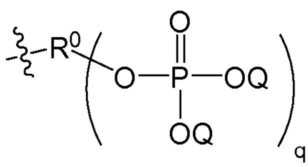
ou
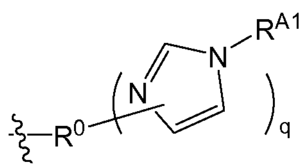
,

où

- Q, $R^{A1}$, q et $R^0$ sont tels que définis précédemment ; et
- et $R^3$ est choisi dans le groupe comprenant un radical -OH, un radical -OY où Y représente un cation alcalin, un halogène, ou un radical $-OR^4$, $-OC(=O)R^4$ ou $-NR^4R^{4'}$, où $R^4$ et $R^{4'}$ sont des radicaux alkyles en $C_{1-12}$ ;

de façon à obtenir ledit solide.

Selon l'invention, le procédé de préparation du solide de l'invention peut comprendre en outre une étape (iii) de fixation sur ledit solide d'au moins un agent de surface organique tel que défini précédemment.

Cette étape de fixation (iii) peut être réalisée pendant ou après l'étape réactionnelle (i) ou encore après une étape d'introduction (ii) d'une molécule d'intérêt. Cf exemples 22, 23 et 24 de WO 2009/77671.

Un certain nombre de solides MOF à surface externe modifiée sont illustrés dans la partie « Exemples ». Il est entendu que ces exemples sont donnés à titre illustratif, et non limitatif. Les méthodes de modification de surface des solides MOF illustrées dans les Exemples sont applicables et/ou adaptables à l'ensemble des solides MOF selon la présente invention (e.g., solides MOF à base de métal M différent de Fe, avec des ligands L différents, et/ou encapsulant ou non au moins un principe actif, un composé d'intérêt cosmétique et/ou un marqueur). Par exemple, ces méthodes peuvent être mises en oeuvre sans difficulté pour la modification des surfaces de l'ensemble des solides MOF décrits

dans la présente demande.

Avantageusement, le ligand L' peut représenter un ligand portant plusieurs fonctions complexantes comprenant les carboxylates, phosphonates, imidazolates, de préférence un groupe carboxylate di-, tri-, tétra- ou hexadentate choisi dans le groupe comprenant :

| | |
|---|---|
| 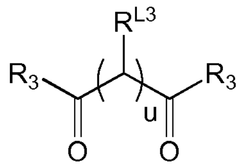 | 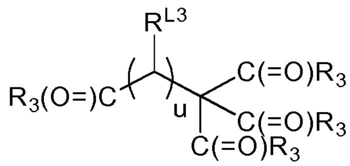 |
| 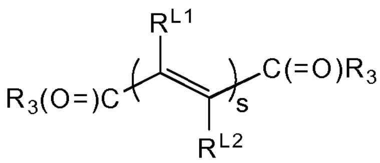 | 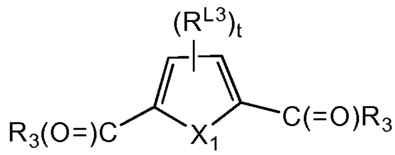 |
| 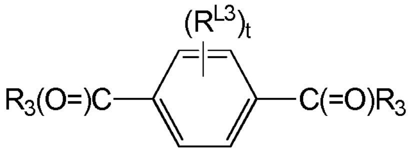 | 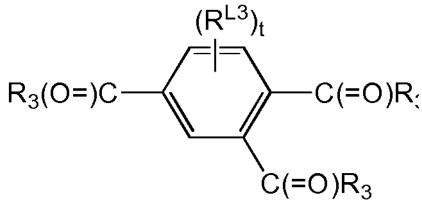 |
| 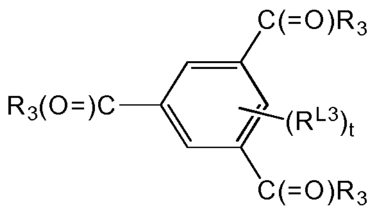 | 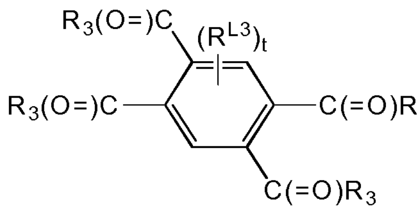 |
| 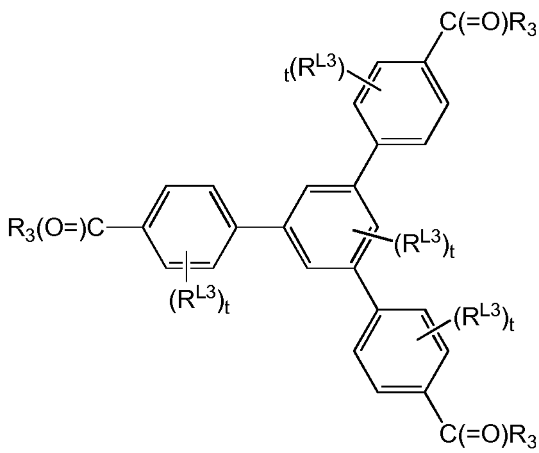 | 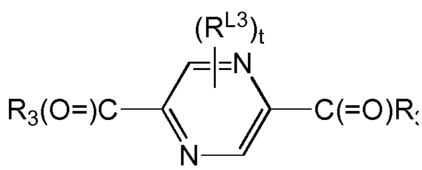 |
| 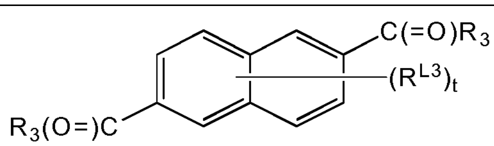 | 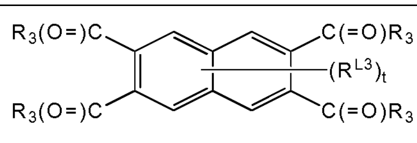 |

(suite)

| | |
|---|---|
| 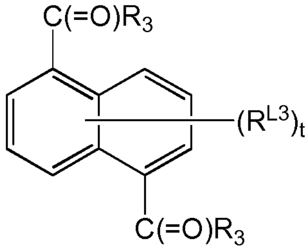 | 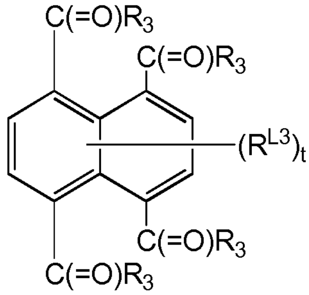 |
| 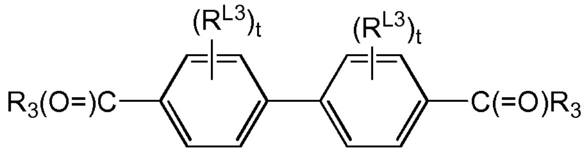 | |
| 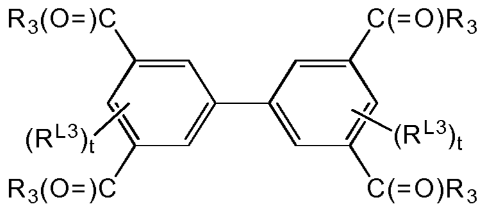 | |
| 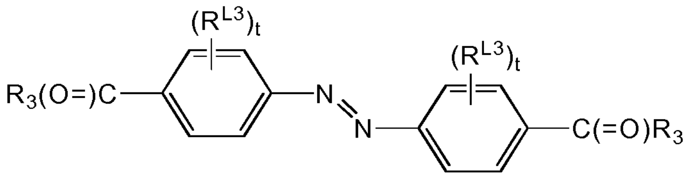 | |
| 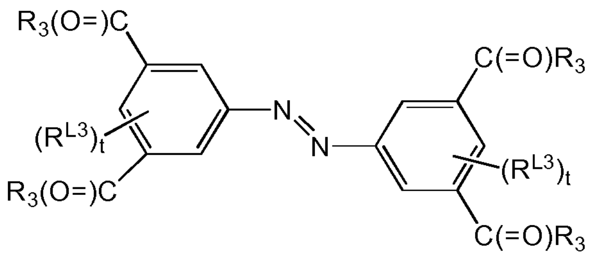 | |
| 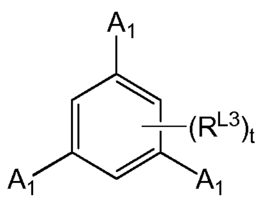 où $A_1$, $A_2$ et $A_3$ représentent indépendamment 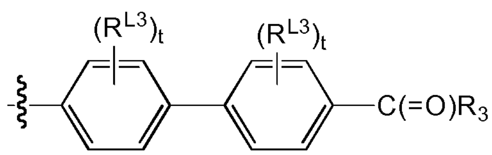 | |

dans lesquels :

$R^3$ est choisi dans le groupe comprenant un radical -OH, un radical -OY où Y représente un cation alcalin, un

halogène, ou un radical $-OR^4$, $-O-C(=O)R^4$ ou $-NR^4R^{4'}$, où $R^4$ et $R^{4'}$ sont des radicaux alkyles en $C_{1-12}$,

$X_1$ représente O ou S,

s représente un entier de 1 à 4,

chaque occurrence de t représente indépendamment un entier de 1 à 4,

u représente un entier de 1 à 7,

$R^{L1}$ et $R^{L2}$ représentent indépendamment H, un halogène ou un alkyle en $C_1$ à $C_6$ (de préférence méthyle ou éthyle), et

chaque occurrence de $R^{L3}$ représente indépendamment H, un halogène (de préférence F, Cl ou Br), OH, $NH_2$, $NO_2$ ou un alkyle en $C_1$ à $C_6$ (de préférence méthyle ou éthyle).

Dans un mode de réalisation, chaque occurrence de $R^3$ représente un atome d'hydrogène.

Dans un mode de réalisation, chaque occurrence des radicaux $R^{L1}$, $R^{L2}$ et $R^{L3}$ représente un atome d'hydrogène.

De préférence, dans l'étape réactionnelle (i), le ligand L' utilisé peut être un acide di-, tri- ou tétra- carboxylique choisi dans le groupe comprenant : $C_2H_2(CO_2H)_2$ (acide fumarique), $C_2H_4(CO_2H)_2$ (acide succinique), $C_3H_6(CO_2H)_2$ (acide glutarique), $C_4H_4(CO_2H)_2$ (acide muconique), $C_4H_8(CO_2H)_2$ (acide adipique), $C_7H_{14}(CO_2H)_2$ (acide azelaique), $C_5H_3S(CO_2H)_2$ (acide 2,5-thiophènedicarboxylique), $C_6H_4(CO_2H)_2$ (acide téréphtalique), $C_6H_2N_2(CO_2H)_2$ (acide 2,5-pyrazine dicarboxylique), $C_{10}H_6(CO_2H)_2$ (acide naphtalène-2,6-dicarboxylique), $C_{12}H_8(CO_2H)_2$ (acide biphényle-4,4'-dicarboxylique), $C_{12}H_8N_2(CO_2H)_2$ (acide azobenzènedicarboxylique), $C_6H_3(CO_2H)_3$ (acide benzène-1,2,4-tricarboxylique), $C_6H_3(CO_2H)_3$ (acide benzène-1,3,5-tricarboxylate), $C_{24}H_{15}(CO_2H)_3$ (acide benzène-1,3,5-tribenzoïque), $C_6H_2(CO_2H)_4$ (acide benzène-1,2,4,5-tétracarboxylique, $C_{10}H_4(CO_2H)_4$ (acide naphtalène-2,3,6,7-tétracarboxylique), $C_{10}H_4(CO_2H)_4$ (acide naphtalène-1,4,5,8-tétracarboxylique), $C_{12}H_6(CO_2H)_4$ (acide biphényl-3,5,3',5'-tétracarboxylique), et les analogues modifiés choisis dans le groupe comprenant l'acide 2-aminotéréphtalique, l'acide 2-nitrotéréphtalique, l'acide 2-méthyltéréphtalique, l'acide 2-chlorotéréphtalique, l'acide 2-bromotéréphtalique, l'acide 2,5-dihydroxotéréphtalique, l'acide tétrafluorotéréphtalique, l'acide tétraméthyltéréphtalique, l'acide diméthyl-4,4'-biphénydicarboxylique, l'acide tétraméthyl-4,4'-biphénydicarboxylique, l'acide dicarboxy-4,4'-biphénydicarboxylique, l'acide 2,5-pyrazyne dicarboxylique. Le ligand L' utilisé peut également être choisi dans le groupe comprenant : l'acide 2,5 diperfluorotéréephthalique, l'acide azobenzène 4,4'-dicarboxylique, l'acide 3,3'-dichloro azobenzène 4,4'-dicarboxylique, l'acide 3,3'-dihydroxo azobenzène 4,4'-dicarboxylique, l'acide 3,3'-diperfluoro azobenzène 4,4'-dicarboxylique, l'acide 3,5,3',5'-azobenzène tétracarboxylique, l'acide 2,5-dimethyl téréphthalique, l'acide perfluoro glutarique.

Avantageusement, le ligand L' peut être aussi un précurseur du ligand imidazolate, tétrazolate, phosphate ou phosphonate tel que l'imidazole, 2-méthylimidazolate, 2-éthylimidazole, 4,(-imidazol-dicarboxyic acid, 1,4-(butanediyl)bis(imidazole), purine, pyrimidine, benzimidazolate, pipérazinediphosphonate, tétrazolylbenzoate.

Il est bien entendu que, dans la mise en oeuvre du procédé, lorsque le ligand L' est de type carboxylate, celui-ci n'est pas nécessairement sous la forme d'un acide carboxylique. Comme indiqué précédemment, celui-ci peut se présenter sous une forme dérivée où une ou plusieurs fonctions carboxyliques est/sont sous la forme $-C(=O)-R^3$ où $R^3$ peut représenter un radical -OY où Y représente un cation alcalin, un halogène, ou un radical $-OR^4$, $-O-C(=O)R^4$ ou $-NR^4R^{4'}$, où $R^4$ et $R^{4'}$ sont indépendamment des radicaux alkyles en $C_{1-12}$.

La synthèse de matériaux MOFs peut être de préférence réalisée en présence d'énergie qui peut être apportée par exemple par le chauffage, comme par exemple des conditions hydrothermales ou solvothermales, mais également par micro-ondes, par ultrasons, par broyage, par un procédé faisant intervenir un fluide supercritique, etc. Les protocoles correspondants sont ceux connus de l'homme du métier. Des exemples non limitatifs de protocoles utilisables pour les conditions hydrothermales ou solvothermales sont décrits par exemple dans les demandes internationales WO 2009/077670 et WO 2009/077670, et dans les références qui y sont citées à cet effet.

Les conditions hydrothermales ou solvothermales, dont les températures de réactions peuvent varier entre 0 et 220°C, sont généralement effectuées dans des récipients en verre (ou en plastique) lorsque la température est inférieure à la température d'ébullition du solvant. Lorsque la température est supérieure ou lorsque la réaction s'effectue en présence de fluor, des corps en téflon insérés dans des bombes métalliques sont employés.

Les solvants utilisés sont généralement polaires. Notamment les solvants suivants peuvent être utilisés : l'eau, les alcools, le diméthylformamide, le diméthylsulfoxide, l'acétonitrile, le tétrahydrofurane, le diéthylformamide, le chloroforme, le cyclohexane, l'acétone, le cyanobenzène, le dichlorométhane, le nitrobenzène, l'éthylèneglycol, le diméthylacétamide ou des mélanges de ces solvants.

Un ou plusieurs co-solvants peuvent également être ajoutés à n'importe quelle étape de la synthèse pour une

meilleure solubilisation des composés du mélange. Il peut s'agir notamment d'acides monocarboxyliques, tels que l'acide acétique, l'acide formique, l'acide benzoïque, etc.

Lorsque le co-solvant est un acide monocarboxylique, celui-ci, outre un effet solubilisateur, permet également d'arrêter la croissance cristalline du solide MOF. En effet, la fonction carboxylique se coordonne avec le fer, lequel ne pourra pas se lier à un autre atome de fer faute de la présence d'une seconde fonction -COOH sur la molécule de co-solvant. Ainsi, la croissance du réseau cristallin s'en trouve ralentie, puis arrêtée. L'ajout d'un co-solvant monocarboxylique, tel que l'acide acétique, l'acide formique, l'acide benzoïque, etc., permet ainsi de réduire la taille des particules de solide MOF obtenues. L'utilisation d'un co-solvant monocarboxylique peut donc favoriser l'obtention de nanoparticules (particules de taille < 1 $\mu$m).

De préférence, l'étape réactionnelle (i) peut être réalisée suivant au moins une des conditions réactionnelles suivantes :

avec une température de réaction de 0°C à 220°C, de préférence de 50 à 150°C ;

avec une vitesse d'agitation de 0 à 1000 rpm (ou rotation par minute), de préférence de 0 à 500 ;

avec un temps de réaction de 1 minute à 96 heures, de préférence de 1 minute à 15 heures ;

avec un pH de 0 à 7, de préférence de 1 à 5 ;

avec l'addition d'au moins un co-solvant au solvant, au précurseur, au ligand ou au mélange de ceux-ci, ledit co-solvant étant choisi dans le groupe comprenant l'acide acétique, l'acide formique, l'acide benzoïque ;

en présence d'un solvant est choisi dans le groupe comprenant l'eau, les alcools $R^S$-OH où $R^S$ est un radical alkyle en $C_1$ à $C_6$ linéaire ou ramifié, le diméthylformamide, diméthylsulfoxide, l'acétonitrile, le tétrahydrofurane, le diéthylformamide, le chloroforme, le cyclohexane, l'acétone, le cyanobenzène, le dichlorométhane, le nitrobenzène, l'éthylèneglycol, le diméthylacétamide ou des mélanges de ces solvants, miscibles ou non ;

dans un milieu supercritique, par exemple dans le $CO_2$ supercritique ;

sous micro-ondes et/ou sous ultra-sons ;

dans des conditions d'électrolyse électrochimique ;

dans des conditions utilisant un broyeur à cylindres ;

dans un flux gazeux.

La synthèse de matériaux MOF peut être de préférence réalisée dans des conditions expérimentales propices à la formation de nanoparticules. Par exemple, un contrôle des paramètres suivants peut être important pour la réalisation de nanoparticules de solides MOF selon l'invention :

- température de réaction,
- temps de réaction,
- concentrations en ligand L' et en précurseur inorganique métallique et/ou
- ajout d'un ou plusieurs additifs tel que des modificateurs de pH (acides, bases), minéralisateurs, ou agents favorisant l'arrêt de la croissante crystalline (monoacide carboxylique).

Les fourchettes de valeurs préférées de chacun de ces paramètres peuvent varier selon que la synthèse des nanoparticules est réalisée par la voie hydro/solvothermale, par ultrasons ou par micro-ondes. Par exemple, une température de réaction plus élevée sera généralement utilisée pour la voie hydro/solvothermale (environ 20-150°C) que pour la voie par ultra-sons (environ 0°C).

Par ailleurs, les inventeurs ont également mis en évidence que les caractéristiques structurales particulières du solide de la présente invention, notamment en terme de flexibilité ou de taille des pores, qui lui confères des propriétés particulièrement intéressantes, notamment en terme de capacité d'adsorption, d'adsorption sélective et de pureté. Ces

matériaux rendent donc possible l'adsorption sélective de molécules, comme par exemple des molécules pharmaceutiques, avec un coût énergétique favorable et un temps de relarguage plus élevé. Ainsi, les travaux de recherche menés par les inventeurs leur ont permis de mettre en évidence l'intérêt des matériaux MOF pour l'adsorption et le transport de principe actifs.

Ainsi, l'invention concerne également l'utilisation du solide MOF selon l'invention, lequel comprenant dans ses pores ou à sa surface au moins un principe pharmaceutiquement actif.

En particulier, l'invention concerne également l'utilisation du solide MOF selon l'invention chargé en principe pharmaceutiquement actif comme médicament. Le principe pharmaceutiquement actif peut être contenu soit dans les pores, soit à la surface du solide selon l'invention. C'est ce que l'on entend dans la suite de ce document par « solide MOF chargé en principe pharmaceutiquement actif ».

Plus généralement, on entend par « solide MOF chargé en composant X » un solide MOF selon l'invention contenant dans ses pores ou à sa surface le composant X. Le composant X peut être adsorbé ou lié par liaison covalente, par liaison hydrogène, par liaison de Van der Waals, par interaction électrostatique à la surface ou dans les pores du solide MOF. Ce composant X peut être, comme indiqué ci-dessus, un principe pharmaceutiquement actif.

Dans la présente, on entend par « principe actif » une molécule qui possède un effet thérapeutique. Par exemple, il peut s'agir de toute molécule ayant des propriétés thérapeutiques entrant dans la composition d'un médicament. On pourra citer par exemple les anti-inflammatoires non stéroïdiens (AINS), les Abortifs, les Alpha-bloquants, les Alpha2-agonistes, les Aminosides, les Analgésiques, les Anesthésiques, les Anesthésiques locaux, les Anorexigènes, les Antagonistes 5HT3, les Antagonistes du calcium, les Antiangoreux, les Antiarythmiques, les Antibiotiques, les Anticholinergiques, les Anticholinestérasiques, les Antidiabétiques, les Antidiarrhéiques, les Antidépresseurs, les Antihistaminiques, les Antihypertenseurs, les Antimycosiques, les Antipaludéens, les Antiparasitaires, les Antipsychotiques, les Antipyrétiques, les Antirétroviraux, les Antiseptiques, les Antispasmodiques, les Antiviraux, les Antiémétiques, les Antiépileptiques, les Anxiolytiques, les Barbituriques, les Benzodiazépines, les Bronchodilatateurs, les Beta-bloquants, les Agents chimiothérapeutiques, les Corticostéroïdes, les Diurétiques, les Diurétiques de l'anse, les Diurétiques osmotique, les Dépresseurs, les Glucocorticoïdes, les Hallucinogènes, les Hypnotiques, les Immunosuppresseurs, les Inhibiteurs de l'anhydrase carbonique, les Inhibiteurs de la neuraminidase, les Inhibiteurs de la pompe à protons, les Inhibiteurs du TNF, les Inhibiteurs sélectifs de la recapture de la sérotonine, les inhibiteurs de la HMG-CoA réductase (ou statines), les Kératolytiques, les Laxatifs, les minéralocorticoïdes, les Myorelaxants, les Neuroleptiques, les Psychotropes, les Spasmolytiques, les Stimulants, les Sédatifs, les Tocolytiques ou les Vasodilatateurs. Cette liste n'est pas exhaustive et s'étend à tout principe actif thérapeutique connu de l'homme du métier.

En effet, le solide MOF selon l'invention a l'avantage de présenter de grandes capacités d'adsorption ou de charge. En effet, le solide de l'invention présente un micro-environnement interne hydrophobe/hydrophile favorable, notamment pour l'incorporation de molécules amphiphiles comme le busulfan. En outre, il permet d'adsorber efficacement des molécules pharmaceutiques qui présentent des difficultés particulières d'encapsulation, par exemple en raison de leur instabilité, leur grande réactivité, leur faible solubilité, leur forte tendance à cristalliser, leur caractère hydrophile, amphiphile, etc...

Par exemple, le solide selon l'invention peut être chargé avec au moins un principe pharmaceutiquement actif qui présente l'une ou plusieurs des caractéristiques suivantes : hydrophile, amphiphile, lipophile, instable, toxique, à forte tendance à cristalliser ou sensiblement insoluble.

On entend par « toxique » un principe pharmaceutiquement actif ayant des effets toxiques susceptibles d'entraver son utilisation dans des applications médicales ou vétérinaires. Il peut s'agir par exemple d'agents alkylants tels que le busulfan, le cisplatine, les nitroso-urées comme la lomustine. Les agents alkylants forment après métabolisation, *des* liaisons covalentes avec les acides nucléiques. La formation de ces liaisons peut entraîner par exemple:

- Des troubles de la transcription et de la réplication de l'ADN
- Des substitutions de bases dans l'ADN
- Des excisions de bases et des ruptures caténaires de l'ADN.

Leur activité pharmacologique principale se manifeste durant la phase de synthèse de l'ADN. Leurs effets toxiques peuvent inclure : la myelosuppression, la stérilité et la leucémie non-lymphocytaire.

Le Cisplatine provoque des ponts ADN intra-caténaire, possède une faible myelotoxicité, mais il est sévèrement émétisant et peut être néphrotoxique.

On entend par «à forte tendance à cristalliser» un principe pharmaceutiquement actif qui a tendance à s'associer à lui-même dans un réseau cristallin au lieu de s'inclure dans d'autres structures. Ainsi, un tel composé tend à former des cristaux lors du procédé d'encapsulation utilisé, au lieu de s'inclure dans les particules. Il résulte donc en fin de procédé un mélange de particules peu chargées en principe pharmaceutiquement actif et des cristaux de celui-ci. Il peut

s'agir par exemple du Busulfan. A forte dose, il présente un effet secondaire grave qui est la maladie veino-occlusive du foie. Celle-ci résulte probablement de la très forte tendance à cristalliser de cette molécule. L'empilement cristallin est régi par de fortes interactions dipôle-dipôle entre les groupements méthylsulfonate de ce principe actif.

On entend par « sensiblement insoluble» un principe pharmaceutiquement actif dont la solubilité est inférieure à 0.1 mg/mL dans l'eau. Il peut s'agir par exemple du Busulfan.

On entend par « instable» un principe pharmaceutiquement actif qui peut se décomposer, cristalliser et/ou reagir en perdant sa structure et son activité. Il peut s'agir par exemple du Busulfan.

En outre, le principe pharmaceutiquement actif peut être toute molécule ayant une activité biologique, comme par exemple, un médicament, notamment un anticancéreux, un agent antiviral, un analogue nucléosidique, modifié ou non, un acide nucléique, un anticorps, une protéine, une vitamine, etc...

Parmi les principes actifs hydrophiles, on peut citer par exemple, l'azidothymidine phosphatée ou non, CDV (cidofovir), le 5-fluoroacil , la citarabine.

Parmi les principes actifs amphiphiles, on peut citer par exemple, le busulfan, le chlorure de doxorubicine, le chlorure d'imipramine.

Parmi les principes actifs lipophiles, on peut citer par exemple, le tamoxifene, le docétaxel, le paclitaxel, l'ibuprofene, la lidocaine, les vitamines liposolubles telles que les vitamines A (rétinol), D (calciférol), E (tocophérol), K1 (phylloquinone), K2 (ménaquinone).

En particulier, le solide selon l'invention peut être chargé avec au moins un principe pharmaceutiquement actif choisi par exemple dans le groupe comprenant le taxotère, le busulfan, l'azidothymidine (AZT), l'azidothymidine phosphatée (AZTP), l'ibuprofène, le cidofovir, les antibiotiques, la gemcitabine, le tamoxifène, la zalcitabine (ddC), la didanosine (ddI).

Avantageusement, le solide selon l'invention peut être chargé avec au moins un principe pharmaceutiquement actif choisi par exemple dans le groupe comprenant le busulfan, l'azidothymidine (AZT), l'azidothymidine phosphatée (AZTP), le cidofovir, la gemcitabine, l'ibuprofène.

En outre, le solide selon l'invention peut être chargé avec au moins un composé d'intérêt en cosmétique.

On entend par « composé d'intérêt cosmétique » toute substance active entrant dans la formulation d'une préparation cosmétique, c'est-à-dire une préparation destinée à être mise en contact avec diverses parties superficielles du corps humain, notamment l'épiderme, les systèmes pileux et capillaires, les organes externes, les dents et les muqueuses, en vue, exclusivement ou principalement, de les nettoyer, protéger, parfumer, maintenir en bon état le corps humain, de modifier son aspect ou d'en corriger l'odeur. Par « substance active », on entend une substance qui assure l'efficacité de la préparation cosmétique.

Le composé d'intérêt cosmétique peut être une substance active entrant dans la préparation de toute préparation cosmétique connue de l'homme du métier, par exemple, les produits d'hygiène (e.g., démaquillant, dentifrice, déodorant, gel douche, savon, shampoing), les produits de soin (e.g., crème antirides, crème de jour, crème de nuit, crème hydratante, eau florale, gommage, lait, masque de beauté, baume pour les lèvres, tonique), les produits capillaires (e.g., après-shampoing, défrisant, gel, huile, laque, masque, teinture), les produits de maquillage (e.g., anti-cerne, autobronzant, eyeliner, fard, fond de teint, khôl, mascara, poudre, produit pour blanchir la peau, rouge à lèvres, vernis à ongles), les parfums (e.g., eau de Cologne, eau de toilette, parfum), les produits solaires (e.g., crèmes, huiles ou lotions après-soleil et solaires), les produits pour le rasage et les produits dépilatoires (e.g., après-rasage, crème dépilatoire, mousse à raser), ou les préparations pour bains et douches (e.g., bain moussant, huile de bain, sels de bain).

Selon l'invention, le composé d'intérêt en cosmétique peut être choisi par exemple dans le groupe comprenant :

- un antioxidant (par exemple, l'acide citrique, le beta-carotene, la vitamine E, l'acide glycolique, le glutathion, la vitamine C, les polyphenols, le lycopene, les flavonoides, les tanins, les anthocyanes, la N-acetylcysteine (antiradicaux libre))
- une vitamine (par exemple, la vitamine A, B3, B5, B6, B2, B1, B9, B8, B12, C, E, D, K, K1, K2)
- un liporégulateur (par exemple, la caféine, la theophylline)
- un agent photoprotecteur (par exemple, la benzophénone 3 (2-Hydroxy-4-Methoxy Benzophenone), la benzophénone 4 (acide 2-Hydroxy-4-methoxybenzophenone-5-sulphonique), le 2-phenylbenzimidazole-5-sulfonique))
- un agent hydratant (par exemple, l'urée, l'acide hyaluronique, le sorbitol).

Par exemple, le solide selon l'invention peut être chargé avec au moins un composé d'intérêt en cosmétique choisi dans le groupe comprenant la benzophénone, la visnadine, l'acide salicylique, l'acide ascorbique, la benzophénone et ses dérivés, la caféine, l'urée, l'acide hyaluronique, etc.

Selon l'invention, l'agent de surface organique peut en outre être fonctionnalisé avec une molécule fluorescente. Par exemple, il peut s'agir des rhodamines (par exemple la rhodamine B), de la fluoresceine, la luciférase, du pyrène et ses dérivés, de l'aminopyrrolidino-7-nitrobenzofurazan ou des quantum dots.

Par exemple, les quantum dots peuvent être choisis parmi le sélénure de cadmium, le sulfure de cadmium, l'arsénure d'indium, le phosphure d'indium ou le sélénure sulfure de cadmium.

Avantageusement, le principe actif peut être une molécule fluorée; c'est-à-dire comprenant au moins un substituant F. Il peut s'agir par exemple de l'une des molécules fluorées citées précédemment. Ces molécules fluorées sont adaptées aux utilisations en imagerie, particulièrement l'imagerie fluorescence telle que la technique TEP précitée.

Ainsi, l'invention concerne également l'utilisation de nanoparticules de MOF encapsulant une ou plusieurs molécules fluorées selon l'invention comme marqueur utilisable en imagerie médicale, telle que l'imagerie TEP.

Par exemple, le solide selon l'invention peut être chargé en principe pharmaceutiquement actif avec une capacité de charge de 1 à 200% en poids de solide sec, par exemple de 1 à 70% en poids de solide sec, soit près de 10 à 700 mg par gramme de solide sec.

Dans le cadre de la présente invention, la capacité de charge signifie la capacité de stockage de molécules ou la quantité de molécules adsorbées dans le matériau. La capacité de charge peut être exprimée en capacité massique (gramme/gramme) ou en capacité molaire (mol/mol) ou en d'autres (mol/gramme, gramme/mol, etc.)

En outre, une autre problématique de l'art antérieur concerne la libération rapide et non contrôlée des molécules transportées en l'absence d'affinité. Le solide MOF à surface externe modifiée selon l'invention présente l'avantage de permettre des temps de relargage plus longs, notamment grâce au micro-environnement interne mais également grâce à la structure des composés. En effet, l'utilisation d'agents de surface ayant une taille supérieure à celle des fenêtres d'accès aux pores des matériaux MOF, fonctionnalisés avec des ligands très complexants des métaux, comme le fer ($Fe^{2+}$, $Fe^{3+}$), le zinc ($Zn^{2+}$), le zirconium ($Zr^{4+}$), le titane ($Ti^{4+}$), le calcium ($Ca^{2+}$), le magnésium ($Mg^{2+}$) et l'aluminium ($Al^{3+}$), permet d'obtenir à la fois des recouvrements stables, sans perturber les molécules encapsulées.

Le solide selon l'invention peut également comporter, par exemple sur les ligands espaceurs, des groupements fonctionnels qui peuvent modifier les interactions entre le solide MOF selon l'invention et la molécule d'intérêt. Ceci peut permettre de contrôler l'encapsulation et la libération des molécules d'intérêt. Les matériaux MOF de l'invention peuvent ainsi être adaptés, formulés et conçus (« designed ») en fonction des molécules d'intérêt à transporter afin de moduler le taux d'encapsulation, la libération des molécules et/ou la dégradabilité du solide.

De plus, le solide MOF selon l'invention a fait l'objet d'études de toxicité très positives, décrites dans la partie « Exemples » ci-dessous.

Ainsi, le solide MOF de la présente invention utilisé pour le transport des principes actifs permet de pallier les problèmes de l'art antérieur cités précédemment, notamment les problèmes liés à l'instabilité des recouvrements du véhicule MOF, notamment en milieu biologique, et à leur interférence avec la libération des molécules encapsulées dans les pores des MOFs à surface externe modifiée.

En outre, le solide MOF selon l'invention rend possible l'incorporation de marqueurs dans ce matériau ce qui présente également un intérêt.

Ainsi, selon un mode particulier de réalisation, le solide selon l'invention, peut être chargé avec au moins une molécule d'intérêt qui peut être un principe pharmaceutiquement actif et/ou un composé d'intérêt en cosmétique et/ou un marqueur. La molécule d'intérêt peut être contenue soit dans les pores, soit à la surface du solide selon l'invention.

Ainsi, les solides MOF selon l'invention peuvent être utilisés pour la fabrication de médicaments, de compositions cosmétiques et/ou de marqueurs utilisables en imagerie médicale.

Ainsi, il est fourni un procédé de traitement d'un sujet affecté par une maladie, ledit procédé comprenant l'administration audit sujet d'un solide MOF selon l'invention comprenant dans ses pores ou à sa surface au moins un principe actif connu pour traiter ladite maladie.

En particulier, le solide MOF selon l'invention peut être chargé avec au moins un marqueur choisi dans le groupe comprenant un marqueur d'imagerie médicale, un agent de contraste, un traceur, un marqueur radioactif, un marqueur fluorescent, un marqueur phosphorescent.

Par exemple, les inventeurs décrivent dans la partie « Exemples » ci-dessous une modification de surface avec un composé fluorescent, en particulier une cyclodextrine ou une polycyclodextrine marqué à la rhodamine. Cette modification permet la détection des particules à l'aide d'un microscope confocal. Le microscope confocal à balayage laser - MCBL (en anglais CLSM pour « *confocal laser scanning microscope* ») est un microscope optique qui a la propriété de réaliser des images de très faible profondeur de champ (environ 600 nm) appelées « sections optiques ». En positionnant le plan focal l'objectif à différents niveaux de profondeur dans l'échantillon, il est possible de réaliser des séries d'images à partir desquelles on peut obtenir une représentation tridimensionnelle de l'objet. Une application possible est l'étude des interactions avec des lignées cellulaires.

Par ailleurs les nanoparticules marquées avec des composés fluorescents peuvent être utilisées en imagerie par fluorescence.

En particulier, le solide selon l'invention peut être chargé avec au moins un marqueur choisi dans le groupe

comprenant : un composé fluorescent, un oxyde de fer, un complexe de gadolinium, des ions gadolinium directement présents dans la structure, par exemple sous forme de complexe avec le ligand organique, etc. Les protocoles de charge en marqueur sont ceux connus de l'homme du métier. Des exemples non limitatifs utilisables sont décrits par exemple dans A.K. Gupta, et al., Nanomed. 2007 2(1), 23-39 **[28] ;** dans P Caravan, Chem. Soc. Rev., 2006, 35, 512-523 **[29] ;** ou dans Yan-Ping Ren, et al., Angew. Chem. Int. Ed. 2003, 42, No. 5, 532 **[30].**

Ainsi, le solide MOF selon l'invention peut être utilisé pour la fabrication, le transport et/ou la vectorisation de marqueurs.

En outre, le solide de l'invention peut être utilisé pour la vectorisation de médicaments lorsqu'il est chargé en principe pharmaceutiquement actif et/ou pour la détection et le suivi de maladies faisant intervenir des cibles biologiques (telles que le cancer) lorsqu'il est utilisé comme marqueur.

En outre, en cumulant ces deux utilisations, le solide de la présente invention permet avantageusement de visualiser la biodistribution d'un médicament. Ceci présente un grand intérêt, notamment pour le suivi d'un traitement thérapeutique et l'étude de la biodistribution d'un médicament.

Selon un mode particulier de réalisation de l'invention, le procédé de préparation du solide selon l'invention, peut comprendre en outre une étape (ii) d'introduction dans les pores ou à la surface du solide MOF, d'au moins une molécule d'intérêt, qui peut être un principe pharmaceutiquement actif et/ou un composé d'intérêt en cosmétique et/ou marqueur.

Ladite étape d'introduction peut être réalisée au cours de l'étape réactionnelle (i) ou après celle-ci de façon à obtenir un solide chargé en molécule d'intérêt.

Toute méthode connue de l'homme du métier peut être utilisée au cours de l'étape d'introduction (ii). La molécule d'intérêt peut être par exemple introduite dans le matériau MOF de la présente invention :

par imprégnation, en immergeant le matériau dans une solution de la molécule d'intérêt ;

par sublimation de la molécule d'intérêt, puis le gaz est adsorbé par le matériau ; ou

par broyage par cylindre rotatif consistant à mélanger mécaniquement le matériau et la molécule d'intérêt.

Les matériaux MOFs de la présente invention constituent donc des composés améliorés capables d'échapper au système immunitaire et/ou à leur capture par certains organes, par exemple le foie, évitant ainsi leur accumulation dans ces organes, et capables de vectoriser des principes actifs vers des cibles spécifiques.

La méthode de modification de surface de MOF améliorée présentée dans la présente demande de brevet permet :

- d'assurer une meilleure stabilité du recouvrement en milieu biologique
- de ne pas interférer avec l'encapsulation et la libération des molécules actives destinées à être vectorisées par les matériaux MOF en question.

Ces composés sont capables de transporter et des principes actifs, par exemple des principes actifs présentant des difficultés particulières d'encapsulation liées à leur instabilité, leur forte tendance à cristalliser, leur faible solubilité, leur caractère amphiphile, hydrophile, etc.

Ils permettent en outre de libérer de façon contrôlée des principes actifs.

Comme décrit dans la partie « Exemples », les cyclodextrines phosphatées ont été utilisées comme agent de surface selon l'invention. Les cyclodextrines (CD) sont de très bons candidats car leurs dimensions sont supérieures à celles des fenêtres d'accès aux pores du trimésate de fer MIL-100(Fe), un des nanoMOFs ayant les plus larges pores (cf. Figures 1 et 2). Ces cyclodextrines ont été fonctionnalisées avec des groupements phosphates capables de se lier de manière stable par coordination avec les sites insaturés de fer, zinc, zirconium, titane, calcium, magnésium et/ou aluminium en surface des MOFs. Afin d'éviter un décrochage rapide des agents de surface des MOFs en milieu physiologique, dû à la fois au pH élevé (7,4) et à la présence d'ions phosphates libres conduisant le plus souvent à la dissolution de la matrice carboxylate de métal pour former des oxydes et/ou des phosphates métalliques, la stratégie proposée dans la présente demande utilise le greffage de groupements, soit de force complexante équivalente ou supérieure à celle des phosphates, soit en nombre suffisant afin d'augmenter l'accrochage en surface du MOF de l'agent de surface organique (effet coopératif).

La modification de surface selon l'invention peut être obtenue par simple incubation dans les solutions de cyclodextrine phosphate (CD-P). Il s'agit d'une méthode facile et rapide (moins de 15 minutes) qui présente par ailleurs l'avantage d'une « galénique verte » (bien que pour l'encapsulation de molécules hydrophobes, un solvant organique puisse être utilisé). En effet, la modification de surface ne nécessite l'addition d'aucun solvant organique, tensioactif ou

autre produit chimique à éliminer en fin de réaction. Cette méthode pourrait donc être extrapolée à l'échelle industrielle.

Comme décrit dans la partie « Exemples », la cristallinité et le volume poreux des MOFs modifiés avec les cyclodextrines phosphatées ne sont pas affectés, contrairement aux agents de surface PEG décrits dans la demande WO 2009/077671. En effet, le volume poreux des échantillons greffés avec la cyclodextrine-phosphate est proche de celui du solide initial (cf. Figure 7A).

La modification de surface a été confirmée par RMN, IR, ITC, mesure de potentiel Zeta, microscopie. Au bout de 15 minutes seulement, les nanoMOFs sont recouvertes de CD-P représentant jusqu'à 20% de leur poids (plus de 32% après 24 h d'incubation), ce qui est très significatif et montre la grande affinité des CD-P pour la surface des nanoMOFs (Figure 5). Ce recouvrement ne disparaît pas après lavage dans l'eau et reste stable dans du PBS même après 24 h d'incubation (Figure 9), contrairement aux recouvrements PEG linéaires et/ou dextrane décrits dans la demande WO 2009/0077671.

Par ailleurs, les recouvrements de CD-P ont un effet bénéfique sur la stabilité des nanoMOFs, en ce que ces derniers ne s'agrègent plus même au bout de plusieurs jours d'incubation dans l'eau (Figure 10). Cet aspect est particulièrement important pour les applications (biologiques, cosmétiques) et pour le stockage des particules de MOF.

Les recouvrements de CD-P ne modifient par ailleurs pas la cinétique de libération des molécules encapsulées. Ceci a été clairement démontré avec l'azidothymidine triphosphate (AZT-TP), forme active de l'antirétroviral AZT (Figure 11). Dans le cas des PEG linéaires, une libération très rapide (« burst effect » en anglais) a lieu dès les premières minutes d'incubation.

Les avantages et propriétés précitées pour les MOFs à surface externe modifiée avec des cyclodextrines-phosphate peuvent également être obtenus avec les autres agents de surface selon la présente invention, à savoir les monomères, oligomères ou polymères de cyclodextrine; les groupements polyéthylèneglycol ramifiés ; les protéines ; les polysaccharides portant une pluralité de chaînes latérales polyéthylèneglycol; ou les polysaccharides insolubles dans l'eau à $6<pH<8$ et solubles dans l'eau à $pH<5$, lesquels sont complexés à un centre métallique M ou à un ligand L situé en surface du solide MOF cristallin via un ou plusieurs groupe(s) phosphate, phosphonate, bisphosphonate, sulfate, carboxylate, azolate (par exemple, imidazolate), amido et/ou amino.

D'autres avantages pourront encore apparaître à l'homme du métier à la lecture des exemples ci-dessous, en référence aux figures annexées, donnés à titre illustratif, et non limitatif.

## Brève description des figures

- Figure 1. Représentation schématique d'un MOF poreux modifié en surface par des agents de surface possédant plusieurs groupes permettant d'interagir avec les centres métalliques ou les ligands en surface, et ayant :

  (A) Une section rigide supérieure à la taille des plus grandes fenêtres du matériau (par exemple, des cyclodextrines);
  (B) Une section rigide inférieure à la taille des plus grandes fenêtres du matériau, mais des groupes repartis sur toute la longueur de la chaine principale de l'agent de surface (par exemple, du dextrane greffé avec des alendronates).

- Figure 2 : Représentation schématique de l'interaction βCDP-MIL-100 (βCDP = β-Cyclodextrine phosphatée).
- Figure 3 : Diffractogrammes rayons X poudre (« X-Ray Powder Diffraction » ou « XRPD » en anglais) d'un MOF MIL-100 avant et après fonctionnalisation par βCDP.
- Figure 4 : Spectre FT-IR de βCDP, de MOF MIL-100 et de MOF MIL-100 à surface modifié par βCDP.
- Figure 5 : Cinétique de modification des MIL-100 par βCDP avec deux ratios poids de nanoparticules/ βCDP différents : ratio massique de 1:0,5 ou 1:2 (trait plein et trait en pointillés respectivement)
- Figure 6 : Représentation du Potentiel-ζ des nanoparticules de MIL-100 modifiées et non modifiées en fonction de leur temps d'incubation.
- Figure 7 A) et B) : Isothermes d'adsorption et désorption d'azote à 77K du MIL-100 avant et après la modification de surface avec la βCDP (A) ou le Meo-PEG-$NH_2$ (B) .
- Figure 8 : Interaction entre les nanoparticules de MIL-100 et la βCD MIL-100 (A) ou la βCDPMIL-100 (B) obtenu par caractérisation ITC.
- Figure 9 : Cinétique de décrochage ourbe de la libération de la βCDP-rhodamine dans du PBS ou dans du RPMI supplémenté avec 10% de sérum bovin.
- Figure 10 : Stabilité aqueuse du MIL-100 non modifié et modifié avec la β-CDP.
- Figure 11 : Cinétique de libération d'AZT-TP depuis le MIL-100 à surface non modifiée, modifiée avec la βCDP et modifiée avec le MeO-PEG-$NH_2$.

- Figure 12: Evaluation de la cytotoxicité de MIL-100 non modifiées et modifiées au βCDP sur la lignée cellulaire J774.A1.
- Figure 13 : Clichés permettant d'observer la cinétique de la pénétration des MIL-100 non modifiées et modifiées par βCDP dans les cellules 774.Al incubées 24H avec un équivalent de solution aqueuse ATP-BODIPY.
- Figure 14 : Illustration des étapes utilisées pour la synthèse des dérivés fluorescents du monomère de β-cyclodextrine phosphate de l'Exemple 4.
- Figure 15 : illustration des étapes de synthèse des dérivés fluorescents du poly-βCD phosphate de l'Exemple 5.
- Figure 16 : illustration des étapes de phosphorylation de poly-βCDs de l'Exemple 5.
- Figure 17 : cinétique de libération de poly-βCDP-rhodamine en milieu PBS à 37°C.
- Figure 18 : Représentation schématique de la modification de surface des nanoparticules MIL-100 par le chitosane *via* des interactions électrostatiques.
- Figure 19 : Evolution du diamètre moyen des nanoparticules MIL-100 et MIL-100/chitosane après dilution des suspensions initiales dans l'eau milliQ. Facteur de dilution : 100.
- Figure 20 : Diffractogrammes RX des MIL-100 et des MIL-100 modifiées par le chitosane.
- Figure 21 : Cinétique de libération de l'AZT-TP à partir des MIL-100 et des MIL-100 modifiées par le chitosane (modification post- encapsulation de l'AZT-TP).
- Figure 22 : Représentation schématique de l'association du chitosane aux nanoparticules MIL-100 par interactions électrostatiques et addition de $Na_2SO_4$.
- Figure 23 : Distribution granulométrique des microparticules MIL-100/Chitosane. Rapport massique MIL-100/chitosane : 20/1.
- Figure 24 : spectres IR du chitosane (trait plein noir), des nanoparticules MIL-100 (pointillés noirs) et des microparticules MIL-100/chitosane rapport massique 10/1 (trait plein gris) et 5/1 (pointillés gris).
- Figure 25 : Diffractogrammes RX des MIL-100 et des MIL-100 modifiées par le chitosane.
- Figure 26 : cinétique de libération de l'AZT-TP à partir des MIL-100 et des microparticules MIL-100 / chitosane (modification post-encapsulation).
- Figure 27 : Bioconjugué PEG-alendronate-dextrane.
- Figure 28 : décrochage de βCDP-rhodamine à partir de nanoparticules de MIL-100(Fe) dans du PBS à 37°C.
- Figure 29 : décrochage de βCDP-rhodamine à partir de nanoparticules de MIL-100(Al) dans du PBS à 37°C.

## EXEMPLES

Dans la partie « Exemples » qui suit, l'abréviation « MIL-100 » désigne le MOF carboxylate de fer de formule $Fe_3O[C_6H_3(CO_2)_3]_2.X.nH_2O$ (X=F, Cl, OH) Dans les exemples suivants, l'unité xg correspond à « fois g » où g = (1.118 x $10^{-5}$).R.$S^2$ où g exprime la force relative centrifuge, R le radius en cm et S la vitesse de centrifugation en rpm et 1 rpm = 1/60 Hz.

### Exemple 1: Synthèses de nanoparticules de divers MOFs MIL-89nano $Fe_3OX[C_4H_4(CO_2)_2]_3$ $nH_2O$ (X=F, Cl, OH)

La synthèse du MIL-89nano se fait a partir de l'acétate de fer (1 mmol; synthétisé selon la procédure décrite dans C.T. Dziobkowski, T.J. Wrobleski, D.B. Brown, Inorg. Chem. 1982, 21, 671 **[51])** et l'acide muconique (1 mmol; Fluka, 97%) en 5 mL d'éthanol (Riedel-de Haën, 99.8%) avec l'addition de 0.25mL de hydroxyde de sodium 2M (Alfa Aesar, 98%) dans un autoclave (Paar bomb) à 100°C pendant 12h. Après refroidissement du container, le produit est récupéré par centrifugation à 5000rpm pendant 10 minutes.

200 mg du solide sont suspendus en 100mL de l'eau distillée sous agitation pendant 15h pour éliminer le solvant qui reste dans les pores. Le solide est ensuite récupéré par centrifugation à 5000rpm 10 minutes.

La taille de particule mesurée par diffusion de lumière est 400 nm.

Les nanoparticules montrent une morphologie arrondie et légèrement allongée, avec une taille de particules très homogène de 50-100 nm.

### MIL-88Anano$Fe_3OX[C_2H_2(CO_2)_2]_3$ $nH_2O$ (X=F, Cl, OH)

Pour l'obtention du matériau MIL-88Anano on ajoute a une solution de $FeCl_3.6H_2O$ (1 mmol; Alfa Aesar, 98%) et acide fumarique (1 mmol; Acros, 99%) dans 15 mL d'ethanol (Riedel-de Haën, 99.8%) 1 mL d'acide acétique (Aldrich, 99.7%). La solution est placée dans un flacon en verre et chauffée à 65°C pendant 2 heures. Le solide est récupéré par centrifugation à 5000rpm 10 minutes.

200 mg du solide sont suspendus en 100mL de l'eau distillée sous agitation pendant 15h pour éliminer le solvant qui reste dans les pores. Le solide est ensuite récupéré par centrifugation à 5000rpm 10 minutes.

La taille de particule mesurée par diffusion de lumière est 250 nm. Les images de MEB montrent des particules

allongées avec des arêtes. Ils existent deux tailles de particules, ~500 nm et 150 nm.

**MIL-100nano** $Fe_3O[C_6H_3(CO_2)_2]_3.X.nH_{2O}$ (X=F, Cl, OH)

Les nanoparticules MIL-100 ont été obtenues par synthèse hydrothermale sous irradiation micro-ondes (Mars 5, CEM), en chauffant une suspension aqueuse contenant 20 ml d'eau déionisée, 8,97 mmol de FeCl3 ($FeCl_3$•$6H_2O$, 98 % Alfa Aesar) et 4,02 mmol d'acide 1,3,5-benzènetricarboxylique (BTC, Sigma Aldrich) 6 min à 130 ° C sous agitation. Les détails de la synthèse sont indiqués dans le tableau 1 (conditions réactionnelles pour la synthèse hydrothermale du MOF MIL-100(Fe)).

**Tableau 1** : Synthèse hydrothermale assistée par irradiation microondes du MOF MIL-100(Fe)

| Réactifs | | | Paramètres | | | | |
|---|---|---|---|---|---|---|---|
| **$FeCl_3$** | **BTC** | **$H_2O$** | **Méthode** | **Puissance** | **Température** | **Temps** | **Agitation** |
| 8,97 mmol | 4,02 mmol | 110,16 mmol | Contrôle standard | 400W | 130°C | 6 min | Élevée |

À la fin de la réaction les nanoparticules ont été récupérées par centrifugation à 5600xg pendant 15 min et elles ont été activées par 6 lavages avec 30 ml d'EtOH absolu. Une fois activé, elles ont été re-dispersées dans de l'EtOH, soniquées avec une sonde u.s. jusqu'à atteindre une taille < 300nm et un PDI < 0,3. Enfin, les MIL-100 ont été centrifugées 15 min à 5600xg, le surnageant a été enlevé et les nanoparticules ont été stockées à température ambiante jusqu'au moment de l'analyse.

**MIL-101nano** $Fe_3OX[C_6H_4(CO_2)_2]_3\ nH_2O$ (X=F, Cl, OH)

Pour l'obtention du solide MIL-101nano une solution de $FeCl_3.6H_2O$ (1 mmol; Alfa Aesar, 98%) et acide 1,4-benzènedicarboxylique (1.5 mmol; 1,4-BDC Aldrich, 98%) dans 10 mL de diméthylformamide (Fluka, 98%) est placée dans une Paar bombe et chauffée à 100°C pendant 15 heures. Le solide est récupéré par centrifugation à 5000rpm 10 minutes.

Pour éliminer l'acide qui reste dans les pores le produit est chauffé à 200°C sous vide pendant 1 jour. Conserver sous vide ou atmosphère inerte car le produit n'est pas stable à l'air.

La taille de particule mesurée par diffusion de lumière est 310 nm.

**MIL-88Btnano** $Fe_3OX[C_6(CH_3)_4(CO_2)_2]_3\ nH_2O$ (X=F, Cl, OH)

Le solide MIL-88Btnano est synthétisé à partir d'une solution de $FeCl_3 \cdot 6H_2O$ (1 mmol; Alfa Aesar, 98%) et acide 1,4-benzènetétraméthyldicarboxylique (1 mmol; Chem Service) dans 10 mL de diméthylformamide (Fluka, 98%) avec 0.4 mL de NaOH 2M. Cette solution est placée dans une Paar bombe et chauffée à 100°C pendant 2 heures. Après refroidisement du container avec de l'eau froide, le produit est récupéré par centrifugation à 5000rpm 10 minutes.

200 mg du solide sont suspendus en 100mL d'eau distillée sous agitation pendant 15h pour éliminer le solvant qui reste dans les pores. Le solide est ensuite récupéré par centrifugation à 5000rpm 10 minutes.

La mesure de la taille de particule par diffusion de lumière montre deux populations de nanoparticules de 50 et 140 nm. Les nanoparticules du solide MIL-88Btnano ont une morphologie sphérique avec une taille de 50 nm. Seulement une fraction minoritaire possède une taille de 200 nm. On peut y observer aussi des agglomérats de petites particules.

**MIL-88Bnano** $Fe_3OX[C_6H_4(CO_2)_2]_3\ nH_2O$ (X=F, Cl, OH)

Le solide MIL-88Bnano est synthétisé à partir d'une solution d'acétate de fer (1 mmol; synthétisé selon la procédure décrite dans C.T. Dziobkowski, T.J. Wrobleski, D.B. Brown, Inorg. Chem. 1982, 21, 671) et acide 1,4-benzènedicarboxylique (1 mmol; 1,4-BDC Aldrich, 98%) dans 5 mL de méthanol (Aldrich, 99%). Cette solution est placée dans une Paar bombe et chauffée à 100°C pendant 2 heures. Après refroidissement du container avec de l'eau froide, le produit est récupéré par centrifugation à 5000rpm 10 minutes.

200 mg du solide sont suspendus en 100mL de l'eau distillée sous agitation et reflux pendant 200 mg du solide sont suspendus en 100mL de l'eau distillée sous agitation pendant 15h pour éliminer le solvant que reste dans le pores. Après, le solide est récupéré par centrifugation à 5000rpm 10 minutes.

La mesure de la taille de particule par diffusion de lumière montre une distribution bimodale de nanoparticules de 156 et 498 nm. La morphologie des particules est très irrégulière, avec une taille de 300 nm.

**ZIF-8 (Zn)_nano** $Zn_6N_{24}C_{48}H_{60}$ n.$H_20$

La synthèse de nanoparticules de l'imidazolate du zinc ZIF-8(Zn)_nano (ref : J. Mater. Chem., 2010, 20, 7676-7681 ; A. Demessence, et al, J. Mater. Chem., 2010, 20, 7676-7681 **[31])** se fait à partir d'une solution de $Zn(NO_3)_2 \cdot 6H_2O$ (2.933 g, 9.87 mmol; 98% Sigma-Aldrich) dans 200 mL de méthanol (Aldrich, 99%). Cette solution est versée dans une solution de 2-méthylimidazole (Hmim; 6.489 g, 79.04 mmol; 99% Aldrich) dans 200 mL de méthanol sous agitation à température ambiante pendant 1 heure. Le produit est récupéré par centrifugation à 10500rpm 15 minutes.

200 mg du solide sont suspendus dans 10 mL d'éthanol absolu sous agitation pendant 15 min. Cette procédure de lavage est répétée trois fois. La taille de particule mesurée par diffusion de lumière est 30 nm.

**UiO-66 (Zr)_nano** $Zr_6O_4(OH)_4(CO_2)_2C_6H_4)_6$ $nH_2O$

La synthèse de nanoparticules du téréphthalate de zirconium UiO-66(Zr)_nano (ref :J.H. Cavka et al., JACS, 2009, 130, 13850-13851 **[32])** est réalisée à partir d'une solution de $ZrOCl_2 \cdot 8H_2O$ (3.22 g, 10 mmol; 99% Sigma-Aldrich) et de l'acide 1,4-benzènedicarboxylique (1.662 g , 10 mmol; 1,4-BDC Aldrich, 98%) dans 50 mL de diméthylformamide (DMF ; Fluka, 98%) à 130°C pendant 2 heures. Le solide est récupéré par centrifugation à 10500rpm 15 minutes.

200 mg du solide sont suspendus dans 10 mL de DMF pendant une nuit. Ensuite, le produit récupéré par centrifugation (10500rpm 15 minutes) est suspendu dans 10 mL de méthanol pendant 1 h. Ce lavage avec du méthanol est répété deux fois. La taille de particule mesurée par diffusion de lumière est 280 nm.

**MIL-100(Al)_nano $Al_3OX[(CO_2)_3C_6H_3]_2$ $nH_2O$** (X=F, Cl, OH, $NO_3$)

La synthèse de nanoparticules du trimésate d'aluminium MIL-100(Al)_nano est réalisée en dispersant 1,21 grammes (0.05 mol) d' 1,3,5-benzèntricarboxylate de méthyle dans 20 mL d'eau à l'aide d'une canne à ultrasons (20 sec à 20% puis 20 sec à 30%). Le mélange est alors mis sous agitation magnétique et 2.6629 g de nitrate d'aluminium nonahydraté est versé. Ensuite, 4 mL d'acide nitrique 4 M sont rajoutés à la dispersion résultante, qui est encore gardée sous agitation pendant 5 minutes avant de chauffer. La synthèse est réalisée sous irradiation microondes à 210°C pendant 30 min avec une rampe de chauffage de 10 min. Le réacteur est sorti du four à microondes quand la température est descendue à 90 °C et le réacteur refroidi sous bain de glace. Le solide est récupéré par centrifugation à 10500rpm 20 minutes.

Lorsque la fraction liquide est prélevée et éliminée, le dépôt (culot) est redispersé dans 30 mL de méthanol et gardé sous agitation magnétique pendant une nuit. La dispersion est enfin centrifugée en utilisant les conditions décrites précédemment pour obtenir un solide jaune.

La taille de particule mesurée par diffusion de lumière est 120 nm.

Dans tous les exemples précédents, la détermination de la taille de particule par diffusion de lumière a été effectué sur un appareil Malvern Zetasizer Nano série - Nano-ZS; modèle Zen 3600 ; serial N° 500180 ; UK.

La microscopie électronique à balayage a été réalisée en utilisant un microscope Topcon (Akashi) EM 002B ultra-haut résolution 200kV.

**Exemple 2: Synthèse de nanoparticules de trimésate de fer MIL-100 fonctionnalisé par βCDP**

Les nanoparticules hybrides organiques-inorganiques (nanoMOF) à base de trimésate de fer, (MIL-100) ont été modifiées avec de la β cyclodextrine phosphate (βCDP, Cyclolab, CY-2017.1, formule moléculaire: $C_{42}H_{70}O_{47}P_4Na_4$). Cette molécule présente deux caractéristiques fondamentales pour une modification superficielle des nanoMOFs efficace: 1) elle possède une structure plus volumineuse que les fenêtres microporeuses des MIL-100, ce qui devrait empêcher son adsorption à l'intérieur des pores, préservant ainsi la porosité des nanoparticules; 2) elle est substituée avec quatre groupements phosphates, qui devraient assurer une interaction stable avec les nanoparticules par la formation de liaisons iono-covalentes avec les atomes de fer à la surface des particules (fig. 2).

2 mg de MIL-100 ont été modifiés par incubation avec 500 μl d'une solution aqueuse de βCDP 2 mg/ml (rapport en poids nanoparticules: βCDP = 1:0.5), 24 h sous agitation à température ambiante. Après l'incubation les nanoparticules modifiées ont été récupérées par centrifugation à 5600xg pendant 10 min. Le culot et le surnageant ont été analysés afin de caractériser l'interaction de la βCDP avec les MIL-100 par des méthodes directes et indirectes. Dans certains cas, un autre rapport en poids (nanoparticules : βCDP = 1:2) et d'autres temps d'incubation ont été testés.

**c) Caractérisation physico-chimique des MIL-100** Analyse DRX (diffraction à rayons X)

Les nanoparticules MIL-100, non modifiées et modifiées avec des βCDP, ont été séchées 8 h à 100° C et leur

structure a été analysée par diffraction à rayons X (diffractomètre à haute résolution Siemens D5000 X'Pert MDP (θ-2θ) (λCu, Kα1, Kα2)). Les résultats ont montré que la méthode de fonctionnalisation de la surface de MIL-100 avec de la βCDP ne modifie pas la structure cristalline de nanoparticules (fig. 3).

Analyse spectroscopique FT-IR

5 mg de MIL-100 ont été incubés avec 1 ml d'une solution aqueuse de βCDP à 2,5 mg/ml (rapport en poids nanoparticules: βCDP = 1:0, 5), 24 h sous agitation à température ambiante. À la fin de l'incubation les nanoparticules ont été récupérées par centrifugation à 5600xg pendant 10 min, lavées trois fois avec 1 ml d'eau, séchées 8 h à 100 ° C et enfin caractérisées par FT-IR. Après modification, le spectre de MIL-100 montre une signature typique de βCDP à environ 1 050 $cm^{-1}$, absente dans l'échantillon non modifié, qui pourrait être lié à la présence de groupements phosphate liés aux nanoparticules.

Ces résultats confirment l'effective fonctionnalisation des nanoparticules de MIL-100 avec la βCDP par incubation simple (fig. 4).

Analyse Élémentaire

20 mg de MIL-100 nanoMOF ont été incubés 15 min, 1 h ou 24 h avec 4 ml d'une solution aqueuse de βCDP 2,5 mg/ml (rapport en poids nanoparticules: βCDP = 1:0, 5) sous agitation, à température ambiante. Après incubation les nanoparticules ont été récupérées par centrifugation à 5600xg 10 min, lavées 2 fois avec 4 ml d'eau et stockées à 100 ° C jusqu'au moment de l'analyse. Le pourcentage en poids de βCDP liées aux MIL-100 (βCDP %p/p) a été calculé sur la base du contenu en phosphore de l'échantillon modifié (P $\%p/p_{MIL\text{-}100(\beta CDP)}$) et de la βCDP (P $\%p/p_{\beta CDP}$) selon la formule suivante :

$$\beta CDP\,\%\frac{p}{p} = \frac{P\,\%\frac{p}{p}(MIL-100(\beta CDP))}{P\,\%\frac{p}{p}(\beta CDP)} x\,100$$

Les résultats obtenus ont montré une cinétique rapide d'interaction entre les βCDP et les MIL-100. La plupart des cyclodextrines est déjà associée aux nanoparticules après seulement 15 min d'incubation. Plus précisément, le pourcentage en poids de βCDP est de 13-14 et de 17,3 % respectivement après 15 min, 1 h et 24 h d'incubation (fig. 5) .

De plus, le rapport entre le pourcentage en poids de Na et celui du phosphore a été analysé:

$$\frac{Na\,\%\frac{p}{p}}{P\,\%\frac{p}{p}}$$

Il a été observé que : a) dans le MIL-100 qui ne contient pas de sodium ce rapport est égal à 0; b) dans la βCDP il est de 0,74, ce qui correspond exactement à la valeur prévue sur la base de la formule moléculaire ($C_{42}H_{70}O_{47}P_4Na_4$) et c) dans les MIL-100 modifiées avec la βCDP, le rapport diminue nettement (environ 0,09), indiquant que la plupart des groupes phosphates est probablement coordonnée avec les nanoparticules.

Enfin l'expérience a été répétée en présence d'un excès de βCDP (rapport en poids MIL-100 :βCDP=1:2), et il a été constaté que dans ce cas, le contenu en poids de βCDP atteint 32,4 % après 24 h d'incubation (fig. 5), qui peut donc être considéré le taux d'association maximal.

Analyse de Potentiel-ζ

2 mg de MIL-100 ont été incubés avec 500μl d'une solution aqueuse de βCDP 2 mg/ml pendant différents temps d'incubation (1 h, 4 h, 8 h, 24 h), sous agitation, à température ambiante. 2mg de nanoparticules non modifiées, utilisées comme contrôle, ont été incubés avec 500 μl d'eau. À la fin de l'incubation, les nanoparticules ont été récupérées par centrifugation à 5600xg 10 min, lavées 2 fois à l'eau, et re-suspendues dans une solution de KCl 0,5 mM. La charge de surface de MIL-100 a été analysée par des mesures de potentiel ζ (Zetasizer Nano 6,12, Malvern Instruments Ltd., UK). Les résultats ont montré que la charge de surface de nanoparticules devient beaucoup plus négative après la modification avec βCDP, diminuant de -12 (±3) mV dans l'échantillon non modifié, à -35 (±3, 5) mV. Ces résultats confirment l'interaction des βCDP avec la surface des MIL-100 au travers de la présence des groupements phosphates

qui rendent la charge de surface plus négative. De plus, cette interaction est très rapide et, après seulement 1 h d'incubation, le potentiel ζ a déjà atteint sa valeur maximale (fig. 6).

Analyse XPS

2 mg de MIL-100 ont été incubées avec 500 μl d'une solution aqueuse de βCDP 2 mg/ml (rapport en poids nanoparticules/βCDP = 1:0, 5) 3 h, sous agitation, à température ambiante. 2 mg de nanoparticules non modifiées, utilisés comme contrôle, ont été incubées avec 500μl d'eau. À la fin de l'incubation, les nanoparticules ont été récupérées par centrifugation à 5600xg 10 min, lavées 3 fois à l'eau et re-suspendues dans 100 μl d'eau deionisée. 10 μl de ces suspensions ont été déposés sur un film en cuivre, laissées sécher à l'air et analysées par XPS.

Les résultats ont montré une augmentation significative du rapport entre le pourcentage atomique du C et celui du fer (C %atomique/Fe %atomique) après modification avec les βCDP (Tab. 2). Dans les MIL-100 la présence du C est liée uniquement au trimésate et l'excès observé après modification est donc en accord avec la présence des βCDP à la surface des nanoparticules. En particulier, il y a 4,6 C appartenant à la βCDP liés à chaque atome de Fe, ce qui correspond environ à 1 βCDP pour chaque 9,1 atomes de Fe, vu que chaque molécule de βCDP contient 42 C. Le même raisonnement peut être appliqué prenant en considération le pourcentage atomique d'oxygène. Dans ce cas, nous avons observé une augmentation du rapport entre le pourcentage atomique d'O et celui du fer (O/Fe % atomique) après modification avec les βCDP. En particulier, nous avons calculé la présence d'environ 4,2 O liée à la βCDP par atome de Fer, ce qui équivaut à la présence d'une molécule de βCDP chaque 11.2 atomes de Fer, vu que chaque molécule de βCDP contienne 47 O. Ces résultats sont en accord avec celui obtenu précédemment à partir du pourcentage atomique du C. Nous pouvons donc conclure qu'il y a environ 1 molécule βCDP chaque 10 atomes de Fe.

Enfin, nous avons répété l'expérience en présence d'un excès de βCDP (rapport en poids nanoparticules :βDP = 1:2). Avec cette même stratégie, nous avons calculé que le plus haut taux d'association correspond effectivement une molécule de βCDP pour 8 atomes de Fe.

Enfin, nous avons répété l'expérience en présence d'un excès de βCDP (rapport en poids nanoparticules :βCDP = 1:2). Avec cette même stratégie, nous avons calculé que le plus haut taux d'association correspond effectivement une molécule de βCDP pour 8 atomes de Fer.

Ces résultats indiquent que les βCDPs sont capables d'interagir avec la surface de MIL-100 formant une couronne externe. Cependant, notre but final était de démontrer que cette couronne externe n'altérait pas la porosité des MIL-100 et leur capacité d'encapsulation de médicaments.

**Tableau 2:** Composition atomique des nanoparticules de MIL-100 non modifiées et modifiées avec des βCDP.

| | C%/Fe% | O%/F | P%/Fe |
|---|---|---|---|
| **nanoMOF** | 7,5 | 4,5 | 0 |
| **nanoMOF(βCDP) (1:0,5)** | 12,1 | 8,7 | 0,5 |
| **nanoMOF(βCDP) (1:2)** | 13,1 | 9,7 | 0,7 |

Porosimètrie d'adsorption d'azote des MIL-100 après modification avec βCDP ou MeO-PEG-$NH_2$

Pour répondre à cette question, nous avons étudié la porosité du MIL-100 avant et après modification avec de la βCDP. 30 mg de nanoparticules ont été incubées avec 6 mL d'une solution aqueuse de βCDP 2,5 mg/ml (rapport en poids nanoparticules:βCDP = 1:0. 5), 24 h sous agitation. Après incubation, les nanoparticules ont été récupérées par centrifugation à 5600 xg 10 min et séchées 6 h à 100 ° C. La porosité des matériaux a été analysée par adsorption d'azote à 77K (Belsorp Mini, Bell, Japon). Les résultats ont montré que la porosité (surface spécifique et volume de pores), en tenant compte de l'augmentation en masse liée au greffage des βCDP à la surface des particules de MIL-100, des particules reste exactement la même après modification, démontrant ainsi que la βCDP, grâce à sa structure volumineuse n'est pas adsorbé à l'intérieur des pores de nanoparticules et interagit uniquement avec la surface de nanoparticules formant une couronne externe qui n'affecte pas la porosité des matériaux et leur capacité d'encapsulation de médicaments (Fig. 7 A).

Au contraire, l'étude de la porosité des MIL-100 avant et après incubation avec Meo-PEG-$NH_2$ (5000 Da), nous avons observé que la porosité des nanoparticules diminue considérablement après la modification (Fig. 7 B). Ces résultats démontrent qu'il n'est pas possible modifier directement la surface des MIL-100 avec du PEG sans altérer leur porosité et la capacité d'encapsulation, probablement parce que la chaîne linéaire du PEG peut pénétrer facilement dans les fenêtres microporeuses des nanoparticules et être ainsi adsorbées à l'intérieur des pores.

Dans cette étude, 30 mg de MIL-100 ont été incubés avec 2 mL d'une solution aqueuse de PEG 5 mg/ml, 3 h, 30 ° C, sous agitation. À la fin de l'incubation, les nanoparticules ont été récupérées par centrifugation à 5600xg 10 min

et séchées 6 h à 100 °. La porosité des matériaux a été analysée par porosimétrie d'adsorption d'azote à 77 K.

**Exemple 3: Synthèses de PEG ramifié**

30 mg de nanoparticules du MIL 100 ont été incubées avec 2 mL d'une solution aqueuse de PEG étoile 0.5 mg/mL (Amino-dPEG™(4)-[dPEG™(12)-OMe]$_3$, $C_{99}H_{197}N_5O_{47}$, 2209, 63 g/mole, Iris Biotech, Allemagne) à température ambiante sous agitation bidimensionnelle, pendant 2 minutes. A la fin de l'incubation, les nanoparticules ont été récupérées par centrifugation à 5600xg 10 min, suivi de deux lavages à l'eau. Le PEG a été dosé dans les surnageants récupérés après la première centrifugation utilisant une méthode colorimétrique (ref Baleaux **[33]).** Seulement 1% pds du PEG reste dans le surnageant, ce qui montre que pratiquement la totalité du PEG (99% pds) interagit avec les nanoparticules. La porosité des nanoparticules séchées a été analysée par adsorption d'azote à 77 K. La surface spécifique BET passe de 1400 à 1225 $m^2/g$, montrant ainsi que le PEG étoile pénètre également dans les pores, tout comme le PEG linéaire (cf ci-dessous). De même, la charge de surface ne change pas avant et après la fonctionnalisation avec le PEG ramifié (potentiel $\xi \sim$ - 25 mV)

Il est à noter que cette pénétration dans les pores est extrêmement rapide, en deux minutes environ. Cet exemple tend à prouver que pour ce matériau à « grandes » fenêtres, des PEG étoilés avec un nombre plus important de ramifications (> 4) serait nécessaire afin de ne pas pénétrer dans les pores.

**PEG linéaires avec différents groupement terminaux**

30 mg de nanoparticules MIL 100 ont été incubées à température ambiante sous agitation bidimensionnelle pendant 2 minutes avec 1 mL d'éthanol et 1 mL d'une solution aqueuse de PEG linéaire 0.5 mg/mL portant à chaque bout de chaine:

- un groupement méthoxy et un groupement carboxyle (alpha-Methoxy-omega-carboxylic acid poly(ethylene glycol), 5.000 Da, Iris Biotech, Allemagne, MeO-PEG-COOH)
- un groupement méthoxy et un groupement amino (alpha-Methoxy-omega-amino poly(ethylene glycol), 5.000 Da, Iris Biotech, Allemagne, MeO-PEG-$NH_2$)
- un groupement amino et un groupement carboxyle (alpha-Amino-omega-carboxy poly(ethylene glycol) hydrochloride, 5.000 Da, Iris Biotech, Allemagne, $NH_2$-PEG-COOH·HCl)
- PEG étoilé (Amino-dPEG™(4)-[dPEG™(12)-OMe]$_3$, $C_{99}H_{197}N_5O_{47}$, 2209, 63 g/mole, Iris Biotech, Allemagne
- un groupement bisphosphonate (PEG-alendronate ; synthèse au laboratoire) :

***Etape 1 : Synthèse de l'acide 4-amino-1-hydroxybutylidene)bisphosphonique (alendronate)***

Dans un tétracol de 250 mL, muni d'un agitateur mécanique, d'un thermomètre, d'une ampoule d'addition, l'acide 4-aminobutyrique (20g, 0.19mol) et un équivalent d'acide phosphoreux $H_3PO_3$ (16g, 0.19) sont introduits. Ils sont dissous dans un minimum d'acide méthane sulfonique puis le milieu réactionnel est chauffé à 65 °C.

Tout en maintenant la température à 65 °C, deux équivalents de trichlorure de phosphore PCl3 (35mL, 0.40mol) sont additionnés goutte à goutte en 20 minutes. Le milieu réactionnel est alors agité à 65 °C pendant une nuit. Par la suite, une trempe du milieu réactionnel est effectuée, à l'aide d'eau distillée glacée puis le milieu obtenu est transvasé dans un tricol de 500 mL. Le mélange est porté à reflux pendant 5 heures.

A l'aide d'un bain de glace, le milieu réactionnel est ramené à température ambiante. En utilisant une solution aqueuse de NaOH à 50 % (en masse), le pH est amené à 4,3 pour favoriser la précipitation de l'acide bisphosphonique. Le précipité est filtré et séché sous vide. La purification de l'alendronate consiste à effectuer plusieurs lavages avec du méthanol anhydre jusqu'à élimination de l'acide méthane sulfonique. Nous séchons ensuite le précipité pendant une nuit au dessiccateur chauffant (à 40 °C).

*Rendement:* 82 %
*I.R. ($cm^{-1}$):* 1524, 1473, 1168, 1073, 913 $cm^{-1}$ *RMN $^1H$ (500 MHz, $D_2O$):* 2.93 (m, 2H), 1.88 (m, 4H) *RMN $^{31}P$ {1H} (80.9 MHz, $H_3PO_4/D_2O$):* 18.5(s).

*Etape 2 : Couplage alendronate-PEG5000-COOH (PEG-alendronate)*

Dans un pilulier, 1 équivalent de m*PEG5000-COOH* (150 mg, $3.10^{-5}$ mol) sont solubilisés dans 5 mL d'eau sous agitation magnétique. Les agents de couplage EDC (45.5 mg, $3.10^{-5}$ mol) et NHS (33.7 mg, $3.10^{-5}$ mol) sont additionnés au mélange réactionnel. La solution est agitée pendant une heure dans l'incubateur à 37°C. Le pH est de 4.5.
L'alendronate (7.8 mg, 0.9éq) est solubilisé dans 1mL d'eau. Le pH est ajusté jusqu'à 10 grâce à quelques gouttes de triéthylamine. La solution est ensuite ajoutée goutte à goutte sur la solution de PEG activée, et le pH est ajusté jusqu'à 10 une fois de plus.
La solution est agitée pendant 24 heures dans l'incubateur à 37°C.
La purification a consisté à évaporer l'eau. Les cristaux sont solubilisés dans un minimum d'eau (200μ) puis le produit désiré est précipité par addition de 20mL d'acétone. Le précipité blanc est filtré puis séché à l'étuve sous vide à 40°C pendant une nuit.

*Masse obtenue:* 110 mg
*Rendement:* 68 %
*RMN $^{31}P$ {1H} (80.9 MHz, $H_3PO_4/D_2O$):* 17.96 (s).

A la fin de l'incubation, les nanoparticules ont été récupérées par centrifugation à 5600xg 10 min, suivi de deux lavages à l'eau. Le PEG a été dosé dans les surnageants récupérés après la première centrifugation utilisant une méthode colorimétrique (ref Baleaux **[33]**). Seulement 1 à 2 % pds du PEG restent dans le surnageant, ce qui montre que pratiquement la totalité du PEG (98-99% pds) interagit avec les nanoparticules.
La porosité des nanoparticules séchées a été analysée par adsorption d'azote à 77 K. La surface spécifique BET a diminué de 1400 à 1050, 1120, 1160, 1270 et 1320 $m^2/g$ dans le cas des MeO-PEG-COOH, MeO-PEG-$NH_2$, $NH_2$-PEG-COOH et, PEG étoilé et PEG-alendronate, respectivement. Ceci démontre que ces PEG linéaires fonctionnalisés en bout de chaine pénètrent rapidement dans les pores des nanoparticules de MIL 100. De même, la charge de surface ne change pas après la fonctionnalisation avec le PEG ramifié (potentiel $\xi \sim -25$ mV) .

**Exemple 4 : Synthèse de dérivés fluorescents du monomère de β-cyclodextrine phosphate :**

**i) β-cyclodextrine phosphate rhodamine BITC βCDP-RBITC)**

**ii) β-cyclodextrine phosphate FITC (βCDP-FITC)**

Les étapes de synthèse utilisées sont illustrées à la Figure 14.

*6-monoamino-6-monodeoxy-βCD (base libre) (**1**)*

11.60 g (0,01 mol) de 6-Monoazido-6-monodeoxy-βCD ont été dispersés à froid, sous agitation, dans un mélange $H_2O$:MeOH-4:1 (100 mL). Dans un premier temps, une suspension de Pd/C (0,58 g, 5 % contenu de Pd dans 3 mL H2O), puis l'hydrazine monohydrate (5 g, 5 mL, 0,1 mol) ont été ajoutées et chauffées à reflux. Après 20 min sous agitation à reflux, le mélange a été refroidi à environ 50 °C, filtré pour éliminer le catalyseur et lavé trois fois avec de l'eau (15 mL). Après évaporation des solvants, le produit brut a été précipité dans une solution aqueuse (50 mL) de $NH_4OH$ 25 % (2 mL). Les cristaux ont été filtrés, lavés trois fois avec du MeOH (10 mL) et séchés à 60 °C, sous pression réduite (10 mbar) pendant la nuit en présence de $P_2O_5$ et KOH. Le produit (***1***) obtenu sous forme de cristaux blancs (10,2 g, 90 %) était dépourvu d'azoture et d'hydrazine, caractérisé par IR et il a été stocké sous vide en présence de KOH.

(***1***): m.p.: 203-205°C (dec.). $R_f$: 0.26-0.29.
IR (KBr) v/$cm^{-1}$: disparition de la bande d'azide (2105), 3428 (O-H), 2928 (C-H), 1080, 1029 (C-O-C).
$^1$H-RMN (DMSO-$d_6$): δ= 5.78-5.63 (m, 14H), 4.90-4.85 (m, 7H), 4.50-4.45 (m, 6H), 3.66-3.54 (m, 28H), 3.42-3.24 (overlap with HDO, m, 16H).
$^{13}$C-RMN (125 MHz, DMSO-$d_6$): δ= 101.8, 82.9, 81.6, 81.5, 73.0, 72.3, 72.1, 59.9.

*6-monodeoxy-6-mono[(5/6)-rhodaminylthioureido]-βCD hydrochloride (**2**)*

Le composé (***1***) fraîchement préparé (227 mg, 0,2 mmol) a été dispersé à température ambiante dans 5 ml de pyridine et 113 mg (0.21 mmol) d'isocyanate de Rhodamine B (« Rhodamine B isothiocyanate » ou « RBITC » en anglais) ont été ajoutés. Le mélange à été chauffé à 60 °C sous agitation. Après 4 h, la température a été portée à 90-100 °C et 54 mg (0,1 mmol) d'isocyanate de Rhodamine B ont été ajoutés. Après 3h sous agitation, le milieu réactionnel à été

porté à température ambiante et précipité en ajoutant 20 ml d'acétone. Le produit brut a été filtré, lavé deux fois avec de l'acétone (1 mL), dispersé dans l'eau (50 mL) et extrait trois fois avec de l'acétate d'éthyle pré-saturé avec de l'eau (50 mL) pour éliminer l'isocyanate de Rhodamine B résiduel. La phase aqueuse a été évaporée à 60 °C, sous pression réduite et le solide violet obtenu a été dispersé dans 10 ml d'eau. De l'acide chlorhydrique (0,1 M) a été ajouté jusqu'à obtenir une solution claire (pH = 4-5). Après lyophilisation, le produit (***2***) à été récupéré sous forme de poudre violette (244 mg, 74 %).

*(**2**)*: m.p.: 214°-215°C (dec.). $R_f$: 0.52-0.54, 0.82-0.84 (RBITC).
IR (KBr) ν/cm$^{-1}$: 3310 (O-H), 2968 (C-H), 2928 (C-H), 1708 (C=O), 1617 (C=C), 1154 (C-O), 944 (C-O), 683.
$^1$H-RMN(DMSO-$d_6$): δ= 0.90-1.30 (t, 12H, methyl-H), 3.10-3.50 (m, 14H, H-2,4), 3.50-3.78 (m, 28H, H-3,5,6), 3.78-4.10 (m, 8H, méthylène-H), 4.51 (s large, 6H, OH-6), 4.84 (s, 6H, H-1), 4.96 (s, 1H, H-1'), 5.74 (s large, 14H, OH-2,3), 6.80-7.00 (m, 6H, aromatic-H), 7.00-7.18 (m, 3H, aromatic-H), 7.23 (s), 7.37 (m), 7.60-7.75 (m), 7.90 (s large), 8.00-8.08 (s large), 8.08-8.25 (m), 8.39 (s large), 8.52 (s large), 8.56 (d, J = 3.4 Hz), 8.74 (d, J = 4.7 Hz),11.00 (s large, 1H, OH-carboxylic)
$^{13}$C-RMN(DMSO-d6): δ= 12.25, 45.12 , 59.71, 71.83, 72.23, 72.85, 81.31, 95.63, 101.10-101.72 (s large), 112.00-115.00 (s large), 125.40, 129.00-130.93 (s large), 141.40, 154.78, 156.88, 165.40.

*6-monodeoxy-6-mono[(5/6)-rhodaminylthioureido]-βCD phosphatesodium salt (**3**)*

$P_2O_5$ a été ajouté (200 mg, 1,4 mmol) à la N-diméthylformamide anhydre (DMF, 3 mL) et le mélange a été dispersé par ultrasonication jusqu'à obtenir une solution limpide. Le composé (***2***) (235 mg, 0,14 mmol) a été ajouté à la solution à température ambiante. Le mélange réactionnel a été porté à 40 °C, laissé 4 h sous agitation et puis refroidi à température ambiante. 20 ml d'eau ont été ajoutés et la solution obtenue a été dialysée pendant 1 journée. La solution à été neutralisée (pH ~ 7) avec du NaOH (1 M) et dialysée plusieurs fois (1 jour). Le pH de la solution a été porté à 7-8 avec du NaOH (1 M) et le produit a été extrait quatre fois avec de l'acétate d'éthyle pré-saturé avec de l'eau (30 mL). L'eau a été évaporée à 60 °C sous pression réduite et le solide obtenu a été dispersé dans 20 ml d'eau, dialysé pendant la nuit et enfin lyophilisé (***3***) sous forme de poudre violette (229 mg, 84 %).

*(**3**)*: m.p.: 229°-232°C (dec.). $R_f$: 0.0, 0.82-0.84 (RBITC).
IR (KBr) ν/cm$^{-1}$: 3390 (O-H), 1647 (C=C), 1594, 1467, 1414, 1348 (P=O), 921, 685, 517.

*6-monodeoxy-6-mono[(5/6)-fluoresceinylthioureido]-βCD hydrochloride (**4**)*

Le composé (1) fraîchement préparé (227 mg, 0,2 mmol) a été dispersé dans 5 ml de pyridine (5 mL) à température ambiante, 82 mg (0.21 mmol) d'isocyanate de fluoresceine (« fluorescein isothiocyanate » ou « FITC » en anglais) ont été ajoutés et le mélange réactionnel a été porté à 60 °C. Après 3 h sous agitation, la température a été porté à 90 °-100 °C et 39 mg (0,1 mmol) d'isocyanate de fluoresceine ont été ajoutés. Après 2h sous agitation, le mélange réactionnel a été refroidi à température ambiante et précipité par ajout 20 ml d'acétone. Le produit brut a été filtré, lavé deux fois avec de l'acétone (1 mL), dispersé dans l'eau (50 mL) et extrait trois fois avec de l'acétate d'éthyle pré-saturé avec de l'eau (50 mL) pour éliminer l'isocyanate de fluoresceine résiduel. La phase aqueuse a été évaporée à 60 °C, sous pression réduite. Le solide jaune-orange a été dispersé dans l'eau (10 mL) et de l'acide chlorhydrique (0,1 M) a été ajouté jusqu'à obtenir une solution claire (pH = 4-5). Après lyophilisation le produit (***4***) à été récupéré sous forme de poudre jaune (256 mg, 85 %).

(***4***): m.p.: 222-225 (dec.). $R_f$: 0.48-0.51, 0.79-0.83 (FITC).
IR (KBr) ν/cm$^{-1}$: 3307 (O-H), 2930 (C-H), 1746 (C-OLacton), 1614 (C=C), 1540, 1505, 1453, 1402, 1365, 1328, 1259, 1154, 945, 850, 593.
$^1$H-RMN (DMSO-d6): δ=3.10-3.44 (m, 14H, H-2,4), 3.50-3.78 (m, 28H, H-3,5,6), 4.23-4.65 (m, 6H, OH-6), 4.83 (d, 6H, H-1, J = 2.9 Hz), 4.90 (s large, 1H, H-1'), 5.55-6.00 (m, 14H, OH-2,3), 6.45-6.72 (m, 6H, Aromatic-H),7.18 (d, 1H, H, J = 7.9 Hz), 7.78 (d, 1H, J = 7.4 Hz), 7.90 (s large, 1H), 10.12 (s large, 2H, OH-phénolique et carboxylique).
$^{13}$C-RMN(DMSO-d6): δ=59.59, 71.83, 72.34, 72.90, 81.41, 99.26, 101.80, 102.10, 109.68, 118.17, 127.37, 128.90, 129.20, 131.30, 152.71, 167.97, 169.51, 180.30.

*6-monodeoxy-6-mono[(5/6)-fluoresceinylthioureido]-βCD phosphatesodiumsalt (**5**)*

$P_2O_5$ a été ajouté (200 mg, 1,4 mmol) dans 3 ml de N-diméthylformamide anhydre (DMF) et le mélange a été dispersé par ultrasonication jusqu'à obtenir une solution limpide. Le composé (***4***) (213 mg, 0,14 mmol) a été ajouté à la solution à température ambiante et le mélange réactionnel a été chauffé à 40 ° C sous agitation pendant 1 h. Le produit

brut a été refroidi à température ambiante, 20 mL d'eau ont été ajoutés et la solution obtenue a été dialysée pendant 1 journée. La solution a été neutralisée (pH ~ 7) avec du NaOH (1 M) et dialysée plusieurs fois (1 jour). Le pH de la solution a été porté à 7-8 avec du NaOH (1 M) et le produit a été extrait quatre fois avec de l'acétate d'éthyle pré-saturaté avec de l'eau (30 mL). L'eau a été évaporée à 60 °C sous pression réduite. Le solide obtenu a été dispersé dans 20 ml d'eau, dialysé pendant la nuit et puis lyophilisé ***(5)*** jusqu'à obtenir une poudre jaune (223 mg, 84 %).

(***5***): m.p.: 268-270 (dec.). $R_f$: 0.0-0.0, 0.79-0.83 (FITC).
IR (KBr) v/cm$^{-1}$: 3399 (O-H), 2924 (C-H), 1750 (C=O), 1639 (C=C),1611,1498, 1470,1428, 1253 (P=O), 1158, 1080, 1034, 918, 525.
$^{31}$P-RMN ($D_2O$): δ=-22.46 (s large), -21.49 (s), -10.70 (s large), -9.44 (s large), -8.86 (m), -2.78 (s large), 0.35 (s large), 0.86 (s large).

**Exemple 5: Synthèse de dérivés fluorescents du polymère de β-cyclodextrine phosphate**

Les étapes de synthèse utilisées pour la préparation des dérivés fluorescents du poly-BCD phosphate sont illustrées dans la Figure 16.

**Note: D**ans le cas du FITC, la dernière étape seulement est différente (FITC au lieu de RBITC, composé (***9***))

*6-monoazido-6-monodeoxy-poly-βCD (**6**) :*

8 mg (0,03 mmol) $PhP_3$ ont été dispersés sous agitation dans 15 ml de DMF anhydre. A cette solution, 8 mg (0,03 mmol) de $I_2$ ont été doucement ajoutés pendant 10 min en faisant attention que la température ne dépasse les 40°C. 2,1 g de poly-BCD ont été ensuite ajoutés, la température portée à 50 °C et la solution a été laissée sous agitation à pression atmosphérique pendant 7 h. La suspension a été refroidie à environ 50 °C, 5 ml de $CH_3OH$ ont été ajoutés et la suspension a été mise sous agitation pendant 30 min. $CH_3OH$ a été ensuite enlevé sous pression réduite et du DMF (~2 mL) ont été ajoutés jusqu'à obtenir une solution. 4 mg (0.062 mmol) de $NaN_3$ ont été ajoutés et la température portée à 100 °C pendant 3h. Après refroidissement à température ambiante, la solution a été dialysée pendant la nuit et a été ensuite diluée avec 100 mL de $H_2O$. 0,5 g de charbon de bois ont été ajoutés et la suspension a été mise sous agitation pendant 30 min. Le charbon a été filtré, le produit de la réaction a été lavé trois fois avec de l'eau (15 mL) et filtré. La solution résultante a été évaporée à 1/3 du volume et la lyophilisation a donné le produit (6) sous forme de poudre blanche (1,9 g).
IR v/cm$^{-1}$. 3408, 2926, 2103, 1663, 1437, 1037, 859, 723, 542. $^1$H-RMN ($D_2O$) δ: 3.0-4.4 (s large), 4.9-5.3 (s large).

*6-monoamino-6-monodeoxy-poly-βCD (**7**)* :

Le composé (6) (1,9 g) a été dissous dans 15 ml d'eau. Premièrement une suspension de Pd/C (0,18 g, 10% de Pd dans 2 mL d'$H_2O$), et ensuite de l'hydrazine monohydrate (1 g, 1 mL, 0,02 mol) ont été ajoutée et chauffée à reflux. Après 30 min, sous agitation sous reflux, le mélange réactionnel a été refroidi à température ambiante, filtré et en fin dialysé pendant la nuit. Le pH de la solution a été porté entre 5 et 6 avec de l'HCl et la lyophilisation a donné le produit (7) sous forme d'une poudre blanche (1,8 g).
IR v/cm$^{-1}$: disparition de la bande azide à 2103.

*6-monodeoxy-6-mono[(5/6)-rhodaminylthioureido]-poly-βCD (**8**)* :

0,8 mg de composé (7) ont été dissous dans 10 ml de pyridine. 10mg (10 μL, 0,05 mmol) de DBU et puis 6 mg (0,01 mmol) de RBITC ont été ajoutés et la solution a été chauffée à 60 °C pendant 18h. Le solvant a été évaporé sous pression réduite (60 °C, 10 mbar). Le produit brut a été redispersé dans 50 ml d'eau et extrait trois fois avec 100 ml de DCM. La phase aqueuse a été évaporée et le solide a été redispersé dans de 50 ml d'eau et extrait trois fois avec de l'acétate d'éthyle pré-saturé avec 100 ml d'eau. La phase aqueuse a été dialysée trois fois et la lyophilisation a donné le produit (8) sous forme d'une poudre violette (0,6 g).
IR v/cm$^{-1}$: 3391, 2924, 1721, 1649, 1591, 1418, 1339, 1157, 1082, 1033, 858, 757.

*6-monodeoxy-6-mono[(5/6)-fluoresceinylthioureido]-poly-βCD (**9**)* :

0,8 mg de composé (7) ont été dispersés dans 10 ml de pyridine. 10 mg (10 μL, 0,05 mmol) de DBU et ensuite 4 mg (0,01 mmol) de FITC ont été ajoutés et la température a été portée à 60 °C pendant 9 h. Le solvant a été évaporé sous pression réduite (60 °C, 10 mbar), le prosuit brut a été redispersé dans 50 ml d'eau et extrait trois fois avec 100 ml de DCM. La phase aqueuse a été évaporée et le solide a été redispersé dans 50 ml d'eau et extrait trois fois avec

de l'acétate d'éthyle pré-saturé avec 100 ml d'eau. La phase aqueuse a été dialysée trois fois et la lyophilisation a donné le produit (9) sous forme de poudre jaune (0,7 g).
IR v/$cm^{-1}$: 3402, 2925, 1738, 1653, 1592, 1506, 1465, 1328, 1151, 1110, 1082, 1041, 853.

Les étapes de phosphorylation de poly-βCDs sont illustrées dans la Figure 17.

**Note:** Dans le cas de FITC, la phosphorylation a été réalisée avec FITC au lieu de RBITC (composé (***11***)).

*6-monodeoxy-6-mono[(5/6)-rhodaminylthioureido]poly-βCD phosphate, sel de sodium (**10**)*

*6-monodeoxy-6-mono[(5/6)-fluoresceinylthioureido]-poly-βCD phosphate, sel de sodium (**11**)*

*poly-βCD phosphate, sel de sodium (**12**)*

**Exemple 6: Caractérisation ITC (« isothermal titration calorimetry » en anglais) des MOFs MIL-100 à surface externe modifiée au β-CDP de l'Exemple 2b)**

Afin d'étudier la nature de l'interaction entre les βCDP et les MIL-100, la thermodynamique de la réaction de modification de surface des nanoparticules par ITC a été caractérisée. Des solutions aqueuse de βCDP et β cyclodextrine (βCD) à 30 et 14 mg/ml respectivement, ont été injectées goutte à goutte dans 2 ml d'une suspension aqueuse de MIL-100 1,5mg/ml. Les résultats ont montré que les βCDP interagissent avec les MIL-100 par une réaction complexe, impliquant des phénomènes exothermiques et endothermiques, qui pourraient être liés respectivement à la coordination entre groupements phosphates et les atomes de fer et à la conséquente déshydratation de nanoparticules. Au contraire aucun signal n'a été observé en titrant la solution de βCD. Ces résultats indiquent que les groupes phosphates jouent un rôle clé dans l'interaction avec le MIL-100, probablement formant des liens iono-covalents avec les atomes de fer (fig 9) .

**Exemple 7: Stabilité du recouvrement à base de βCDP.**

Une fois vérifié que la βCDP est capable d'interagir efficacement avec les MIL-100, la stabilité de cette interaction dans des conditions physiologiques a été étudiée. Pour cela, nous avons modifié les MIL-100 avec de la βCDP-rhodamine, une molécule de βCD substituée avec 3 groupes phosphates et une unité de rhodamine-BITC. 2 mg de nanoMOF ont été incubés avec 500 μl d'une solution aqueuse de βCDP-rhodamine, 24 h sous agitation, à température ambiante. À la fin de l'incubation, les nanoparticules ont été récupérées par centrifugation à 5600xg 10 min, et le surnageant a été analysé par spectrofluorimétrie afin de définir la quantité de βCDP-rhodamine liée aux nanoparticules. Les résultats obtenus indiquent que la quantité de βCDP-rhodamine associée aux MIL-100 représente 30,5($\pm$0,1) % p/p. Ces résultats sont en accord avec ceux obtenus par analyse élémentaire. Les nanoparticules modifiées ont été lavées avec 1 ml d'eau, afin d'éliminer l'excès de βCDP-rhodamine et enfin incubées dans 1 ml de PBS (Phosphate Buffered Saline, Lonza) ou RPMI 10% FBS (RPMI 1640 GlutaMAX ™ additionné de sérum foetal bovin 10%(v/v), 100 UI/mL de pénicilline-streptomycine), à 37 °C, sous agitation. Après différents temps d'incubation (0,5-2, 5-5-8-24 h) la suspension a été centrifugée 10 min à 5600xg, 500μL du surnageant ont été prélevés et remplacés par la même quantité de milieu frais. Les surnageant ont été analysés par spectrofluorimétrie, afin de quantifier la libération de βCDP-rhodamine dans le milieu. Les résultats ont montré que seulement 11,6 % de βCDP-rhodamine liés aux nanoparticules est libéré après 24 h d'incubation dans du PBS et ce pourcentage ne dépasse pas 16,1 % dans l' RPMI 10 % SBF (fig. 9). Il peut être conclu que la couronne externe de βCDP est stable dans des conditions physiologiques, probablement grâce à l'effet coopératif entre les quatre groupes phosphates qui complexent le fer.

*Effet de la couronne externe à base de βCDP*

**Exemple 8: Stabilité dans l'eau des MIL-100**

2 mg de MIL-100 ont été incubés avec 500 μl d'une solution aqueuse de βCDP 2 mg/ml (rapport en poids nanoparticules : βCDP = 1:0, 5) 24 h, sous agitation, à température ambiante. À la fin de l'incubation, les MIL-100 ont été récupérés par centrifugation à 5600xg 10 minutes, lavés à l'eau et re-suspendues dans 1 ml d'eau. 2 mg de MIL-100 non modifiées, utilisées comme le contrôle, ont été incubées dans 1 ml d'eau. La taille des nanoparticules a été évaluée après différents temps d'incubation (1-2-3 jours) par DLS (Zetasizer Nano 6,12, Malvern Instruments Ltd., UK). Les résultats ont montré que, tandis que les nanoparticules non modifiées s'agrègent immédiatement dans l'eau, une fois modifiées avec le βCDP elles gardent une taille petite et constante pendant 3 jours d'incubation (fig. 10). Il peut en être conclu que la couronne externe de βCDP confère une bonne stabilité dans l'eau aux MIL-100.

**Exemple 9 ; Utilisation des MIL-100 pour libération de l'AZT-TP après modification avec de la βCDP ou du MeO-PEG-$NH_2$**

2,5 mg de MIL-100 ont été incubées avec 500 μl d'une solution aqueuse d'azidothymidine triphosphate (AZT-TP, triples) 400ug/ml, contenant 1 % d'AZT-TP[$^3$H] (AZT-TP-méthyl [$^3$H], Moravek), 24 h, sous agitation, à température ambiante. À la fin de l'incubation, les nanoparticules ont été récupérées par centrifugation à 5600xg 10 min, et le surnageant a été analysé par un compteur à scintillation, afin de calculer le taux de médicament encapsulé. Les nanoparticules ont ensuite été incubées avec 500 μl d'une solution aqueuse de βCDP 2,5 mg/ml (rapport en poids nanoparticules: bCDP = 1:0, 5), 500 μl d'une solution aqueuse de MeO-PEG-NH2 1,67 mg/ml (rapport en poids nanoparticules: PEG = 1:0,33) ou 500μl d'eau (échantillon contrôle), 3 h, sous agitation, à température ambiante. À la fin de l'incubation, nous avons récupéré les nanoparticules par centrifugation à 5600xg 10 min et nous avons analysé le surnageant à l'aide d'un compteur à scintillation pour quantifier la libération de médicaments après la modification de surface. Enfin les nanoparticules ont été incubées dans 1 ml de PBS à 37 °C sous agitation. Après différents temps d'incubation la suspension a été centrifugée 10 min à 5600xg, 500μl de surnageant ont été prélevés et remplacés par une même quantité de milieu frais. Le surnageant a été analysé par un compteur à scintillation afin d'évaluer la libération de l'AZT-TP par des nanoparticules non modifiées, modifiées avec de la βCDP ou du MeO-PEG-NH2. Les résultats résumés dans la fig. 11 montrent que la modification de surface avec de la βCDP n'affecte pas la libération du médicament, probablement parce que, comme il a été déjà précisé, ils interagissent seulement avec la surface de nanoparticules sans perturber la porosité interne des particules. Au contraire, après de la modification avec du MeO-PEG-$NH_2$ un «burst effect » (libération rapide, non contrôlée) a été observé. Les chaînes linéaires de PEG peuvent pénétrer à l'intérieur des pores MIL-100 et remplacer les molécules de l'AZT-TP encapsulées, favorisant leur libération une fois dans le PBS.

**Exemple 10 :Cytotoxicité des nanoparticules de MIL-100 non modifiées et modifiées avec de la βCDP**

La ligne cellulaire de macrophages murines, J774, (ECACC no 91051511) a été cultivées dans du RPMI 1640 GlutaMAX ™ additionné avec du sérum foetal bovin inactivé (10%(v/v)) de la pénicilline (100 UI/mL) et de la streptomycine (100 μg/mL). La cytotoxicité des MIL-100 vers cette ligne a été déterminée par un test MTT en plaques de 96 puits ($10^4$ cellules par puits) après 48 h d'incubation avec des solutions de nanoparticules différemment concentrées. Les résultats ont montré que les MIL-100 n'ont aucun effet cytotoxique significatif à des concentrations élevées (100 mM), et les mêmes résultats ont été observes après modification avec de la βCDP.

Pénétration des MIL-100 dans la lignée cellulaire J774

Pour cette analyse les MIL-100 ont été marquées avec un fluorophore: l'adénosine 5'-triphosphate BODIPY-FL (ATP-BODIPY, adénosine 5'-triphosphate, BODIPY ® FL, Invitrogen). 1,25 mg de MIL-100 ont été incubés avec 500μL d'une solution aqueuse d'ATP-BODIPY 10 nmol/ml, 1 h, sous agitation, dans le noir, à température ambiante. À la fin de l'incubation, les nanoparticules ont été récupérées par centrifugation à 5600xg 10 min, et nous avons analysé le surnageant par spectrofluorimétrie afin de quantifier le taux d'absorption du fluorophore. Les nanoparticules ont ensuite été incubées dans 500μl d'eau (échantillon non modifié) ou d'une solution aqueuse de βCDP 1,25 mg/ml, 1 h sous agitation, à température ambiante, dans le noir. À la fin de l'incubation, les nanoparticules ont été récupérées par centrifugation à 5600xg 10 min et re-suspendues dans du RMPI 10% SFV à la concentration finale de 50 μg/ml. Les cellules de la ligne J774 ont été précédemment cultivées dans des plaques de 24-puits (5 x $10^4$ cellules par puits) sur une lamelle de verre stérile. Après 24 h d'incubation dans un incubateur humidifié à 37 ° C, remplacé le milieu de culture a été remplacé par 1 ml des solutions de nanoparticules chargées avec de l'ATP-BODIPY (non modifiées ou modifiées en surface), ou d'ATP-BODIPY libre. Après différents temps d'incubation (15 min, 1 h, 4 h, 24 h), le milieu de culture a été aspiré et les cellules fixées. Par CLSM, il a été observé que les MIL-100 sont très rapidement intériorisées, probablement par phagocytose, dans les cellules, après seulement 15 minutes d'incubation. Le même comportement a été observé dans le cas de nanoparticules modifiées en surface avec de la βCDP. Au contraire, l'ATP-BODIPY libre, en raison de son caractère hydrophile, n'est pas capable de traverser la membrane cellulaire et aucune fluorescence n'a été observée dans les cellules, ce qui démontre le rôle actif des MIL-100 dans l'internalisation du fluorophore (fig. 13).

**Exemple 11** : **Exemple comparatif de modification de la surface d'un MOF avec un PEG non-fonctionalisé avec un groupement complexant selon l'invention : porosité et libération de principes actifs**

Les chaînes PEG utilisées dans l'art antérieur [2] pour fonctionnaliser la surface des MOFs pénètrent aisément dans les pores du matériau, conduisant ainsi à une perte très significative de la porosité accessible, et donc de la capacité à encapsuler les molécules actives. Par ailleurs, il a été mis en évidence que les chaînes de PEG délogeaient les molécules actives encapsulées qui étaient ainsi libérées de manière incontrôlée.

**Exemple 12 : Exemple comparatif de modification de la surface d'un MOF avec un dextrane-biotine non-fonctionnalisé avec un groupement complexant selon l'invention**

Il a été mis en évidence récemment que les recouvrements basés sur des interactions hydrophobes (dextrane-biotine) [2] n'étaient pas stables dans les solutions de tampon phosphate. Ces recouvrements ont été réalisés en incubant les nanoparticules de MIL 100 avec une solution de dextrane-biotine (Sigma Aldrich) pendant une heure suivi de la récupération des nanoparticules par centrifugation à 9500 g et resuspension dans l'eau ou dans le PBS. En effet, au bout d'une demi-heure, 30% du recouvrement disparaissait. Au bout d'une heure, plus de 40% du dextrane était décroché et 70% au bout de 24 heures. Cette instabilité du recouvrement n'est pas adaptée pour des applications biomédicales in vivo.

**Exemple 13: Modification superficielle des nanoparticules de MIL-100 avec du poly-βCDP :**

2 mg de MIL-100 ont été modifiés par incubation avec 500 μl d'une solution aqueuse de poly-βCDP-rhodamine (1 :10, 1 phosphate/10 cyclodextrines) 2 mg/ml (rapport en poids nanoparticules:βCDP = 1:0.5), 24 h sous agitation à température ambiante. Après incubation, nous avons récupéré les nanoparticules modifiées par centrifugation (9500 g, 10 min) et le surnageant a été analysé par spectrofluorimétrie afin de définir la quantité de poly-βCDP-rhodamine asociée aux nanoparticules. Les résultats obtenus indiquent que la quantité de βCDP-rhodamine associée aux MIL-100 représente 34(±0,5) % p/p. Les nanoparticules modifiées ont été lavées avec 1 ml d'eau, afin d'éliminer l'excès de poly-βCDP-rhodamine et enfin incubées dans 1 ml de PBS (Phosphate Buffered Saline, Lonza) à 37 °C, sous agitation à la concentration de 2 mg/ml. Après différents temps d'incubation (0,3-6-24 h) la suspension a été centrifugée 10 min à 9500 g, puis 500μL du surnageant ont été prélevés et remplacés par la même quantité de milieu frais. Les surnageants ont été analysés par spectrofluorimétrie, afin de quantifier la libération de poly-βCDP-rhodamine dans le milieu. Les résultats ont montré que moins de 20% de βCDP-rhodamine liés aux nanoparticules sont libérés après 4 h d'incubation dans du PBS (fig. 17). Il peut être conclu que la couronne externe de poly-βCDP-rhodamine est stable dans des conditions physiologiques, probablement grâce à l'effet coopératif de plusieurs groupes phosphates qui se coordinent aux nano-MOFs.

**Exemple 14 : Modification superficielle post-synthèse des nanoparticules MIL-100 avec du chitosane.**

**Concept:**

Les nanoparticules hybrides organiques-inorganiques (nanoMOF) à base de trimésate de fer (MIL-100), ont été modifiées avec le chitosane.

Le chitosane est un polysaccharide d'origine naturelle, obtenu par déacétylation de la chitine, dont la formule est donnée ci-dessous (DDA est le degré de déacétylation du chitosane. (DDA : degré de déacétylation)).

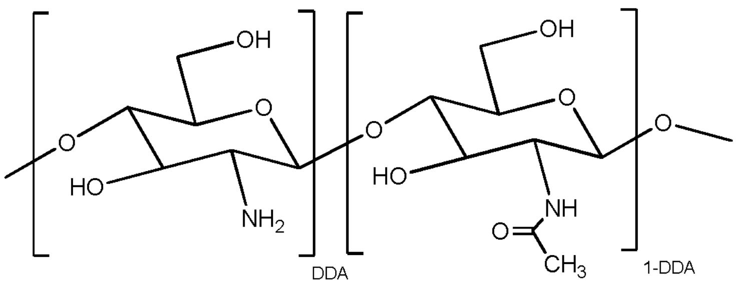

Ce polymère, constitué d'unités glucosamine liées entre elles par des liaisons β(1-4), présente l'avantage d'être biodégradable et biocompatible. De plus, il présente des propriétés bioadhésives. Le chitosane insoluble dans l'eau peut être solubilisé en milieu acide tel qu'une solution d'acide acétique à 1% (AA1%); les groupements amines répartis le long de la chaîne polymère sont alors protonés et le polymère est chargé positivement.

Les nanoparticules de MIL-100 comportent des cations $Fe^{3+}$ et aussi des groupements COOH provenant de l'acide trimésique utilisé pour leur synthèse (pKa 3,4).

La modification de surface par le chitosane se base sur la multitude d'interactions électrostatiques entre le polymère et les nanoparticules de MIL-100 en milieu AA1% **(Fig.18).**

L'interaction entre le chitosane et les nanoparticules peut être renforcée par addition d'un sel tel que le $Na_2SO_4$ qui entraîne la perte de solubilité du chitosane et permet d'augmenter la quantité de polymère accroché à la surface des nanoparticules.

**Méthodes :**

Synthèse de nanoparticules de trimésate de fer MIL-100: *décrite au paragraphe* ***a*** *de l'Exemple 2.*

Les nanoparticules MIL-100 ont été obtenues par synthèse hydrothermale sous irradiation micro-ondes (Mars 5, CEM), en chauffant une suspension aqueuse contenant 20 mL d'eau désionisée, 8,97 mmol de $FeCl_3$ (FeCl3•6H2O, 98 % Alfa Aesar) et 4,02 mmol d'acide 1,3,5-benzènetricarboxylique (BTC, Sigma Aldrich) pendant 6 min à 130 °C sous agitation.

À la fin de la réaction les nanoparticules ont été récupérées par centrifugation à 5600xg pendant 15 min. L'activation des particules (càd l'extraction du BTC des pores) a été réalisée par 6 lavages consécutifs avec 30 mL d'Ethanol absolu. Une fois activées, les nanoparticules de taille <300 nm et monodisperses (PDI<0,2) ont été re-dispersées dans de l'ethanol.

Modification superficielle de MIL-100 avec le chitosane:

5 mg de MIL-100 ont été modifiés par incubation avec 500 μL d'une solution aqueuse de chitosane à 2,5 mg/mL dans l'acide acétique 1% (rapport massique nanoparticules/chitosane = 4/1). Après homogénéisation (vortex et ultrasons), les échantillons sont placés sous agitation rotative pendant 24H. Les nanoparticules sont ensuite centrifugées 16 870 xg/20 min/ 25°C. Un culot uniforme est obtenu. Il est resuspendu dans 200μL d'AA1% puis l'échantillon est à nouveau centrifugé afin d'éliminer l'excès de chitosane. Le culot de nanoparticules MIL-100/chitosane est au final repris dans 1mL d'eau milliQ.

Dans certains cas, d'autres rapports massiques nanoparticules / chitosane ainsi que d'autres temps d'incubation ont été testés.

**Caractérisation physico-chimique des nanoparticules MIL-100/chitosane :**

Diamètre moyen et stabilité colloïdale:

La taille des nanoparticules a été mesurée par diffusion quasi-élastique de la lumière, (Nanosizer, Malvern, France). Le tableau 3 présente les résultats obtenus le jour de la formation des MIL-100/chitosane (j0) et après 30 jours de conservation à température ambiante (20°C). L'analyse a montré une légère augmentation du diamètre moyen des nanoparticules après recouvrement par le chitosane (213 ±3 nm *vs* 178 ±3 nm). De même, l'indice de polydispersité passe de 0,05 pour les MIL-100 à 0,26 pour les MIL-100/chitosane. Après 30 jours de conservation, une agrégation des particules a été observée dans l'échantillon non modifié. En revanche, les nanoparticules MIL-100 recouvertes de chitosane ont conservé leur diamètre moyen.

**Tableau 3** : Diamètre moyen des nanoparticules MIL-100 recouvertes ou non de chitosane.

| | j0 | | j30 | |
|---|---|---|---|---|
| | d (nm) | IP | d (nm) | IP |
| MIL 100 | 178 ± 3 | 0,05 | 973±144 | 0,50 |
| MIL 100_Chitosane | 213 ± 3 | 0,26 | 187±3 | 0,11 |

La stabilité colloïdale des nanoparticules a aussi été évaluée en diluant les suspensions d'un facteur 100 dans l'eau milliQ et en suivant l'évolution du diamètre moyen dans l'échantillon dilué **(Fig. 19).**

Il a été observé que 2 heures après dilution dans l'eau, les MIL-100 s'agrégeaient en absence de recouvrement. Le diamètre moyen passait de 160 nm à 360 nm après 2 h et atteignait 1100 nm après 4H dans l'eau. En revanche, dans le cas des MIL-100/chitosane, les nanoparticules gardaient un diamètre constant au cours du temps voisin de 170 nm.

Stabilité du recouvrement :

Des nanoparticules MIL-100/chitosane ont été préparées en utilisant du chitosane marqué à la rhodamine isothiocyanate (chitosane-RITC) avec un rapport massique MIL-100/chitosane : 4/1. Après mise en contact des MIL-100 et de la solution de chitosane pendant 1, 2 et 24 h, les échantillons ont été centrifugés, purifiés et re-suspendus dans l'eau milliQ comme décrit précédemment (cf méthodes). Les surnageants issus de la centrifugation des échantillons ainsi que de la purification ont été analysés par spectrofluorimétrie. Il a été montré que le chitosane était associé aux

MIL-100 avec un taux voisin de 2% p/p.

Les nanoparticules marquées à la rhodamine-ITC ont été re-suspendues dans 1 mL de tampon phosphate pH 7.4. Après 24 h d'incubation à TA dans ce milieu, les échantillons ont été centrifugés et les surnageants ont été analysés par spectrofluorimétrie. Le chitosane-RITC n'a pas été détecté dans les surnageants indiquant que l'association MIL-100/chitosane était stable dans les conditions expérimentales testées.

DRX

Les nanoparticules MIL-100 non modifiées et modifiées avec le chitosane ont été séchées à 100°C pendant 18 h puis leur structure a été analysée par diffraction des rayons X. Les analyses de diffraction des rayons X ont montré que le procédé de modification de la surface des nanoparticules MIL-100 par le chitosane n'altérait pas la structure cristalline du matériau **(Fig.20).**

Porosimétrie d'adsorption d'azote des MIL-100 après modification avec le chitosane

30 mg de nanoparticules ont été incubés avec 3 mL d'une solution aqueuse de chitosane à 2,5 mg/mL (rapport massique nanoparticules/chitosane = 4/1). Après 24 h de contact, l'excès de chitosane a été éliminé par centrifugation à 16870xg/20 min/25°C. Les nanoparticules ont été purifiées comme décrit précédemment puis elles ont été séchées à 100 °C pendant 18 h. Un échantillon témoin de MIL-100 (sans chitosane) a été préparé de la même façon que l'échantillon MIL-100/chitosane. La porosité des matériaux MIL-100 et MIL-100/chitosane a été analysée par adsorption d'azote à 77K.

Les résultats ont montré que la surface spécifique des nanoparticules de MIL-100 n'était pas modifiée par le recouvrement de chitosane. Ainsi, les MIL-100 ont montré une surface de Langmuir de 2100 $m^2/g$ tandis que les MIL-100/chitosane ont présenté une surface de Langmuir égale à 2030 $m^2/g$. Cela démontre également que le chitosane ne bloque pas l'accès des pores et donc cela est en accord avec la présence du chitosane uniquement à la surface des particules.

Encapsulation et libération de l'AZT-TP

Des nanoparticules modifiées avec du chitosane ont été incubées avec une solution d'AZT-TP. Après 24 h de contact, les échantillons ont été centrifugés et l'AZT-TP encapsulé a été déterminé. Il a été montré que même après modification, les nanoparticules de MIL-100 conservaient leur capacité à encapsuler l'AZT-TP. Le rendement d'encapsulation était de 99% correspondant à un taux d'association aux nanoparticules de 7,8 % (p/p). Ces résultats étaient similaires à ceux obtenus pour les MIL-100 en absence de tout recouvrement. Par ailleurs, des nanoparticules de MIL-100 encapsulant l'AZT-TP ont été modifiées par le chitosane (modification post-encapsulation). Il a été montré que le procédé de modification ne délogeait que 4% du PA encapsulé et ce, par un effet de dilution dû au procédé de recouvrement.

La libération de l'AZT-TP à partir des MIL-100 modifiées post-encapsulation a été étudiée en milieu PBS pH 7.4 à 37°C et comparée à celle à partir de MIL-100 traitées dans les mêmes conditions que les MIL-100/chitosane. Les résultats obtenus sont présentés sur la **Fig.21.** Ils montrent que la libération du principe actif n'est pas altérée par le recouvrement de chitosane.

**Augmentation de la quantité de chitosane à la surface des nanoparticules MIL-100 :**

5 mg de MIL-100 ont été modifiés par incubation avec 500 µL d'une solution aqueuse de chitosane à 2,5 mg/mL dans l'acide acétique 1% (rapport massique nanoparticules/chitosane = 4/1). Après homogénéisation des échantillons (vortex et ultrasons) permettant l'interaction chitosane / MIL-100, une solution de $Na_2SO_4$ a été ajoutée goutte à goutte à l'échantillon placé dans un bain à ultrasons à température ambiante. Des microparticules ont ainsi été obtenues **(Fig.22).** Elles ont été recueillies par centrifugation douce (1380 xg/ 1 min/ 25°C). Un culot uniforme est obtenu. Les microparticules ont été ensuite purifiées par lavage à l'eau désionisée et centrifugation (1380xg/ 1min/ 25°C). L'opération a été répétée 3 fois.

Dans certains cas, d'autres rapports massiques nanoparticules / chitosane ont été testés.

**Caractérisations physico-chimiques des microparticules**

Taille et morphologie des particules :

La taille des microparticules a été mesurée par granulométrie laser en milieu liquide (Mastersizer 2000, Malvern,

France). Elles ont présenté un diamètre moyen de 6,4 μm avec une distribution étroite comme indiqué par l'indice Span égal à 1,5. (**Fig. 23, tableau 4**). 10 % des particules ont un diamètre inférieur à 3,3 μm et 90% des particules ont un diamètre inférieur à 12,6 μm.

**Tableau 4** : Paramètres granulométriques d'une suspension de microparticules MIL-100/chitosane. Rapport massique MIL/chitosane : 20/1.

| D (0,1) μm | D(0,5) μm | D(0,9) μm | Span |
|---|---|---|---|
| 3,3 | 6,4 | 12,6 | 1,5 |

Microanalyse élémentaire :

Des microparticules ont été préparées à partir de 5 mg de MIL-100 et de 0,25 mg de chitosane. Après purification, le culot de microparticules a été séché à 100°C pendant 18 h puis analysé par microanalyse élémentaire (**tableau 5**). L'atome d'azote étant retrouvé spécifiquement dans le chitosane, il a été possible de quantifier sa présence dans les microparticules. L'analyse a montré que tout le chitosane était retrouvé associé au MIL 100.

**Tableau 5** : Pourcentages de C, H et N déterminés par microanalyse élémentaire dans les échantillons de MIL-100, de chitosane et de microparticules MIL-100/chitosane.

| | MIL-100 | chitosane | MIL-100/chitosane |
|---|---|---|---|
| | % mesuré | % mesuré | % mesuré |
| carbone | 36,5 | 45,5 | 31,7 |
| hydrogène | 2,7 | 6,7 | 2,1 |
| azote | 0 | 8,5 | 0,4 |

Stabilité de l'association MIL-100/chitosane :

Des microparticules ont été préparées en utilisant du chitosane marqué à la rhodamine isothiocyanate (chitosane-RITC) avec un rapport massique MIL-100/chitosane : 4/1 ; 8/1 et 20/1. Après formation des microparticules, les échantillons ont été centrifugés et lavés à l'eau désionisée comme décrit précédemment. Les surnageants issus de la centrifugation des échantillons ainsi que les différents lavages ont été analysés par spectrofluorimétrie. Il a été montré qu'ils ne contenaient pas de chitosane, ce dernier étant totalement associé aux MIL-100 pour former les microparticules. Ce résultat confirme les résultats de microanalyse élémentaire.

Les microparticules marquées à la rhodamineITC ont été re-suspendues dans 1 mL de tampon phosphate pH 7.4. Après 24 h d'incubation à TA dans ce milieu, les échantillons ont été centrifugés et les surnageants ont été analysés par spectrofluorimétrie. Le chitosane -RITC n'a pas été détecté dans les surnageants indiquant que l'association MIL/chitosane était stable dans le tampon phosphate.

Spectroscopie Infra-rouge :

Les microparticules ont été séchées à 100°C pendant 18H puis analysées par FT-IR. Les spectres obtenus montrent clairement la présence d'une bande d'absorption caractéristique du chitosane vers 1030 $cm^{-1}$ (bande d'élongation de la liaison éther C-O-C) dans les échantillons de microparticules. (Fig. 24)

DRX

Les matériaux MIL-100 et MIL-100/chitosane, ont été séchés 18H à 100°C. Leur structure a été analysée par diffraction à rayons X (diffractomètre à haute résolution Siemens D5000 X'Pert MDP (θ-2θ) (λCu, Kα1, Kα2). Les résultats ont montré la méthode de modification de la surface des MIL 100 avec le chitosane ne modifie pas la structure cristalline des MIL-100 **(Fig. 25).**

Encapsulation et libération de l'AZT-TP

Des particules micrometriques modifiées avec du chitosane ont été incubées avec une solution d'AZT-TP. Après 24 h de contact, les échantillons ont été centrifugés et l'AZT-TP encapsulé a été déterminé. Il a été montré que

même après modification, les particules de MIL-100 conservaient leur capacité à encapsuler l'AZT-TP. Le rendement d'encapsulation était de 99% correspondant à un taux d'association de 7,8 % (p/p). Ces résultats étaient similaires à ceux obtenus pour les MIL-100 en absence de tout recouvrement.

Des microparticules MIL-100/chitosane chargées en AZT-TP ont aussi été préparées à partir des nanoparticules encapsulant l'AZT-TP (modification post-encapsulation). Il a été montré que le procédé de modification (post-encapsulation de l'AZT-TP) ne délogeait que 0,8% du PA encapsulé et ce, par un effet de dilution dû au procédé de recouvrement.

La libération de l'AZT-TP à partir des microparticules MIL-100/chitosane (modification post-encapsulation) a été étudiée à 37°C en milieu PBS pH 7.4. Les résultats obtenus sont présentés sur la **Fig.26.** Ils montrent une libération plus lente du principe actif du fait de la présence du chitosane.

**Exemple 15: Modification superficielle post-synthèse des nanoparticules MIL-100 avec du bioconjugué PEG-alendronate-dextrane**

**Concept :**

Un bioconjugué PEG-alendronate-dextrane a été synthétisé (Fig. 27) en vue de la modification superficielle des MOFs. Cet exemple concerne celles à base de trimésate de fer (MIL-100).

Le dextrane est un polysaccharide hydrophile linéaire constitué d'unités de glucose liées entre elles par des liaisons $\alpha$-(1,6). Ce polymère présente l'avantage d'être neutre, soluble dans l'eau, biocompatible et biodégradable. De plus, il est utilisé pour des applications biomédicales et donne la possibilité de greffer différents ligands d'intérêt pour le ciblage.

Le couplage des chaines de polyéthylène glycol (PEG) et des molécules d'alendronate sur le squelette du dextrane se base sur des réactions « click ». Concrètement, nous avons employé la variété catalysée par du cuivre (I) de la cycloaddition 1,3-dipolar Huisgen d'azides et d'alcynes, pour la formation de 1,2,3-triazoles. A fin de pouvoir utiliser cette réaction « click », nous avons synthétisé d'abord les dérivés azido-PEG, azido-alendronate et alcyne-dextrane.

L'alendronate est une molécule utilisée pour le traitement de l'ostéoporose. Le rôle de l'alendronate sera d'assurer l'encrage et la stabilité du recouvrement de dextrane-PEG à la surface des nanoMOF, par coordination avec le Fer.

**Méthodes :**

**Abréviations**

Acétate d'éthyle (AcOEt), diméthylformamide (DMF), dichlorométhane (DCM), N-hydroxysuccinimide (NHS), 1-éthyl-3-(3'-diméthylaminopropyl)- N-(3-diméthylaminopropyl)-N'-éthylcarbodiimide hydrochloride (EDC), polyéthylène glycol (PEG), 4-Diméthylaminopyridine (DMAP), tétrahydrofurane (THF), 1,1-carbonyldiimidazole (DCI), Poids moléculaire (PM), chromatographie d'exclusion stérique (d'après l'anglais Size Exclusion Chromatography, SEC).

**Matériels**

Tous les réactifs et solvants ont été obtenus des sources commerciales indiquées entre parenthèses et ont été utilisés sans aucunes purifications additionnelles autrement stipulées. Acide 6-bromohexanoique (97%, Aldrich), $NaN_3$ (99%, Acros Organics), alendronate sodique (Molekula), NHS (purum, Fluka), EDC (Sigma-Aldrich), MePEG (Sigma-Aldrich), THF (Merck), Dextrane T40 (masse moyenne PM 40000 Daltons, Pharmacosmos, SEC PM (g/mol) 45360$\pm$8.0%), LiCl (99%, Acros), DCI (Aldrich), propargylamine (98%, Aldrich), 2-propanol (VWR), $CuSO_4(5H_2O)$ (Prolabo), L-ascorbate sodique ($\geq$98%, Sigma-Aldrich), $Et_2O$ (Carlo Erba), AcOEt (Acros Organics), acetonitrile (Carlo Erba), DCM (Carlo Erba), EtOH (Carlo Erba), DMF anhydre a été directement distillé, de l'eau dé-ionisée Milli-Q a été obtenue d'un appareil Millipore avec un filtre de 0.22$\mu$m, tous les solvants deuterés (Euriso-top).

**Synthèse du fragment azido-alendronate**

L'azido-alendronate a été synthétisé à travers une procédure synthétique de trois étapes utilisant l'acide 6-bromohexanoique comme produit de départ.

1. Synthèse de l'acide 6-azidohexanoique

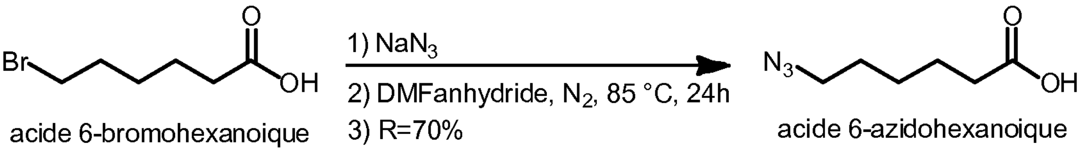


acide 6-bromohexanoique

acide 6-azidohexanoique

L'acide 6-azidohexanoique a été synthétisé sur la base des méthodes déjà publiées, en optimisant les procédures décrites.[1,2] Concrètement, l'acide 6-bromohexanoique (PM 195,05g/mol, 1.5 g, 7.7 mmol, 1 equiv.) a été dissous dans du DMF anhydride (10 mL). Ensuite, $NaN_3$ (PM 65.01, 1 g, 15.4 mmol, 2 équiv.) a été ajouté. Ce mélange a été chauffé à 85 °C sous agitation et sous $N_2$ pendant 24h. À la fin de la réaction le DMF a été évaporé sous vide. Le solide résultant a été dissous dans l'eau (20 mL) et extrait avec AcOEt (30 mL x3). La phase organique a été séchée avec $MgSO_4$, filtrée et concentrée sous vide pour donner l'acide 6-azidohexanoique sous la forme d'une huile légèrement jaune (0.872 g, 5.5 mmol, 70% de rendement). ESI-MS/MS (m/z) 156 (M-H)$^-$; $^1$H RMN (400 MHz, $^3J_{HH}$ (Hz), $CDCl_3$) $\delta$: 11.04 (s, 1H, COO*H*), 3.24 (t, $^3J_{HH}$ 6.9, 2 H, $CH_2N_3$), 2.32 (t, $^3J_{HH}$ 7.3, 2H, $CH_2COOH$), 1.70-1.50 (m, 2H chacun, $CH_2CH_2COOH$, $CH_2CH_2N_3$), 1.40 (m, 2H, $CH_2CH_2CH_2N_3$) ; $^{13}$C RMN (100 MHz, $CDCl_3$) $\delta$: 179.1 1 (*C*OOH), 51.2 ($CH_2N_3$), 34.0 ($CH_2COOH$), 28.6 ($CH_2CH_2N_3$), 26.2 ($CH_2CH_2CH_2N_3$), 24.3 ($CH_2CH_2COOH$).

1] Aleman EA, Pedini HS, Rueda D, ChemBioChem, 2009, 10, 2862-2866
[2] Kuil J, Branderhorst HM, Pieters RJ, de Mol NJ, Liskamp RM, Org. Biomol. Chem., 2009, 7, 4088-94

2. Synthèse de 2,5-dioxopyrrolidin-1-yl-6-azidohexanoate

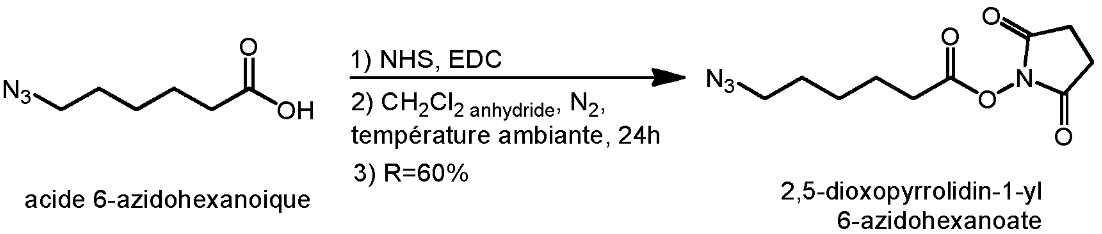


acide 6-azidohexanoique

2,5-dioxopyrrolidin-1-yl 6-azidohexanoate

2,5-dioxopyrrolidin-1-yl-6-azidohexanoate a été synthétisé sous la base des méthodes déjà publiées, en optimisant la procédure.[3,4] Concrètement, l'acide 6-azidohexanoique (PM 157 g/mol, 0.6 g, 3.82 mmol, 1 équiv.) a été dissous dans le DCM anhydride (15 mL). Ensuite, le NHS (PM 115.05, 0.48g, 4.2 mmol, 1.1 equiv.) a été ajouté sous atmosphère d'azote et mélangé sous agitation pendant 5 min. Postérieurement, l'EDC (PM 191.7, 0.8 g, 4.2 mmol, 1.1 equiv.) a été ajouté. Ce mélange a été placé sous une atmosphère d'azote et agité à température ambiante pendant 24h. À la fin de la réaction, le brut de la réaction a été rincé avec une solution aqueuse de HCl 1N (20 mL, x2) et une solution de $NaHCO_3$ saturée (20 mL, x2). Ensuite, les phases aqueuses obtenues ont été nettoyées avec le DCM (20 mL, x2). Ensuite, les phases organiques ont été mélangées, et la phase organique résultante a été séchée avec du $MgSO_4$, filtrée et concentrée sous vide à sec. Le produit a été purifié par chromatographie de gel de silice (DCM pur comme phase mobile). Le produit a été obtenu sous forme de liquide transparent (0.59 g, 2.32 mmol, 60% de rendement). $^1$H RMN (300 MHz, $^3J_{HH}$ (Hz), $CDCl_3$) $\delta$: 3.23 (t, $^3J_{HH}$ 6.74, 2H, $CH_2N_3$), 2.75 (s, 4H, $COCH_2CH_2CO$), 2.56 (t, $^3J_{HH}$ 7.34, 2H, $CH_2COON$), 1.90-1.30 (m, 6H, $CH_2CH_2CH_2CH_2N_3$) ; $^{13}$C NMR (75 MHz, $CDCl_3$) $\delta$: 169.1 (N*C*OCH$_2$), 168.4 (CH$_2$*C*OON), 51.1 ($CH_2N_3$), 30.8 ($CH_2COON$), 28.4 ($CH_2$), 25.9 ($CH_2$), 25.6 (2CO$CH_2CH_2$CO), 24.1 ($CH_2$).

[3] Liu XM, Lee HT, Reinhardt RA, Marky LA, Wang D., Journal of Controlled Release, 2007, 122, 54-62
[4] Grandjean C, Boutonnier A, Guerreiro, C, Fournier J-M, Mulard L-A, J.Org. Chem., 2005, 70, 7123-7132.

3. Synthèse de 4-(6-azidohexanamido)-1-hydroxy-1-phoshphonobutyl)phosphonate hydrogène sodique (composé azido-alendronate)

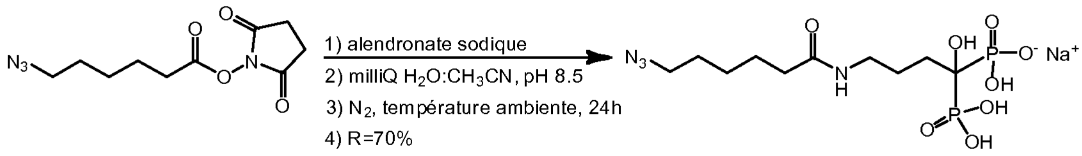


2,5-dioxopyrrolidin-1-yl 6-azidohexanoate

(4-(6-azidohexanamido)-1-hydroxy-1-phosphonobutyl) phosphonate sodium hydrogène sodique

Le composé azido-alendronate a été synthétisé sous la base des méthodes déjà publiées, en optimisant la procédure.[5,6] Concrètement, l'alendronate sodique (PM 271.08, 0.445 g, 1.64 mmol, 1 equiv.) a été dissout dans l'eau dé-ionisée (10 mL). Ensuite, une solution aqueuse de NaOH (0.1 M, ~ 20 mL) a été ajoutée goutte à goutte jusqu'à un pH de ~8.5. À ce moment-là, 2,5-dioxopyrrolidin-1-yl-6-azidohexanoate (0.514 g, 2 mmol, 1.2 equiv.) a été dissout dans d'acetonitrile (10 mL) et ajouté dans la solution aqueuse en 4 portions toutes les 15 min. Ce mélange a été placé sous agitation à température ambiante. Avant l'incorporation de chaque portion, le pH a été réajusté entre 8 et 8.5 en obtenant un volume final d'environ 60 mL. Après avoir additionnée la dernière portion, la réaction a été placée sous agitation à température ambiante pendant 24h. À la fin de la réaction la solution a été concentrée sous vide à sec. Postérieurement, le produit a été dissout dans de l'eau (3 mL) et précipité avec l'EtOH (30 mL). Le produit solide a été récupéré et séché pour obtenir finalement le produit approprié sous forme solide, blanche (0.47 g, 1.15 mmol, 70% de rendement). $^1H$ RMN (400 MHz, $^3J_{HH}$ (Hz), $D_2O$) $\delta$: 3.32 (t, $^3J_{HH}$ 6.8, 2H, $CH_2N_3$), 3.19 (t, $^3J_{HH}$ 7.0, 2H, $CONHCH_2$) 2.25 (t, $^3J_{HH}$ 7.4, 2H, $CH_2CONH$), 2.00-1.72 (m, 4H, $CH_2CH_2C(PO_3H_2)_2(OH)$), 1.67-1.54 (m, 2H chacun, $CH_2CH_2CONH$, $CH_2CH_2N_3$), 1.38 (m, 2H, $CH_2CH_2CH_2N_3$) ; $^{13}C$ RMN (100 MHz, $^2J_{CP}$, $^3J_{CP}$ (Hz), $D_2O$) $\delta$: 177.4 (CONH), 74.5 (t, $^1J_{CP}$ 129.0, $C(PO_3H_2)_2(OH)$, 51.6($CH_2N_3$), 40.8 ($CONHCH_2$), 36.3 ($CH_2CONH$) 32.1 ($CH_2C(PO_3H_2)_2(OH)$),), 28.3 ($CH_2CH_2N_3$), 26.1 ($CH_2CH_2CH_2N_3$), 25.5 ($CH_2CH_2CONH$), 24.2 (t, $^3J_{CP}$ 6.5, $CH_2CH_2C(PO_3H_2)2(OH)$); $^{31}P$ (162 MHz, $D_2O$) $\delta$: 19.25 (2*P*).

[5] Hein CD, Liu X-M, Chen F, Cullen DM, Wang D, Macromol. Biosci. 2010, 10, 1544-1556.
[6] Liu X-M, Lee H-T, Reinhardt R-A, Marky LA, Wang D, J. Control. Release 2007, 122, 54-62.

**Synthèse du composé azido-PEG**

1. Synthèse de méthanesulfonate de méthoxy poly(éthylène glycol) ($MePEG_{43}Ms$)

Une solution de $MeOPEG_{43}OH$ (35 g, 17.5 mmol), DMAP (427 mg, 3.5 mmol) et $Et_3N$ distillée (4.05 g, 40 mmol) en DCM (40 mL) a été refroidie à 0 °C. Ensuite, le MsCl (4.01 g, 35 mmol) a été ajouté goutte à goutte avec une seringue pendant 15 min. Le mélange a été agité à 0 °C pendant 30 min puis à température ambiante pendant toute la nuit. À la fin de la réaction le mélange a été rincé trois fois avec une solution aqueuse de HCl (0.5 M) et une fois avec de la saumure. Ultérieurement, la phase organique a été séchée sur $MgSO_4$, filtrée et concentrée sous vide en obtenant le $MePEG_{43}OMs$ sous forme de poudre blanche (28.36 g; (81% de rendement). IR (neat, $cm^{-1}$) v = 2890, 1467, 1340, 1279, 1240, 1175, 1147, 1105, 1059, 1017, 960; $^1H$ RMN (300 MHz, $CDCl_3$) $\delta$: 4.19 (m, 2 H, $CH_2SO_3CH_3$), 3.70 - 3.21 (m, 224 H, $OCH_2CH_2O$), 3.19 (s, 3 H, $CH_3O$-PEG), 2.91 (s, 3 H, $CH_2SO_3CH_3$) ; $^{13}C$ RMN (75 MHz, $CDCl_3$) $\delta$: 71.3 ($CH_2$, $CH_3OCH_2$), 69.9 ($CH_2$, $OCH_2CH_2O$), 68.8 ($CH_2$, $CH_2CH_2OSO_2CH_3$), 68.4 ($CH_2$, $CH_2CH_2SO_3CH_3$), 58.4 ($CH_3$, $CH_3OCH_2$), 37.1 ($CH_3$, $CH_2OSO_2CH_3$).

2. Synthèse d'azido méthoxy poly(éthylène glycol) ($MePEG_{43}N_3$) Le $NaN_3$ (1.755 g, 27 mmol) a été incorporé sur une solution de $MePEG_{43}Ms$ (28 g, 14 mmol) en DMF (40 mL). Le mélange a été chauffé à 60 °C sous agitation pendent 24h. À la fin de la réaction, le DMF a été évaporé sous vide et une quantité minimum de THF a été ajoutée. Le solide résultant a été filtré et le THF a été éliminé sous vide. Le solide obtenu a été dissout dans une quantité minimum de DCM et précipité par l'addition goutte à goutte d'un volume large d'$Et_2O$ froid. Le produit a ensuite été récupéré par filtration. Enfin, le solide a été repris dans de l'eau et extrait avec du DCM (5 x25 mL). La phase organique a été séchée sur le $MgSO_4$, filtrée, concentrée et séchée sous vide obtenant au final une poudre légèrement jaune (24.3 g, 87% de rendement). $^1H$ RMN (400 MHz, $D_2O$) $\delta$: 3.80-3.56 (m, $OCH_2CH_2O$), 3.51 (m, $CH_2N_3$), 3.38 (s, $CH_3O$) ; $^{13}C$ RMN (100 MHz, $D_2O$) $\delta$: 71.7 ($CH_3OCH_2$), 70.3 ($OCH_2CH_2O$), 70.2 ($CH_3OCH_2CH_2O$), 70.0 ($OCH_2CH_2N_3$), 58.8 ($OCH_3$), 50.9 ($CH_2N_3$).

**Synthèse du composé alcyne-dextrane**

unité sucrée

unité sucrée modifiée

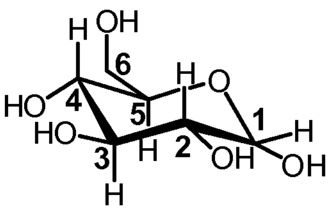

+

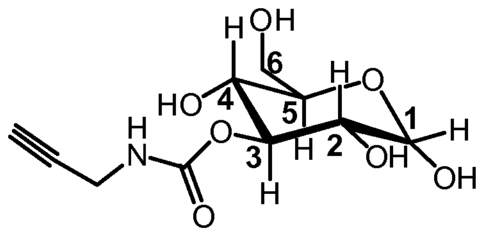

Le dextrane propargylcarbamate a été synthétisé sous la base des méthodes déjà publiées, en optimisant la procédure.[7,8] Concrètement, le protocole suivi est:

Séchage des réactifs : le Dextrane T40 (GPC PM (g/mol) 45360±8.0%, 0.5 g, 0.0110 mmol, 1 equiv.) et le LiCl (PM 42.38, 0.125 g, 2.95 mmol, 268 equiv.) ont été individuellement séchés sous vide à 80 °C pendant toute la nuit. Puis, le deux produits de départ ont été mélangés et séchés deux fois avec de toluène anhydre et une fois avec de la DMF anhydre.

Solubilisation du dextrane : Une solution du mélange contenant le dextrane T40 et le LiCl secs en DMF (10 mL) a été chauffée à 80 °C sous atmosphère d'azote et sous agitation jusqu'à dissolution complète du dextrane T40.

Synthèse du dextrane propargylcarbamate : Après solubilisation du dextrane T40, le mélange a été refroidi jusqu'à température ambiante. Ensuite, le DCI (PM 162, 0.099 g, 0.613 mmol, 49 equiv.) a été ajouté et la solution a été agitée pendant 2h à température ambiante. Puis, la propargylamine (PM 55.3, d 0.86, 0.4 mL, 6.125 mmol, 490 equiv.) a été ajoutée et la solution a été sous agitation pendant 24h à température ambiante. À la fin de la réaction, le produit a été récupéré pour précipitation par l'addition goutte à goutte d'un volume large de 2-propanol. Le produit solide a été filtré et solubilisé dans de l'eau dé-ionisé milliQ (~5 mL). Puis, la solution aqueuse a été soumise à dialyse pendant 48h. Pour la dialyse, des membranes avec un seuil de coupure de 12000-14000 g/mol ont été utilisées. Ensuite, le produit dialysé a été récupéré et lyophilisé obtenant au final une poudre blanche (SEC PM (g/mol): 45 450 (±2.1%), 0.40 g, 0.0088 mmol, 80% de rendement). AE (%) C 40.7, H 6.39, N 0.79 . $^1H$ RMN (400 MHz, $^3J_{HaxHax}$ et $^3J_{HaxHeq}$ (Hz), $D_2O$) δ: 4.98 (d, $^3J_{HaxHeq}$ 3.3, C*H*($H_1$)), 4.1-3.8 (m, C$H_2$($H_6$), C*H*($H_5$)), 3.8-3.65 (m, C$H_2$($H_6$), C*H*($H_3$)), 3.57 (dd, $^3J_{HaxHax}$ 9.6, $^3J_{HaxHeq}$ 3.3, C*H*($H_2$)), 3.52 (t, $^3J_{HaxHax}$ 9.6, C*H*($H_4$)); $^{13}C$ RMN (100 MHz, $D_2O$) δ: 98.4 (*C*H($C_1$)), 74.0 (*C*H($C_3$)), 72.1 (*C*H($C_2$)), 70.9 (*C*H($C_5$)), 70.2 (*C*H($C_4$)), 66.2 (*C*$H_2$($C_6$)).

Les suivantes signales ont été assignés grâce à le spectre de HSQC : $^1H$ RMN (400 MHz, $^3J_{HaxHax}$ et $^3J_{HaxHeq}$ (Hz), $D_2O$) δ: 3.9 (C$H_2$NHCOO) ; $^{13}C$ RMN (100 MHz, $D_2O$) δ: 30.9 (*C*$H_2$NHCOO).

[7] Mohamad Othman Ph.D thesis, Châtenay Malabry, 2010.
[8] Lukyanov AN et al, J Biomater Sci Polym Ed. 2004, 15(5), 621-30

**Synthèse du bioconjugué PEG-alendronate-dextrane basé sur des réactions « click »**

Une réaction « click » a été utilisée comme méthode de synthèse. Concrètement, la réaction de cycloaddition d'azides et d'alcynes catalysé par le cuivre (I) a été employée. [5, 9, 10]

1. Synthèse du bioconjugué PEG-dextrane

unité sucreé modifiée

unité du bioconjugué MePEG-dextrane

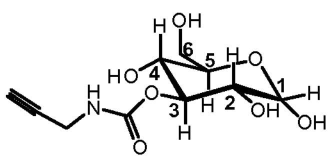

+

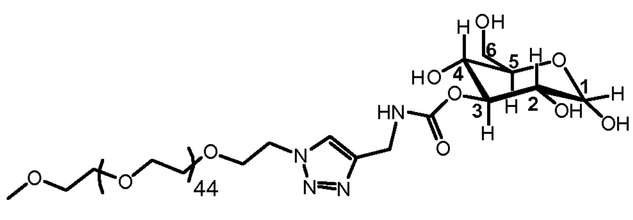

Le dextrane propargylcarbamate (SEC PM (g/mol): 45 450 (±2.1%), 0.3 g, 0.0068 mmol, 1 equiv.) et le méthoxy poly(éthylène glycol) azide ($MeOPEG_{43}N_3$, mass monoisotopique théorique 2054.2 m/z, 0.14 g, 0.068 mmol, 10 equiv.) ont été mélangés sous atmosphère d'azote. Puis, une solution aqueuse (5 mL) contenant du $CuSO_4(5H_2O)$ (PM 249.69, 0.017 g, 0.067 mmol, 10 equiv.) précédemment purgé à l'azote a été ajoutée. La solution résultante a été placée à température ambiante sous agitation et $N_2$ pendant 5 min. Ensuite, une solution aqueuse d'ascorbate sodique (PM 198.1, 0.016 g, 0.08 mmol, 12 equiv.) (5 mL) a été ajoutée. Puis, le mélange a été agité sous azote à température ambiante pendant 24h. À la fin de la réaction la solution a été soumise à dialyse pendant 48h (membranes avec un seuil de coupure de 12000-14000 g/mol). Ensuite, le produit dialysé a été récupéré et lyophilisé obtenant au final une poudre blanche légèrement verte. (SEC PM (g/mol): 62 120 (±2.3%), 0.33 g, 0.0053 mmol, 78% de rendement). $^{1}H$ RMN (400 MHz, T 320K (47 °C), $^{3}J_{HaxHax}$ et $^{3}J_{HaxHeq}$ (Hz), $D_2O$) δ: 7.98 (s, C*H*-triazole), 4.98 (d, $^{3}J_{HaxHeq}$ 3.1, C*H*($H_1$)), 4.08-3.84 (m, C*$H_2$*($H_6$), C*H*($H_5$)), 3.8-3.42 (m), 3.38 (s, OC*$H_3$*) ; $^{13}C$ RMN (100 MHz, T 320K (47 °C), $D_2O$) δ: 98.5 (*C*H($C_1$)), 74.2 (*C*H($C_3$)), 72.2 (*C*H($C_2$)), 71.0 (*C*H($C_5$)), 70.4 (*C*H($C_4$)), 70.3 (O*C*$H_2$$CH_2$O), 66.5 (*C$H_2$*($C_6$)), 50.8 (*C*$H_2$N-triazole).

[7] Lewis WG, Magallon FG, Fokin VV, Finn MG, J. Am. Chem. Soc., 2004, 126, 9152-9153

[8] Phaimanolis N, Vesterinen A-H, Rich J, Seppala J, Carbohydrate Polymers, 2010, 82, 78-82

2. Synthèse du bioconjugué PEG-alendronate-dextrane

unité du bioconjugué MePEG-dextrane

unité du bioconjugué MePEG-dextrane-alendronate

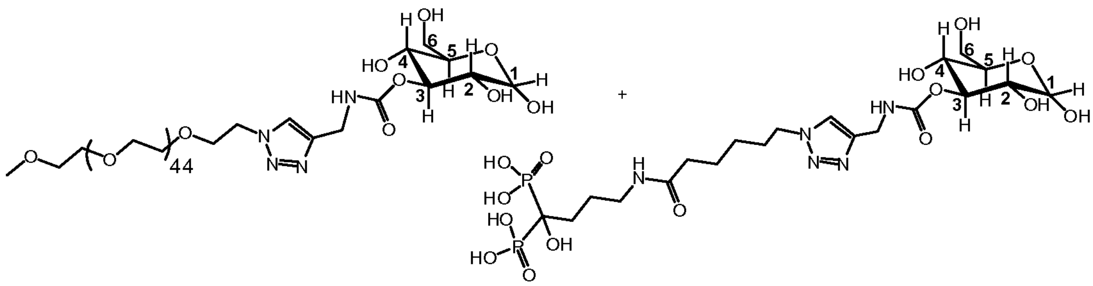

Le bioconjugué PEG-dextrane (SEC PM (g/mol): 62 120 (±2.3%)) et l'azido-alendronate ont été mélangés sous une atmosphère d'azote. Puis, une solution aqueuse (5 mL) contenant $CuSO_4(5H_2O)$ (PM 249.69, 0.017 g, 0.067 mmol, 10 equiv.) précédemment purgé à l'azote a été ajoutée. La solution résultante a été placée à température ambiante sous agitation et $N_2$ pendant 5 min. Ensuite, une solution aqueuse d'ascorbate sodique (PM 198.1, 0.016 g, 0.08 mmol, 12 equiv.) (5 mL) a été ajoutée. Puis, le mélange a été agité sous l'azote à température ambiante pendant 24h. À la fin de la réaction la solution a été soumise à dialyse pendant 48h (membranes avec un seuil de coupure de 12000-14000 g/mol). Ensuite, le produit dialysé a été récupéré et lyophilisé.

**Exemple 16: Modification superficielle de nanoparticules de MIL-100 avec poly-βCDP :**

2 mg de nanoparticules de MIL-100 ont été modifiés par incubation avec 500 μl d'une solution aqueuse de poly-βCDP-rhodamine(1 :10, 1 phosphate/10 cyclodextrines) 2 mg/ml (rapport en poids nanoparticules : βCDP = 1:0.5), 24 h sous agitation à température ambiante. Après incubation, les nanoparticules modifiées ont été récupérées par centrifugation à 9500 g, 10 min et le surnageant a été analysé par spectrofluorimétrie afin de définir la quantité de poly-βCDP-rhodamine liée aux nanoparticules. Les résultats obtenus indiquent que la quantité de βCDP-rhodamine associée aux MIL-100 représente 34(±0,5) % p/p. Les nanoparticules modifiées ont été lavées avec 1 ml d'eau, afin d'éliminer l'excès de poly-βCDP-rhodamine et enfin incubées dans 1 ml de PBS (Phosphate Buffered Saline, Lonza) à 37 °C, sous agitation à la concentration de 2 mg/ml. Après différents temps d'incubation (0,3-6-24 h) la suspension a été centrifugée 10 min à 9500 g, 500μL du surnageant ont été prélevés et remplacés par la même quantité de milieu frais. Les surnageant ont été analysés par spectrofluorimétrie, afin de quantifier la libération de poly-βCDP-rhodamine dans le milieu. Les résultats ont montré que seulement 27.24 % de βCDP-rhodamine liés aux nanoparticules sont libérés après 6 h d'incubation dans du PBS (fig. 28). Il peut être conclu que la couronne externe de poly-βCDP-rhodamine est stable dans des conditions physiologiques, probablement grâce à l'effet coopératif de plusieurs groupes phosphates dans le polyCD.

**Exemple 17: Modification superficielle des nanoparticules de MIL-100(Al) à base de βCDP.**

2 mg de nanoMOF de MIL-100(Al) ont été incubés avec 500 μl d'une solution aqueuse de βCDP-rhodamine, 24 h sous agitation, à température ambiante. À la fin de l'incubation, les nanoparticules ont été récupérées par centrifugation à 9491 xg 10 min, et le surnageant a été analysé par spectrofluorimétrie afin de définir la quantité de βCDP-rhodamine liée aux nanoparticules. Les résultats obtenus indiquent que la quantité de βCDP-rhodamine associée aux MIL-100(Al) représente 29.6(±0,6) % p/p. Les nanoparticules modifiées ont été lavées avec 1 ml d'eau, afin d'éliminer l'excès de βCDP-rhodamine et enfin incubées dans 1 ml de PBS (Phosphate Buffered Saline, Lonza) à 37 °C, sous agitation. Après différents temps d'incubation (0,3, 6,24h) la suspension a été centrifugée 10 min à 9491xg, 500μL du surnageant ont été prélevés et remplacés par la même quantité de milieu frais. Les surnageant ont été analysés par spectrofluorimétrie, afin de quantifier la libération de βCDP-rhodamine dans le milieu. Les résultats ont montré que seulement 26.5(±4) % de βCDP-rhodamine liés aux nanoparticules sont libérés après 6 h d'incubation et ce pourcentage ne dépasse pas 37.1(3.5) % après 24h (fig. 29). Il peut être conclu que la couronne externe de βCDP est stable dans des conditions physiologiques, et que cette méthode de modification superficielle est valide aussi pour nanoMOFs à base de métaux différents du Fer, comme le MIL-100(Al).

**LISTE DES REFERENCES**

**[1]** WO 2009/077670

**[2]** WO 2009/077671

**[3]** Bone Marrow Transplant. 2002.30 (12), 833-841

**[4]** US 4,329,332

**[5]** J. Bouligand, et al., Int. J.Pharm., 2004

**[6]** E. Renard et al., European Polymer Journal, vol. 33, No 1, pp 49-57 (1997)

**[7]** Gref et al., International Journal of Pharmaceutics, Vol. 332, Issues 1-2, Pages 185-191 (2007)

**[8]** Gref et al., J. Control Release, 111(3):316-24 (2006)

**[9]** Gref et al., Journal of colloid and interface science, 307(1) : 83-93 (2007)

**[10]** Blanchemain et al., Acta Biomaterialia, Volume 4, Issue 6, November 2008, Pages 1725-1733

**[11]** Elif Yilmaz Ozmen et al. Bioresource Technology, Volume 99, Issue 3, Pages 526-531 (2008)

**[12]** Cesteros et al., European Polymer Journal, Volume 45, Issue 3, Pages 674-679 (2009) (PEG acylé)

**[13]** Salmaso et al., International Journal of Pharmaceutics, Volume 345, Issues 1-2, Pages 42-50 (2007) (PEG diaminé)

**[14]** Yang et al., Biomaterials, Volume 28, Issue 21, Pages 3245-3254 (2007)

**[15]** G. Oros, T. Cserhati, E. Forgacs, Chemosphere 52, 2003, 185

**[16]** A.M. Badawi, E.M.S. Azzam, S.M.I. Morsy, Bioorg. Med. Chem., 14, 2006, 8661

**[17]** W-J. Tsai, Y-J Shiao, S-J Lin, W-F Chiou, L-C Lin, T-H Yang, C-M teng, T-S Wu, L-M Yang, Bioorg. Med. Chem. Letters 16, 2006, 4440

**[18]** Whitfield, T. R.; Wang, X.; Liu, L.; Jacobson, A. J. Solid State Sci. 2005, 7, 1096

[19] T. Loiseau et al, C. R. Chimie, 8 765 (2005).

[20] Serre et al., « Role of solvent-host interactions that lead to very large swelling of hybrid frameworks », Science, 2007, Vol. 315, 1828-1831

[21] Surblé et al., « A new isoreticular class of metal-organic frameworks with the MIL-88 topology », Chem. Comm., 2006, 284-286

[22] Mellot-Draznieks et al., « Very large swelling in hybrid frameworks : a combined computational and power diffraction study », J. Am. Chem. Soc., 2005, Vol. 127, 16273-16278

[23] C. Serre, F. Millange, S. Surblé, G. Férey Angew. Chem. Int. Ed. 2004, 43, 6286: A new route to the synthesis of trivalent transition metals porous carboxylates with trimeric SBU

[24] Horcajada et al., « Synthesis and catalytic properties of MIL-100(Fe), an iron(III) carboxylate with large pores », Chem. Comm., 2007, 2820-2822

[25] Férey et al., « A chromium terephthalate-based solid with unusally large pore volumes and surface area », Science, 2005, Vol. 309, 2040-2042

[26] S. Surblé, F. Millange, C. Serre, T. Düren, M. Latroche, S. Bourrelly, P.L. Llewellyn and G. Férey « MIL-102: A Chromium Carboxylate Metal Organic Framework with Gas Sorption Analysis » J. Am. Chem. Soc. 128 (2006), 46, 14890

[27] Mulder WJ, et al. Nanomed. 2007 Jun, 2(3), 307-324

[28] A.K. Gupta, et al., Nanomed. 2007 2(1), 23-39

[29] P Caravan, Chem. Soc. Rev., 2006, 35, 512-523

[30] Yan-Ping Ren, et al., Angew. Chem. Int. Ed. 2003, 42, No. 5, 532

[31] J. Mater. Chem., 2010, 20, 7676-7681 ; A. Demessence, et al, J. Mater. Chem., 2010, 20, 7676-7681

[32] J.H. Cavka et al., JACS, 2009, 130, 13850-13851

[33] Baleaux B. et al., « Chimie analytique-dosage colorimétrique d'agents de surface non ioniques polyoxyéthylènes à l'aide d'une solution iode-iodurée », C.R. Acad. Sciences Paris, 1972, série C, 274, 1617-1620.

[34] Navath R S, Menjoge A R, Dai H, Romero R , Kannan S, Kannan R M., Injectable PAMAM dendrimer-PEG hydrogels for the treatment of genital infections: formulation and in vitro and in vivo evaluation, Mol Pharm. 2011 Aug 1;8(4):1209-23

[35] R C. Hedden et B. J. Bauer,Structure and Dimensions of PAMAM/PEG Dendrimer_Star Polymers, Macromolecules, 2003, 36 (6), pp 1829_1835

[36] N Bhattarai, H R. Ramay, J Gunn, F A. Matsen, M Zhan, PEG-grafted chitosane as an injectable thermosensitive hydrogel for sustained protein release, J Controlled Release, 103 (3), 609-624, 2005

[37] J.A Wieland, T.L. Houchin-Ray, L.D. Shea, Non-viral vector delivery from PEG-hyaluronic acid hydrogels, J. Controlled Release, 120 (3), 233-241, 2007

[38] X.M. Liu, H. Lee, R. Reinhardt, L. Marky, W Dong, J. Controlled Release vol 122, 2007, 54-62, Novel biomineral-binding cyclodextrins for controlled drug delivery in the oral cavity

[39] Chalati et al., « Optimisation of the synthesis of MOF nanoparticules made of flexible porous iron fumarate MIL-88A », J .Mater. Chem., 2011, 21, 2220

[40] Cavka, J.; Jakobsen, S.; Olsbye, U.; Guillou, N.; Lamberti, C.; Bordiga, S.; Lillerud, K., J. Am. Chem. Soc. 2008, 130, 13850

[41] Kandiah, M.; Nilsen, M.H.; Usseglio, S.; Jakobsen, S.; Olsbye, U.; Tilset, M.; Larabi, C.; Quadreli, E.A.; Bonino, F.; Lillerud K.P., Chem. Mater., 2010, 22(24), 6632

[42] Garibay S.J.; Cohen S.M., Chem. Commun., 2010, 46, 7700

[43] Park et al., « Exceptional chemical and thermal stability of zeolitic imidazolate frameworks

[44] M. Dan-Hardi, C. Serre, T. Frot, L. Rozes, G. Maurin, C. Sanchez and G. Férey : J. Am. Chem. Soc. Comm., 131, 2009, 10857-10859 A New Photoactive Crystalline Highly Porous Titanium (IV) Dicarboxylate », Proc. Natl. Acad. Sci. U.S.A., 2006, 103, 10186

[45] C. Zlotea, D. Phanon, M. Mazaj, D. Heurtaux, V. Guillerm, C. Serre, P. Horcajada, T. Devic, E. Magnier, F. Cuevas, G. Férey, P. L. Llewellyn and M. Latroche : "Effect of NH2 and CF3 functionalization on the hydrogen sorption properties of MOFs" Dalton Trans., 2011, 40, 4879-4881

[46] A.E. Platero-Prats, V.A. de la Pena-O'Shea, N. Snejko, A. Monge,E. Gutierrez-Puebla, « Dynamic calcium metal-organic framework acts as a selective organic solvent sponge », Chemistry, 16(38), 11632

[47] C. Volkringer, J. Marrot, G. Ferey, T. Loiseau, »Hydrothermal crystallization of three calcium-based hybrid solids with 2,6-naphthalene or 4,4'-biphenyl-dicarboxylates » Crystal Growth Design, 2008, 8, 685

[48] Horcajada et al., « How linker's modification controls swelling properties of highly flexible iron(III) dicarboxylates MIL-88 », J. Am. Chem. Soc., 2011, 133, 17839

[49] Devic et al., "Functionalization in flexible porous solids: effects on the pore opening and the host-guest interactions", J. Am. Chem. Soc., 2010, 132, 1127

[50] C. Serre, S. Surblé, C. Mellot-Draznieks, Y. Filinchuk, G. Férey Dalton Trans., 2008, 5462-5464 : Evidence of flexibility in the nanoporous iron(III) carboxylate MIL-89

[51] C.T. Dziobkowski, T.J. Wrobleski, D.B. Brown, Inorg. Chem. 1982, 21, 671

**Revendications**

**1.** Solide MOF cristallin poreux comprenant une succession tridimensionnelle de motifs répondant à la formule (I) suivants :

$M_mO_kX_lL_p$ Formule **(I)**

dans laquelle :

chaque occurrence de **M** représente indépendamment un ion métallique choisi dans le groupe comprenant $Fe^{2+}$, $Fe^{3+}$, $Zn^{2+}$, $Zr^{4+}$, $Ti^{4+}$, $Ca^{2+}$, $Mg^{2+}$ et $Al^{3+}$ ;

- **m, k, 1** et **p** sont des nombres ≥ 0 choisis de façon à respecter la neutralité des charges du motif ;
- **X** est un ligand choisi dans le groupe comprenant $OH^-$, $Cl^-$, $F^-$, $I^-$, $Br^-$, $SO_4^{2-}$, $NO_3^-$, $ClO_4^-$, $R^1-(COO)_n^-$, $R^1-(SO_3)_n^-$, $R^1-(PO_3)_n^-$, où $R^1$ est un hydrogène, un alkyle en $C_1$ à $C_8$, linéaire ou ramifié, n = 1 à 6 ; et
- L est un ligand espaceur comprenant un radical $R^0$ et q occurrences d'un groupe complexant A, où

  • q est un nombre entier compris entre 2 et 6 ;
  • chaque occurrence de A est indépendamment :

    (i) un carboxylate

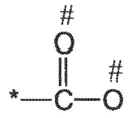 ;

(ii) un phosphonate

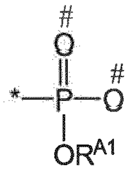 ;

ou

(iii) un groupe imidazolate

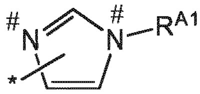 ;

dans lequel $R^{A1}$ représente un atome d'hydrogène ou un radical $C_{1-6}$alkyle ;

où * désigne le point d'attachement du groupement A avec le radical $R^0$ ;
# désigne les points d'attachement possibles du groupement A à l'ion métallique M ;

- $R^0$ représente
  - ◦ un radical $C_{1-12}$alkylène, $C_{2-12}$alcènylène ou $C_{2-12}$alcynylène ;
  - ◦ un radical aryle mono- ou poly-cyclique, fusionné ou non, comprenant de 6 à 50 atomes de carbone,
  - ◦ un radical hétéroaryle mono- ou poly-cyclique, fusionné ou non, comprenant de 4 à 50 atomes de carbone,

le radical $R^0$ étant éventuellement substitué par un ou plusieurs groupes indépendamment choisis dans le groupe halogène, OH, $NH_2$, $NO_2$ ou un alkyle en $C_1$ à $C_6$ ;

dans lequel la surface externe du MOF est modifiée en ce qu'elle comprend au moins un agent de surface organique complexé à un centre métallique M ou à un ligand L situé sur la surface externe du solide MOF cristallin, l'agent de surface organique comprenant : i) au moins un groupement phosphate, phosphonate, bisphosphonate, sulfate, carboxylate, hydroxy, cathécolate, thiolate (-SR ou $-S^-$), hétérocyclique azoté, amido ($-C(=O)N(R)_2$), amino ($-N(R)_2$), ou une combinaison de ces groupes, où chaque occurrence de R représente indépendamment H, $C_{1-6}$alkyle ou phényle);

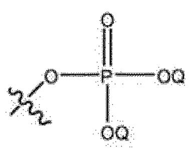

Phosphate

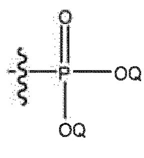

Phosphonate

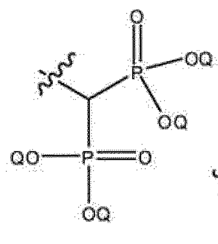

Bisphosphonate

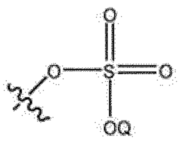

Sulfate

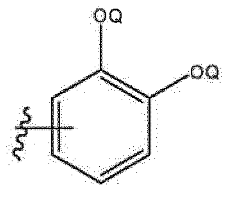

Cathécolate

où chaque occurrence de Q représente indépendamment H ou un cation de métal alcalin ;
et/ou ii) une dimension du petit axe du volume rigide occupé par l'agent de surface supérieure à la taille des fenêtres d'accès aux pores de plus grande dimension du matériau MOF ; l'agent de surface organique étant choisi parmi un monomère, oligomère ou polymère de cyclodextrine; un groupement polyéthylèneglycol ramifié ; une protéine ; un

polysaccharide portant une pluralité de chaînes latérales polyéthylèneglycol; ou un polysaccharide insoluble dans l'eau à 6<pH<8 et soluble dans l'eau à pH<5 ;
ledit agent de surface organique étant en interaction avec un centre métallique M ou à un ligand L situé en surface du solide MOF cristallin via ledit un ou plusieurs groupe(s) phosphate, phosphonate, bisphosphonate, sulfate, carboxylate, hydroxy, cathécolate, thiolate, hétérocyclique azoté, amido, amino, ou une combinaison de ces groupes.

**2.** Solide selon la revendication 1, dans lequel le ligand L est un espaceur portant plusieurs fonctions complexantes comprenant les carboxylates, phosphonates, imidazolates, dans lequel le carboxylate est un di-, tri-, tétra- ou hexa-carboxylate choisi dans le groupe comprenant :

| | |
|---|---|
| 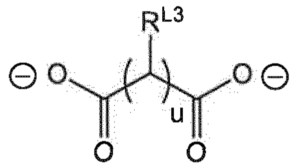 | 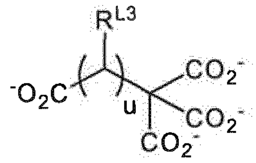 |
| 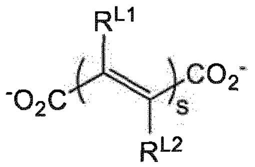 | 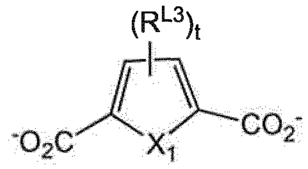 |
| 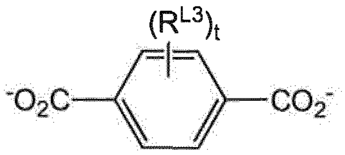 | 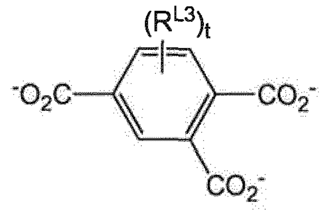 |
| 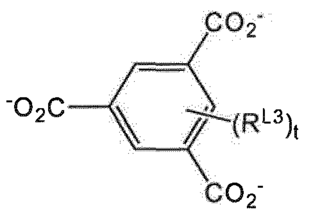 | 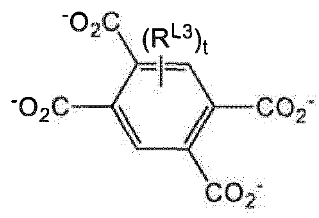 |
| 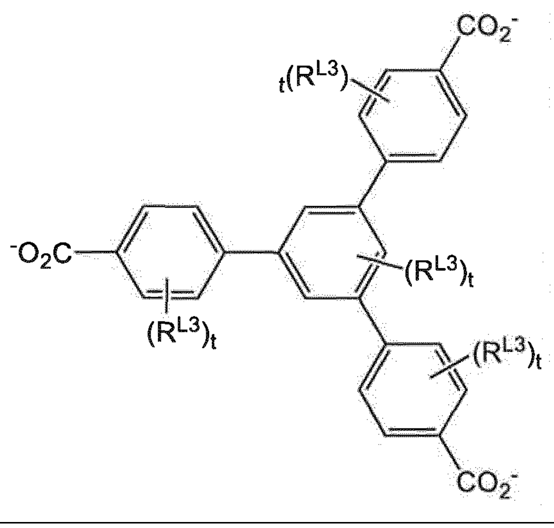 | 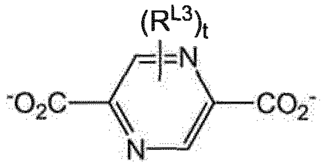 |

(suite)

| | |
|---|---|
| 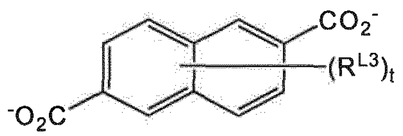 | 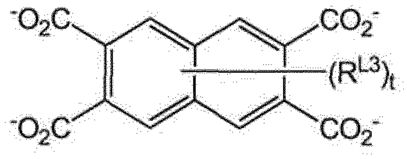 |
| 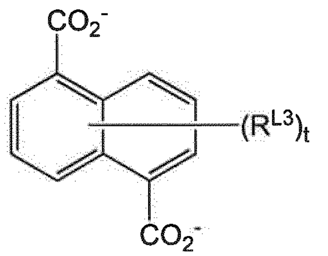 | 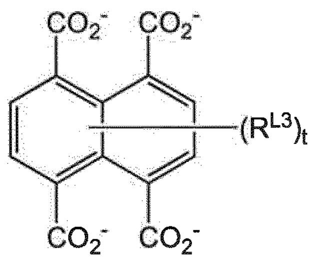 |
| 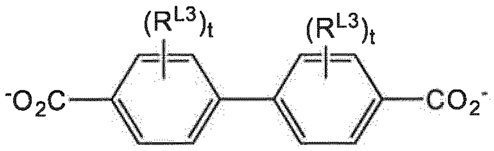 | |
| 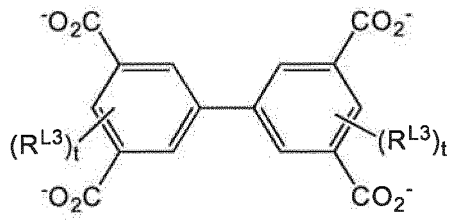 | |
| 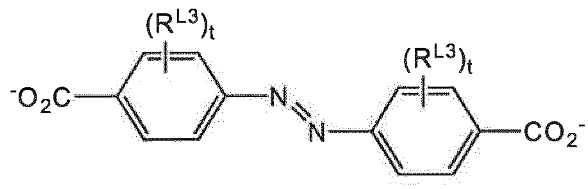 | |
| 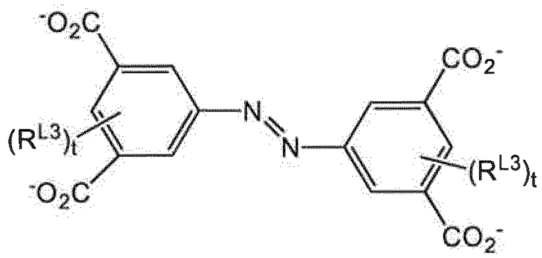 | |
| 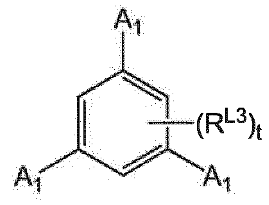 où $A_1$, $A_2$ et $A_3$ représentent indépendamment | |

(suite)

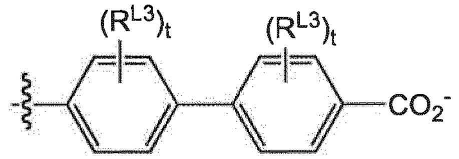

dans lesquels :

$X_1$ représente O ou S,
s représente un entier de 1 à 4,
chaque occurrence de t représente indépendamment un entier de 1 à 4,
u représente un entier de 1 à 7,
$R^{L1}$ et $R^{L2}$ représentent indépendamment H, un halogène ou un alkyle en $C_1$ à $C_6$, et
chaque occurrence de $R^{L3}$ représente indépendamment H, un halogène, OH, $NH_2$, $NO_2$ ou un alkyle en $C_1$ à $C_6$.

**3.** Solide selon la revendication 1 ou 2, dans lequel le ligand L est un ligand di-, tri- ou tétra- carboxylate choisi dans le groupe comprenant : $C_2H_2(CO_2^-)_2$ (fumarate), $C_2H_4(CO_2^-)_2$ (succinate), $C_3H_6(CO_2^-)_2$ (glutarate), $C_4H_4(CO_2^-)_2$ (muconate), $C_4H_8(CO_2^-)_2$ (adipate), $C_7H_{14}(CO_2^-)_2$ (azelate), $C_5H_3S(CO_2^-)_2$ (2,5-thiophènedicarboxylate), $C_6H_4(CO_2^-)_2$ (téréphtalate), $C_6H_2N_2(CO_2^-)_2$ (2,5-pyrazine dicarboxylate), $C_{10}H_6(CO_2^-)_2$ (naphtalène-2,6-dicarboxylate), $C_{12}H_8(CO_2^-)_2$ (biphényle-4,4'-dicarboxylate), $C_{12}H_8N_2(CO_2^-)_2$ (azobenzènedicarboxylate), $C_6H_3(CO_2^-)_3$ (benzène-1,2,4-tricarboxylate), $C_6H_3(CO_2^-)_3$ (benzène-1,3,5-tricarboxylate), $C_{24}H_{15}(CO_2^-)_3$ (benzène-1,3,5-tribenzoate), $C_6H_2(CO_2^-)_4$ (benzène-1,2,4,5-tétracarboxylate, $C_{10}H_4(CO_2^-)_4$ (naphtalène-2,3,6,7-tétracarboxylate), $C_{10}H_4(CO_2^-)_4$ (naphtalène-1,4,5,8-tétracarboxylate), $C_{12}H_6(CO_2^-)_4$ (biphényl-3,5,3',5'-tétracarboxylate), et les analogues modifiés choisis dans le groupe comprenant le 2-aminotéréphtalate, le 2-nitrotéréphtalate, le 2-méthyltéréphtalate, le 2-chlorotéréphtalate, le 2-bromotéréphtalate, le 2,5-dihydroxotéréphtalate, le tétrafluorotéréphtalate, le le tétraméthyltéréphtalate, le diméthyl-4,4'-biphénydicarboxylate, le tétraméthyl-4,4'-biphénydicarboxylate, le dicarboxy-4,4'-biphénydicarboxylate, le 2,5-pyrazyne dicarboxylate, le 2,5 diperfluorotéréphthalate, azobenzène 4,4'-dicarboxylate, 3,3'-dichloro azobenzène 4,4'-dicarboxylate, 3,3'-dihydroxo azobenzène 4,4'-dicarboxylate, 3,3'-diperfluoro azobenzène 4,4'-dicarboxylate, 3,5,3',5'-azobenzène tétracarboxylate, 2,5-dimethyl téréphthalate, perfluorosuccinate, perfluoromuconate, perfluoro glutarate, 3,5,3',5' perfluoro-4,4'-azobenzène dicarboxylate, 3,3'-diperfluoro azobenzène 4,4'-dicarboxylate.

**4.** Solide selon l'une quelconque des revendications 1 à 3, dans lequel le ligand L est un ligand fluoré choisi dans le groupe comprenant tétrafluorotéréphtalate, perfluorosuccinate, perfluoromuconate, perfluoro glutarate, 2,5 diperfluorotéréephtalate, 3,6 perfluoro 1,2,4,5 benzènetétracarboxylate, 3,5,3',5' perfluoro-4,4'-azobenzène dicarboxylate, 3,3'-diperfluoro azobenzène 4,4'-dicarboxylate.

**5.** Solide selon l'une quelconque des revendications 1 à 3, dans lequel le ligand L est un ligand biologiquement actif choisi dans le groupe comprenant $C_7H_{14}(CO_2^-)_2$ ; aminosalicylate ; chlodronate, pamidrontate, alendronate, etidronate ; meprobamate ; des porphyrines comprenant des groupes carboxylates, phosphonates et/ou amino ; des aminoacides ; des azobenzènes comprenant des groupes carboxylates, phosphonates, et/ou amino ; le dibenzofuran-4,6-dicarboxylate, le dipicolinate; le glutamate, le fumarate, le succinate, le suberate, l'adipate, le nicotinate, nicotinamide, purines, pyrimidines.

**6.** Solide selon l'une quelconque des revendications 1 à 5, dans lequel l'agent de surface organique est choisi dans le groupe comprenant :

- les $\alpha$-, $\beta$- ou $\gamma$-cyclodextrines ;
- les oligomères de $\alpha$-, $\beta$- ou $\gamma$-cyclodextrines ;
- les poly-$\alpha$, poly-$\beta$ ou poly-$\gamma$-cyclodextrines,
- les copolymères de $\alpha$, $\beta$ et/ou $\gamma$-cyclodextrines,
- les PEG dendrimères,
- le chitosane,
- le chitosane portant une pluralité de chaînes latérales PEG,
- l'albumine,

- les immunoglobulines

comprenant un ou plusieurs groupe(s) phosphate(s), phosphonate, sulfate, carboxylate, hydroxy, cathécolate, thiolate, azote hétérocyclique, amido ou amino, ou une combinaison de ces groupes.

7. Solide selon la revendication 6, dans lequel les unités cyclodextrine du poly-$\alpha$, poly-$\beta$ ou poly-$\gamma$-cyclodextrine ou le copolymère de $\alpha$,$\beta$ et/ou $\gamma$-cyclodextrine sont liées entre elles par des chaînes hydrocarbonées répondant à la formule $-O-(CH_2-CHOR^1-CH_2)_n-O-$ où n est un entier compris entre 1 et 50 et, dans chacune des unités $(CH_2-CHOR^1-CH_2)$, $R^1$ désigne soit un atome d'hydrogène, soit une chaine $-CH_2-CHOH-CH_2-O-$reliée à une unité cyclodextrine dudit polymère ou copolymère.

8. Solide selon l'une quelconque des revendications 1 à 5, dans lequel l'agent de surface organique est un polyéthylèneglycol ramifié répondant à la structure suivante :

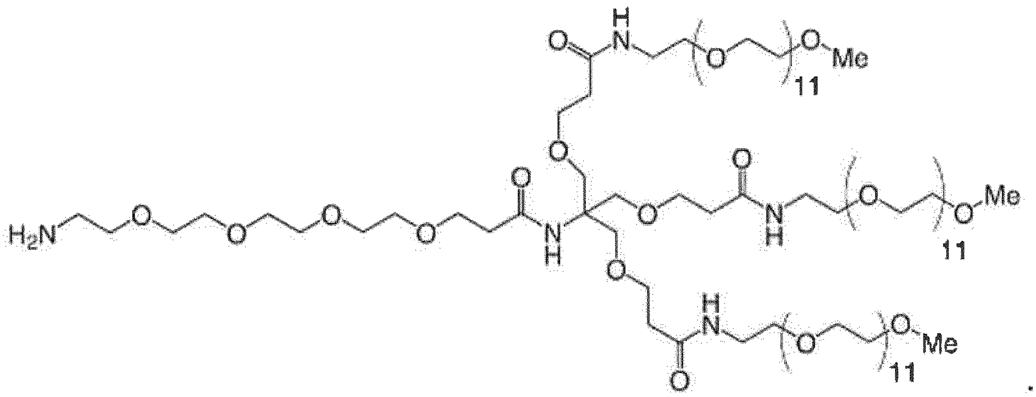

.

9. Solide selon l'une quelconque des revendications 1 à 8, dans lequel l'agent de surface organique est en outre fonctionnalisé avec une molécule fluorescente.

10. Solide selon la revendication 9, dans lequel la molécule fluorescente est la rhodamine, la fluoresceine, la luciférase, le pyrène et dérivés, l'aminopyrrolidino-7-nitrobenzofurazan, ou un quantum dot.

11. Solide selon l'une quelconque des revendications 1 à 10, ledit solide comprenant dans ses pores ou à sa surface au moins un principe pharmaceutiquement actif et/ou une substance active entrant dans la formulation d'une préparation cosmétique et/ou un marqueur.

12. Solide selon la revendication 11, dans lequel le principe pharmaceutiquement actif est un agent anticancéreux, antiviral, antibiotique, antiinflammatoire ou analgésique.

13. Solide selon la revendication 12, dans lequel l'agent anticancéreux est choisi dans le groupe comprenant : le busulfan, l'azidothymidine (AZT), l'azidothymidine phosphatée (AZTP), le cidofovir, la gemcitabine, la zalcitabine (ddC), la didanosine (ddI), l'ibuprofène.

14. Procédé de préparation d'un solide tel que défini dans l'une quelconque des revendications 1 à 13, comprenant :

a) au moins une étape réactionnelle (i) consistant à mélanger dans un solvant polaire :

- au moins une solution comprenant au moins un précurseur inorganique métallique se présentant sous la forme de métal M, d'un sel de métal M ou d'un complexe de coordination comprenant l'ion métallique M dans laquelle M est tel que défini dans la revendication 1 ;
- au moins un ligand L' de formule $-R^0(COR^3)_q$,

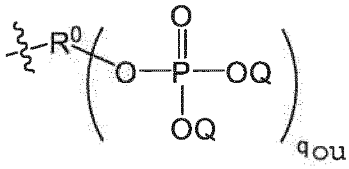 ou 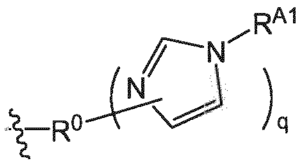 ,

où Q, $R^{A1}$, q et $R^0$ sont tels que définis dans la revendication 1, et $R^3$ est choisi dans le groupe comprenant un radical -OH, un radical -OY où Y représente un cation alcalin, un halogène, ou un radical $-OR^4$, -O-$C(=O)R^4$ ou $-NR^4R^{4'}$, où $R^4$ et $R^{4'}$ sont des radicaux alkyles en $C_{1-12}$ ;

b) une étape (ii) de fixation sur la surface externe dudit solide d'au moins un agent de surface organique comprenant: i) au moins un groupement phosphate, phosphonate, bisphosphonate, sulfate, carboxylate, hydroxy, cathécolate, thiolate (-SR ou $-S^-$), hétérocyclique azoté, amido ($-C(=O)N(R)_2$), amino ($-N(R)_2$), ou une combinaison de ces groupes, où chaque occurrence de R représente indépendamment H, $C_{1-6}$alkyle ou phényle) ;

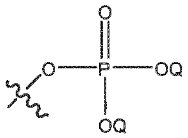
Phosphate

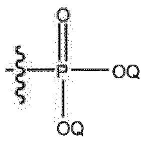
Phosphonate

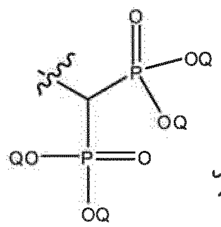
Bisphosphonate

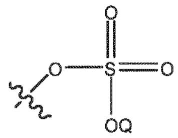
Sulfate

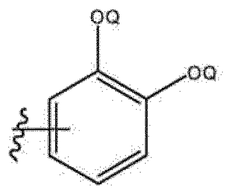
Cathécolate

où chaque occurrence de Q représente indépendamment H ou un cation de métal alcalin ;
et/ou ii) une dimension du petit axe du volume rigide occupé par l'agent de surface supérieure à la taille des fenêtres d'accès aux pores de plus grande dimension du matériau MOF l'agent de surface organique étant choisi parmi un monomère, oligomère ou polymère de cyclodextrine; ou un groupement polyéthylèneglycol ramifié ; une protéine ; un polysaccharide portant une pluralité de chaînes latérales polyéthylèneglycol; ou un polysaccharide insoluble dans l'eau à 6<pH<8 et soluble dans l'eau à pH<5 ;
de façon à obtenir ledit solide dans lequel ledit agent de surface organique est en interaction avec un centre métallique M ou à un ligand L en surface du solide MOF cristallin via ledit un ou plusieurs groupe(s) phosphate, phosphonate, bisphosphonate, sulfate, carboxylate, hydroxy, cathécolate, thiolate, hétérocyclique azoté, amido, amino, ou une combinaison de ces groupes.

**15.** Procédé selon la revendication 14, dans lequel le ligand L' représente un ligand portant plusieurs fonctions complexantes comprenant les carboxylates, phosphonates, imidazolates, dans lequel le groupe carboxylate est un un ligand di-, tri-, tétra- ou hexadentate choisi dans le groupe comprenant :

| | |
|---|---|
| 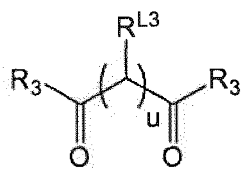 | 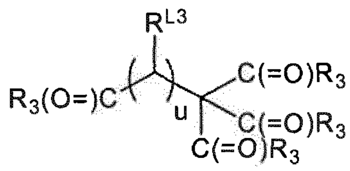 |
| 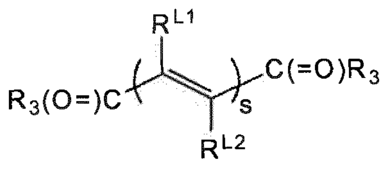 | 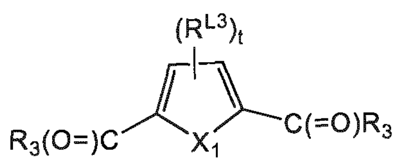 |

(suite)

| | |
|---|---|
| 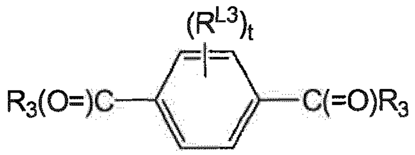 | 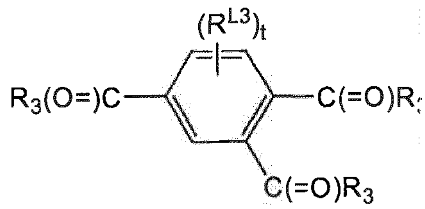 |
| 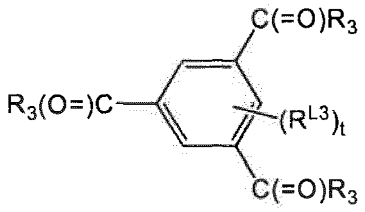 | 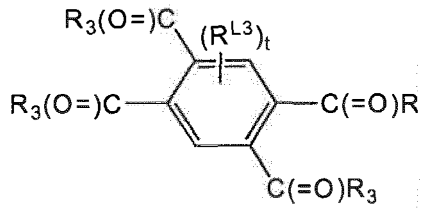 |
| 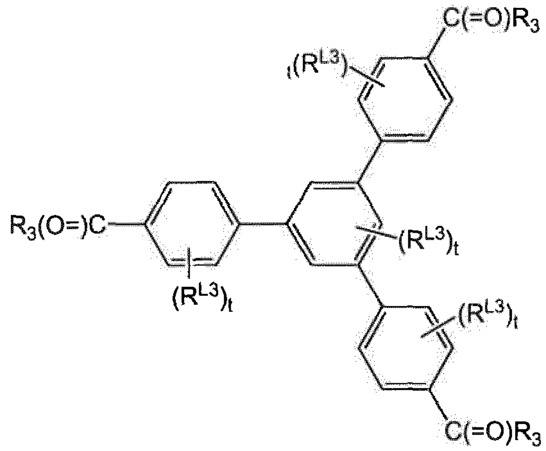 | 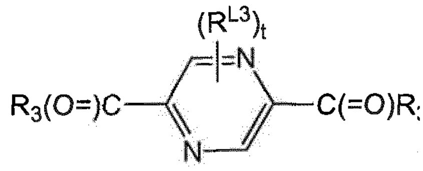 |
| 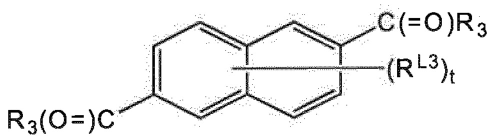 | 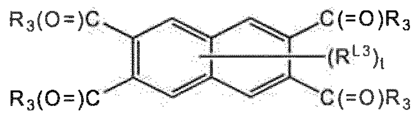 |
| 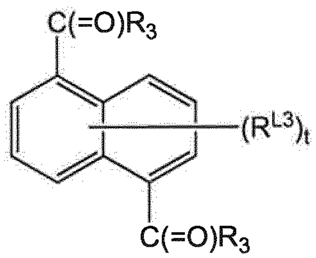 | 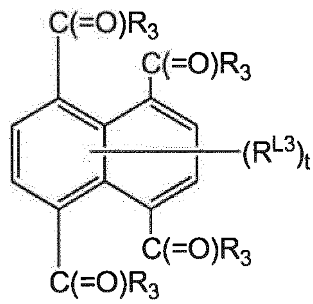 |
| 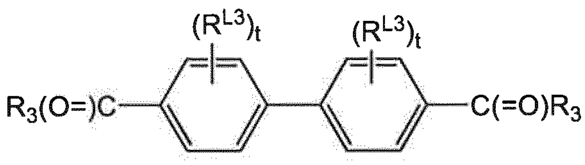 | |

(suite)

| |
|---|
| 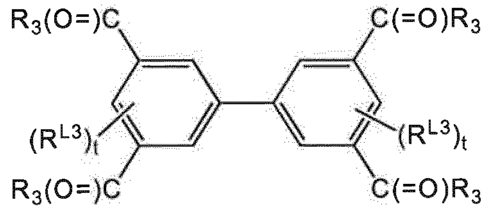 |
| 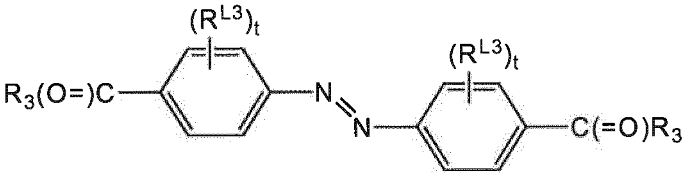 |
| 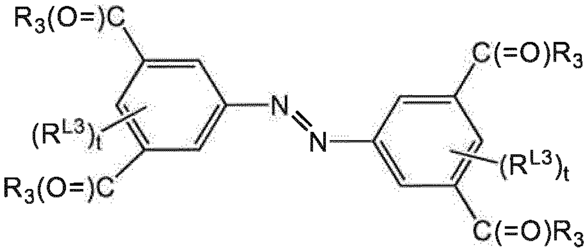 |
| 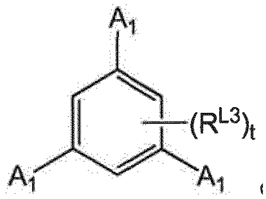<br>où $A_1$, $A_2$ et $A_3$ représentent indépendamment<br>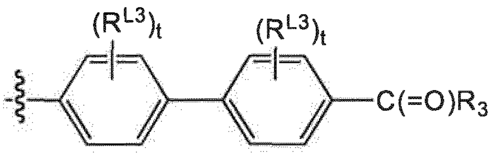 |

dans lesquels :

$R_3$ est choisi dans le groupe comprenant un radical -OH, un radical -OY où Y représente un cation alcalin, un halogène, ou un radical $-OR^4$, $-O-C(=O)R^4$ ou $-NR^4R^{4'}$, où $R^4$ et $R^{4'}$ sont des radicaux alkyles en $C_{1-12}$,
$X_1$ représente O ou S,
s représente un entier de 1 à 4,
chaque occurrence de t représente indépendamment un entier de 1 à 4,
u représente un entier de 1 à 7,
$R^{L1}$ et $R^{L2}$ représentent indépendamment H, un halogène ou un alkyle en $C_1$ à $C_6$, et

chaque occurrence de $R^{L3}$ représente indépendamment H, un halogène, OH, $NH_2$, $NO_2$ ou un alkyle en $C_1$ à $C_6$.

**16.** Procédé selon l'une quelconque des revendications 14 à 15, dans lequel l'agent de surface organique est tel que défini à l'une quelconque des revendications 1 et 6 à 10.

**17.** Procédé de préparation d'un solide selon l'une quelconque des revendications 14 à 16, dans lequel l'étape réactionnelle (i) est réalisée avec au moins une des conditions réactionnelles ci-dessous :

(i) une température de réaction de 0°C à 220°C ;
(ii) une vitesse d'agitation de 0 à 1000 rpm wherein 1 rpm = 1/60 Hz ;
(iii) un temps de réaction de 1 minute à 96 heures ;
(iv) un pH de 0 à 7 ;
(v) l'addition d'au moins un co-solvant au solvant, au précurseur, au ligand ou au mélange de ceux-ci, ledit co-solvant étant choisi dans le groupe comprenant l'acide acétique, l'acide formique, l'acide benzoïque ;
(vi) le solvant est choisi dans le groupe comprenant l'eau, les alcools $R^s$-OH où $R^s$ est un radical alkyle en $C_1$ à $C_6$ linéaire ou ramifié, le diméthylformamide, diméthylsulfoxide, l'acétonitrile, le tétrahydrofurane, le diéthylformamide, le chloroforme, le cyclohexane, l'acétone, le cyanobenzène, le dichlorométhane, le nitrobenzène, l'éthylèneglycol, le diméthylacétamide ou des mélanges de ces solvants, miscibles ou non ;
(vii) dans un milieu supercritique ;
(viii) sous micro-ondes et/ou sous ultra-sons ;
(ix) dans des conditions d'électrolyse électrochimique ;
(x) dans des conditions utilisant un broyeur à cylindre ;
(xi) dans un flux gazeux.

**18.** Procédé selon l'une quelconque des revendications 14 à 17, comprenant en outre une étape (iii) d'introduction dans ledit solide d'au moins un principe pharmaceutiquement actif.

**19.** Solide selon l'une des revendications 1 à 13 pour son utilisation comme médicament.

**20.** Solide selon l'une des revendications 1 à 13 pour son utilisation dans le traitement du cancer.

**21.** Utilisation d'un solide selon l'une des revendications 1 à 13 pour la fabrication d'un marqueur utilisable en imagerie médicale.

**Patentansprüche**

**1.** Festes poröses kristallines MOF (MOF - Metallorganisches Gerüst) umfassend eine dreidimensionale Abfolge von Einheiten der Formel (I):

$$M_mO_kX_lL_p \qquad \text{Formel } \mathbf{(I)}$$

worin:

jedes Vorkommen von **M** unabhängig ein Metallion darstellt, das aus der Gruppe ausgewählt ist, die $Fe^{2+}$, $Fe^{3+}$, $Zu^{2+}$, $Zr^{4+}$, $Ti^{4+}$, $Ca^{2+}$, $Mg^{2+}$ und $Al^{3+}$ umfasst;

- **m**, **k**, **l und p** Zahlen $\geq 0$ sind, die so gewählt sind, dass die Ladungsneutralität der Einheit gewahrt bleibt;
- **X** ein Ligand ist, der aus der Gruppe ausgewählt ist, die $OH^-$, $Cl^-$, $F^-$, $I^-$, $Br^-$, $SO_4^{2-}$, $NO_3^-$, $ClO_4^-$, $R^1$-$(COO)_n^-$, $R^1$-$(SO_3)_n^-$, $R^1$-$(PO_3)_n^-$ umfasst, wobei $R^1$ Wasserstoff, ein lineares oder verzweigtes $C_1$ bis $C_8$-Alkyl ist, n = 1 bis 6 ist; und
- **L** ein Abstandshalter-Ligand ist, der ein Radikal $R^0$ und q umfasst, die in einer komplexbildenden Gruppe A vorkommen, wobei

  • q eine ganze Zahl zwischen 2 und 6 ist ;
  • jedes Vorkommen von A unabhängig folgendes ist:

    (i) ein Carboxylat

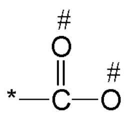
;

    (ii) ein Phosphonat

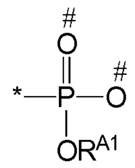

;

oder

(iii) eine Imidazolat-Gruppe

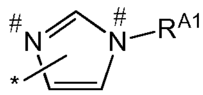

;

worin $R^{A1}$ ein Wasserstoffatom oder ein $C_{1-6}$ Alkylradikal darstellt ;

worin * einen Bindungspunkt der Gruppe A mit dem Radikal $R^0$ bezeichnet;
# mögliche Bindungspunkte der Gruppe A mit dem Metallion M bezeichnet;

• $R^0$ folgende darstellt

• ein $C_{1-12}$Alkylen-, $C_{2-12}$-Alcenylen- oder $C_{2-12}$-Alcynylen-Radikal;
• ein mono- oder polyzyklisches fusioniertes oder nicht fusioniertes Arylradikal, das 6 bis 50 Kohlenstoffatome umfasst,
• ein mono- oder polyzyklisches fusioniertes oder nicht fusioniertes Heteroarylradikal, das 6 bis 50 Kohlenstoffatome umfasst,

wobei das Radikal $R^0$ gegebenenfalls durch eine oder mehrere Gruppen substituiert werden kann, unabhängig ausgewählt aus der Gruppe Halogen, OH, $NH_2$, $NO_2$ oder ein $C_1$ bis $C_6$-Aryl;

in dem die Außenoberfläche des MOFs derart modifiziert ist, dass sie mindestens ein komplexiertes organisches oberflächenaktives Mittel mit einem metallischen Kern M oder einem an der Außenoberfläche des festen kristallinen MOFs befindlichen Liganden L umfasst, wobei das organische oberflächenaktive Mittel folgendes umfasst : i) mindestens eine Phosphat-, Phosphonat-, Bisphosphonat-, Sulfat-, Carboxylat-, Hydroxy-, Catecholat-, Thiolat- (-SR oder $-S^-$), heterozyklischen Stickstoff-, Amido ($-C(=O)N(R)_2$), Amino ($-N(R)_2$)-Rest, oder eine Kombination dieser Gruppen umfasst, wobei jedes Vorkommen von R unabhängig H, $C_{1-6}$ Alkyl oder Phenyl darstellt);

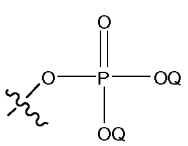

Phosphat

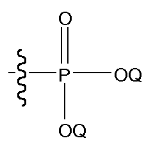

Phosphonat

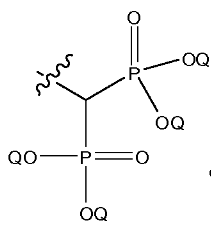

Bisphosphonat

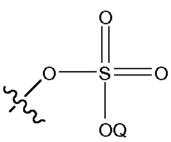

Sulfat

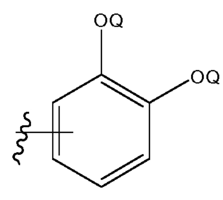

Catecholat

worin jedes Vorkommen von Q unabhängig H oder ein Alkalimetallkation darstellt;
und/oder ii) eine Dimension der kurzen Achse des festen Volumens ist, das von dem oberflächenaktiven Mittel eingenommen wird und grösser als die Zugangsfenster zu Poren größerer Dimension des MOF-Materials ist;
das organische oberflächenaktive Mittel ausgewählt ist aus einem Monomer, Oligomer oder Cyclodextrin-Polymer; einem verzweigten Polyethylenglycolrest; einem Protein; einem Polysaccharid mit einer Vielzahl von Polyethylenglycolseitenketten; oder einem bei einem 6<pH<8 in Wasser nicht löslichen und bei einem ph< 5 in Wasser löslichen Polysacharid;
wobei das organische oberflächenaktive Mittel in Wechselwirkung mit dem metallischen Kern M steht oder einen Liganden L an der Oberfläche des festen kristallinen MOF hat über die eine oder mehrere Phosphat-, Phosphonat-, Bisphosphonat-, Sulfat-, Carboxylat-, Hydroxy-, Catecholat-, Thiolat-, heterozyklische Stickstoff-, Amido-, Amino- -

Gruppe(n), oder eine Kombination dieser Gruppen.

2. Feststoff nach Anspruch 1, worin der Ligand L ein Abstandshalter mit mehreren komplexierenden Funktionen ist, umfassend Carboxylate, Phosphonate, Imidazolate, wobei das Carboxylat ein Di-, Tri-, Tetra- oder Hexacarboxylat ist, ausgewählt aus der Gruppe umfassend :

| | |
|---|---|
| 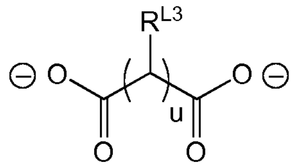 | 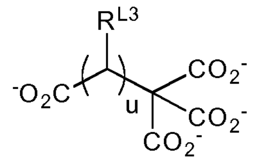 |
| 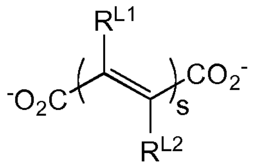 | 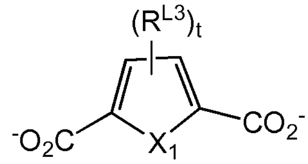 |
| 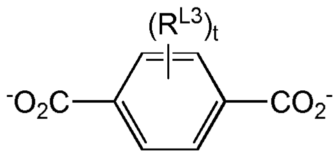 | 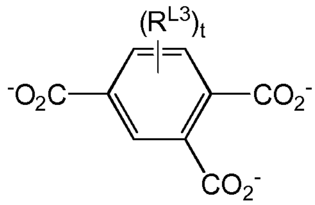 |
| 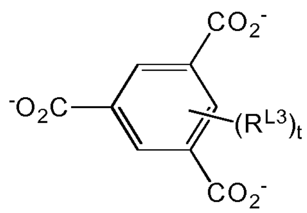 | 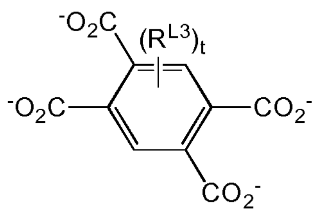 |
| 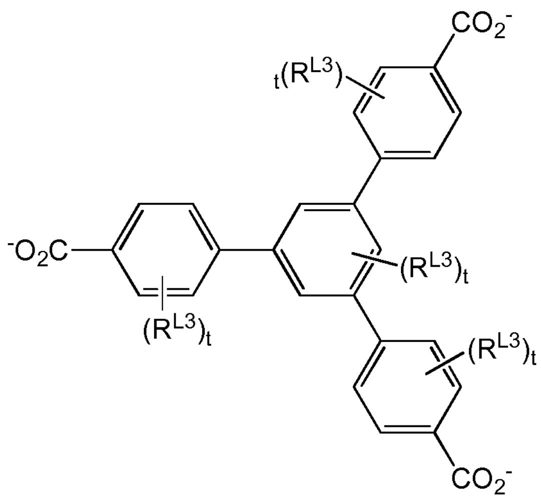 | 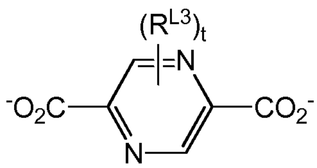 |
| 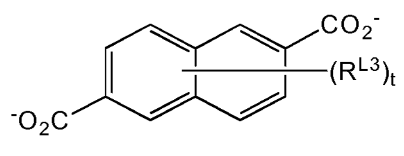 | 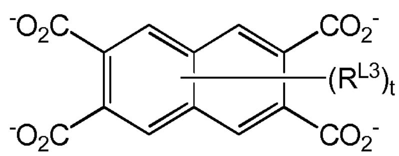 |

(fortgesetzt)

| | |
|---|---|
| 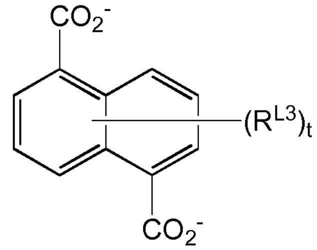 | 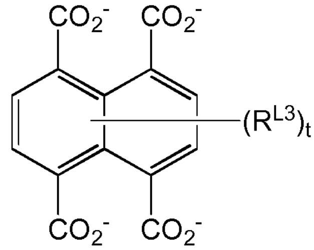 |
| 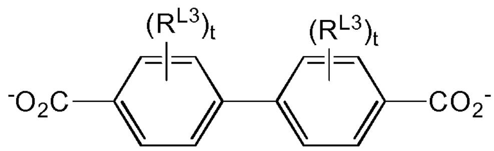 | |
| 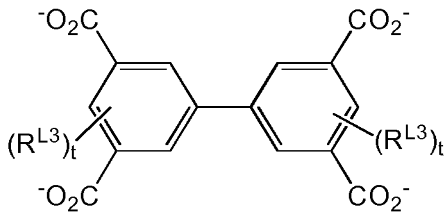 | |
| 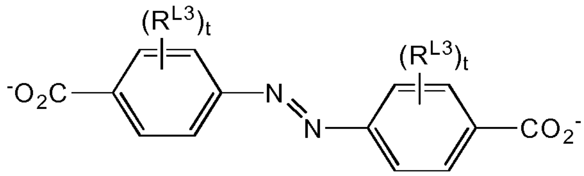 | |
| 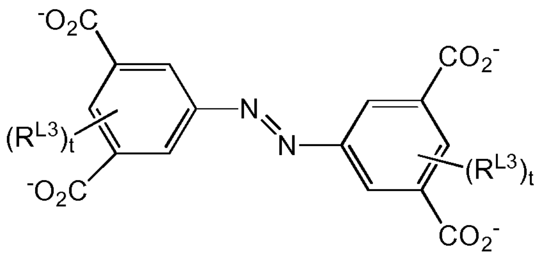 | |
| 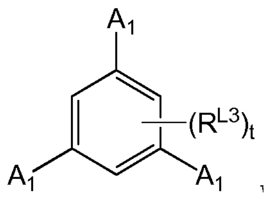 , wobei $A_1$, $A_2$ und $A_3$ unabhängig 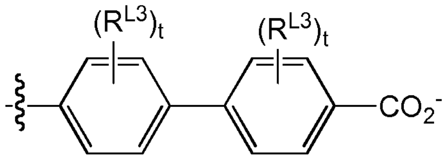 darstellen | |

worin:

$X_1$ O oder S darstellt,
s eine ganze Zahl von 1 bis 4 darstellt,

jedes Vorkommen von t unabhängig eine ganze Zahl von 1 bis 4 darstellt,
u eine ganze Zahl von 1 bis 7 darstellt,
$R^{L1}$ und $R^{L2}$ unabhängig H, ein Halogen oder ein $C_1$ bis $C_6$-Alkyl darstellen, und
jedes Vorkommen von $R^{L3}$ unabhängig H, ein Halogen, OH, $NH_2$, $NO_2$ oder ein $C_1$ bis $C_6$-Alkyl darstellt.

**3.** Feststoff nach Anspruch 1 oder 2, worin der Ligand L ein Di-, Tri- oder Tetra-Carboxylatligand ist, ausgewählt aus der Gruppe umfassend : $C_2H_2(CO_2^-)_2$ (Fumarat), $C_2H_4(CO_2^-)_2$ (Succinat), $C_3H_6(CO_2^-)_2$ (Glutarat), $C_4H_4(CO_2^-)_2$ (Muconat), $C_4H_8(CO_2^-)_2$ (Adipat), $C_7H_{14}(CO_2^-)_2$ (Azelat), $C_5H_3S(CO_2^-)_2$ (2,5-Thiophenedicarboxylat), $C_6H_4(CO_2^-)_2$ (Terephtalat), $C_6H_2N_2(CO_2^-)_2$ (2,5-Pyrazin-Dicarboxylat), $C_{10}H_6(CO_2^-)_2$ (Naphtalin-2,6-Dicarboxylat), $C_{12}H_8(CO_2^-)_2$ (Biphenyl-4,4'-Dicarboxylat), $C_{12}H_8N_2(CO_2^-)_2$ (Azobenzoldicarboxylat), $C_6H_3(CO_2^-)_3$ (Benzol-1,2,4-Tricarboxylat), $C_6H_3(CO_2^-)_3$ (Benzol-1,3,5-Tricarboxylat), $C_{24}H_{15}(CO_2^-)_3$ (Benzol-1,3,5-Tribenzoat), $C_6H_2(CO_2^-)_4$ (Benzol -1,2,4,5-Tetracarboxylat, $C_{10}H_4(CO_2^-)_4$ (Naphtalin-2,3,6,7-Tetracarboxylat), $C_{10}H_4(CO_2^-)_4$ (Naphtalin-1,4,5,8-Tetracarboxylat), $C_{12}H_6(CO_2^-)_4$ (Biphenyl-3,5,3',5'-Tetracarboxylat), und modifizierte Analoga ausgewählt aus der Gruppe umfassend 2-Aminoterephtalat, 2-Nitroterphtalat, 2-Methylterephtalat, 12-Chloroterephtalat, 2-Bromoterephtalat, 2,5-Dihydroxoterephtalat, Tetrafluorterephtalat, Tetramethylterephtalat, Dimethyl-4,4'-Biphenyldicarboxylat, Tetramethyl-4,4'-Biphenyldicarboxylat, Dicarboxy-4,4'-Biphenyldicarboxylat, 2,5-Pyrazyndicarboxylat, 2,5 Diperfluorterephthalat, Azobenzol 4,4'-Dicarboxylat, 3,3'-Dichloroazobenzol 4,4'-Dicarboxylat, 3,3'-Dihydroxoazobenzol 4,4'-Dicarboxylat, 3,3'-Diperfluorazobenzol 4,4'-Dicarboxylat, 3,5,3',5'-Azobenzoltetracarboxylat, 2,5-Dimethylterephthalat, Perfluorsuccinat, Perfluormuconat, Perfluorglutarat, 3,5,3',5' Perfluor-4,4'-Azobenzoldicarboxylat, 3,3'-Diperfluorazobenzol 4,4'-Dicarboxylat.

**4.** Feststoff nach einem der Ansprüche 1 bis 3, worin der Ligand L ein fluorierter Ligand ist, ausgewählt aus der Gruppe umfassend Tetrafluorterephtalat, Perfluorsuccinat, Perfluormuconat, Perfluorglutarat, 2,5 Diperfluorterephtalat, 3,6 Perfluor 1,2,4,5 Benzoltetracarboxylat, 3,5,3',5' Perfluor-4,4'-Azobenzoldicarboxylat, 3,3'-Diperfluorazobenzol 4,4'-Dicarboxylat.

**5.** Feststoff nach einem der Ansprüche 1 bis 3, in dem der Ligand L ein biologisch aktiver Ligand ist, ausgewählt aus der Gruppe umfassend $C_7H_{14}(CO_2^-)_2$ ; Aminosalicylat ; Chlodronat, Pamidrontat, Alendronat, Etidronat ; Meprobamat ; Porphyrine umfassend Carboxylat-, Phosphonat und/oder Aminogruppen ; Aminosäuren ; Azobenzole umfassend Carboxylate-, Phosphonate-, und/oder Aminogruppen ; Dibenzofuran-4,6-Dicarboxylat, Dipicolinat; Glutamat, Fumarat, Succinat, Suberat, Adipat, Nicotinat, Nicotinamid, Purine, Pyrimidine.

**6.** Feststoff nach einem der Ansprüche 1 bis 5, worin das organische oberflächenaktive Mittel aus der Gruppe ausgewählt ist, die folgende umfasst, :

- $\alpha$-, $\beta$ oder $\gamma$-Cyclodextrine ;
- Oligomere von $\alpha$-, $\beta$- oder $\gamma$-Cyclodextrinen ;
- Poly-$\alpha$, Poly-$\beta$ oder Poly-$\gamma$-Cyclodextrine,
- Coploymere von $\alpha$, $\beta$ und/oder $\gamma$-Cyclodextrinen,
- PEG-Dendrimere,
- Chitosan,
- Chitosan mit einer Vielzahl von PEG-Seitenketten,
- Albumin,
- Immunoglubuline

umfassend eine oder mehrere Phosphat-, Phosphonat-, Sulfat-, Carboxylat-, Hydroxy-, Catecholat-, Thiolat-, heterocyclische Stickstoff-, Amido- oder Aminogruppen, oder eine Kombination dieser Gruppen.

**7.** Feststoff nach Anspruch 6, worin die Cyclodextrineinheiten von poly-$\alpha$, poly-$\beta$ oder poly-$\gamma$-Cyclodextrin oder das Copolymer von $\alpha$,$\beta$ und/oder $\gamma$-Cyclodextrin miteinander durch Kohlenwasserstoffketten entsprechend der Formel $-O-(CH_2-CHOR^1-CH_2)_n-O-$ verbunden sind, worin n eine ganze Zahl zwischen 1 und 50 ist und in jeder der $(CH_2-CHOR^1-CH_2)$-Einheiten $R^1$ entweder ein Wasserstoffatom oder eine $-CH_2-CHOH-CH_2-O-$Kette bezeichnet, die an eine Cyclodextrineinheit des Polymers oder Copolymers gebunden ist.

**8.** Feststoff nach einem der Ansprüche 1 bis 5, worin das oberflächenchenaktive Mittel ein verzweigtes Polyethylenglycol mit folgender Struktur ist :

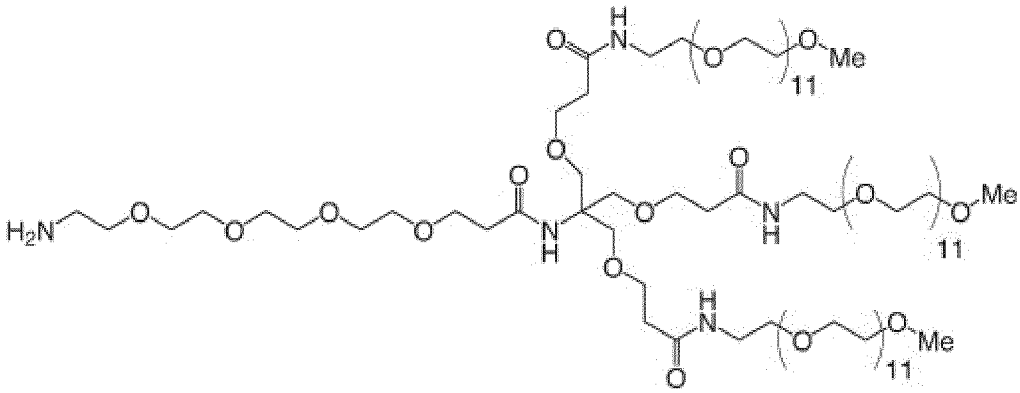
.

9. Feststoff nach einem der Ansprüche 1 bis 8, worin das oberflächenaktive Mittel ferner mit einem fluoreszierenden Molekül funktionalisiert ist.

10. Feststoff nach Anspruch 9, worin das fluoreszierende Molekül Rhodamin, Fluorescein, Luciferase, Pyren und Derivate davon, Aminopyrrolidino-7-Nitrobenzofurazan, oder ein Quantenpunkt ist.

11. Feststoff nach einem der Ansprüche 1 bis 10, wobei der Feststoff in seinen Poren oder an seiner Oberfläche mindestens einen pharmazeutisch aktiven Bestandteil und/oder einen Wirkstoff umfasst, der in der Formulierung einer kosmetischen Zubereitung und/oder als Marker verwendet wird.

12. Feststoff nach Anspruch 11, worin der pharmazeutisch aktive Bestandteil ein Antikrebsmittel, ein antivirales Mittel ein Antibiotikum, ein entzündungshemmendes Mittel oder ein Analgetikum ist.

13. Feststoff nach Anspruch 12, wobei das Antikrebsmittel aus der Gruppe ausgewählt wird, die folgende umfasst: Busulfan, Azidothymidin (AZT), Azidothymidinphosphat (AZTP), Cidofovir, Gemcitabin, Zalcitabin (ddC), Didanosin (ddI), Ibuprofen.

14. Verfahren zur Herstellung eines Feststoffs wie in einem der Ansprüche 1 bis 13 definiert umfassend:

a) mindestens einen Reaktionsschritt (i), der daraus besteht in einem polaren Lösungsmittel folgende zu mischen:

- mindestens eine Lösung, die mindestens einen anorganischen metallischen Vorläufer in Form eines Metalls M, eines Metallsalzes M oder eines Koordinationskomplexes mit einem Metallion M umfasst, worin M so ist, wie in Anspruch 1 definiert ;
- mindestens ein Ligand L' der Formel $-R^0(COR^3)_q$,

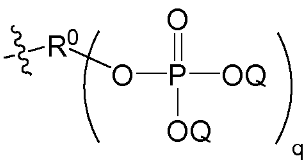 ou 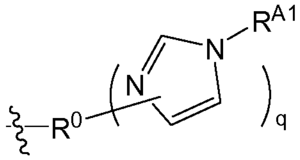 ,

worin Q, $R^{A1}$, q und $R^0$ so sind wie in Anspruch 1 definiert, und $R^3$ aus der Gruppe ausgewählt ist, die ein Radikal -OH, ein Radikal -OY umfasst, worin Y ein Alkalikation, ein Halogen, oder ein-$OR^4$-, -O-C(=O)$R^4$- oder -$NR^4R^{4'}$-Radikal darstellt, worin $R^4$ und $R^{4'}$ $C_{1-12}$-Alkylradikale sind;

b) ein Fixierungsschritt (ii) an der Außenoberfläche des Feststoffs eines organischen oberflächenaktiven Mittels, das folgende umfasst: i) mindestens eine Phosphat-, Phosphonat-, Bisphosphonat-, Sulfat-, Carboxylat-, Hydroxy-, Catecholat-, Thiolat- (-SR oder -S-), heterozyklische Stickstoff-, Amido (-C(=O)N(R)$_2$), Amino (-N(R)$_2$)-Rest, oder eine Kombination dieser Gruppen umfasst, wobei jedes Vorkommen von R unabhängig H, $C_{1-6}$-Alkyl oder Phenyl darstellt);

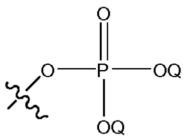 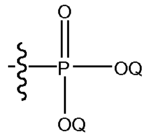 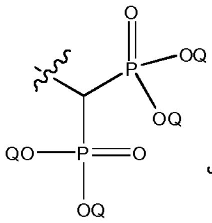 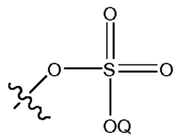 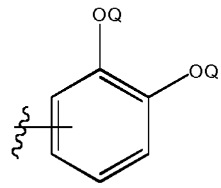

Phosphat Phosphonat Bisphosphonat Sulfat Catecholat

worin jedes Vorkommen von Q unabhängig H oder ein Alkalimetallkation darstellt;
und/oder ii) eine Dimension der kurzen Achse des festen Volumens, das von dem oberflächenaktiven Mittel eingenommen wird und grösser als die Zugangsfenster zu Poren größerer Dimension des MOF-Materials ist;
wobei das organische oberflächenaktive Mittel ausgewählt ist aus einem Monomer, Oligomer oder Cyclodextrin-Polymer; oder einem verzweigten Polyethylenglycolrest; einem Protein; einem Polysaccharid mit einer Vielzahl von Polyethylenglycolseitenketten; oder einem bei einem 6<pH<8 in Wasser nicht löslichen und bei einem ph< 5 in Wasser löslichen Polysacharid;
um das organische oberflächenaktive Mittel zu erhalten, worin das organische oberflächenaktive Mittel in Wechselwirkung mit dem metallischen Kern M steht oder einen Liganden L an der Oberfläche des festen kristallinen MOF hat über die eine oder mehrere Phosphat-, Phosphonat-, Bisphosphonat-, Sulfat-, Carboxylat-, Hydroxy-, Catecholat-, Thiolat-, heterozyklische Stickstoff-, Amido, Amino-Gruppe(n), oder eine Kombination dieser Gruppen.

**15.** Verfahren nach Anspruch 14, worin der Ligand L' ein Ligand mit mehreren komplexierenden Funktionen darstellt, umfassend Carboxylate, Phosphonate, Imidazolate, wobei die Carboxylatgruppe ein Di-, Tri-, Tetra- oder Hexacarboxylatligand ist, ausgewählt aus der Gruppe umfassend:

| | |
|---|---|
| 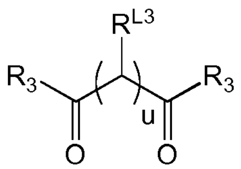 | 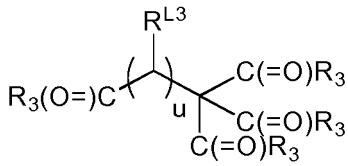 |
| 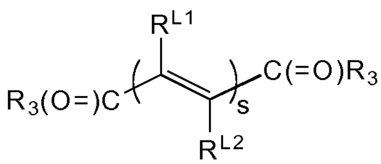 | 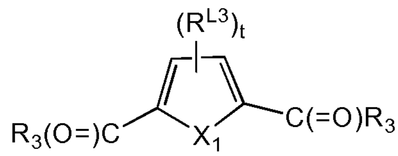 |
| 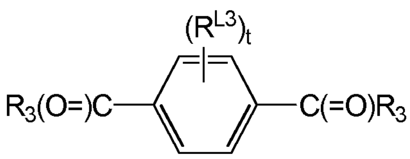 | 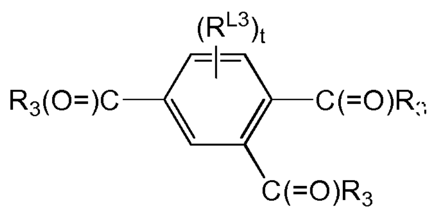 |
| 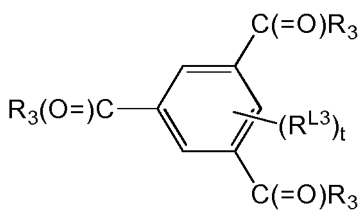 | 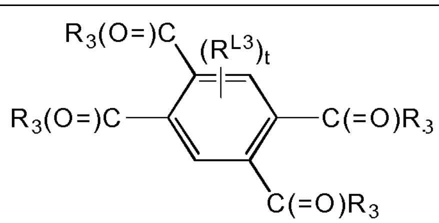 |

(fortgesetzt)

| | |
|---|---|
| 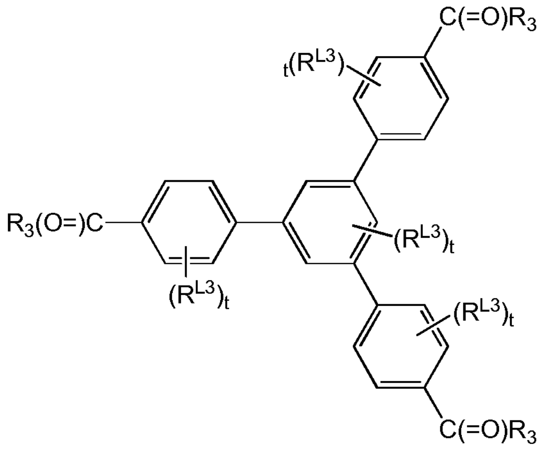 | 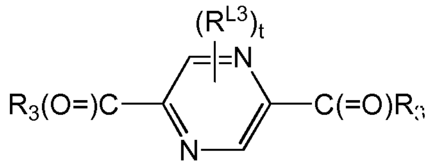 |
| 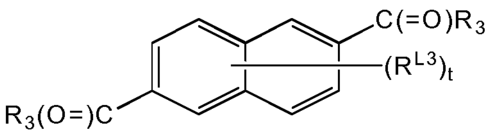 | 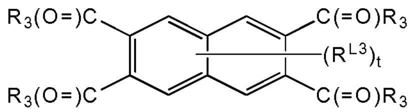 |
| 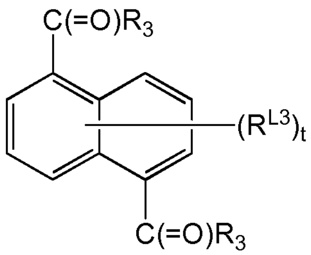 | 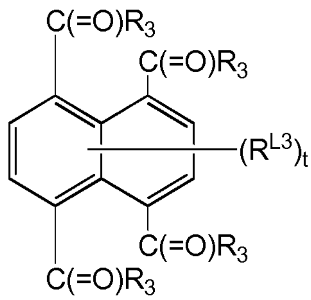 |
| 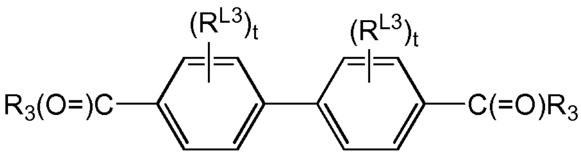 | |
| 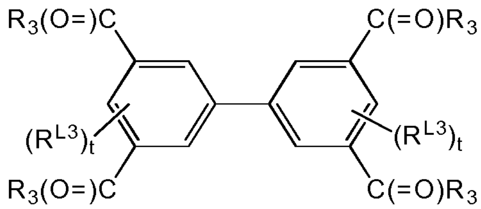 | |
| 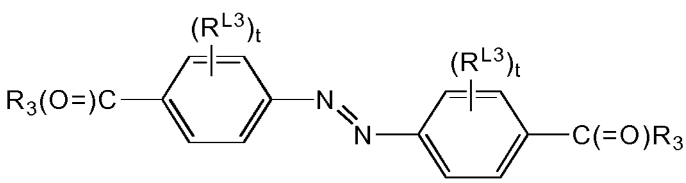 | |

(fortgesetzt)

| |
|---|
| 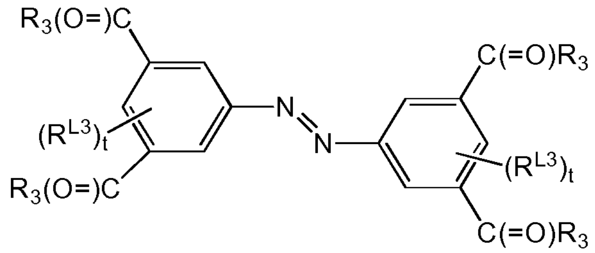 |
| 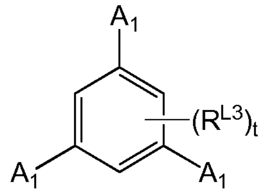 worin $A_1$, $A_2$ und $A_3$ unabhängig 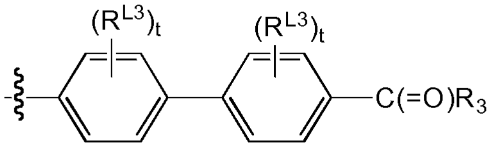 darstellen |

worin:

$R^3$ aus der Gruppe ausgewählt ist, die ein Radikal -OH, ein Radikal -OY umfasst, worin Y ein Alkalikation, ein Halogen, oder ein-$OR^4$-, -O-C(=O)$R^4$- oder -$NR^4R^{4'}$-Radikal darstellt, worin $R^4$ und $R^{4'}$ $C_{1-12}$-Alkylradikale sind,
$X_1$ O oder S darstellt,
s eine ganze Zahl von 1 bis 4 darstellt,
jedes Vorkommen von t unabhängig eine ganze Zahl von 1 bis 4 darstellt,
u eine ganze Zahl von 1 bis 7 darstellt,
$R^{L1}$ und $R^{L2}$ unabhängig H, ein Halogen oder ein $C_1$ bis $C_6$-Alkyl darstellen, und
jedes Vorkommen von $R^{L3}$ unabhängig H, ein Halogen, OH, $NH_2$, $NO_2$ oder ein $C_1$ bis $C_6$-Alkyl darstellt.

**16.** Verfahren nach einem der Ansprüche 14 bis 15, worin das organische oberflächenaktive Mittel so ist, wie in einem der Ansprüche 1 und 6 bis 10 definiert.

**17.** Verfahren zur Herstellung eines Feststoffs nach einem der Ansprüche 14 bis 16, worin der Reaktionsschritt (i) unter mindestens einer der nachfolgenden Reaktionsbedingungen durchgeführt wird :

(i) eine Reaktionstemperatur von 0°C bis 220°C ;
(ii) eine Rührgeschwindigkeit von 0 bis 1000 rpm , wobei 1 rpm 1/60 Hz ist;
(iii) eine Reaktionszeit von 1 Minute bis 96 Stunden ;
(iv) ein pH-Wert von 0 bis 7 ;
(v) das Hinzufügen von mindestens einem Co-Lösungsmittel zu dem Lösungsmittel, dem Vorläufer, dem Liganden oder einer Mischung hiervon, wobei das Co-Lösungsmittel aus der Gruppe ausgewählt ist, die Essigsäure, Ameisensäure, Benzoesäure umfasst ;
(vi) das Co-Lösungsmittel aus der Gruppe ausgewählt ist, die Wasser, $R^S$-OH-Alkohole umfasst, worin $R^S$ ein lineares oder verzweigtes $C_1$ bis $C_6$ - Alkylradikal ist, Dimethylformamid, Dimethylsulfoxid, Acetonitril, Tetrahydrofuran, Diethylformamid, Chloroform, Cyclohexan, Aceton, le Cyanobenzol, Dichloromethan, Nitrobenzol, Ethylenglycol, Dimethylacetamid oder Mischungen dieser Lösungsmittel, mischbar oder nicht;
(vii) in einem überkritischen Medium ;
(viii) unter Mikrowellen und/oder Ultraschall ;
(ix) unter elektrochemischen Elektrolysebedingungen;
(x) unter Bedingungen mit Verwendung einer Walzmühle ;
(xi) in einem Gasstrom.

**18.** Verfahren nach einem der Ansprüche 14 bis 17, ferner umfassend einen Schritt (iii) des Einbringens mindestens eines pharmazeutisch aktiven Bestandteils in den Feststoff.

**19.** Feststoff nach einem der Ansprüche 1 bis 13 zu seiner Verwendung als Medikament.

**20.** Feststoff nach einem der Ansprüche 1 bis 13 zu seiner Verwendung in der Krebsbehandlung.

**21.** Verwendung eines Feststoffs nach einem der Ansprüche 1 bis 13 zur Herstellung eines Markers, der in der Medizinbildtechnik verwendet werden kann.

## Claims

**1.** A porous crystalline MOF solid comprising a three-dimensional succession of units having the following formula (I):

$M_mO_kX_lL_p$ Formula **(I)**

wherein:

each occurrence of **M** independently represents a metal ion selected from the group comprising $Fe^{2+}$, $Fe^{3+}$, $Zn^{2+}$, $Zr^{4+}$, $Ti^{4+}$, $Ca^{2+}$, $Mg^{2+}$ and $Al^{3+}$;

- **m**, **k**, **l and p** numbers $\geq 0$ selected so as to respect charge neutrality of the unit;
- **X** is a ligand selected from the group comprising $OH^-$, $Cl^-$, $F^-$, $I^-$, $Br^-$, $SO_4^{2-}$, $NO_3^-$, $ClO_4^-$, $R^1-(COO)_n^-$, $R^1-(S0_3)_n^-$, $R^1-(PO_3)_n^-$, wherein $R^1$ is a hydrogen atom, a linear or branched $C_1$ to $C_8$ alkyl, n = 1 to 6; and
- **L** is a spacer ligand comprising a radical $R^0$ and q occurrences of a complexing group A, where

  • q is an integer between 2 and 6;
  • each occurrence of A is independently:

(i) a carboxylate

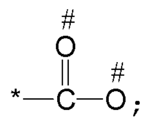

(ii) a phosphonate

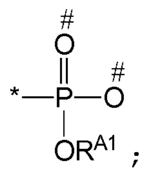

or
(iii) an imidazolate group

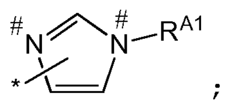

wherein $R^{A1}$ represents a hydrogen atom or a $C_{1-6}$alkyl radical;

wherein * denotes the point of attachment of group A to the radical $R^0$;
# denotes the possible points of attachment of group A to the metal ion M;

- $R^0$ represents
  - a $C_{1-12}$alkylene, $C_{2-12}$alkenylene or $C_{2-12}$alkynylene radical;
  - a mono- or polycyclic aryl radical, fused or not, comprising from 6 to 50 carbon atoms,
  - a mono- or polycyclic heteroaryl radical, fused or not, comprising from 4 to 50 carbon atoms,

the radical $R^0$ optionally being substituted with one or more groups independently selected from the group halogen atom, OH, $NH_2$, $NO_2$ or a $C_1$ to $C_6$ alkyl;

wherein the outer surface of the MOF is modified in that it comprises at least one organic surface agent complexed with a metal center M or with a ligand L located on the outer surface of the crystalline MOF solid, the organic surface agent comprising: i) at least one phosphate, phosphonate, bisphosphonate, sulfate, carboxylate, hydroxy, cathecolate, thiolate (-SR or $-S^-$), N-containing heterocyclic, amido ($-C(=O)N(R)_2$), amino ($-N(R)_2$) group, or a combination of these groups, wherein each occurrence of R represents independently H, $C_{1-6}$alkyl or phenyl);

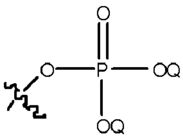
Phosphate

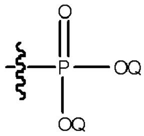
Phosphonate

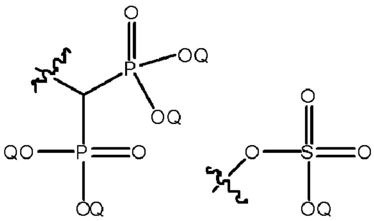
Bisphosphonate Sulfate

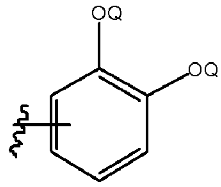
Cathecolate

wherein each occurrence of Q represents independently H or an alkali metal cation;
and/or ii) a dimension of the minor axis of the rigid volume occupied by the surface agent, that is larger than the largest sized pore access windows of the MOF material;
the organic surface agent being selected from a cyclodextrin monomer, oligomer or polymer; a branched polyethylene glycol group; a protein; a polysaccharide bearing a plurality of polyethylene glycol side chains; or a polysaccharide that is water-insoluble at 6<pH<8 and water-soluble at pH<5;
said organic surface agent interacting with a metal center M or with a ligand L located on the surface of the crystalline MOF solid via said one or more phosphate, phosphonate, bisphosphonate, sulfate, carboxylate, hydroxy, cathecolate, thiolate, N-containing heterocyclic, amido, amino group(s), or a combination of these groups.

2. A solid according to claim 1, wherein the ligand L is a spacer bearing several complexing groups selected from carboxylate, phosphonate, imidazolates group, wherein the carboxylate group is a di-, tri-, tetra- or hexa-carboxylate selected from the group comprising:

| | |
|---|---|
| 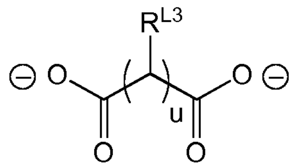 | 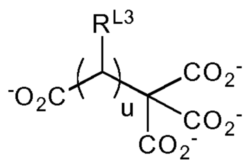 |
| 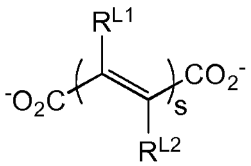 | 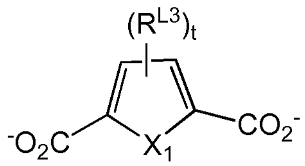 |

(continued)

| | |
|---|---|
| 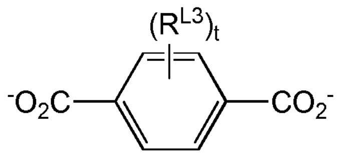 | 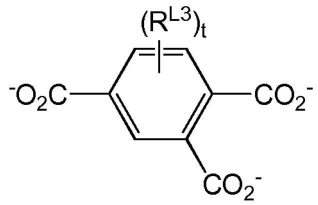 |
| 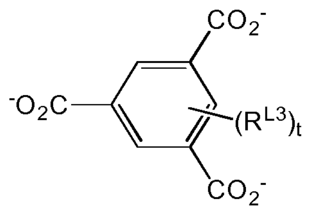 | 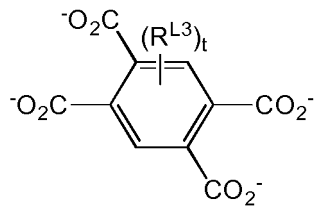 |
| 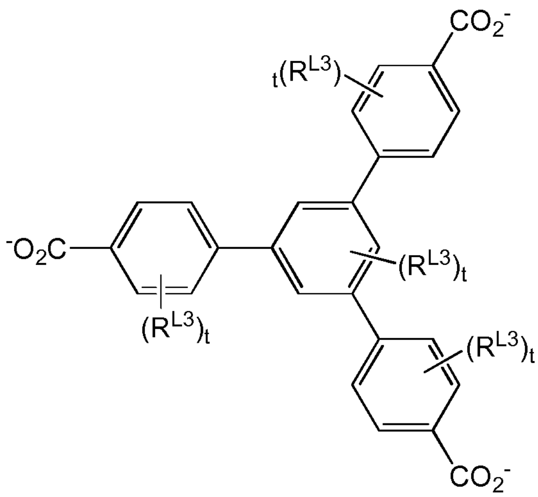 | 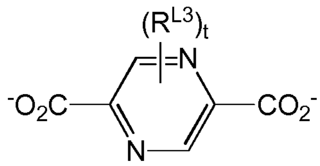 |
| 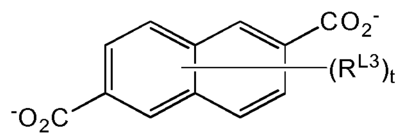 | 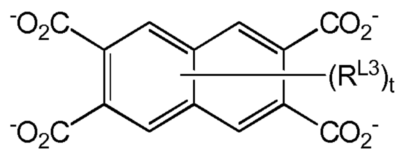 |
| 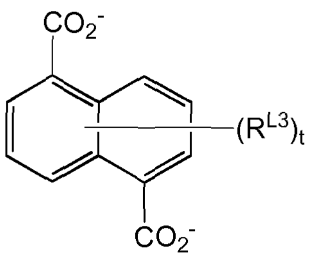 | 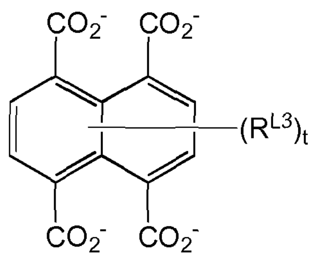 |
| 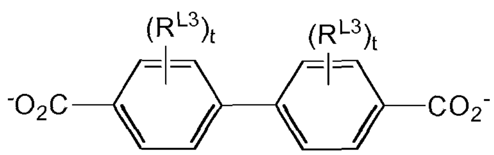 | |

(continued)

| |
|---|
| 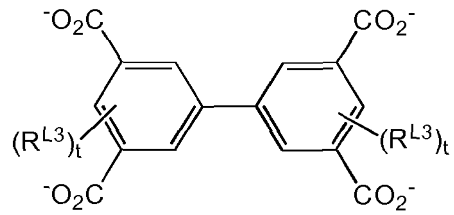 |
| 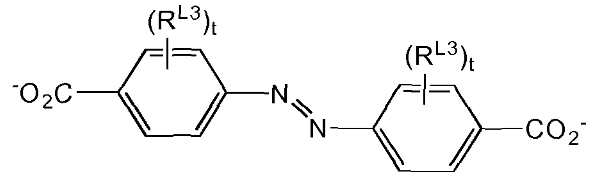 |
| 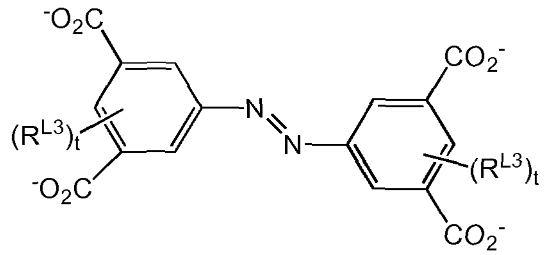 |
| 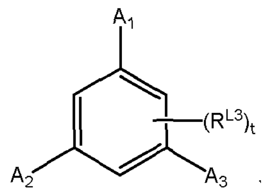 wherein $A_1$, $A_2$ and $A_3$ represent independently 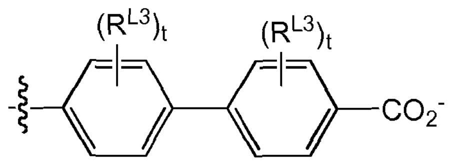 |

wherein:

$X_1$ represents O or S,
s represents an integer from 1 to 4,
each occurrence of t represents independently an integer from 1 to 4,
u represents an integer from 1 to 7,
$R^{L1}$ and $R^{L2}$ represent independently H, a halogen atom or a $C_1$ to $C_6$ alkyl, and
each occurrence of $R^{L3}$ represents independently H, a halogen atom, OH, $NH_2$, $NO_2$ or a $C_1$ to $C_6$ alkyl.

**3.** A solid according to claim 1 or 2, wherein the ligand L is a di-, tri- or tetracarboxylate ligand selected from the group comprising: $C_2H_2(CO_2^-)_2$ (fumarate), $C_2H_4(CO_2^-)_2$ (succinate), $C_3H_6(CO_2^-)_2$ (glutarate), $C_4H_4(CO_2^-)_2$ (muconate), $C_4H_8(CO_2^-)_2$ (adipate), $C_7H_{14}(CO_2^-)_2$ (azelate), $C_5H_3S(CO_2^-)_2$ (2,5-thiophenedicarboxylate), $C_6H_4(CO_2^-)_2$ (terephthalate), $C_6H_2N_2(CO_2^-)_2$ (2,5-pyrazine dicarboxylate), $C_{10}H_6(CO_2^-)_2$ (naphthalene-2,6-dicarboxylate), $C_{12}H_8(CO_2^-)_2$ (biphenyl-4,4'-dicarboxylate), $C_{12}H_8N_2(CO_2^-)_2$ (azobenzenedicarboxylate), $C_6H_3(CO_2^-)_3$ (benzene-1,2,4-tricarboxylate), $C_6H_3(CO_2^-)_3$ (benzene-1,3,5-tricarboxylate), $C_{24}H_{15}(CO_2^-)_3$ (benzene-1,3,5-tribenzoate), $C_6H_2(CO_2^-)_4$ (benzene-1,2,4,5-tetracarboxylate, $C_{10}H_4(CO_2^-)_4$ (naphthalene-2,3,6,7-tetracarboxylate), $C_{10}H_4(CO_2^-)_4$ (naphthalene-1,4,5,8-tetracarboxylate), $C_{12}H_6(CO_2^-)_4$ (biphenyl-3,5,3',5'-tetracarboxylate), and the modified analogs selected from the group comprising 2-aminoterephthalate, 2-nitroterephthalate, 2-methylterephthalate, 2-chloroterephthalate

thalate, 2-bromoterephthalate, 2,5-dihydroxoterephthalate, tetrafluoroterephthalate, tetramethylterephthalate, dimethyl-4,4'-biphenyldicarboxylate, tetramethyl-4,4'-biphenyldicarboxylate, dicarboxy-4,4'-biphenyldicarboxylate, 2,5-pyrazyne dicarboxylate, 2,5-diperfluoroterephthalate, azobenzene-4,4'-dicarboxylate, 3,3'-dichloro azobenzene-4,4'-dicarboxylate, 3,3'-dihydroxo azobenzene-4,4'-dicarboxylate, 3,3'-diperfluoro-azobenzene-4,4'-dicarboxylate, 3,5,3',5'-azobenzene tetracarboxylate, 2,5-dimethylterephthalate, perfluorosuccinate, perfluoromuconate, perfluoroglutarate, 3,5,3',5' perfluoro-4,4'-azobenzene dicarboxylate, 3,3'-diperfluoro-azobenzene-4,4'-dicarboxylate.

**4.** A solid according to any one of claims 1 to 3, wherein the ligand L is a fluorinated ligand selected from the group comprising tetrafluoroterephthalate, perfluorosuccinate, perfluoromuconate, perfluoroglutarate, 2,5-diperfluoroterephthalate, 3,6-perfluoro-1,2,4,5-benzenetetracarboxylate, 3,5,3',5'-perfluoro-4,4'-azobenzene dicarboxylate, 3,3'-diperfluoro-azobenzene-4,4'-dicarboxylate.

**5.** A solid according to any one of claims 1 to 3, wherein the ligand L is a biologically active ligand selected from the group comprising $C_7H_{14}(CO_2^-)_2$; aminosalicylate; clodronate, pamidrontate, alendronate, etidronate; meprobamate; porphyrins comprising carboxylate, phosphonate and/or amino groups; amino acids; azobenzenes comprising carboxylate, phosphonate, and/or amino groups; dibenzofuran-4,6-dicarboxylate, dipicolinate; glutamate, fumarate, succinate, suberate, adipate, nicotinate, nicotinamide, purines, pyrimidines.

**6.** A solid according to any one of claims 1 to 5, wherein the organic surface agent is selected from the group comprising:

- α-, β- or γ-cyclodextrins;
- oligomers of α-, β- or γ-cyclodextrins;
- poly-α, poly-β or poly-γ-cyclodextrins,
- copolymers of α, β and/or γ-cyclodextrins,
- PEG dendrimers,
- chitosan,
- chitosan bearing a plurality of PEG side chains,
- albumin,
- immunoglobulins

comprising one or more phosphate, phosphonate, sulfate, carboxylate, hydroxy, cathecolate, thiolate, nitrogen heterocyclic, amido or amino group(s), or a combination of these groups.

**7.** The solid as claimed in claim 6, wherein the cyclodextrin units of the poly-α, poly-β or poly-γ-cyclodextrin or the copolymer of α, β and/or γ-cyclodextrin are linked together by hydrocarbon chains of formula $-O-(CH_2-CHOR^1-CH_2)_n-O-$ wherein n is an integer between 1 and 50 and, in each of the units $(CH_2-CHOR^1-CH_2)$, $R^1$ denotes either a hydrogen atom, or a $-CH_2-CHOH-CH_2-O-$ chain bound to a cyclodextrin unit of said polymer or copolymer.

**8.** A solid according to any one of claims 1 to 5, wherein the organic surface agent is a branched polyethylene glycol having the following structure:

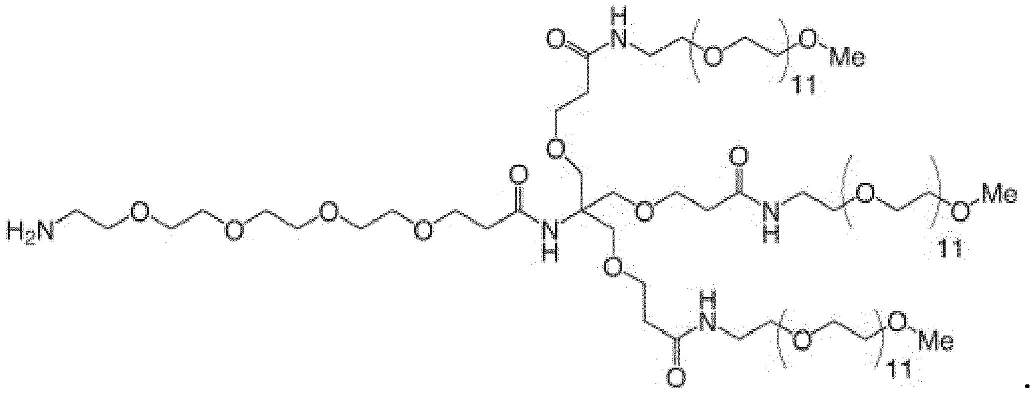

**9.** A solid according to any one of claims 1 to 8, wherein the organic surface agent is further functionalized with a

fluorescent molecule.

**10.** A solid according to claim 9, wherein the fluorescent molecule is rhodamine, fluorescein, luciferase, pyrene or derivatives thereof, aminopyrrolidino-7-nitrobenzofurazan, or a quantum dot.

**11.** A solid according to any one of claims 1 to 10, said solid comprising in its pores or on its surface at least one pharmaceutically active ingredient and/or an active substance included in the formulation of a cosmetic preparation and/or a marker.

**12.** A solid according to claim 11, wherein the pharmaceutically active ingredient is an anticancer, antiviral, antibiotic, anti-inflammatory or analgesic agent.

**13.** A solid according to claim 12, wherein the anticancer agent is selected from the group comprising: busulfan, azidothymidine (AZT), azidothymidine phosphate (AZTP), cidofovir, gemcitabine, zalcitabine (ddC), didanosine (ddI), ibuprofen.

**14.** A method for preparing a solid as defined in any one of claims 1 to 13, comprising:

a) at least one reaction step (i) consisting in mixing, in a polar solvent:

- at least one solution comprising at least one inorganic metal precursor, said precursor being in the form of metal M, of a salt of metal M or of a coordination complex comprising the metal ion M wherein M is as defined in claim 1;
- at least one ligand L' of formula $-R^0(COR^3)_q$,

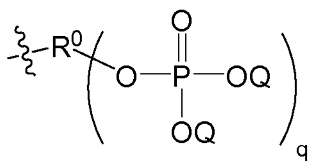 or 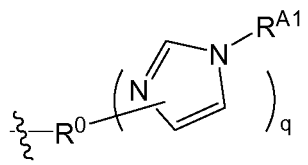 ,

wherein Q, $R^{A1}$, q and $R^0$ are as defined in claim 1, and $R^3$ is selected from the group comprising a radical -OH, a radical -OY wherein Y represents an alkali metal cation, a halogen atom, or a radical $-OR^4$, $-O-C(=O)R^4$ or $-NR^4R^{4'}$, wherein $R^4$ and $R^{4'}$ are $C_{1-12}$ alkyl radicals;

b) a step (ii) of fixation, on the outer surface of said solid, of at least one organic surface agent comprising: i) at least one phosphate, phosphonate, bisphosphonate, sulfate, carboxylate, hydroxy, cathecolate, thiolate (-SR or $-S^-$), N-containing heterocyclic, amido ($-C(=O)N(R)_2$), amino ($-N(R)_2$) group, or a combination of these groups, wherein each occurrence of R represents independently H, $C_{1-6}$alkyl or phenyl;

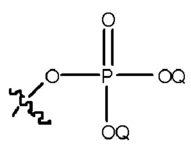
Phosphate

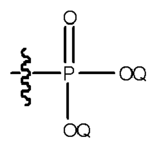
Phosphonate

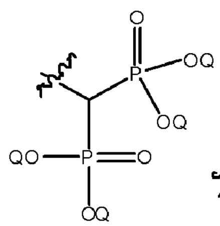
Bisphosphonate

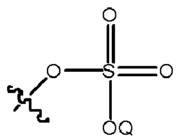
Sulfate

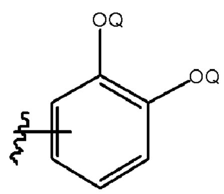
Cathecolate

wherein each occurrence of Q represents independently H or an alkali metal cation;
and/or ii) a dimension of the minor axis of the rigid volume occupied by the surface agent, that is larger than the largest sized pore access windows of the MOF material the organic surface agent being selected from a cyclodextrin monomer, oligomer or polymer; or a branched polyethylene glycol group; a protein; a polysaccharide bearing a plurality of polyethylene glycol side chains; or a polysaccharide that is water-insoluble at 6<pH<8 and water-soluble at pH<5;
so as to obtain said solid wherein said organic surface agent interacts with a metal center M or with a ligand L on

the surface of the crystalline MOF solid via said one or more phosphate, phosphonate, bisphosphonate, sulfate, carboxylate, hydroxy, cathecolate, thiolate, N-containing heterocyclic, amido, amino group(s), or a combination of these groups.

**15.** A method according to claim 14, wherein the ligand L' represents a ligand bearing several complexing groups comprising carboxylate, phosphonate, imidazolate groups, wherein the carboxylate group is a di-, tri-, tetra- or hexadentate ligand selected from the group comprising:

| | |
|---|---|
| 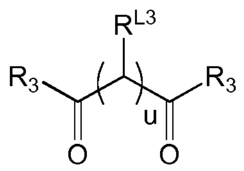 | 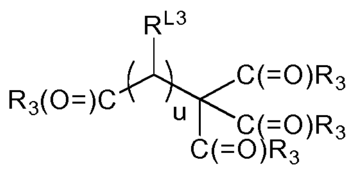 |
| 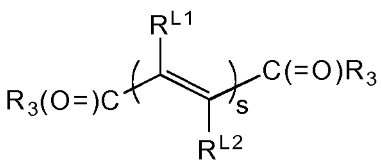 | 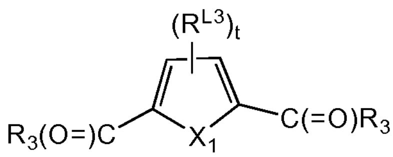 |
| 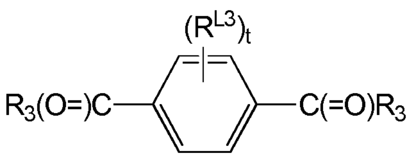 | 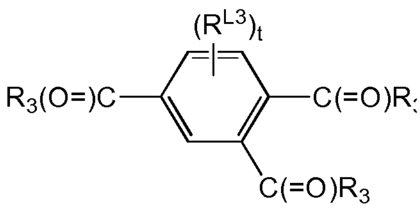 |
| 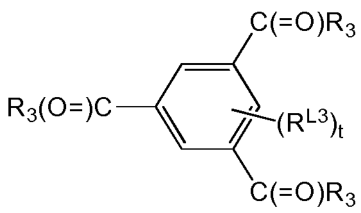 | 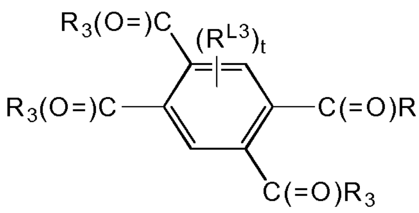 |
| 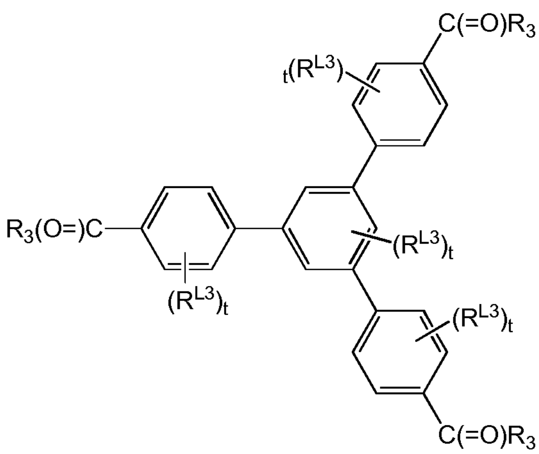 | 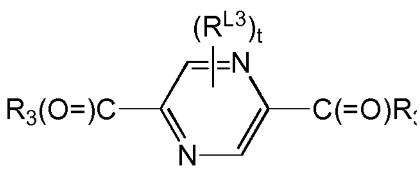 |
| 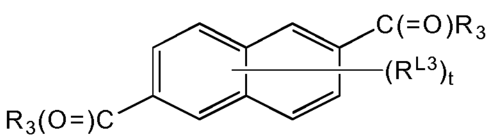 | 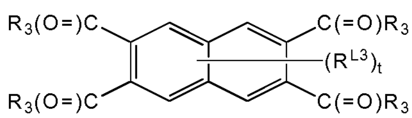 |

(continued)

| | |
|---|---|
| 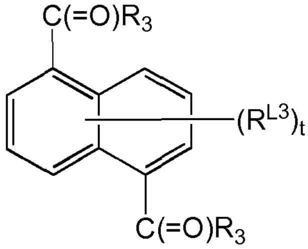 | 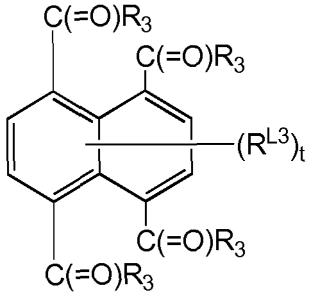 |
| 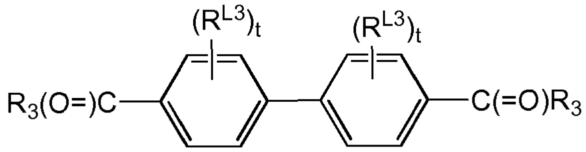 | |
| 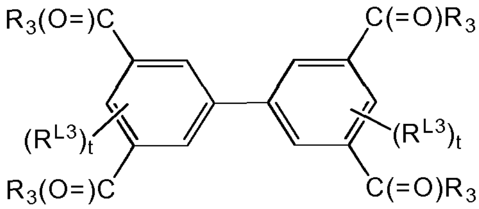 | |
| 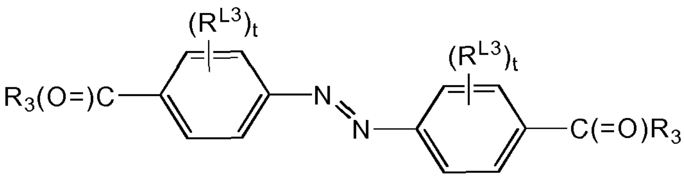 | |
| 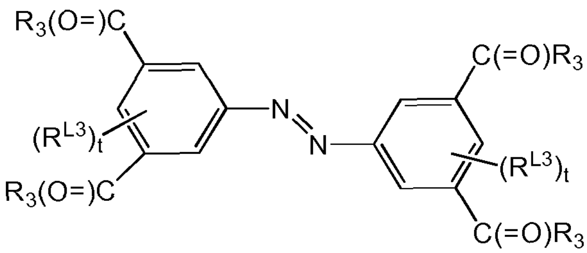 | |
| 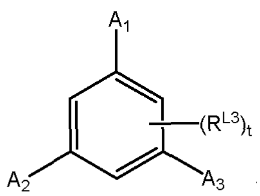 wherein $A_1$, $A_2$ and $A_3$ represent independently | |
| 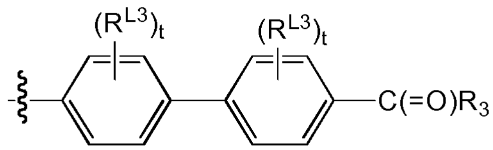 | |

wherein:

$R_3$ is selected from the group comprising a radical -OH, a radical -OY wherein Y represents an alkali metal

cation, a halogen atom, or a radical $-OR^4$, $-O-C(=O)R^4$ or $-NR^4R^{4'}$, wherein $R^4$ and $R^{4'}$ are $C_{1-12}$ alkyl radicals,
$X_1$ represents O or S,
s represents an integer from 1 to 4,
each occurrence of t represents independently an integer from 1 to 4,
u represents an integer from 1 to 7,
$R^{L1}$ and $R^{L2}$ represent independently H, a halogen atom or a $C_1$ to $C_6$ alkyl, and

each occurrence of $R^{L3}$ represents independently H, a halogen atom, OH, $NH_2$, $NO_2$ or a $C_1$ to $C_6$ alkyl.

**16.** A method according to any one of claims 14 to 15, wherein the organic surface agent is as defined in any one of claims 1 and 6 to 10.

**17.** A method for preparing the solid as claimed in any one of claims 14 to 16, wherein reaction step (i) is carried out with at least one of the following reaction conditions:

(i) a reaction temperature from 0°C to 220°C;
(ii) a stirring speed from 0 to 1000 rpm, wherein 1 rpm = 1/60 Hz;
(iii) a reaction time from 1 minute to 96 hours;
(iv) a pH from 0 to 7;
(v) addition of at least one co-solvent to the solvent, to the precursor, to the ligand or to a mixture thereof, said co-solvent being selected from the group comprising acetic acid, formic acid, benzoic acid;
(vi) the solvent is selected from the group comprising water, the alcohols $R^S$-OH wherein $R^S$ is a linear or branched $C_1$ to $C_6$ alkyl radical, dimethylformamide, dimethylsulfoxide, acetonitrile, tetrahydrofuran, diethylformamide, chloroform, cyclohexane, acetone, cyanobenzene, dichloromethane, nitrobenzene, ethylene glycol, dimethylacetamide or mixtures of these solvents, miscible or not;
(vii) in a supercritical medium;
(viii) under microwaves and/or under ultrasound;
(ix) under conditions of electrochemical electrolysis;
(x) under conditions using a rolling crusher;
(xi) in a gas stream.

**18.** A method according to any one of claims 14 to 17, further comprising a step (iii) of introducing at least one pharmaceutically active ingredient into said solid.

**19.** A solid according to any one of claims 1 to 13 for use as a medicament.

**20.** A solid according to any one of claims 1 to 13 for use in the treatment of cancer.

**21.** The use of a solid according to any one of claims 1 to 13 for manufacturing a marker usable in medical imaging.

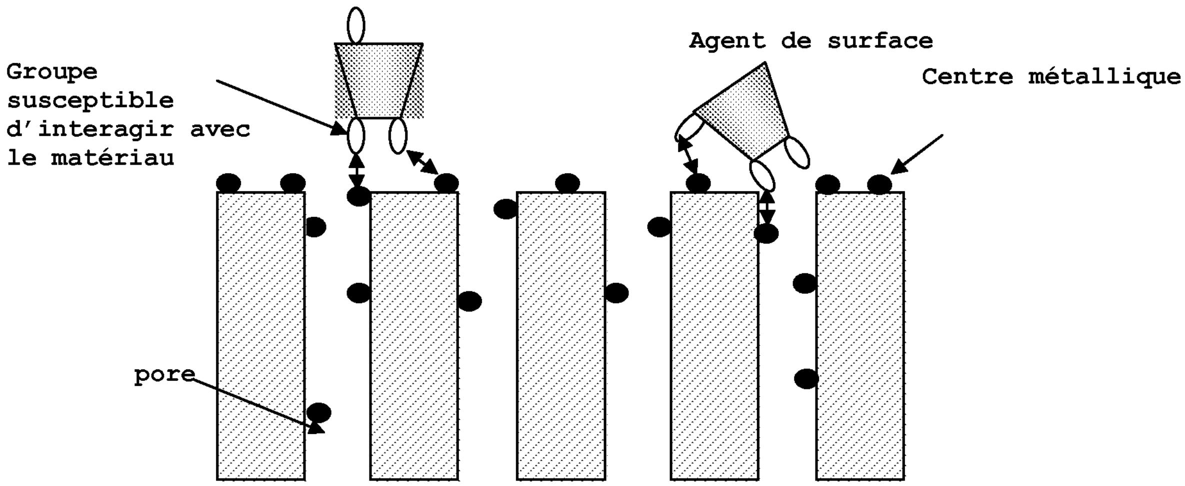


Figure 1A

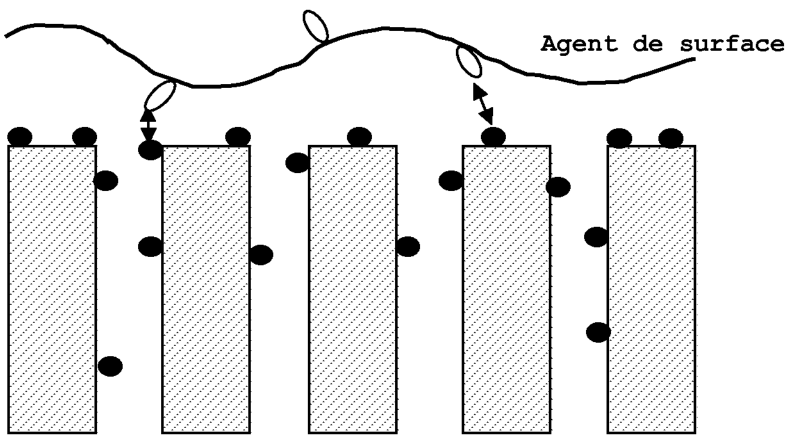


Figure 1B

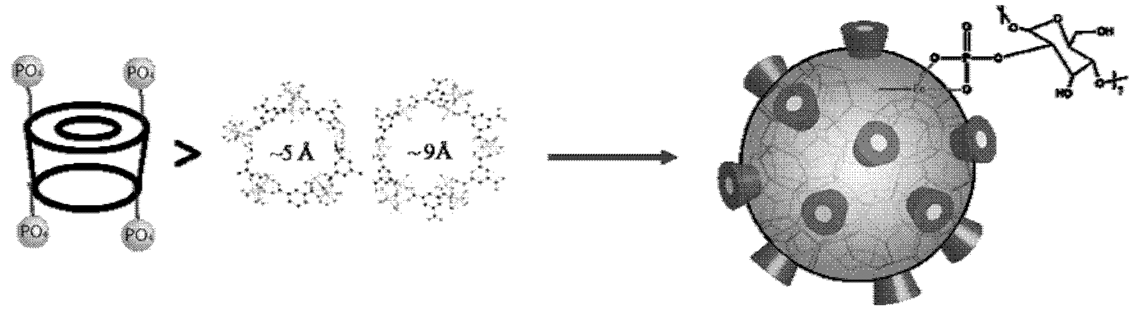


Figure 2

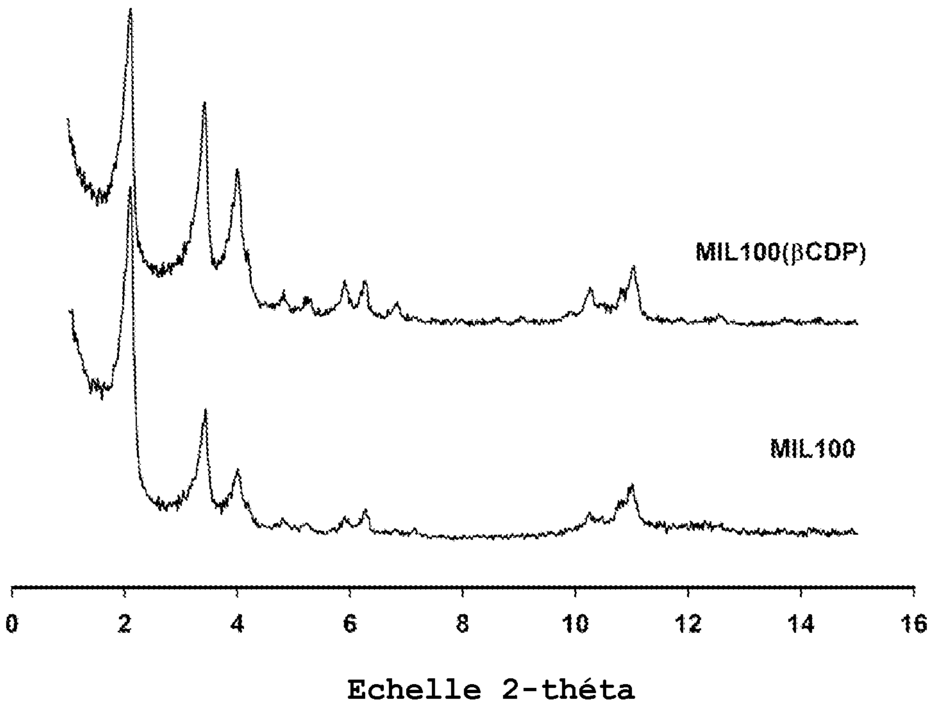


Figure 3

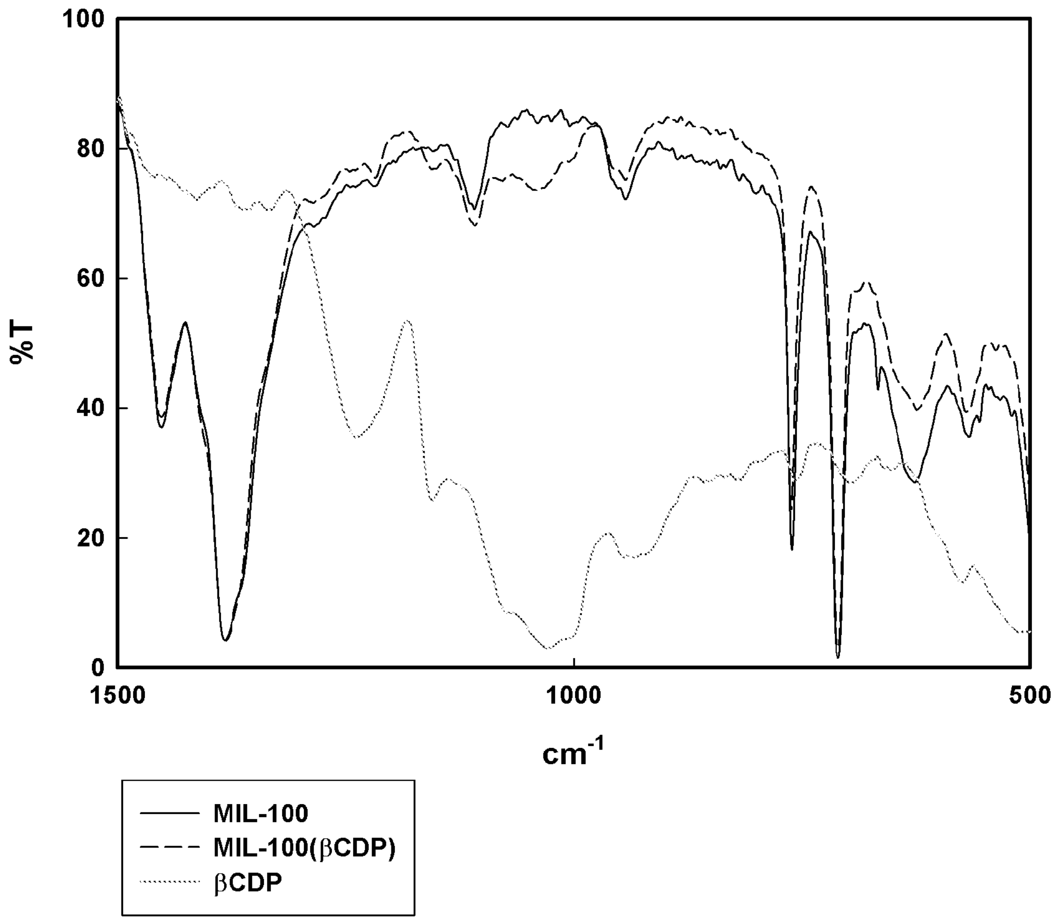


Figure 4

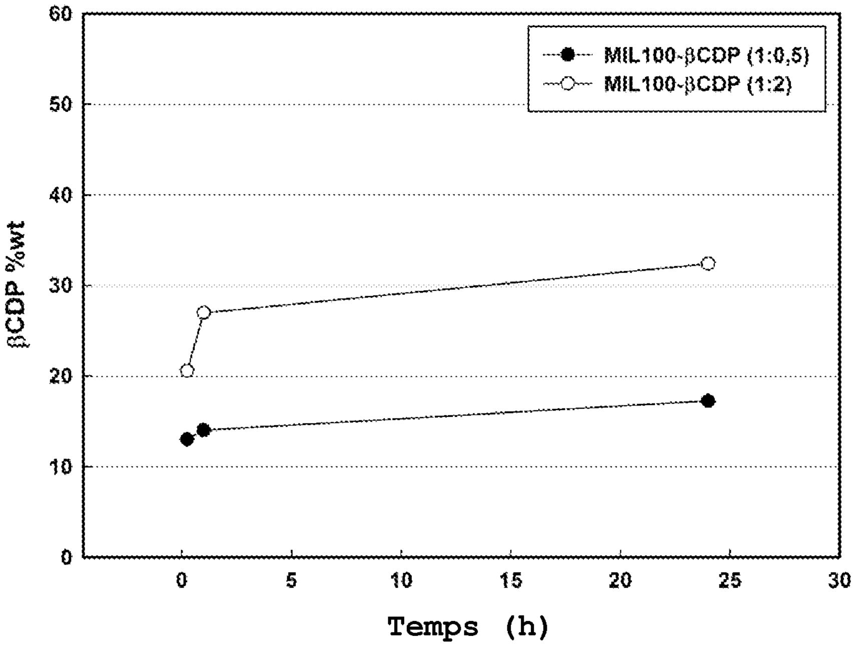


Figure 5

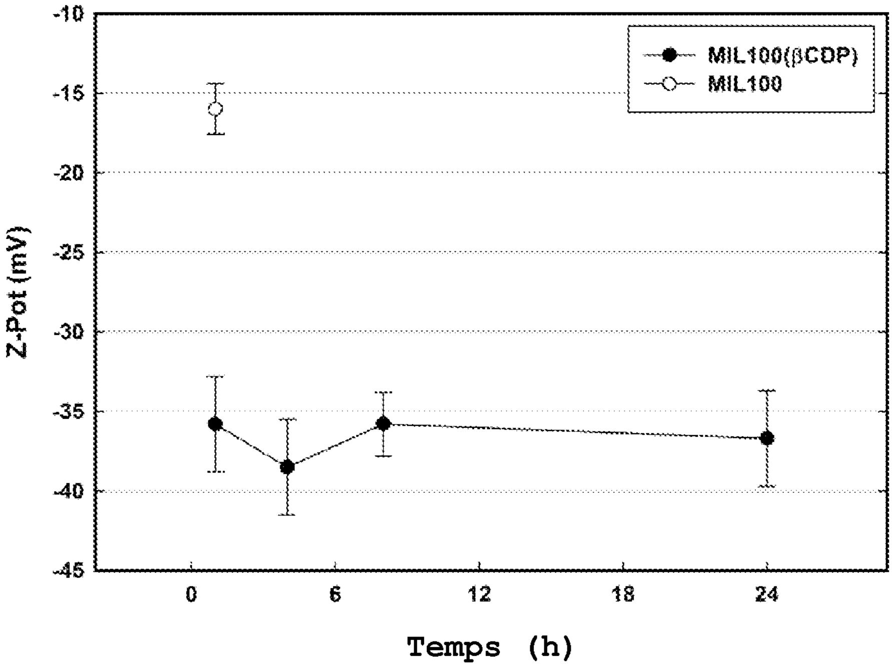
-10
-15
-20
-25
-30
-35
-40
-45
Z-Pot (mV)
0
6
12
18
24
Temps (h)
MIL100(βCDP)
MIL100

Figure 6

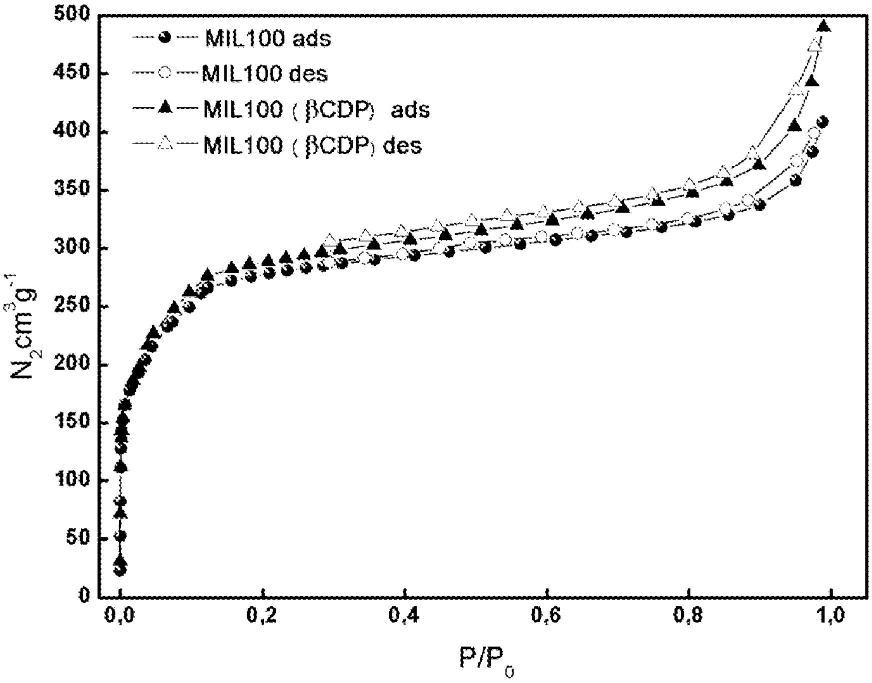


Figure 7 A

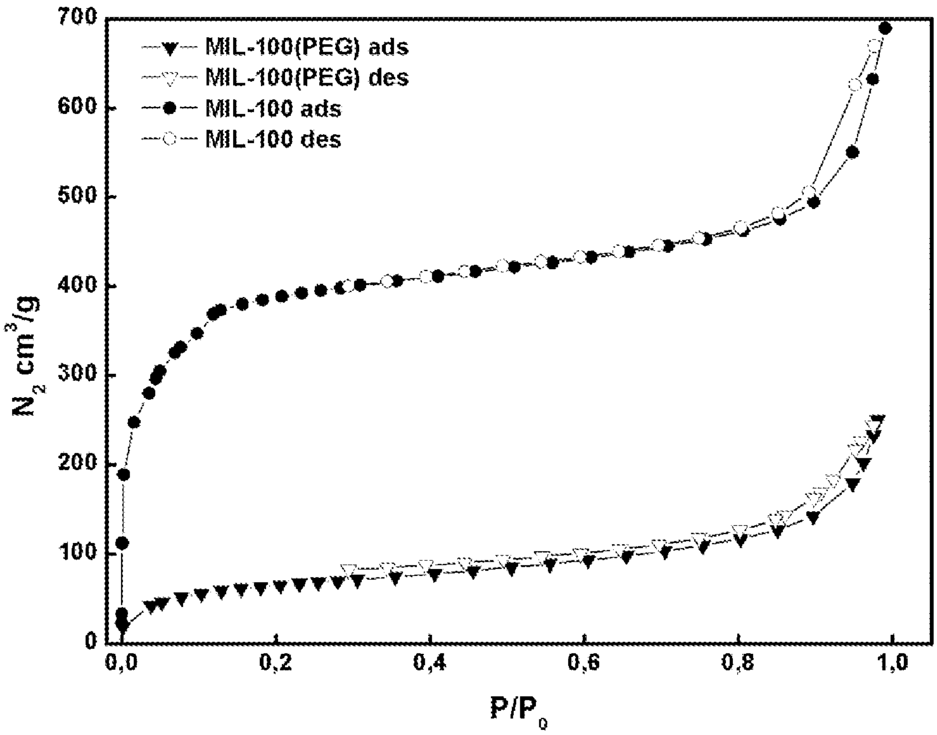


Figure 7 B

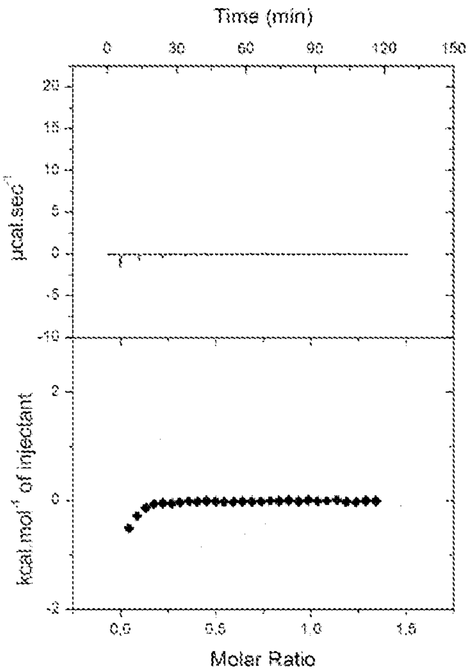


Figure 8A

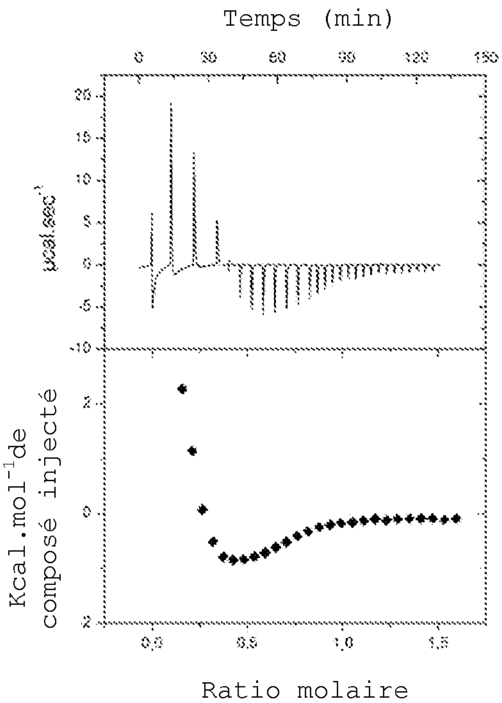


Figure 8B

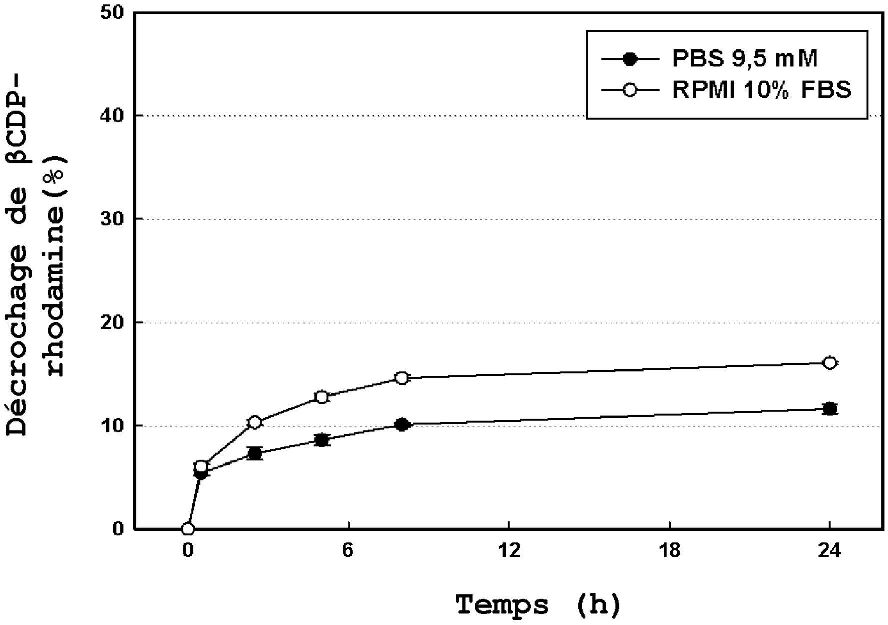


Figure 9

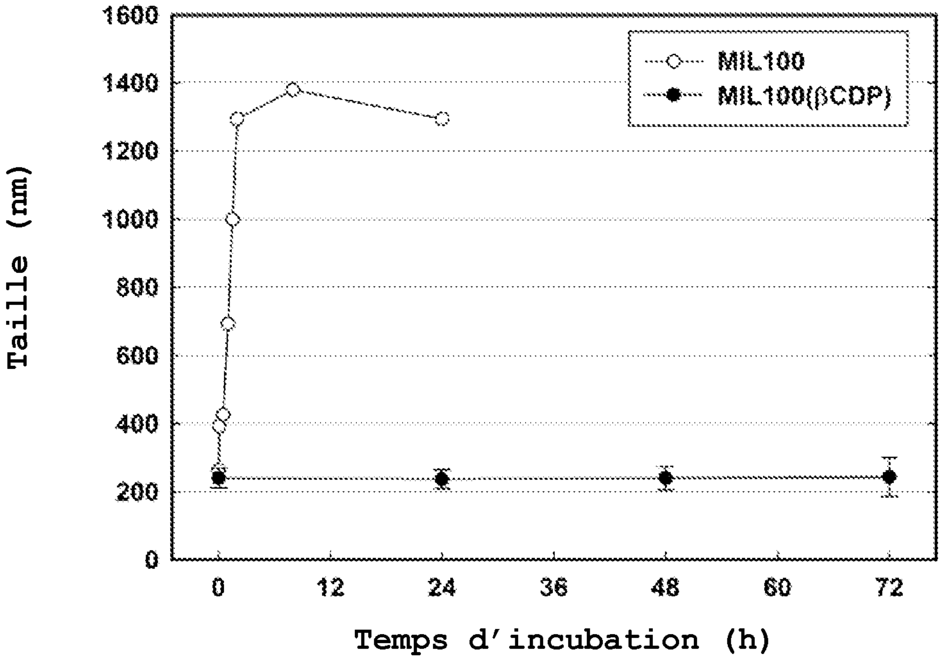


Figure 10

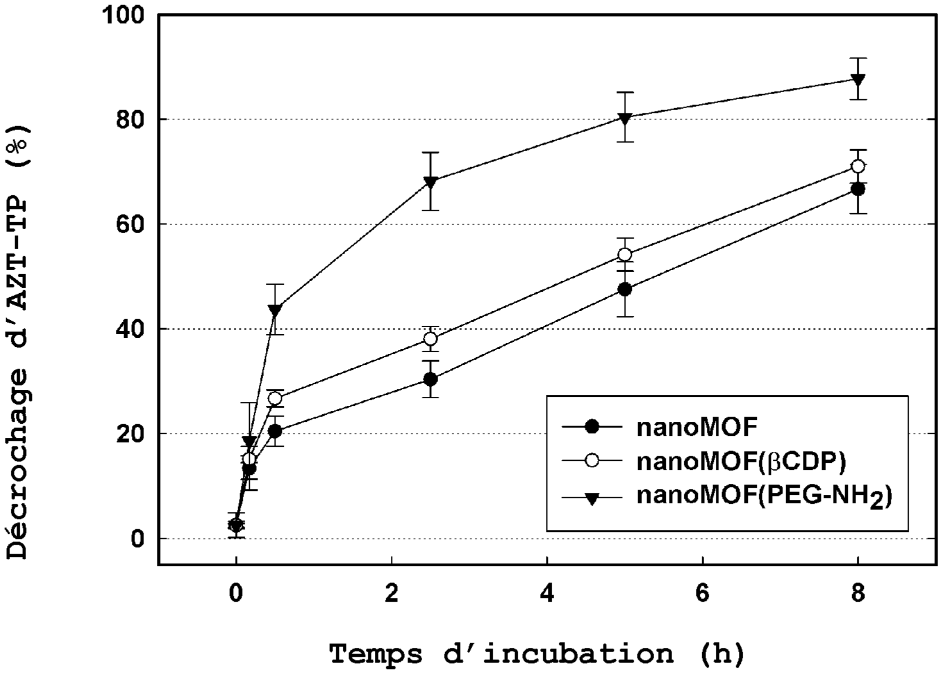


**Figure 11**

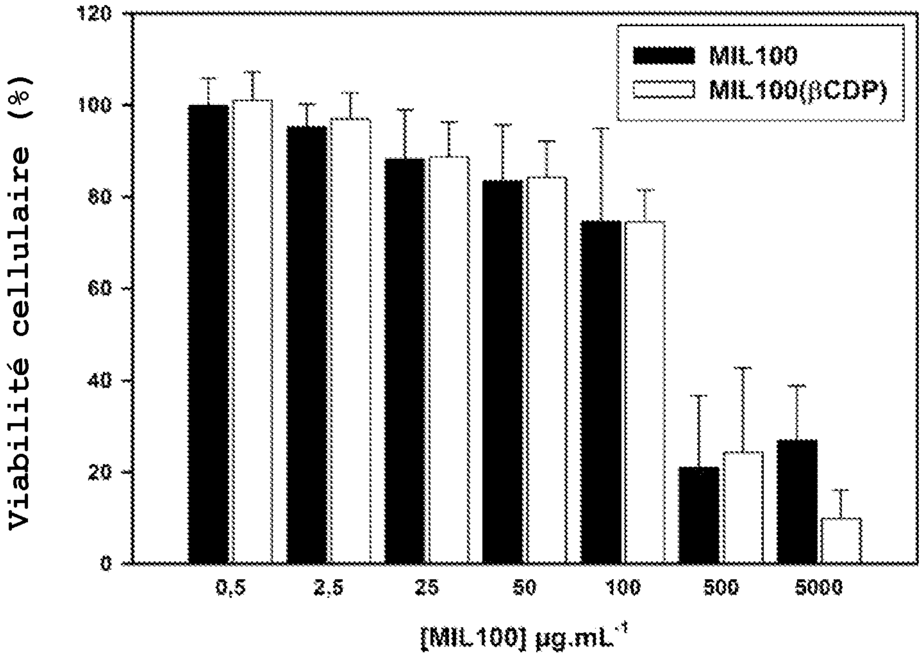


**Figure 12**

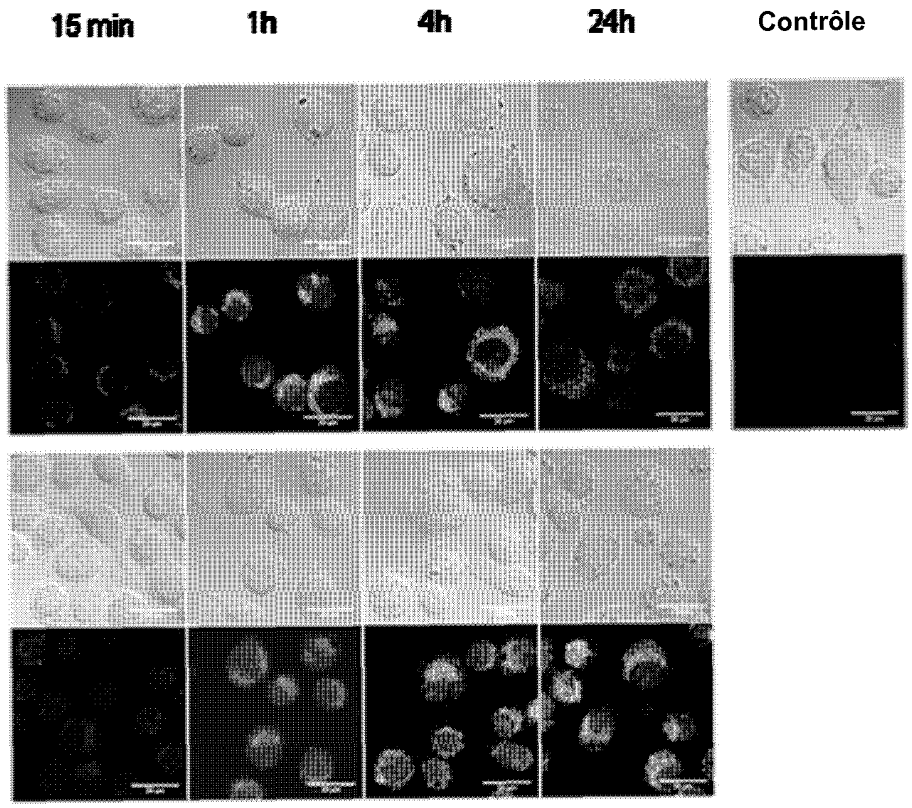


Figure 13

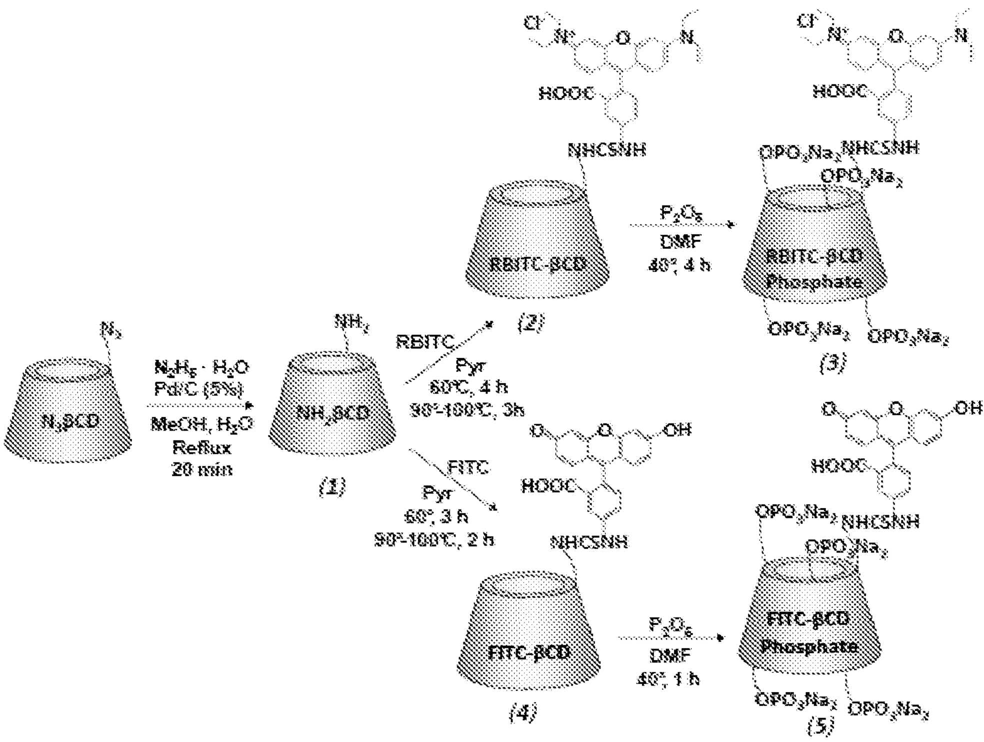


Figure 14

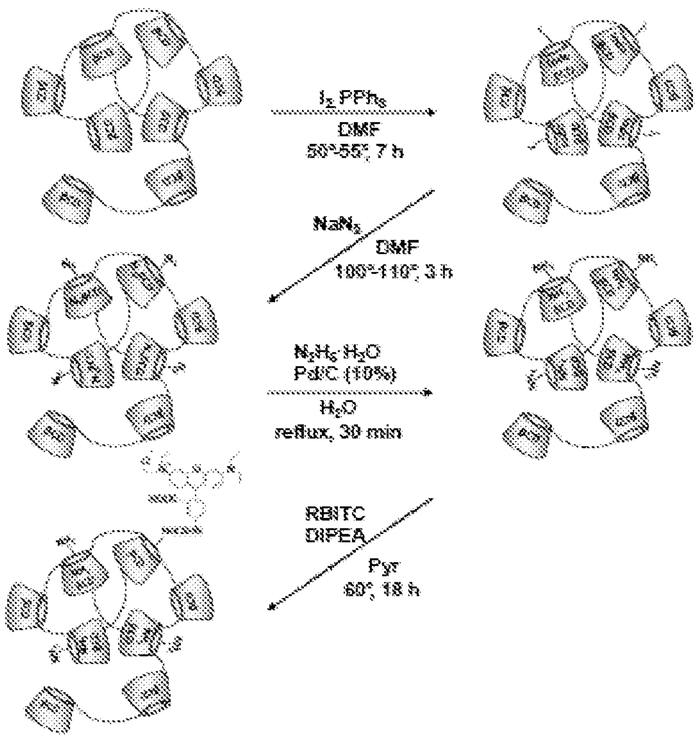


**Figure 15**

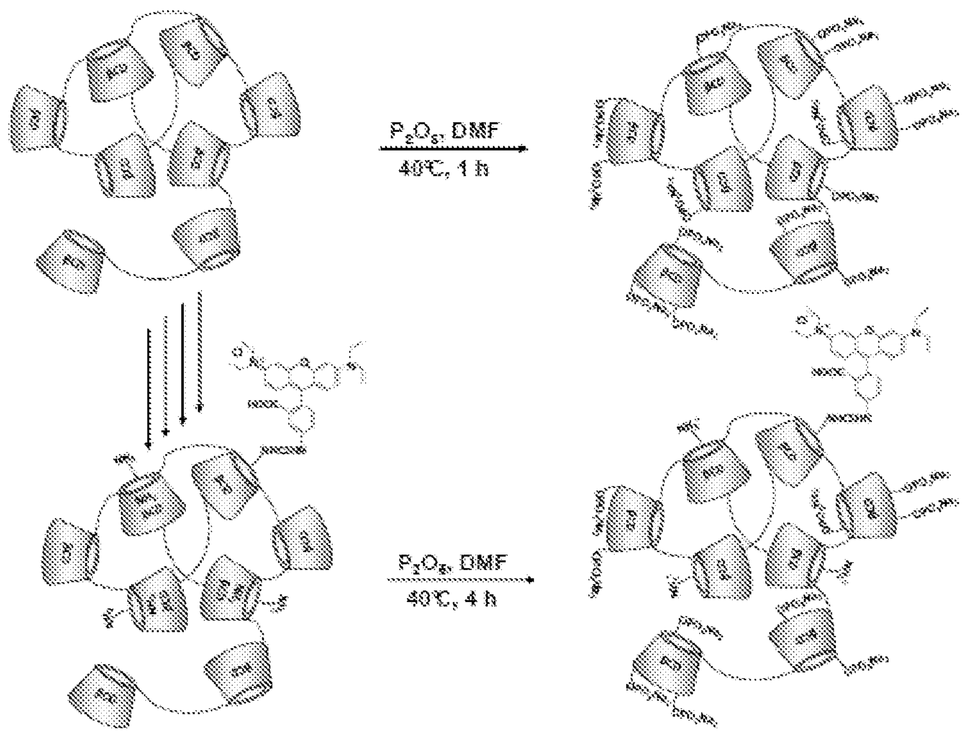


**Figure 16**

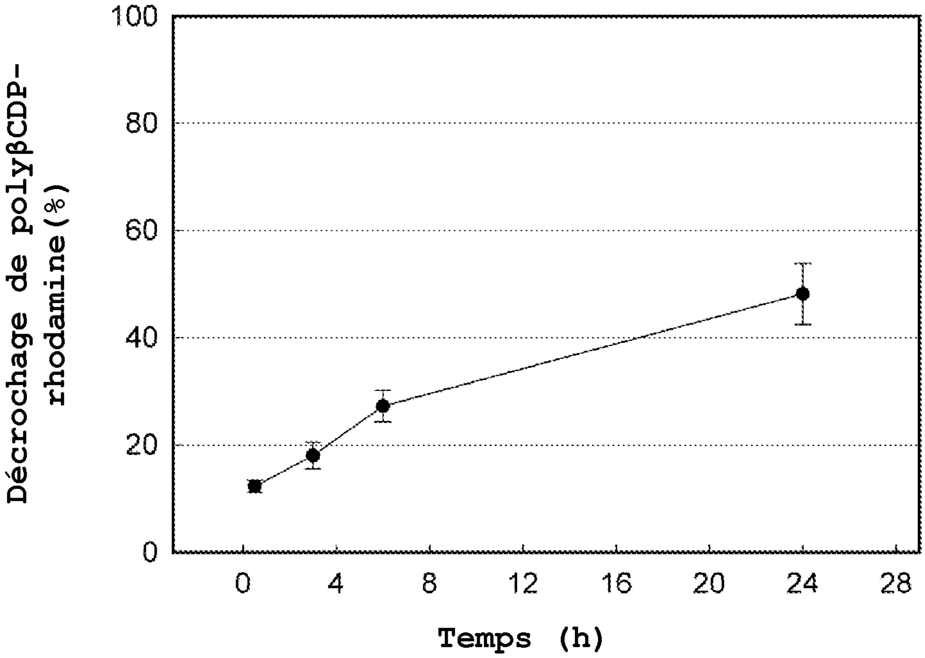


Figure 17

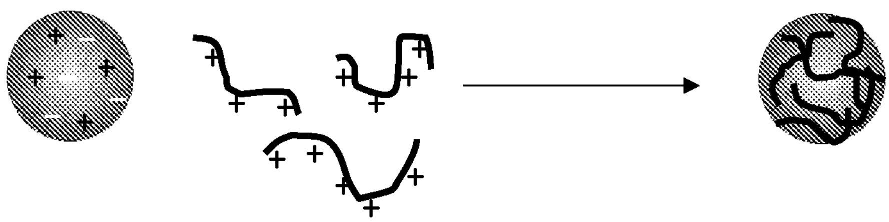

Figure 18

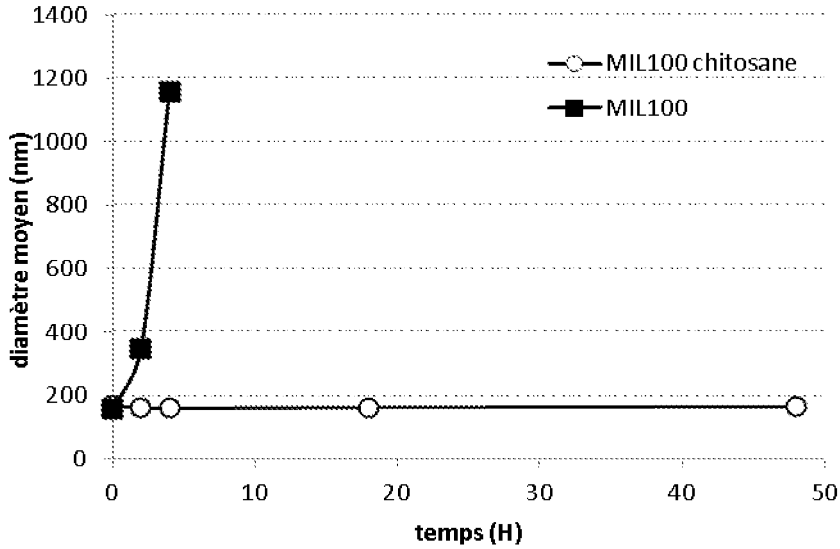


Figure 19

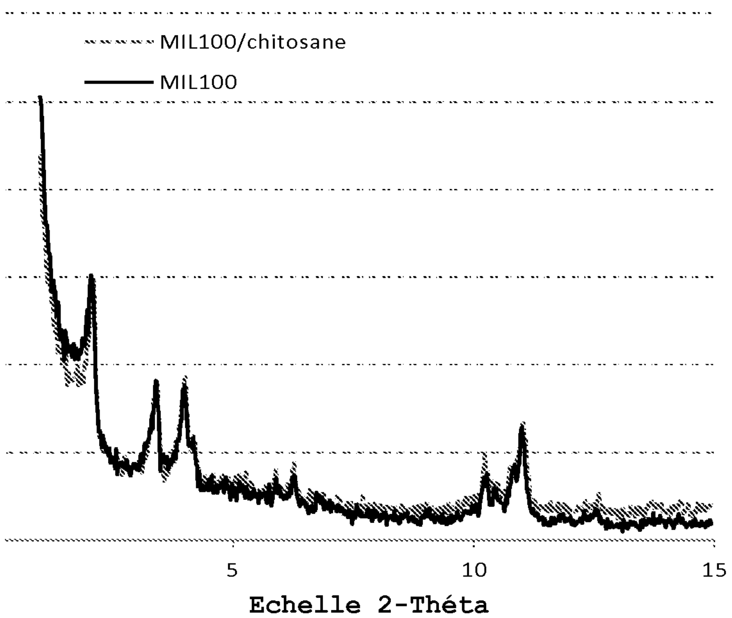


Figure 20

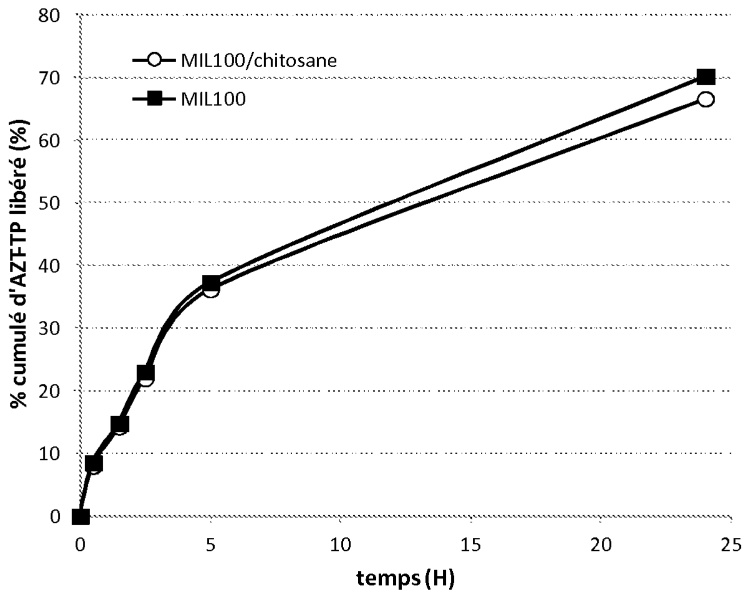


Figure 21

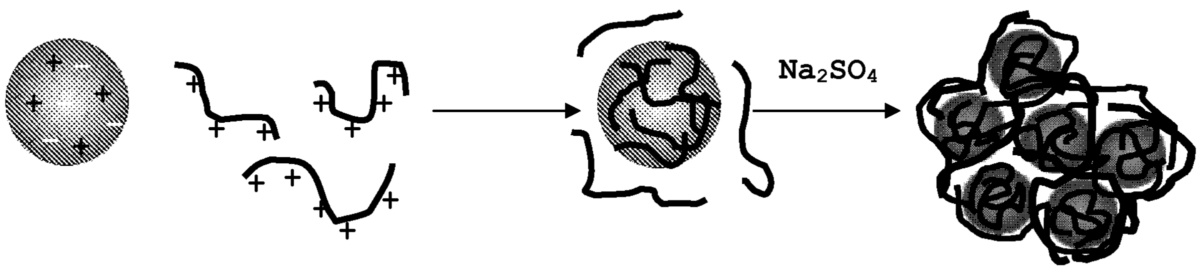


Figure 22

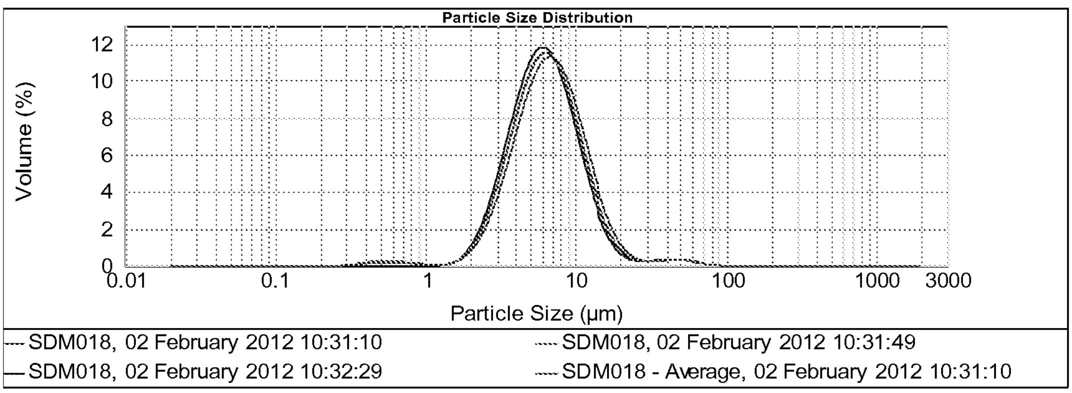


Figure 23

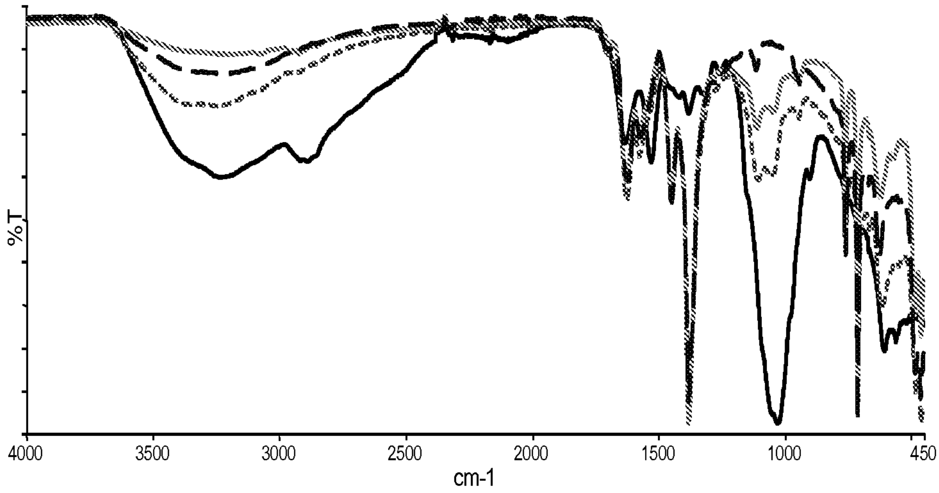


Figure 24

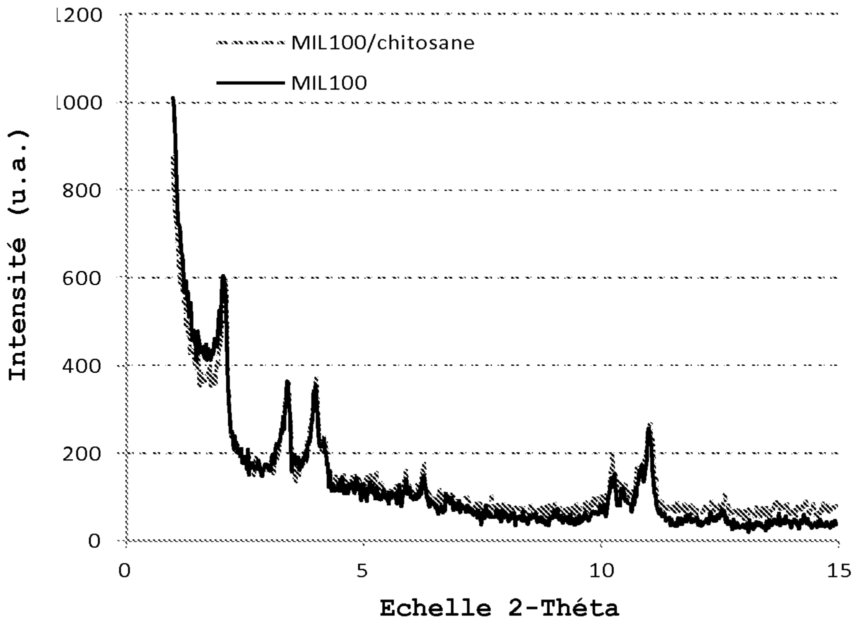


Figure 25

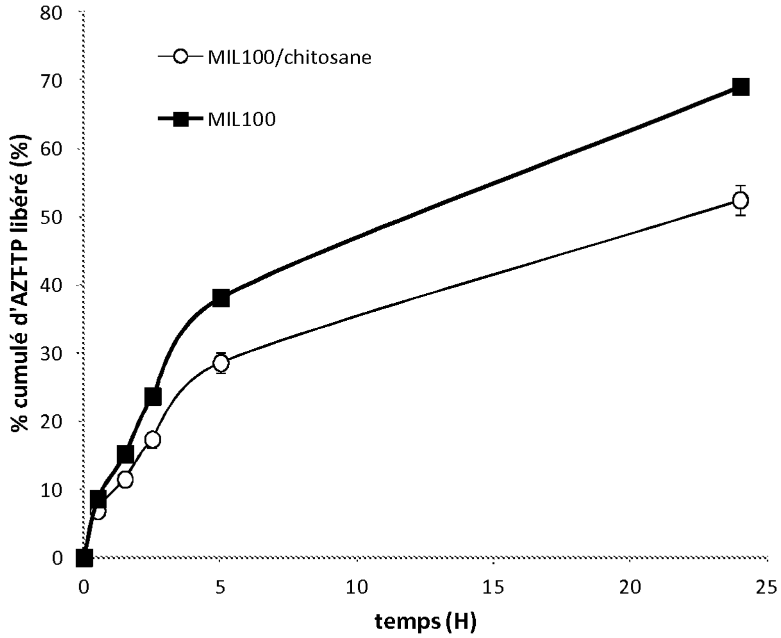


Figure 26

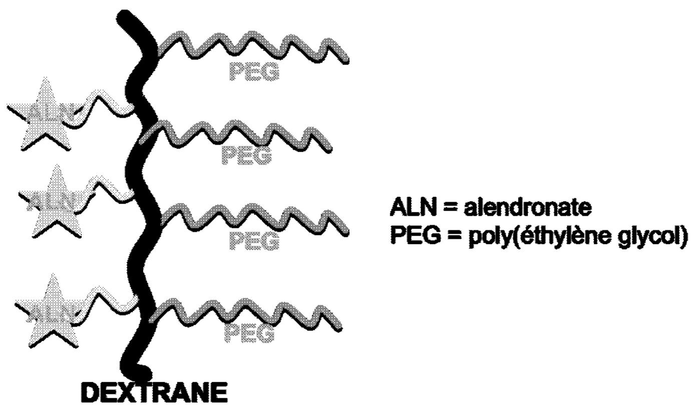


Figure 27

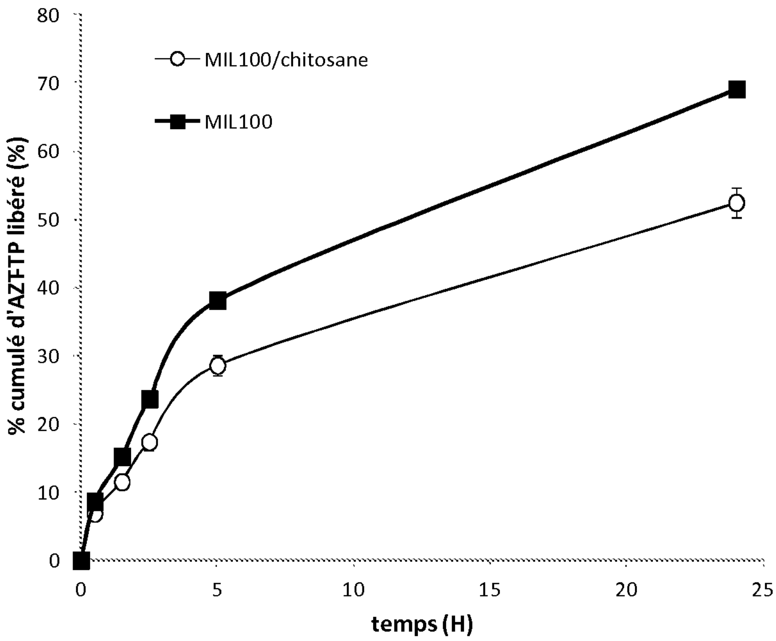


Figure 28

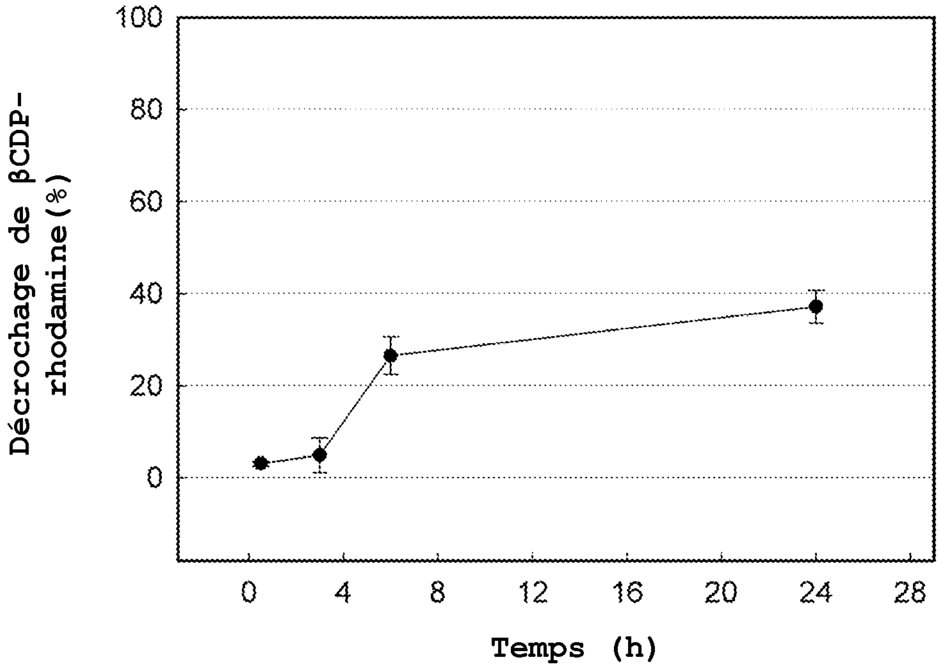


Figure 29

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2009077670 A
- WO 2009077671 A
- WO 20090077671 A
- WO 200977670 A
- WO 200977671 A
- US 4329332 A


### Littérature non-brevet citée dans la description


- **E. RENARD et al.** *European Polymer Journal,* 1997, vol. 33 (1), 49-57
- **GREF et al.** *International Journal of Pharmaceutics,* 2007, vol. 332 (1-2), 185-191
- **GREF et al.** *J. Control Release,* 2006, vol. 111 (3), 316-24
- **GREF et al.** *Journal of colloid and interface science,* 2007, vol. 307 (1), 83-93
- **BLANCHEMAIN et al.** *Acta Biomaterialia,* Novembre 2008, vol. 4 (6), 1725-1733
- **ELIF YILMAZ OZMEN et al.** *Bioresource Technology,* 2008, vol. 99 (3), 526-531
- **CESTEROS et al.** *European Polymer Journal,* 2009, vol. 45 (3), 674-679
- **SALMASO et al.** *International Journal of Pharmaceutics,* 2007, vol. 345 (1-2), 42-50
- **YANG et al.** *Biomaterials,* 2007, vol. 28 (21), 3245-3254
- **NAVATH R S ; MENJOGE A R ; DAI H ; ROMERO R ; KANNAN S ; KANNAN R M.** Injectable PAMAM dendrimer-PEG hydrogels for the treatment of génital infections: formulation and in vitro and in vivo evaluation. *Mol Pharm.,* 01 Août 2011, vol. 8 (4), 1209-23
- **R C. HEDDEN ; B. J. BAUER.** Structure and Dimensions of PAMAM/PEG Dendrimer-Star Polymers. *Macromolecules,* 2003, vol. 36 (6), 1829-1835
- **N BHATTARAI ; H R. RAMAY ; J GUNN ; F A. MATSEN ; M ZHAN.** PEG-grafted chitosane as an injectable thermosensitive hydrogel for sustained protein release. *J Controlled Release,* 2005, vol. 103 (3), 609-624
- **J.A WIELAND ; T.L. HOUCHIN-RAY ; L.D. SHEA.** Non-viral vector delivery from PEG-hyaluronic acid hydrogels. *J. Controlled Release,* 2007, vol. 120 (3), 233-241
- **X.M. LIU ; H. LEE ; R. REINHARDT ; L. MARKY ; W DONG.** *J. Controlled Release,* 2007, vol. 122, 54-62
- **G. OROS ; T. CSERHATI ; E. FORGACS.** *Chemosphere,* 2003, vol. 52, 185
- **A.M. BADAWI ; E.M.S. AZZAM ; S.M.I. MORSY.** *Bioorg. Med. Chem.,* 2006, vol. 14, 8661
- **W-J. TSAI ; Y-J SHIAO ; S-J LIN ; W-F CHIOU ; L-C LIN ; T-H YANG ; C-M TENG ; T-S WU ; L-M YANG.** *Bioorg. Med. Chem. Letters,* 2006, vol. 16, 4440
- **WHITFIELD, T. R. ; WANG, X. ; LIU, L. ; JACOBSON, A. J.** *Solid State Sci.,* 2005, vol. 7, 1096
- **T. LOISEAU et al.** *C. R. Chimie,* 2005, vol. 8, 765
- **SERRE et al.** Role of solvent-host interactions that lead to very large swelling of hybrid frameworks. *Science,* 2007, vol. 315, 1828-1831
- **SURBLÉ et al.** A new isoreticular class of metal-organic frameworks with the MIL-88 topology. *Chem. Comm.,* 2006, 284-286
- **MELLOT-DRAZNIEKS et al.** Very large swelling in hybrid frameworks : a combined computational and power diffraction study. *J. Am. Chem. Soc.,* 2005, vol. 127, 16273-16278
- **CHALATI et al.** Optimisation of the synthesis of MOF nanoparticules made of flexible porous iron fumarate MIL-88A. *J .Mater. Chem.,* 2011, vol. 21, 2220
- **C. SERRE ; F. MILLANGE ; S. SURBLÉ ; G. FÉREY.** *Angew. Chem. Int. Ed.,* 2004, vol. 43, 6286
- **HORCAJADA et al.** Synthesis and catalytic properties of MIL-100(Fe), an iron(III) carboxylate with large pores. *Chem. Comm.,* 2007, 2820-2822
- **FÉREY et al.** A chromium terephthalate-based solid with unusally large pore volumes and surface area. *Science,* 2005, vol. 309, 2040-2042

- **S. SURBLÉ ; F. MILLANGE ; C. SERRE ; T. DÜREN ; M. LATROCHE ; S. BOURRELLY ; P.L. LLEWELLYN ; G. FÉREY.** MIL-102: A Chromium Carboxylate Metal Organic Framework with Gas Sorption Analysis. *J. Am. Chem. Soc.,* 2006, vol. 128 (46), 14890
- **CAVKA, J. ; JAKOBSEN, S. ; OLSBYE, U. ; GUILLOU, N. ; LAMBERTI, C. ; BORDIGA, S. ; LILLERUD, K.** *J. Am. Chem. Soc.,* 2008, vol. 130, 13850
- **KANDIAH, M. ; NILSEN, M.H. ; USSEGLIO, S. ; JAKOBSEN, S. ; OLSBYE, U. ; TILSET, M. ; LARABI, C. ; QUADRELI, E.A. ; BONINO, F. ; LILLERUD K.P.** *Chem. Mater.,* 2010, vol. 22 (24), 6632
- **GARIBAY S.J. ; COHEN S.M.** *Chem. Commun.,* 2010, vol. 46, 7700
- **PARK et al.** Exceptional chemical and thermal stability of zeolitic imidazolate frameworks. *Proc. Natl. Acad. Sci. U.S.A.,* 2006, vol. 103, 10186
- **M. DAN-HARDI ; C. SERRE ; T. FROT ; L. ROZES ; G. MAURIN ; C. SANCHEZ ; G. FÉREY.** *J. Am. Chem. Soc. Comm.,* 2009, vol. 131, 10857-10859
- **C. ZLOTEA ; D. PHANON ; M. MAZAJ ; D. HEURTAUX ; V. GUILLERM ; C. SERRE ; P. HORCAJADA ; T. DEVIC ; E. MAGNIER ; F. CUEVAS.** Effet of NH2 and CF3 functionalization on the hydrogen sorption properties of MOFs. *Dalton Trans.,* 2011, vol. 40, 4879-4881
- **A.E. PLATERO-PRATS ; V.A. DE LA PENA-O'SHEA ; N. SNEJKO ; A. MONGE ; E. GUTIERREZ-PUEBLA.** Dynamic calcium metal-organic framework acts as a selective organic solvent sponge. *Chemistry,* vol. 16 (38), 11632
- **C. VOLKRINGER ; J. MARROT ; G. FEREY ; T. LOISEAU.** Hydrothermal crystallization of three calcium-based hybrid solids with 2,6-naphthalene or 4,4'-biphenyl-dicarboxylates. *Crystal Growth Design,* 2008, vol. 8, 685
- **HORCAJADA et al.** How linker's modification controls swelling properties of highly flexible iron(III) dicarboxylates MIL-88. *J. Am. Chem. Soc.,* 2011, vol. 133, 17839
- **DEVIC et al.** Functionalization in flexible porous solids: effects on the pore opening and the host-guest interactions. *J. Am. Chem. Soc.,* 2010, vol. 132, 1127
- **C. SERRE ; S. SURBLÉ ; C. MELLOT-DRAZNIEKS ; Y. FILINCHUK ; G. FÉREY.** *Dalton Trans.,* 2008, 5462-5464
- **A.K. GUPTA et al.** *Nanomed.,* 2007, vol. 2 (1), 23-39
- **P CARAVAN.** *Chem. Soc. Rev.,* 2006, vol. 35, 512-523
- **YAN-PING REN et al.** *Angew. Chem. Int. Ed.,* 2003, vol. 42 (5), 532
- **C.T. DZIOBKOWSKI ; T.J. WROBLESKI ; D.B. BROWN.** *Inorg. Chem.,* 1982, vol. 21, 671
- *J. Mater. Chem.,* 2010, vol. 20, 7676-7681
- **A. DEMESSENCE et al.** *J. Mater. Chem.,* 2010, vol. 20, 7676-7681
- **J.H. CAVKA et al.** *JACS,* 2009, vol. 130, 13850-13851
- **ALEMAN EA ; PEDINI HS ; RUEDA D.** *ChemBioChem,* 2009, vol. 10, 2862-2866
- **KUIL J ; BRANDERHORST HM ; PIETERS RJ ; DE MOL NJ ; LISKAMP RM.** *Org. Biomol. Chem.,* 2009, vol. 7, 4088-94
- **LIU XM ; LEE HT ; REINHARDT RA ; MARKY LA ; WANG D.** *Journal of Controlled Release,* 2007, vol. 122, 54-62
- **GRANDJEAN C ; BOUTONNIER A ; GUERREIRO, C ; FOURNIER J-M ; MULARD L-A.** *J.Org. Chem.,* 2005, vol. 70, 7123-7132
- **HEIN CD ; LIU X-M ; CHEN F ; CULLEN DM ; WANG D.** *Macromol. Biosci.,* 2010, vol. 10, 1544-1556
- **LIU X-M ; LEE H-T ; REINHARDT R-A ; MARKY LA ; WANG D.** *J. Control. Release,* 2007, vol. 122, 54-62
- **MOHAMAD OTHMAN PH.** *D thesis,* 2010
- **LUKYANOV AN et al.** *J Biomater Sci Polym Ed.,* 2004, vol. 15 (5), 621-30
- **LEWIS WG ; MAGALLON FG ; FOKIN VV ; FINN MG.** *J. Am. Chem. Soc.,* 2004, vol. 126, 9152-9153
- **PHAIMANOLIS N ; VESTERINEN A-H ; RICH J ; SEPPALA J.** *Carbohydrate Polymers,* 2010, vol. 82, 78-82
- **BONE MARROW.** *Transplant.,* 2002, vol. 30 (12), 833-841
- **J. BOULIGAND et al.** *Int. J.Pharm.,* 2004
- **MULDER WJ et al.** *Nanomed.,* Juin 2007, vol. 2 (3), 307-324
- **BALEAUX B. et al.** Chimie analytique-dosage colorimétrique d'agents de surface non ioniques polyoxyéthylènes à l'aide d'une solution iode-iodurée. *C.R. Acad. Sciences Paris,* 1972, vol. 274, 1617-1620
- **NAVATH R S ; MENJOGE A R ; DAI H ; ROMERO R ; KANNAN S ; KANNAN R M.** Injectable PAMAM dendrimer-PEG hydrogels for the treatment of genital infections: formulation and in vitro and in vivo evaluation. *Mol Pharm.,* 01 Août 2011, vol. 8 (4), 1209-23
- **R C. HEDDEN ; B. J. BAUER.** Structure and Dimensions of PAMAM/PEG Dendrimer_Star Polymers. *Macromolecules,* 2003, vol. 36 (6), 1829-1835
- **PARK et al.** *Exceptional chemical and thermal stability of zeolitic imidazolate frameworks*

- A New Photoactive Crystalline Highly Porous Titanium (IV) Dicarboxylate. *Proc. Natl. Acad. Sci. U.S.A.,* 2006, vol. 103, 10186
- **C. ZLOTEA ; D. PHANON ; M. MAZAJ ; D. HEURTAUX ; V. GUILLERM ; C. SERRE ; P. HORCAJADA ; T. DEVIC ; E. MAGNIER ; F. CUEVAS.** Effect of NH2 and CF3 functionalization on the hydrogen sorption properties of MOFs. *Dalton Trans.,* 2011, vol. 40, 4879-4881